(19) **Europäisches Patentamt**
**European Patent Office**
**Office européen des brevets**

(11) **EP 2 124 800 B1**

(12) **EUROPEAN PATENT SPECIFICATION**

(45) Date of publication and mention
of the grant of the patent:
**17.11.2010 Bulletin 2010/46**

(51) Int Cl.:
*A61B 19/00* (2006.01) *A61M 25/01* (2006.01)

(21) Application number: **08730065.3**

(22) Date of filing: **15.02.2008**

(86) International application number:
**PCT/US2008/054185**

(87) International publication number:
**WO 2008/101228 (21.08.2008 Gazette 2008/34)**

(54) **ROBOTIC MEDICAL INSTRUMENT SYSTEM**

MEDIZINISCHES ROBOTERINSTRUMENTENSYSTEM

SYSTÈME D'INSTRUMENT MÉDICAL ROBOTISÉ

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.02.2007 US 902144 P**
**25.05.2007 US 931827 P**
**15.06.2007 US 934639 P**

(43) Date of publication of application:
**02.12.2009 Bulletin 2009/49**

(73) Proprietor: **Hansen Medical, Inc.**
**Mountain View, California 94043-5207 (US)**

(72) Inventors:
• **STAHLER, Gregory, J.**
**San Jose, California 95117 (US)**
• **WALLACE, Daniel, T.**
**Burlingame, California 94010 (US)**
• **SALISBURY, J., Kenneth**
**Cambridge, Massachusetts 02140 (US)**

(74) Representative: **Lecomte, Didier**
**Lecomte & Partners Sàrl**
**B.P. 1623**
**1016 Luxembourg (LU)**

(56) References cited:
**WO-A-2005/117688** **US-A1- 2002 087 049**
**US-A1- 2006 084 945** **US-A1- 2006 229 587**

## EP 2 124 800 B1

**Description**

The invention relates generally to surgical tools, and more particularly, to flexible catheter instruments for performing minimally invasive diagnostic and therapeutic procedures with a robotic catheter system.

BACKGROUND

Robotic interventional systems and devices are well suited for use in performing minimally invasive medical procedures as opposed to conventional procedures that involve opening the patient's body to permit the surgeon's hands to access internal organs. Traditionally, surgery utilizing conventional procedures meant significant pain, long recovery times, lengthy work absences, and visible scarring. However, advances in technology have lead to significant changes in the field of medical surgery such that less invasive surgical procedures are increasingly popular, in particular, minimally invasive surgery (MIS). A "minimally invasive medical procedure" is generally a procedure that is performed by entering the body through the skin, a body cavity, or an anatomical opening utilizing small incisions rather than large open incisions in the body. Various medical procedures are considered to be minimally invasive including, for example, mitral and tricuspid valve procedures, patent formen ovale, atrial septal defect surgery, colon and rectal surgery, laparoscopic appendectomy, laparoscopic esophagectomy, laparoscopic hysterectomies, carotid angioplasty, vertebroplasty, endoscopic sinus surgery, thoracic surgery, donor nephrectomy, hypodermic injection, air-pressure injection, subdermal implants, endoscopy, percutaneous surgery, laparoscopic surgery, arthroscopic surgery, cryosurgery, microsurgery, biopsies, videoscope procedures, keyhole surgery, endovascular surgery, coronary catheterization, permanent spinal and brain electrodes, stereotactic surgery, and radioactivity-based medical imaging methods. With MIS, it is possible to achieve less operative trauma for the patient, reduced hospitalization time, less pain and scarring, reduced incidence of complications related to surgical trauma, lower costs, and a speedier recovery.

Special medical equipment may be used to perform minimally invasive procedures. Typically, a surgeon inserts small tubes or ports into a patient and uses endoscopes or laparoscopes having a fiber optic camera, light source, or miniaturized surgical instruments. Without a traditional large and invasive incision, the surgeon is not able to see directly into the patient. Thus, the video camera serves as the surgeon's eyes. The images of the interior of the body are transmitted to an external video monitor to allow la surgeon to analyze the images, make a diagnosis, visually identify internal features, and perform surgical procedures based on the images presented on the monitor.

Minimally invasive procedures may involve minor surgery as well as more complex operations that involve robotic and computer technologies, which may be used during more complex surgical procedures and have led to improved visual magnification, electromechanical stabilization, and reduced number of incisions. The integration of robotic technologies with surgeon skill into surgical robotics enables surgeons to perform surgical procedures in new and more effective ways.

Although minimally invasive surgical techniques have advanced, physical limitations of certain types of medical equipment still have shortcomings and can be improved. For example, during a minimally invasive medical procedure, catheters, endoscopes or laparoscopes may be inserted into a body cavity duct or vessel. A catheter is an elongated tube that may, for example, allow for drainage or injection of fluids or provide a path for delivery of working or surgical instruments to a surgical or treatment site. Such devices, however, may have rigid shafts and lack flexibility along their lengths. As a result, they lack the required or desired degrees of freedom and range of controllable motion. These issues may be particularly relevant in procedures involving routing of surgical devices around a number of turns or bends. Consequently, control of a tool or working instrument at the distal tip of an instrument that has traversed a number of curves may be difficult with known devices, thereby resulting in more complicated and/or less effective procedures.

US 2006/0084945 discloses a device according to the preamble of appended claim 1.

Accordingly, robotic interventional systems, devices and related procedures can be improved to provide enhanced robotic controls, maneuverability and positioning of controllable surgical instruments.

SUMMARY

According to the invention as defined in appended claim 1, an instrument driver operable to control coaxially aligned elongate catheter instruments in response to control signals generated by an input device comprises a frame and first and second carriages. The first carriage is movably coupled to the frame and configured to support a first catheter instrument, and the second carriage is movably coupled to the frame and configured to support a second catheter instrument. The first and second carriages are independently rotatable about a longitudinal axis of the respective first and second catheter instruments.

According to another embodiment, an instrument driver operable to control coaxially aligned elongate catheter instruments in response to control signals generated by an input device, the instrument driver comprises a frame and first and second carriages. The first carriage is movably coupled to the frame and configured to support a first catheter instrument. The second carriage is movably coupled to the frame and configured to support a second catheter instrument. The first and second carriages are independently movable in at least three different directions with at least two different types of motion.

In a further embodiment, a robotic medical system comprises an input device, first and second elongate catheter instruments, and an instrument driver. The input device is configured to generate control signals in response to user input. The first and second elongate catheter instruments are coaxially aligned. The instrument driver is operable to control the first and second catheter instruments in response to control signals generated by the input device. The instrument driver comprises a frame, a first carriage movably coupled to the frame and configured to support a first catheter instrument, and a second carriage movably coupled to the frame and configured to support a second catheter instrument. The first and second carriages are independently rotatable about a longitudinal axis of the respective first and second catheter instruments.

In one or more embodiments, the first carriage is positioned relative to the second carriage such that the first catheter instrument coupled to the first carriage may be positioned within a lumen of a second catheter instrument coupled to the second carriage. The first and second carriages may also be positioned within the frame such that respective longitudinal axes of the first and second catheter instruments are aligned with a longitudinal axis of the frame.

Further, in one or more embodiments, the first and second carriages are independently and simultaneously rotatable about a longitudinal axis of the frame. The first and second carriages may also be rotatable in different directions about a longitudinal axis of the frame. The frame may also be rotatable, and the first and second carriages are independently rotatable relative to each other and to the frame when the frame is rotated. Independent carriage rotation can be implemented by use of respective drive elements that are controllable to rotate the respective carriage relative to the frame.

In another configuration, the disclosure is directed to a robotic medical instrument system that comprises an elongate catheter body and a catheter drive shaft. The catheter body includes proximal and distal ends, has longitudinal axis, and defines a lumen. The catheter drive shaft is positioned within the catheter body lumen. An inner surface of the catheter body distal end and an outer surface of a distal end of the catheter drive shaft are operatively coupled such that axial displacement of the catheter drive shaft relative to the catheter body causes a corresponding rotation of one of the drive shaft and catheter body relative to the other.

The distal end of the catheter drive shaft may be controllably extendable out of, and controllably retractable into, a distal end opening of the catheter body in communication with the catheter body lumen. Further, the outer surface of the distal end of the catheter drive shaft and the inner surface of the distal end of the catheter body have complimentary threaded surfaces or form a BNC connector.

A further configuration is directed to a robotic medical instrument system that comprises an elongate catheter, an actuation element and a control element. The catheter body includes a proximal end and a controllable distal end. The catheter body further includes a longitudinal axis and defines a lumen. The actuation element is positioned within the catheter body lumen and coupled to an internal portion of the catheter body, e.g., a distal segment of the catheter. The control element extends through the catheter body, and the actuation element and the catheter body are rotatable together in response to manipulation of the control element.

The actuation element may include a gear, a guide and a pin. The gear includes teeth defining corresponding grooves, and the guide is disposed on an inner surface of the distal end of the catheter body adjacent to the gear. For example, the guide may have a triangular shape and be formed within an inner surface of the catheter body distal end. The pin is movable along the guide. A control element is attached to the pin such that manipulation of the control element results in movement of the pin along the guide and within the groove to rotate the actuation element and the catheter body. The pin may be movable in multiple directions along the guide in response to manipulation of the control element. Further, the pin may traverse a guide surface that is non-uniform such that the pin is positionable at different depths as the pin traverses the guide surface.

Another alternative configuration is directed to a robotic medical instrument system that includes an elongate catheter body, first and second actuation elements and first and second control elements. The elongate catheter body has a proximal end and a controllable distal end. The catheter body has a longitudinal axis and defines a lumen. The first actuation element is positioned within the catheter body lumen and is coupled to an internal portion of the catheter body, and the second actuation element is positioned within the catheter body lumen distal to the first internal actuation element and coupled to an internal portion of the catheter body. The first control element extends through the catheter body lumen and is coupled to the first actuation element, and the second control element extends through the catheter body lumen and is coupled to the second actuation element. At least one of the first and second actuation elements and the catheter body are rotatable together in response to manipulation of at least one of the first and second control elements. The first and second actuation elements may rotate in opposite directions.

According to yet another configuration, a robotic medical instrument system comprises an elongate catheter body and a harmonic drive element. The catheter body includes a proximal end, a controllable distal end and a longitudinal axis. The harmonic drive element is located at the distal end of the catheter body and operable to generate a harmonic signal to thereby rotate the distal end of the catheter body

The harmonic drive element may include a harmonic wave generator having an elliptically shaped and rotatable element, a flexible spline, the wave generator being positioned within a bore of the flexible spine to impart an elliptical shape to the flexible spline, and an outer circular spline. The elliptically shaped flexible spline is positioned within a bore of the circular spine. An outer surface of the flexible spline rotatably engages an inner surface of the circular spline at opposite locations due to the elliptical shape imparted to the flexible spline by the wave generator. The flexible spline is rotatable within the circular spine to generate a harmonic signal that rotates the distal end of the catheter body. The circular spine is also rotatable to generate a harmonic signal that rotates the distal end of the catheter body. Alternatively, the flexible spline may be fixed relative to the circular spline.

A further configuration is directed to a robotic medical instrument system that comprises an elongate catheter body and a wobble plate drive element. The catheter body has a proximal end and a controllable distal end. The catheter body has a longitudinal axis and defines a lumen. The wobble plate drive element is located at the distal end of the catheter body and is operable to rotate the distal end of the catheter body.

In one embodiment, the wobble plate drive element is operable to rotate the distal end of the catheter body based on differential rotation of different gear elements of the wobble plate drive element. In one embodiment, the wobble plate drive element comprises a rotatable shaft, a first, stationary gear element, a second gear element coaxial with the shaft and rotatable about the first gear element plate and around the shaft, a tension element disposed between the first and second gear elements, the tension element urging the second gear element away from the first gear element, and a drive element configured to rotate the second gear element to urge a portion of the second gear element, against the force of the spring, to engage a portion of the first gear element, while an opposite portion of the second gear element does not engage the first gear element. Further, in another embodiment, the drive element comprises a plurality of control elements attached to different sections of the second gear element. The plurality of control elements can be manipulated to urge a portion of the second gear element to engage a portion of the first gear element. The drive element may also include a rotatable arm that extends from the shaft and engaging a top surface of the second gear element to urge a portion of the second gear element, against the force of the spring, to engage a portion of the first gear element. Thus, the location at which the second gear element engages the first gear element changes as the rotatable arm is rotated.

In yet another alternative configuration, a robotic medical instrument system comprises an elongate catheter and a planetary gear drive element. The elongate catheter body has a proximal end, a controllable distal end, and a longitudinal axis, and defines a lumen.

The planetary gear drive element is located at the distal end of the catheter body and is operable to rotate the distal end of the catheter body. The planetary gear drive element can include an outer ring gear, a central gear, and a plurality of intermediate gears rotatably engaged between the outer ring gear and the central gear.

In one or more configuration, a control cable extends through a lumen of the catheter drive shaft to a working instrument coupled to a distal end of the catheter drive shaft.

According to yet another configuration, a robotic medical instrument system comprises an elongate catheter body and an elongate, flexible support structure that extends through a lumen of the catheter body. The catheter body has flexible distal segment, and the flexible support structure comprises a plurality of substantially spherical elements and a plurality of non-spherical elements.

According to another configuration, a robotic medical instrument system comprises an elongate catheter body, an elongate, flexible support structure, and a control element. The catheter body has a controllable and flexible distal end, and a lumen extending longitudinally through the catheter body. The support structure extends through the catheter body lumen and comprises a series of alternating substantially spherical and non-spherical elements. The control element extends through the catheter body. Bending of the respective catheter body and the flexible support structure are controllable by manipulation of the control element.

A further alternative configuration is directed to a robotic medical instrument system that comprises an elongate catheter body, an elongate, flexible support structure, an interface, and a control element. The catheter body has a proximal end, a controllable and flexible distal end, and a lumen extending longitudinally through the catheter body. The support structure extends through the catheter body lumen and comprises a series of alternating substantially spherical elements and non-spherical elements. The interface is coupled to the distal end of the catheter body for controlling an orientation of a working instrument. The interface includes a base member coupled to the distal end of the catheter body, a spacer element, and a platform member. The spacer element is retained between and separates the base member and the platform member. The control element extends through the flexible catheter, through an aperture defined by the base member, and terminates at the platform member. Bending of the respective catheter body and the support structure, an orientation of the platform member, and an orientation of the working instrument are each controllable by manipulation of the control element.

In one or more configuration, the non-spherical elements comprise cylindrical elements, and the support structure may comprise a series of alternating substantially spherical elements and non-spherical elements. Respective substantially spherical elements are seated and movable within indentations or concave cavities of respective adjacent non-spherical elements.

In one or more configurations, the support structure is bendable with the catheter body lumen, and the respective substantially spherical and non-spherical elements (e.g. cylindrical elements) collectively define a central, inner lumen. Further, in one or more embodiments, respective substantially spherical elements are seated and movable within indentations or concave cavities of respective adjacent non-spherical members. Additionally, in one or more embodiments, a plurality of control elements extend through the catheter body and terminate at the platform member. Bending of the catheter body, bending of the support structure, an orientation of the platform member, and an orientation of the working instrument are each controllable by manipulation of one or more of the plurality of control elements.

In one or more configurations, a spacer element has a substantially spherical shape. Further, in one or more embodiments, a base member is fixed to the distal end of the catheter body, and the platform member is pivotable about the spacer element. Further, the base member and the platform member may each define a respective indentation or concave cavity occupied by the spacer element. An adapter may also be utilized to couple a working instrument to the platform members.

According to another configuration, an interface assembly for controlling an orientation of a working instrument of a robotic medical instrument system comprises a base member, a platform member, a spacer element, and a control element. The base member is coupled to a distal end of an elongate instrument of the robotic medical instrument system. The spacer element is retained between the base member and the platform member, which is movable relative to the base member about the spacer element. The control element extends through an aperture defined by the base member and between the base and platform members. An orientation of the platform member and an orientation of the working instrument are controllably adjustable by manipulation of the control element.

According to yet another configuration, an interface assembly for controlling an orientation of a working instrument of a robotic medical instrument system comprises a base member, a platform member, a spacer element and a control element. The base member is coupled to a distal end of an elongate instrument of the robotic medical instrument system. The spacer element is retained between the base member and the platform member, which is movable relative to the base member about the spacer element. The control element extends through an aperture defined by the base member and between the base member and the platform member at an angle, which is defined between longitudinal axes of the control element and the base member. An orientation of the platform member and an orientation of the working instrument are controllably adjustable by manipulation of the control element.

In accordance with a further alternative configuration, a multi-level interface assembly for controlling an orientation of a working instrument of a robotic medical instrument system comprises a base member, spacer elements, platform members and a control element. The base member coupled to a distal end of an elongate instrument of the robotic medical instrument system. The first spacer element is retained between the base member and a proximal platform member, and the second spacer element is retained between the proximal platform member and a distal platform member, which is movable relative to the base member about the second spacer element. The control element extends through an aperture defined by the base member and between the base member and the distal platform member. An orientation of the distal platform member and an orientation of the working instrument are controllably adjustable by manipulation of the control element.

In one or more configurations, the base member is fixed or stationary, and the platform member is pivotable about the spacer element. Further, in one or more configurations, each of the base member and the platform member defines a concave cavity in which the spacer element, which may be spherical, is positioned. Assembly components may also define a lumen such that the collection of assembly component lumens defines a central lumen that in communication with a lumen of the elongate instrument, which may be a robotically controlled catheter.

Further, in one or more configurations an assembly includes a plurality of control elements that extend through respective apertures defined by the base member and terminate at the platform member. Orientations of the platform member and the working instrument, which may be coupled to the platform member via an adapter, are controllably adjustable by manipulation of one or more control elements assuming biasing forces of springs extending between the base and platform members are overcome. Further, control element manipulation may be used to control an orientation of the distal end of the elongate instrument.

In one or more configurations, control elements terminate at the platform member. Control elements may also be oriented at an angle relative to a longitudinal axis of the base member, such as an angle of at least 30 degrees. Two or more control elements may also overlap or cross each other, e.g., if they extend through misaligned apertures defined by the base member and the platform member at an angle in cases in which a control element extends through a platform member aperture. Angled of off-axis control element arrangements may result in a control element extending across a substantial width of the base member. Further, a control element may also extend through a platform member and across a portion of a top or distal surface of the platform member, and the, for example, back down through the platform member and through the base member.

BRIEF DESCRIPTION OF THE DRAWINGS

The foregoing arid other aspects of various configurations of the disclosure will best be appreciated with reference to the detailed description of embodiments in conjunction with the accompanying drawings, wherein:

Fig. 1 illustrates an configuration of a robotic medical instrument system including a flexible instrument such as a flexible catheter;

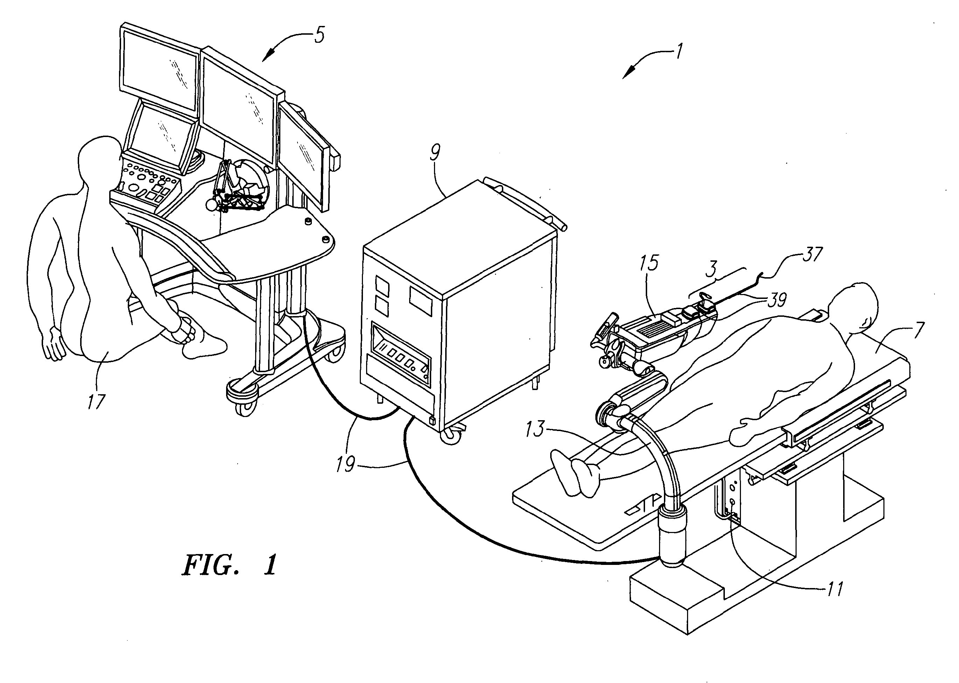

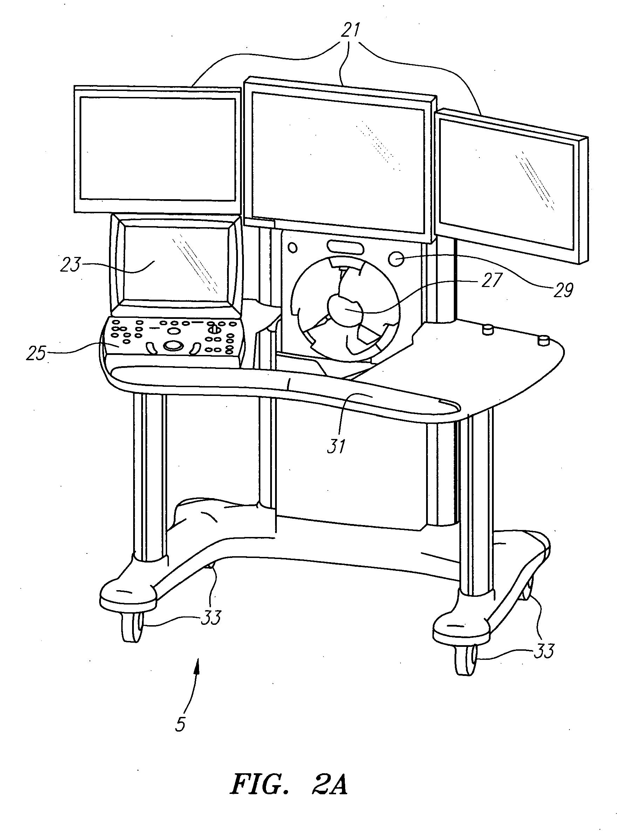

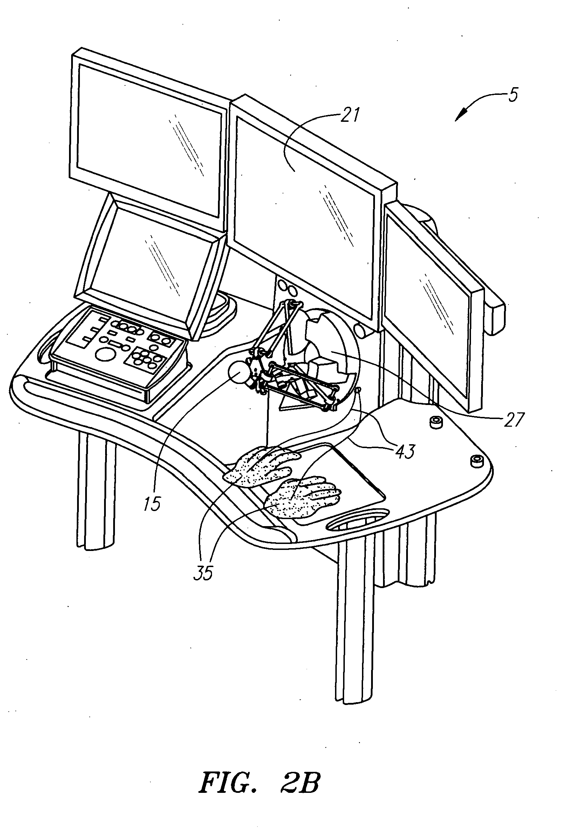

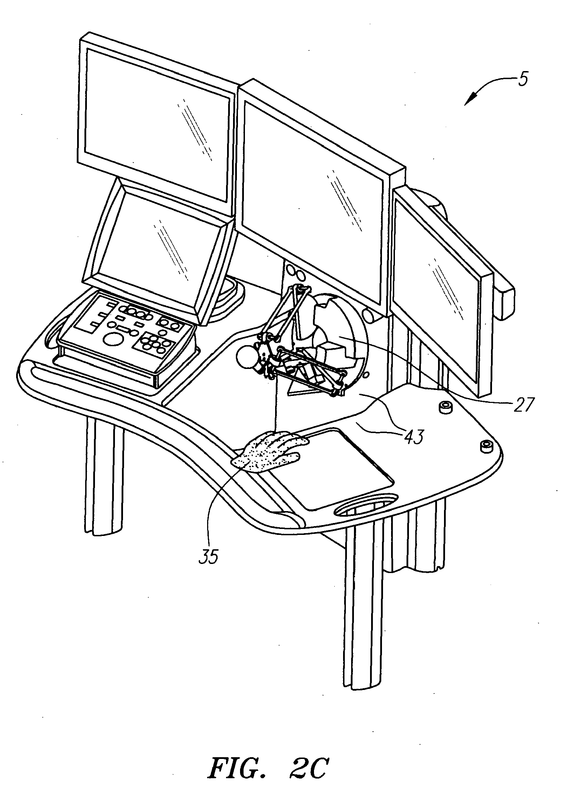

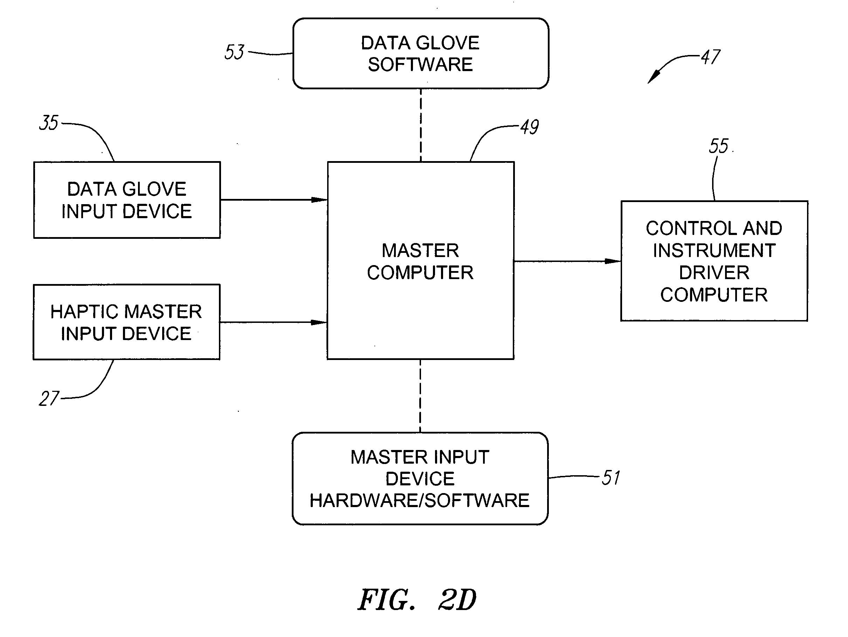

Figs. 2A-D illustrate configurations of a workstation and robotic medical instrument system, wherein Fig. 2A illustrates an operator workstation of an configuration of the system illustrated in Fig. 1, Fig. 2B illustrates an configuration of an operator workstation that includes a master input device and data gloves, Fig. 2C illustrates another configuration of an operator workstation with which a flexible instrument control can be input using a master input device and wireless data gloves, and Fig. 2D is a block diagram illustrating a system architecture of one configuration of a robotic medical instrument system;

Fig. 3 illustrates an configuration of a setup joint supporting an instrument driver above an operating table;

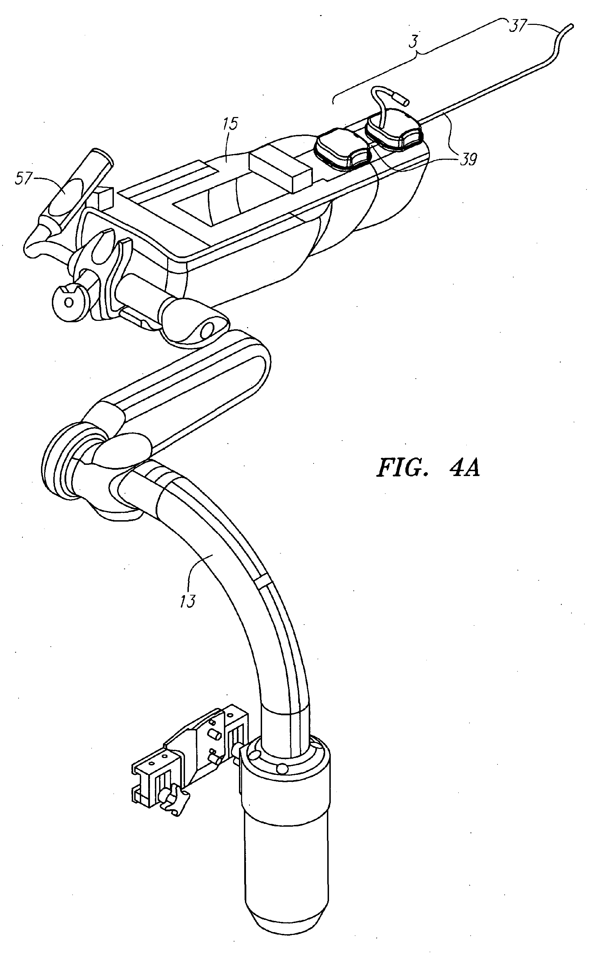

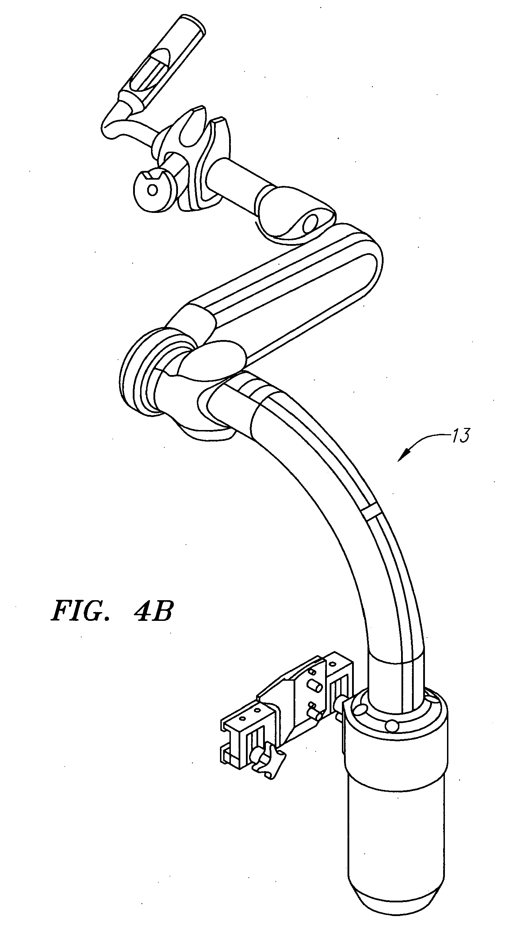

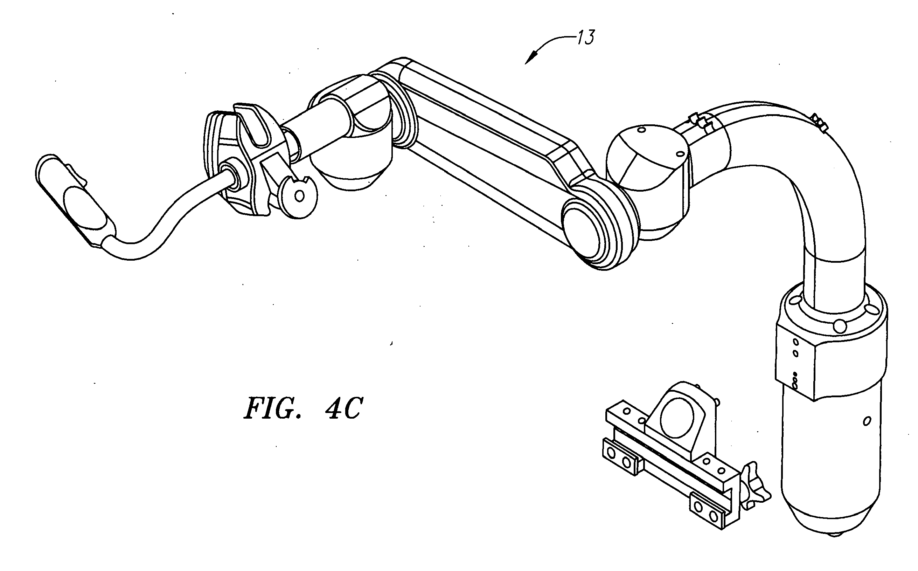

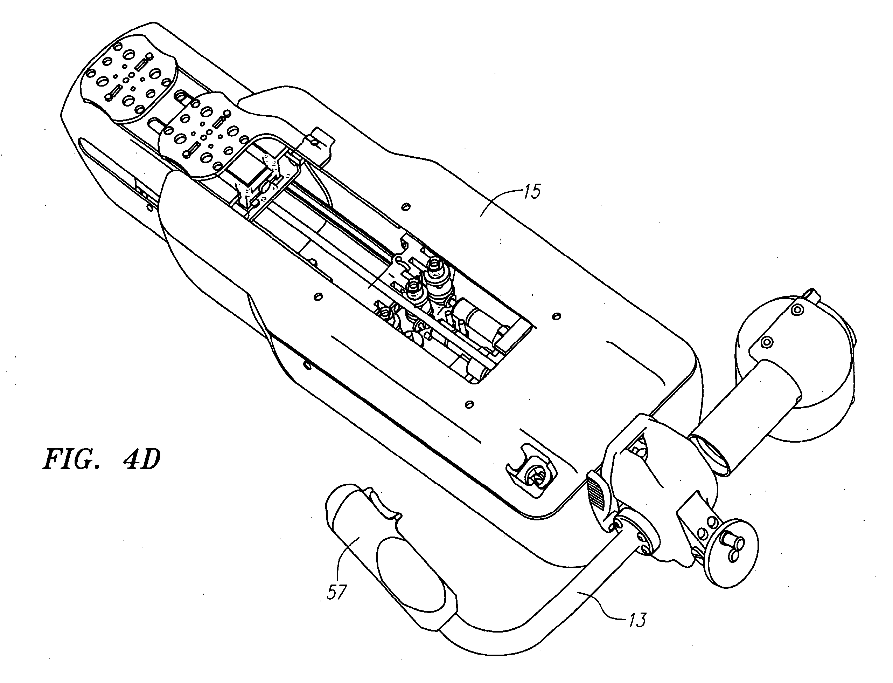

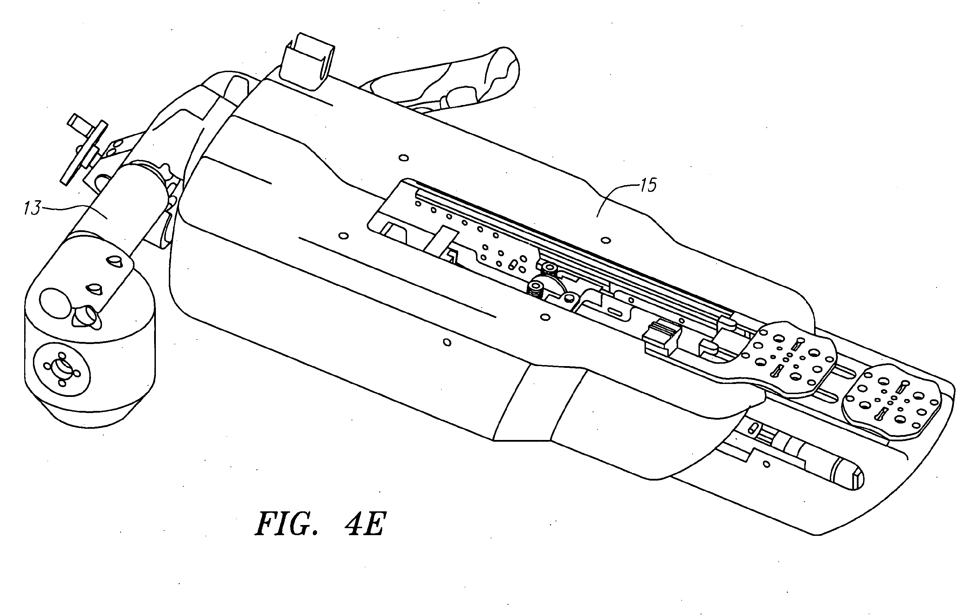

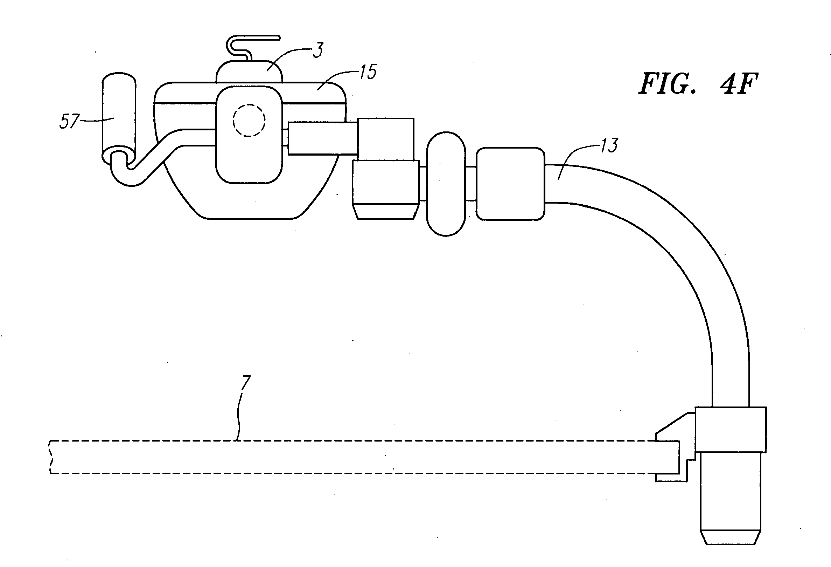

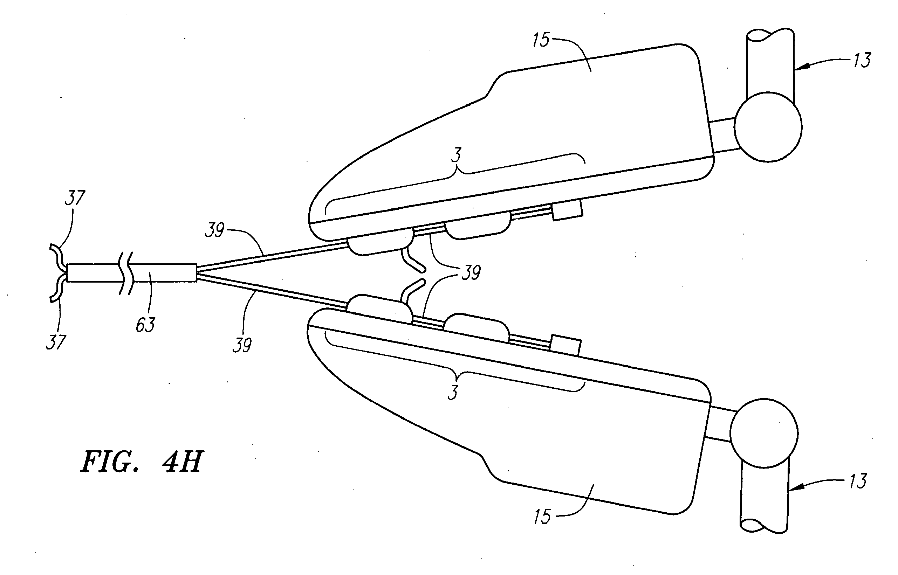

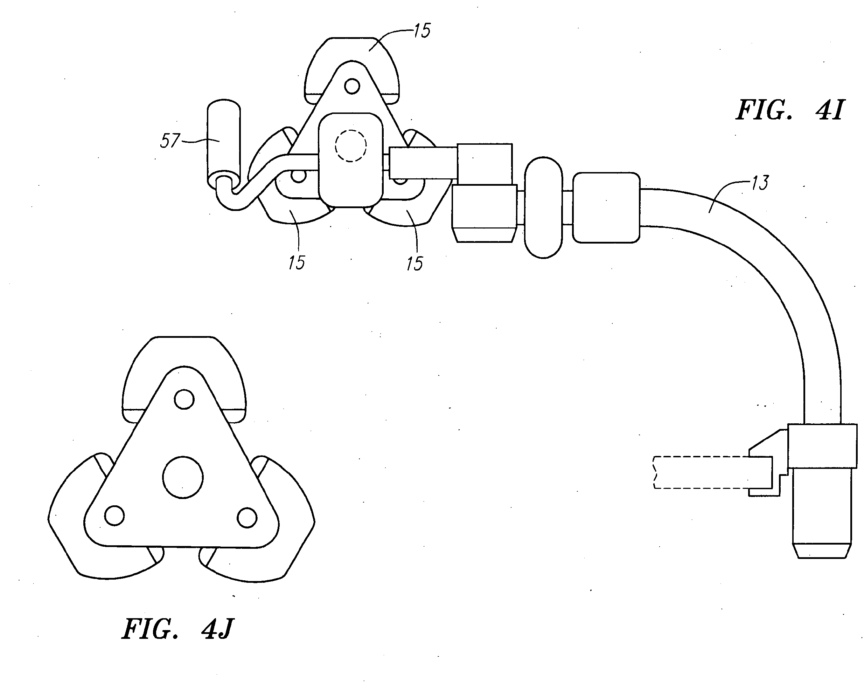

Fig. 4A-F illustrate a setup joint constructed according to one configuration, wherein Fig. 4A is a rear perspective view of one embodiment of a setup joint having an instrument driver mounted thereto, Fig. 4B illustrates the setup joint separately from the instrument driver, Fig. 4C is another perspective view of the setup joint shown in Fig. 4B, Fig. 4D is a rearward perspective view of an assembly including a mounting plate and locking lever, and Fig. 4E is a forward perspective view of the assembly shown in Fig. 4D and showing front and top portions of the instrument driver, and Fig. 4F illustrates a setup joint mounted to a patient bed;

Fig. 4G-J illustrate other configurations of a setup joint, wherein Figs. 4G-H illustrate a setup joint configured to support a pair of instrument drivers, and Figs. 4I-J illustrate a setup joint configured to support three instrument drivers;

Fig. 5A-D illustrate an arrangement for controlling a robotic medical instrument using an instrument driver, wherein Fig. 5A is a forward perspective view of one configuration an instrument driver having a flexible instrument assembly mounted thereon, Fig. 5B is a rear perspective view of the arrangement shown in Fig. 5A, Fig. 5C is a forward perspective view of the arrangement shown in Figs. 5A-B, and Fig. 5D is a rear perspective view of the arrangement;

Figs. 5E-L illustrate an embodiment of an instrument driver according to the invention for controlling a robotic medical instrument and including independently rotatable carriages, wherein Figs. 5E-F are cross-sectional side views illustrating positioning and axial motion of carriages, Figs. 5G-H are rear views of the instrument driver shown in Figs. 5E-F and that show independent carriage rotation, Fig. 5I is a perspective view of an instrument driver having independently rotatable carriages, and Fig. 5J illustrates one embodiment of a gear arrangement for rotating a carriage;

Fig. 5K is a top view of an embodiment of an instrument driver that includes independently rotatable carriages, and Fig. 5L is a side elevation view of the arrangement shown in Fig. 5K;

Figs. 6A-E illustrate embodiments of a flexible catheter assembly, wherein Fig. 6A is a forward perspective view of catheter assembly, Fig. 6B is a rear perspective view of Fig. 6A, Fig. 6C illustrates a flexible sheath instrument, and Fig. 6D illustrates a flexible catheter instrument, and Fig. 6E illustrates an embodiment of a flexible catheter assembly having splayers with their housings removed to show their control knobs;

Figs. 7A-D illustrate embodiments of a flexible catheter having instrument members with varying degrees of flexibility, wherein Fig. 7A illustrates a catheter having a flexible distal end, Fig. 7B illustrates a catheter having a flexible distal end and flexible segment disposed between rigid segments, Fig. 7C illustrates a catheter having a rigid proximal segment, a flexible medial segment, and a flexible distal segment, and Fig. 7D illustrates a catheter having a flexible proximal segment and a flexible distal segment;

Figs. 8A-F illustrate a configuration of a flexible catheter assembly having various degrees of freedom;

Figs. 9A-9G illustrate further embodiments of a flexible catheter having various degrees of freedom, and Figs. 9B-G are cross sectional views along line A-A in Fig. 9A illustrating different opening shapes;

Figs. 10A-C illustrate flexible catheter constructed according to configurations and having coil, braid and skeletal hinge spine or support structures;

Figs. 11A-B illustrate a flexible catheter constructed according to one configuration having a spine or support structure including a series of alternating spherical and cylindrical elements;

Figs. 12A-B illustrate a flexible catheter constructed according to another configuration that includes pivoting skeletal rings;

Figs. 13A-B illustrate another embodiment of a flexible catheter that includes a support spine including pivoting skeletal rings;

Figs. 14A-C illustrate a configuration of a flexible catheter having a support spine constructed with saddle joint blocks, wherein Fig. 14A is a perspective view of two cylindrical saddle joint segments that are mated together, Fig. 14B is an exploded view of two segments of Fig. 14A, and Fig. 14C illustrates a spine created with a plurality of saddle joint segments;

Figs. 15A-G illustrate flexible catheters constructed according to other configuration and including support spines constructed of wedges;

Figs. 16A-H illustrate embodiments of flexible catheters having support spines that include ball links;

Figs. 17A-B illustrate routing of control elements in one embodiment of a flexible catheter arrangement, wherein Fig. 17A includes cutaway views of catheter instrument members, and Fig. 17B is a cross-sectional view of the catheter assembly shown in Fig. 17A along cross-section B-B;

Fig. 18 illustrates one implementation of an uber-sheath including a first flexible catheter and a scope;

Fig. 19 illustrates another implementation of an uber-sheath including a pair of flexible catheters;

Fig. 20 illustrates a further configuration of an uber-sheath including three flexible catheters;

Fig. 21 illustrates yet another configuration of an uber-sheath including three flexible catheters and a scope;

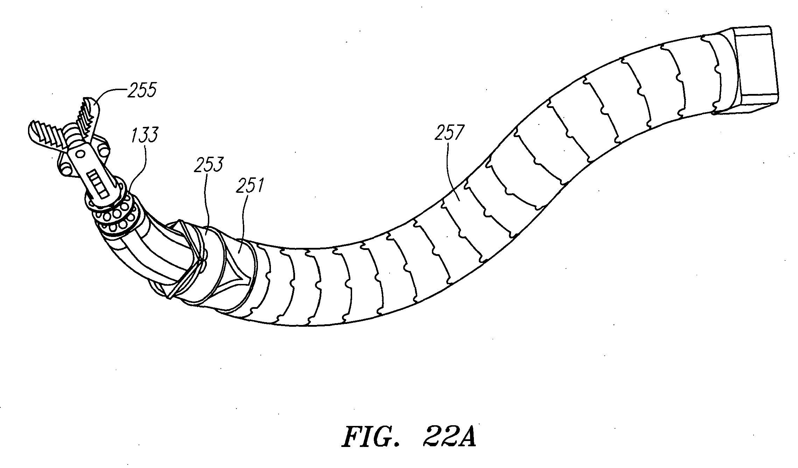

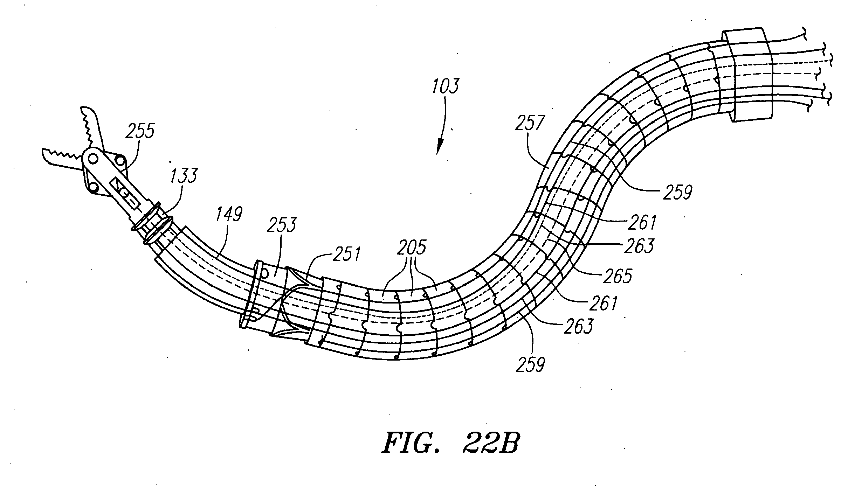

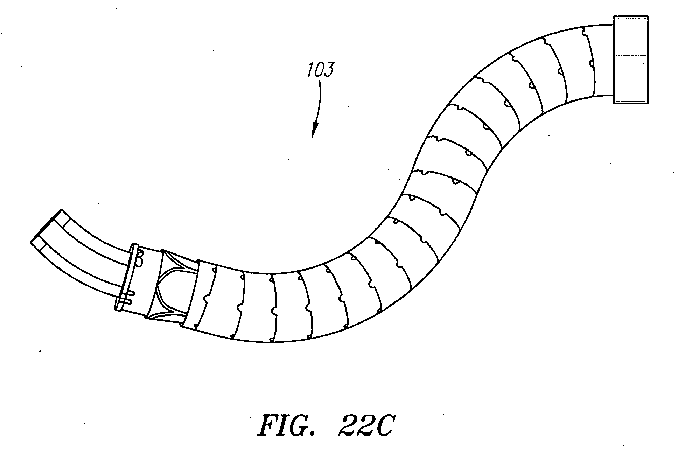

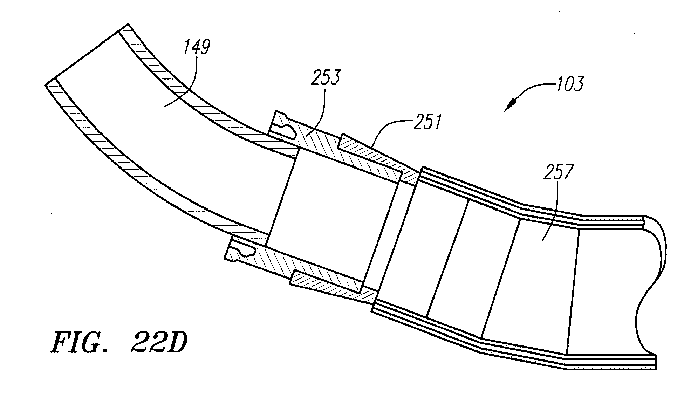

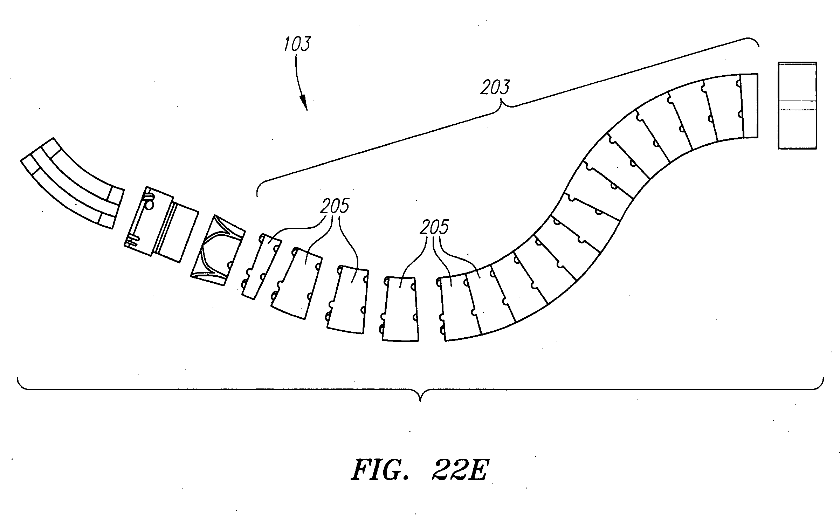

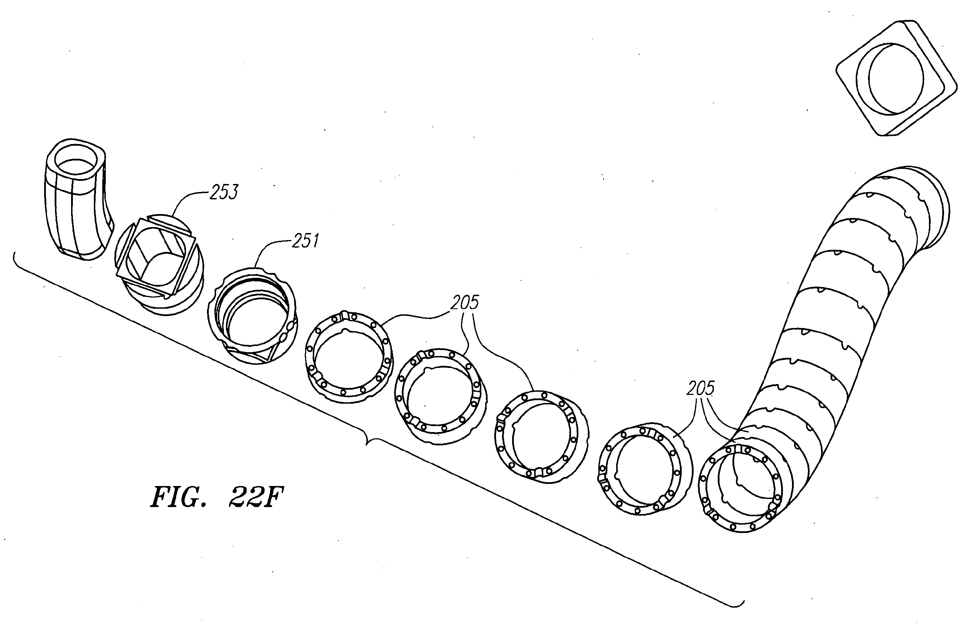

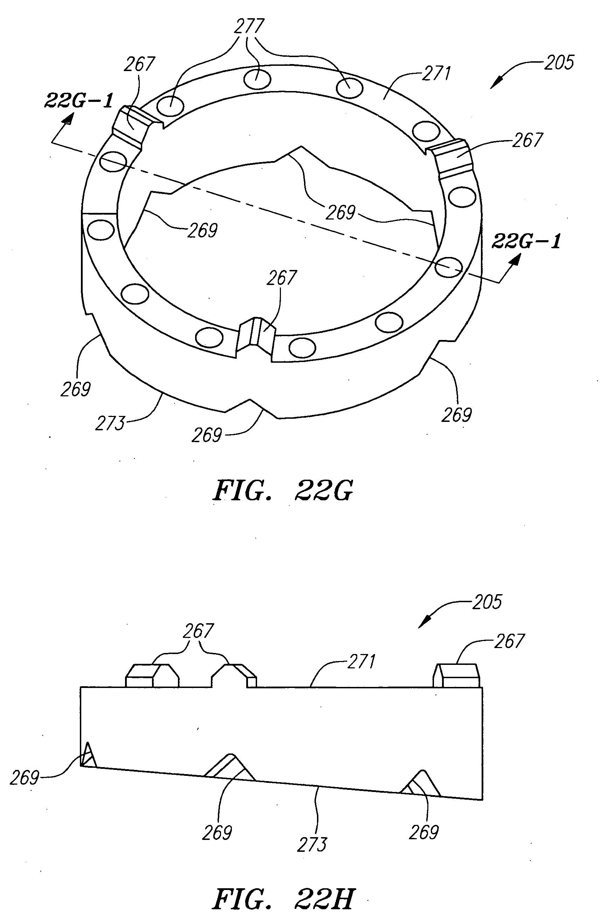

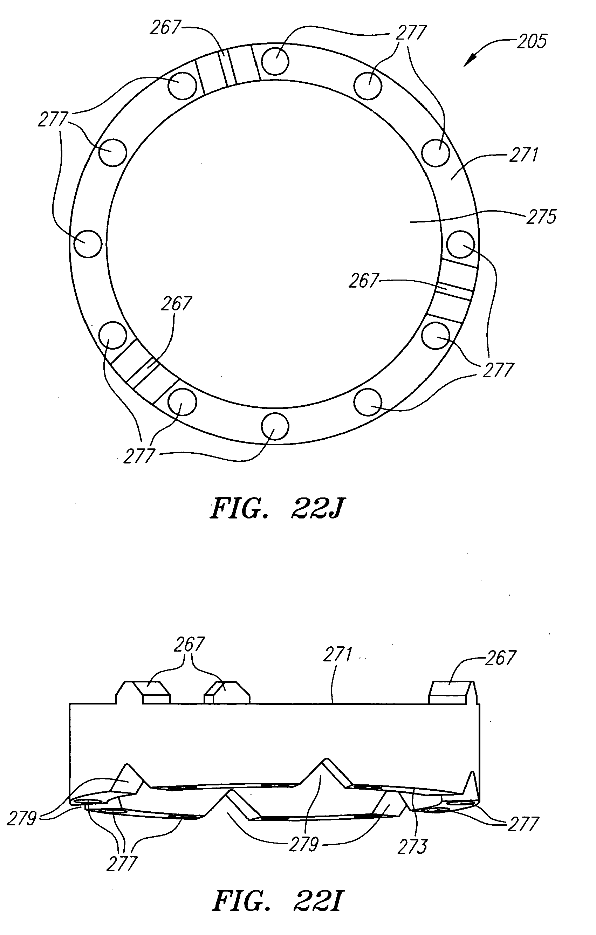

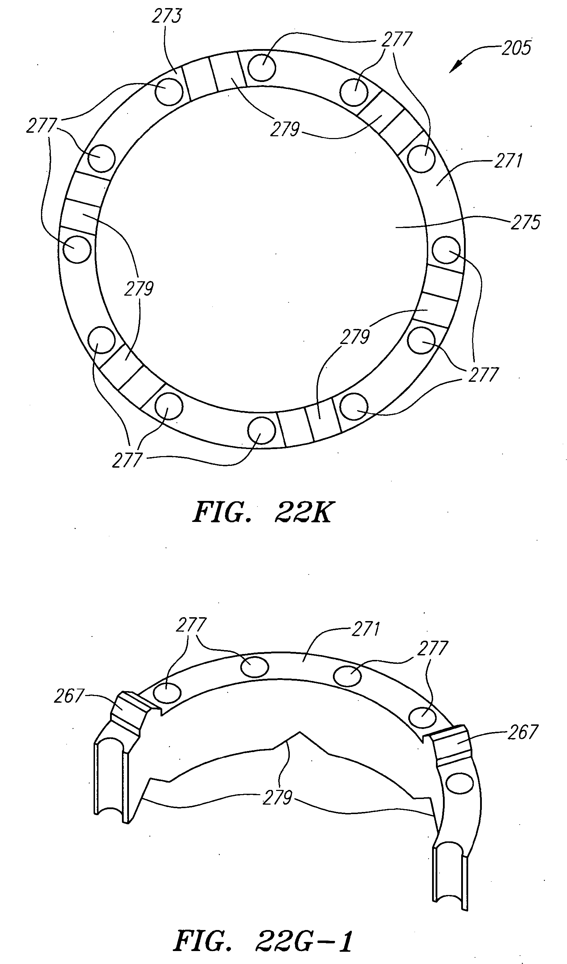

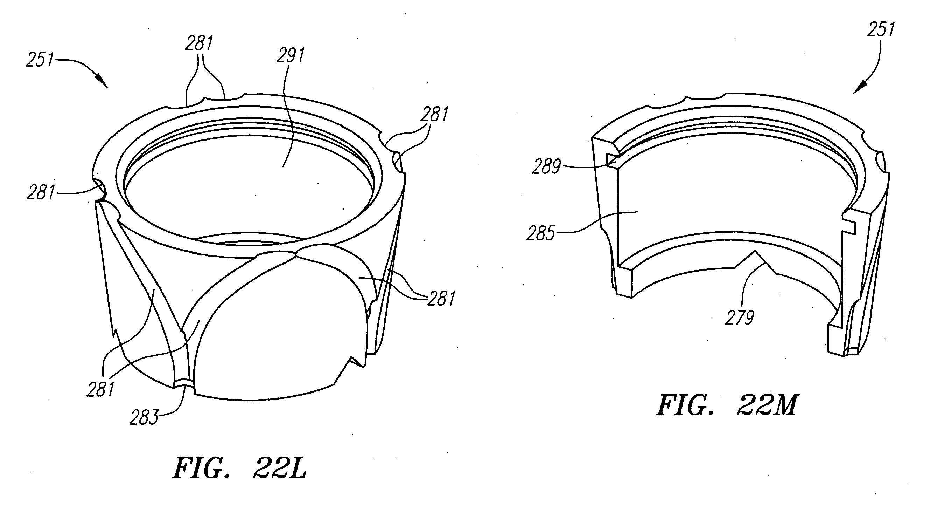

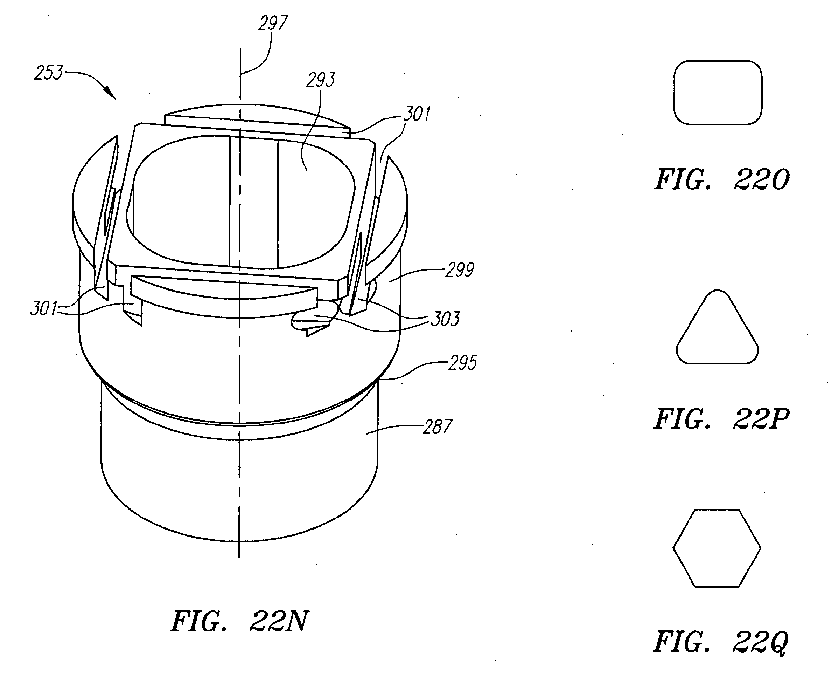

Fig. 22A-Q illustrate a flexible catheter assembly constructed according to other configurations wherein Fig. 22A illustrates a flexible catheter assembly having spine or support structure including wedges, Fig. 22B provides an alternative perspective view of the flexible catheter assembly shown in Fig. 22A, Fig. 22C is a side view of the flexible catheter assembly along its longitudinal axis, Fig. 22D is a cross-sectional view of the assembly shown in Fig. 22C, Figs. 22E-F are exploded views of a flexible catheter assembly, Figs. 22G-K provide more detailed views of spine wedges, wherein Fig. 22G is a perspective view of a wedge, Fig. 22H is a side view of a wedge, Fig. 22I is a perspective view, and Fig. 22J is a top view of a wedge, and Fig. 22K is a bottom view, Fig. 22L illustrates an interface piece, and Fig. 22M illustrates a cross-sectional view of the interface piece shown in Fig. 22L, Fig. 22N is a perspective view of a rotational collar, Fig. 22O illustrates an opening having a square shape with rounded corners, Fig. 22P illustrates an opening having a triangular shape with rounded corners, and Fig. 22Q illustrates an opening having a hexagon shape;

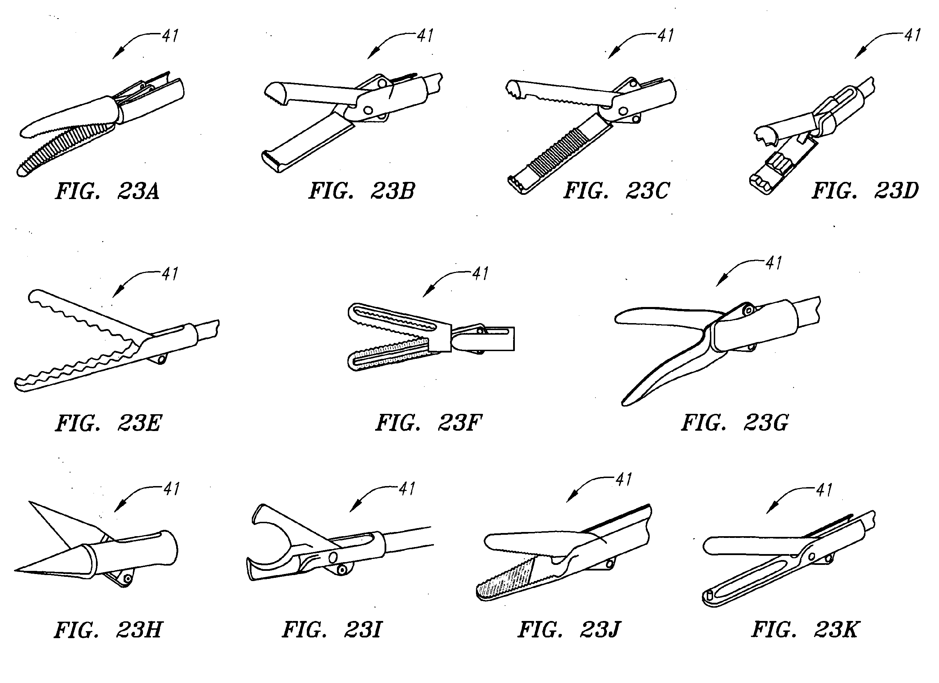

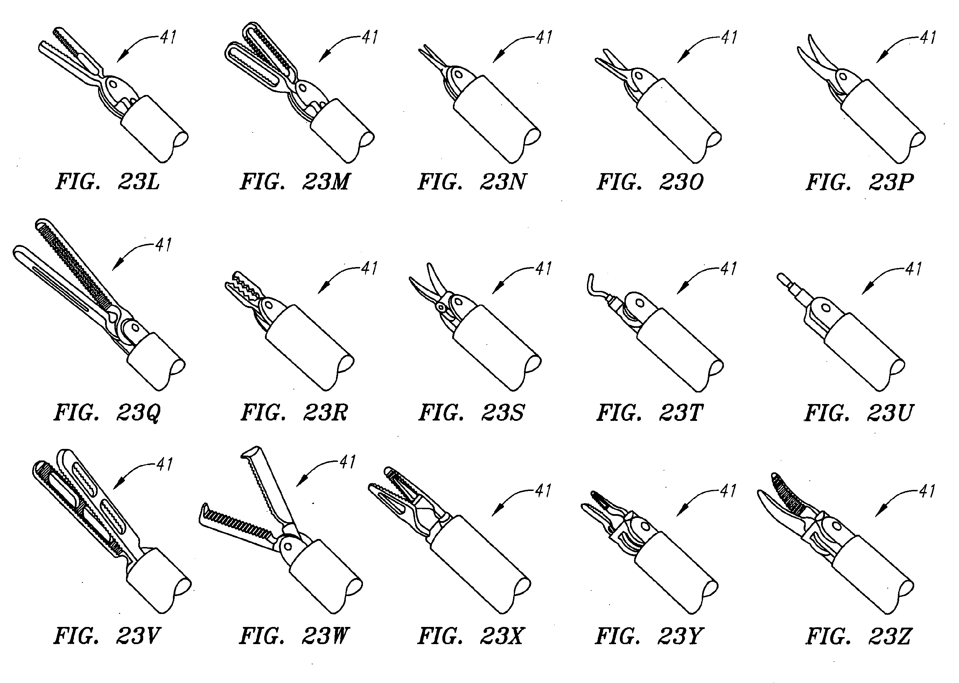

Figs. 23A-Z illustrate different types of working instruments that may be used with flexible catheter configurations wherein Fig. 23A illustrates a curved Maryland dissector, Fig. 23B illustrates a serrated Manhes grasping forceps, Fig. 23C illustrates surgical and serrated Manhes grasping forceps, Fig. 23D illustrates cobra type forceps with claw and twin rows of teeth for myomis, Fig. 23E illustrates Davis & Geak forceps, Fig. 23F illustrates Johann atraumatic grasping forceps, Fig. 23G illustrates a Metzenbaum type of serrated curved scissors, Fig. 23H illustrates a pair of straight micro dissection scissors, Fig. 23I illustrates a pair of hook scissors, Fig. 23J illustrates needle holder forceps with short jaws, Fig. 23K illustrates biopsy forceps with up and down thorns, Fig. 23L illustrates long tip forceps, Fig. 23M illustrates Cadiere forceps, Fig. 23N illustrates a pair of Potts scissors, Fig. 23O illustrates a pair of round tip scissors, Fig. 23P illustrates a pair of curved scissors, Fig. 23Q illustrates a bowel grasper, Fig. 23R illustrates Resano forceps, Fig. 23S illustrates hot shears, Fig. 23T illustrates a cautery hook, Fig. 23U illustrates a cautery spatula, Fig. 23V illustrates a double fenestrated grasper, Fig. 23W illustrates a cobra grasper, Fig. 23X illustrates a bipolar cautery instrument, Fig. 23Y illustrate a micro bipolar cautery instrument, and Fig. 23Z illustrates a Maryland bipolar cautery instrument;

Figs. 24A-B illustrate an configuration of catheter assembly that includes a catheter drive shaft including a helical drive element and configured such that axial displacement of a catheter drive shaft causes a corresponding rotation;

Figs. 25A-B illustrate an embodiment of catheter assembly that includes a catheter drive shaft including a BNC drive element that is operable such that axial displacement of a catheter drive shaft causes a corresponding rotation;

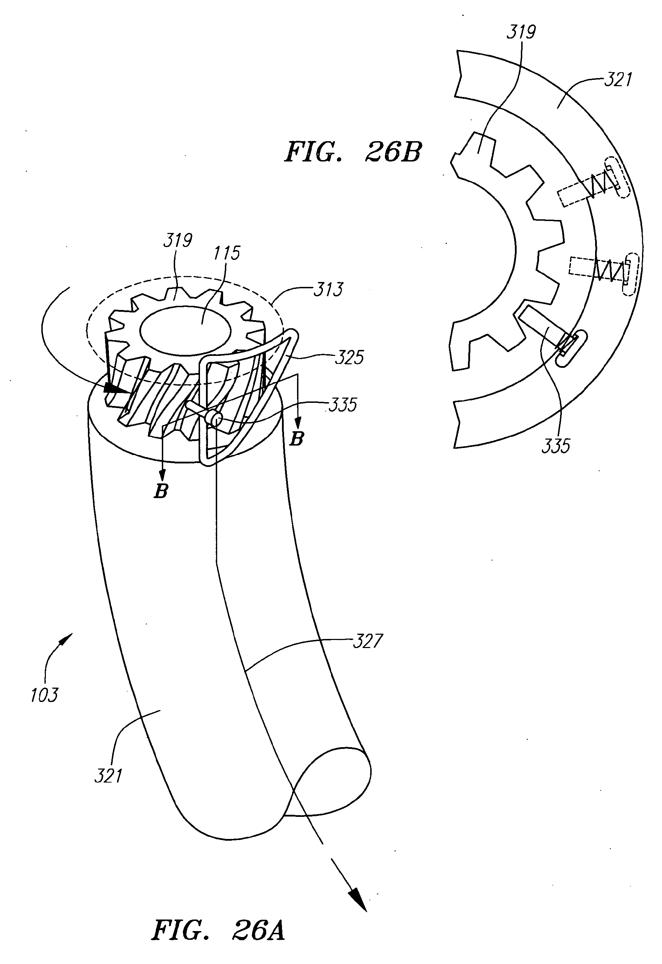

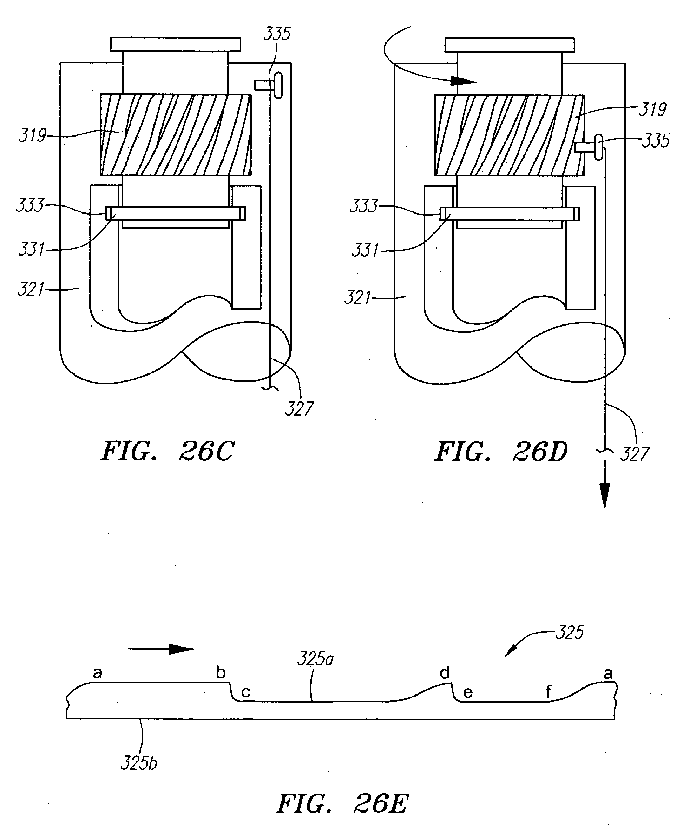

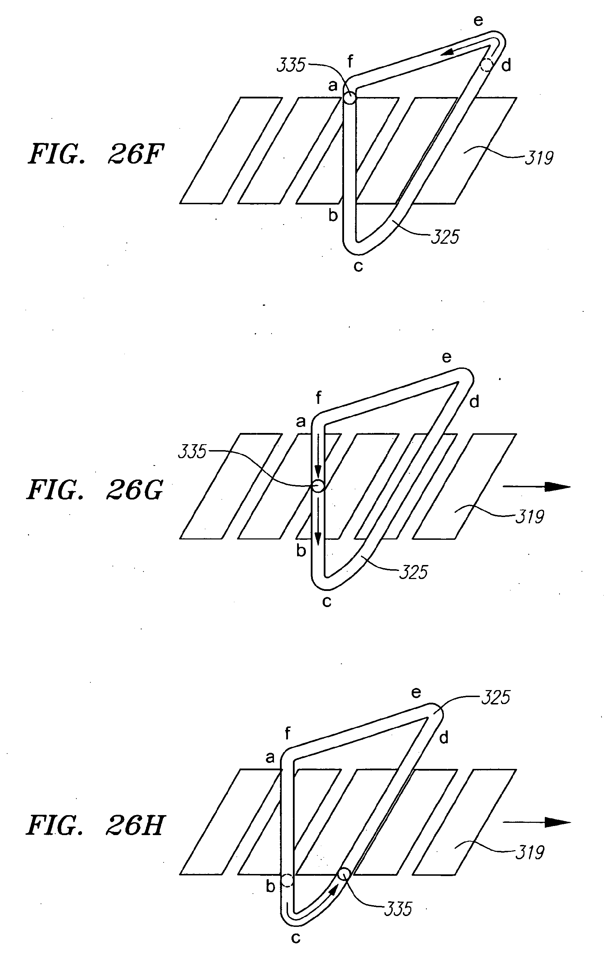

Figs. 26A-H illustrate one configuration of a catheter assembly that includes a ratchet drive element to rotate a segment of a flexible catheter, wherein Fig. 26A is a perspective view of a distal portion of an instrument member, Fig. 26B is partial top view of a portion of a helical gear and associated pin, Fig. 26C is a cross-sectional view of a helical gear and its associated pin in a first position, Fig. 26D is a cross-sectional view of a helical gear and its associated pin in another position, Fig. 26E is cross-sectional view of a surface of a slotted track or guide upon which a pin traverses, Fig. 26F illustrates a pin carried by a guide and positioned at a top of a track or groove of a gear, Fig. 26G illustrates the pin shown in Fig. 26F moving along the guide and through a track or groove of the gear, and Fig. 26H illustrates the pin traversing a different portion of the guide and the gear;

Figs. 27A-E illustrate an embodiment of a catheter assembly that includes a dual ratchet drive element to allow bi-directional rotation of a segment of a flexible catheter, wherein

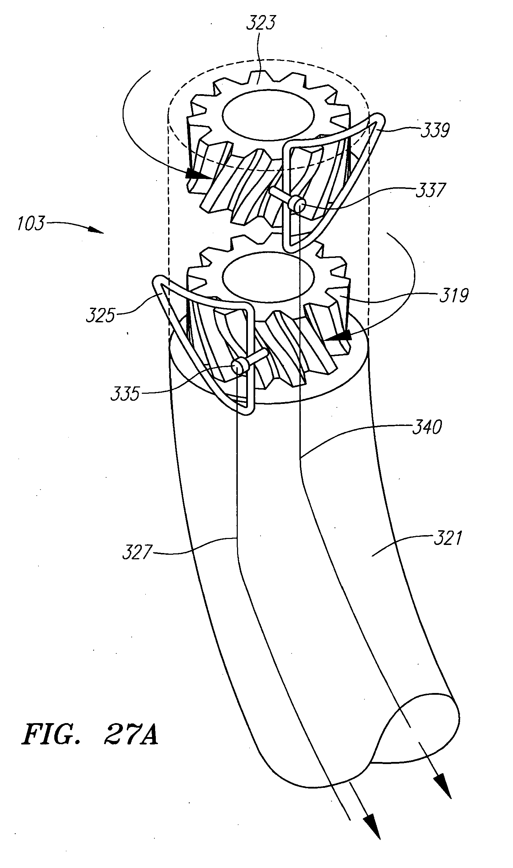

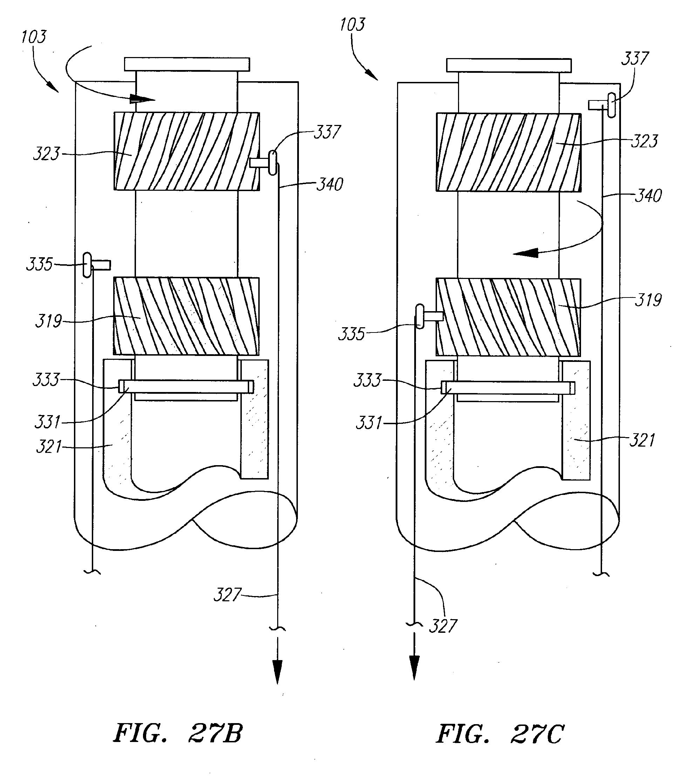

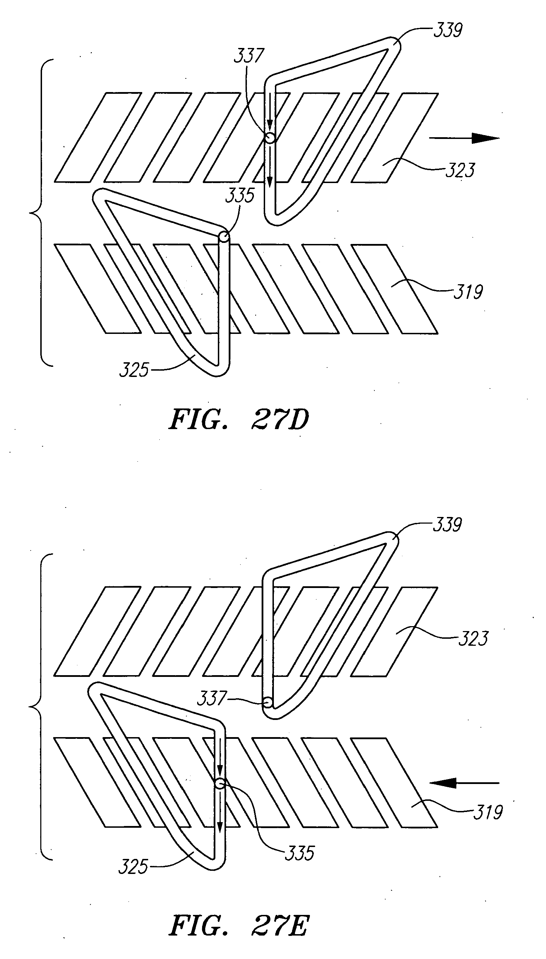

Fig. 27A is a perspective view of internal components of a distal portion of an instrument member, Fig. 27B is a cross-sectional view helical gears and associated pins in a first position, Fig. 27C is a cross-sectional view of helical gears and pins at different positions, Fig. 27D illustrates pins carried by respective guides and at respective initial positions, and Fig. 27E illustrates pins carried by respective guides being moved along the guides and through tracks of associated gears;

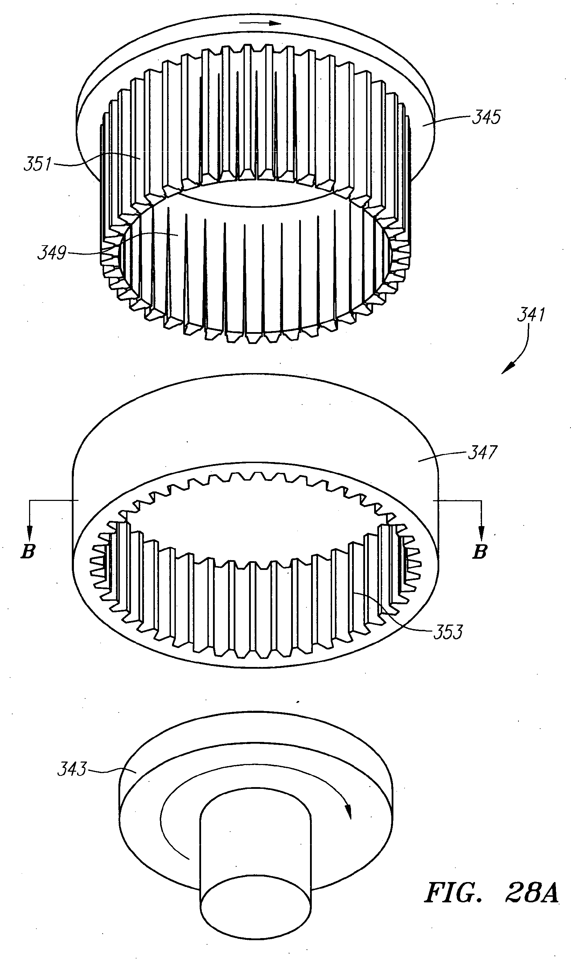

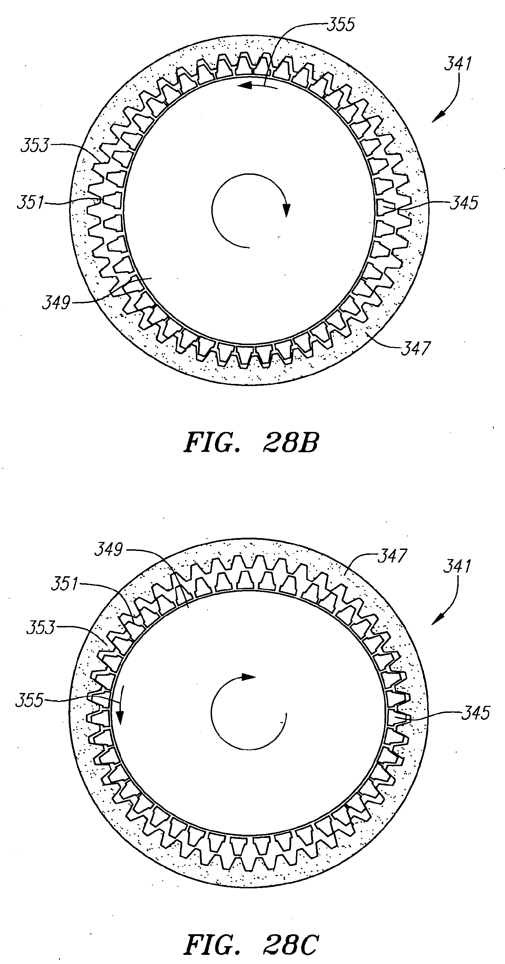

Figs. 28A-C illustrate an configuration of a catheter assembly that includes a harmonic drive element to rotate a segment of a flexible catheter, wherein Fig. 28A illustrates various components of a harmonic drive element, Fig. 28B is a cross-sectional view of Fig. 28A along line B-B with engagement at the tops and bottoms of gears, and Fig. 28C is a cross-sectional view of Fig. 28A along line B-B with engagement at the sides of gears;

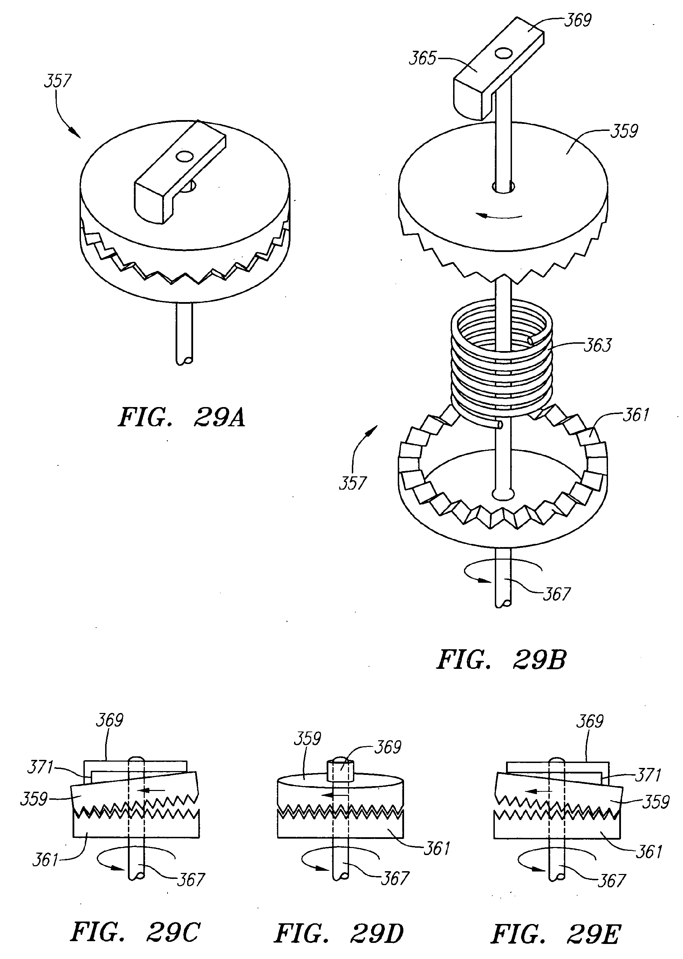

Figs. 29A-E illustrate an embodiment of a catheter assembly that includes a wobble plate drive plate to rotate a segment of a flexible catheter utilizing an arm or finger element that engages a top surface of a gear element of the wobble plate drive, wherein Fig. 29A is a perspective view of one configuration of a wobble plate drive element, Fig. 29B is an expanded view further illustrating components of the wobble drive element shown in Fig. 29A, and Figs. 29C-E illustrates operation of the wobble plate drive element as force is applied to different portions of a top surface of a gear element;

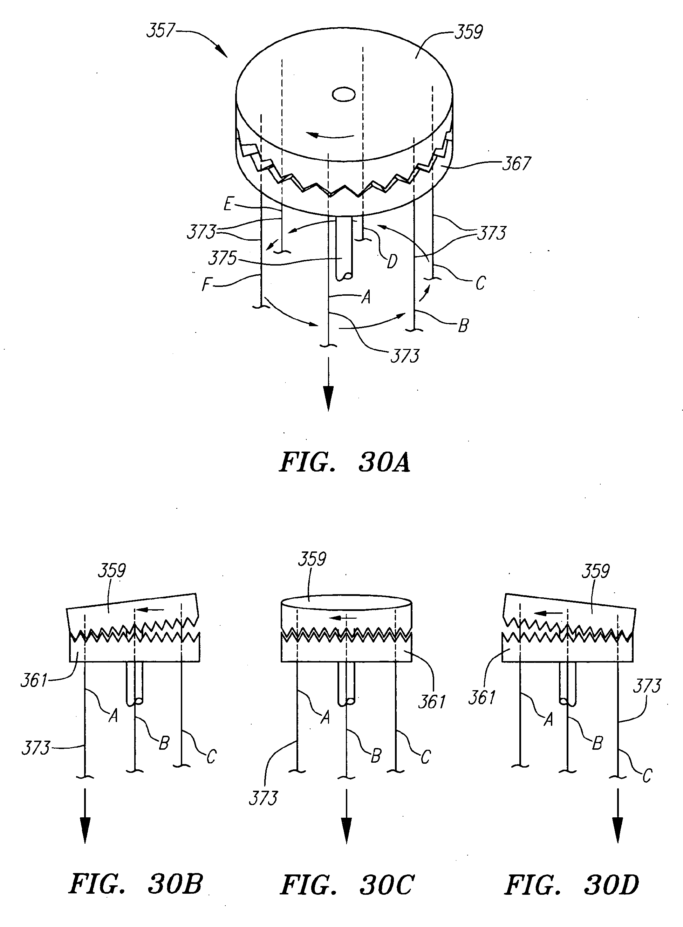

Figs. 30A-D illustrate a configuration of a catheter assembly that includes a wobble plate drive plate to rotate a segment of a flexible catheter utilizing control elements, wherein Fig. 30A is a perspective view of a wobble plate drive element driven by control elements, and Figs.

Figs. 30C-E illustrate operation of the wobble plate drive element as force sequentially applied to different portions of a top surface of a gear element by sequentially pulling control elements;

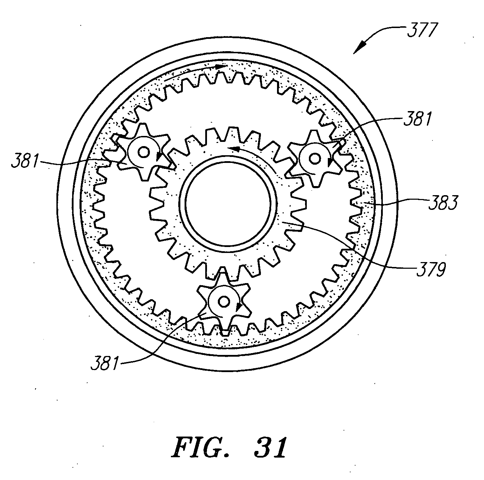

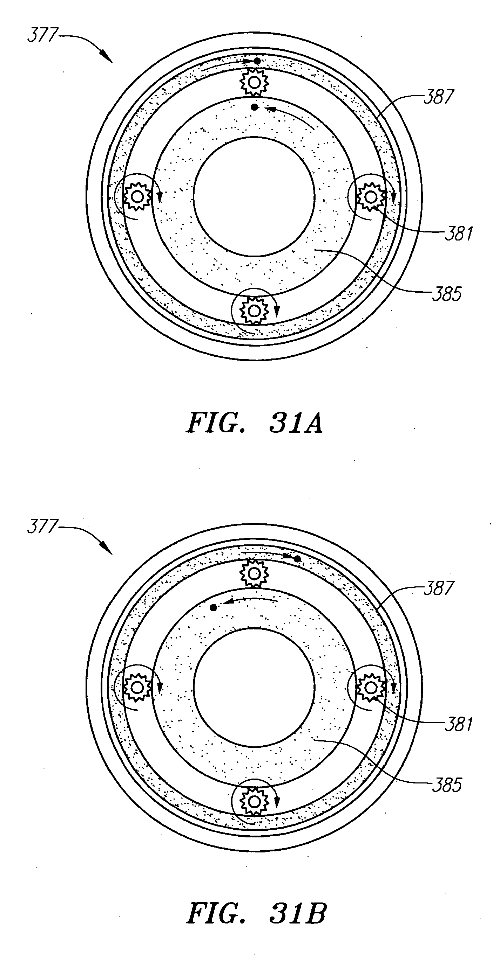

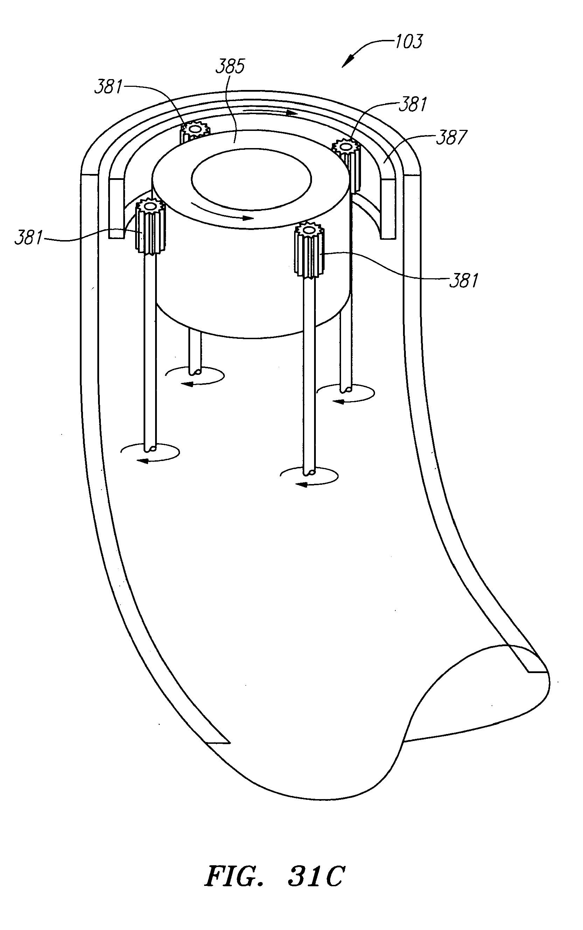

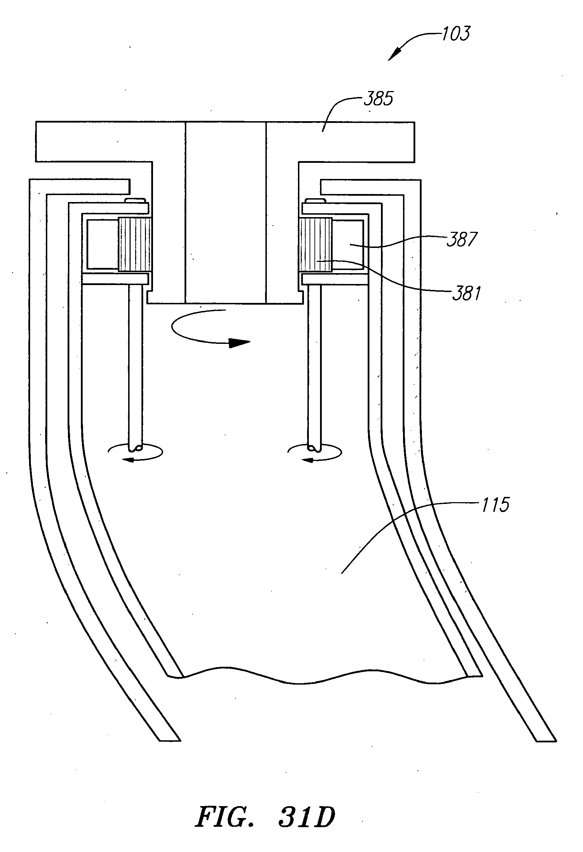

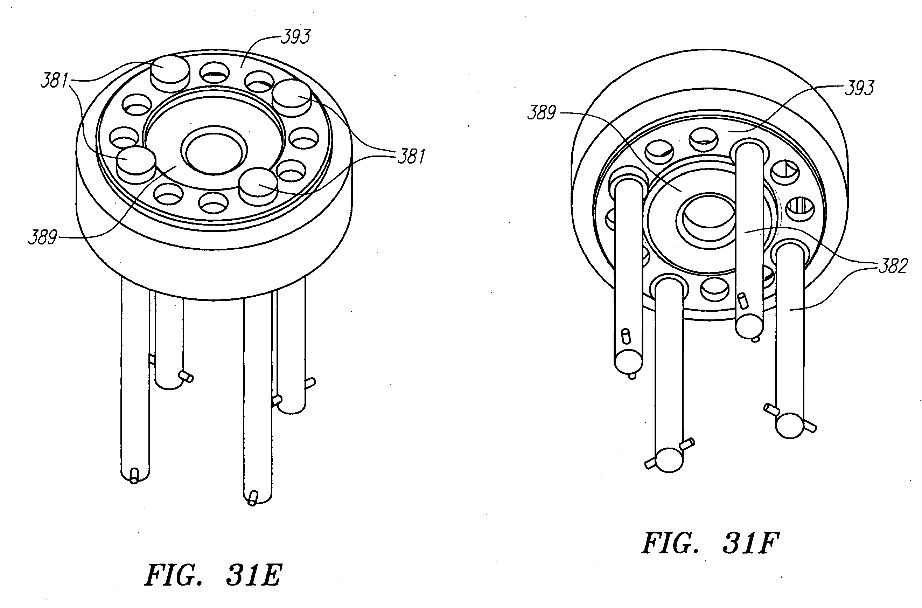

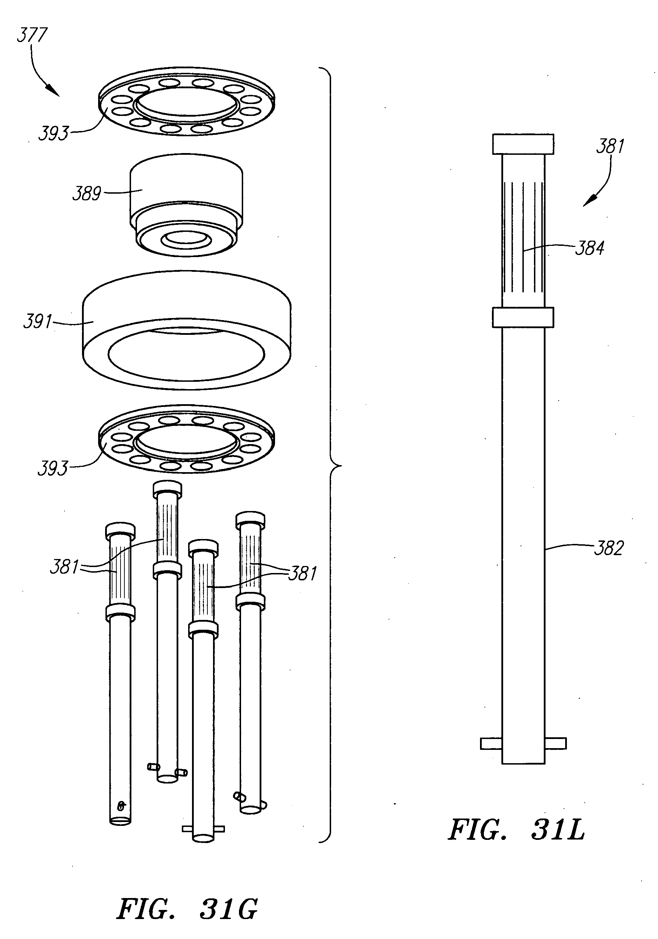

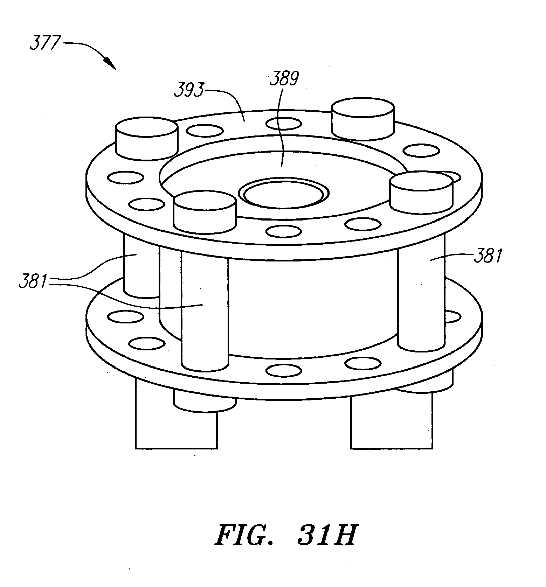

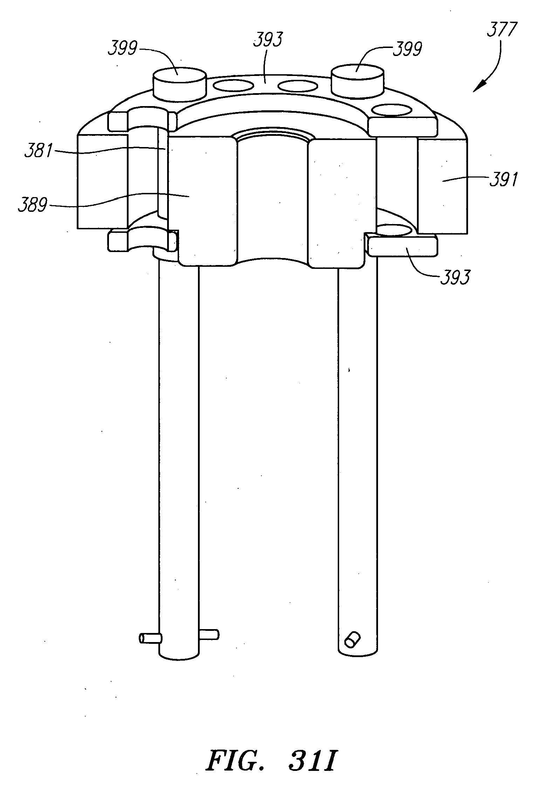

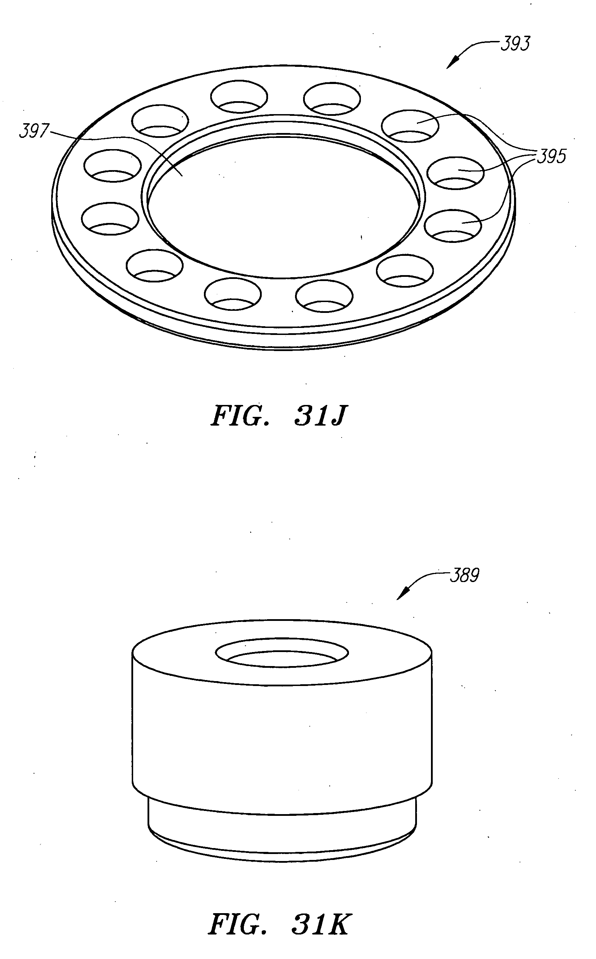

Fig. 31 illustrates one configuration of a planetary gear drive to rotate a segment of a flexible catheter;

Figs. 31A-K illustrate other configurations of planetary gear drives to rotate a segment of a flexible catheter, wherein Figs. 31A is a top view of a planetary gear drive element and showing driving of planetary gears, Fig. 31B is a top view of a planetary gear drive element and showing rotation of a sun gear after a revolution of a planetary gear, Fig. 31C is a cross-sectional view of the drive assembly within a flexible instrument member, Fig. 31D is an exploded cross-sectional view of a drive assembly, Fig. 31Eis a top perspective view of a planetary gear drive, Fig. 31F is a bottom perspective view of a planetary gear drive, Fig. 31G further illustrates components of a planetary gear drive assembly, Fig. 31H is a further perspective view of a planet gear drive element, Fig. 31I is a cross-sectional view of a planet gear drive element, Fig. 31J is a perspective view of a retention disc, Fig. 31K is a perspective view of a sun band piece, Fig. 31L further illustrates a planet gear component;

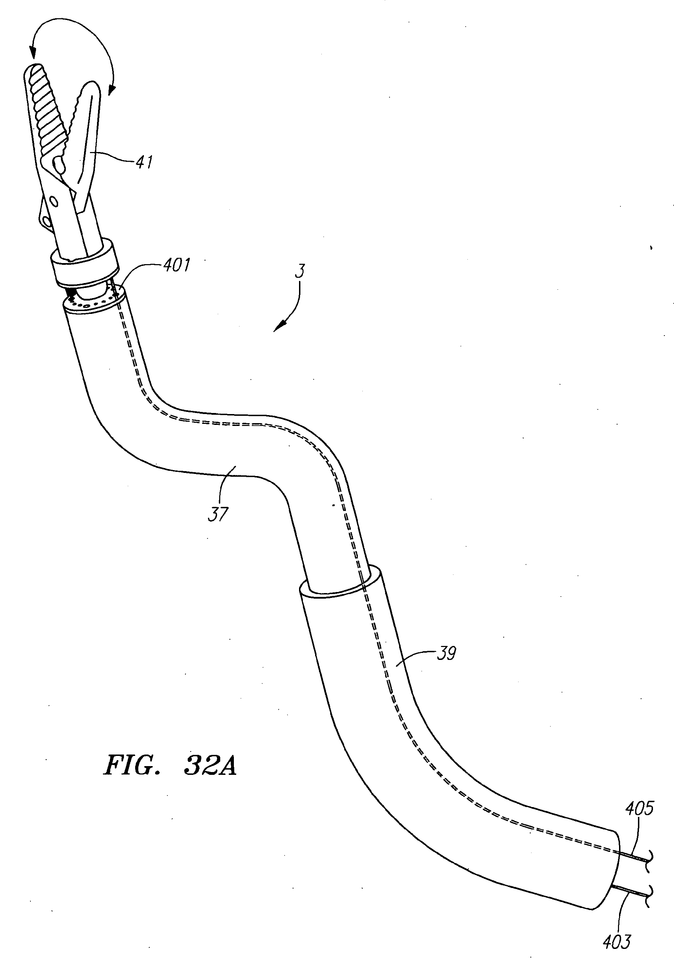

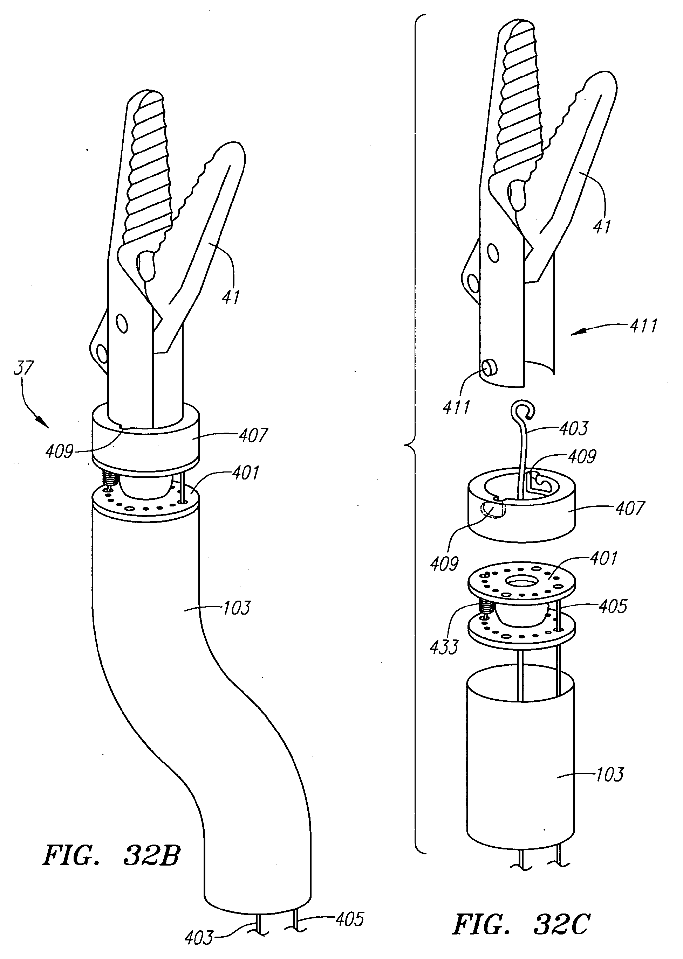

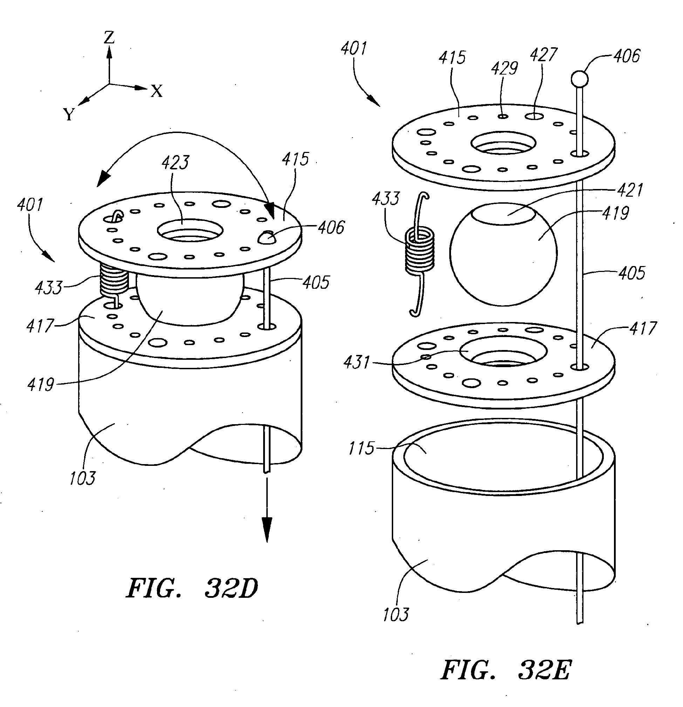

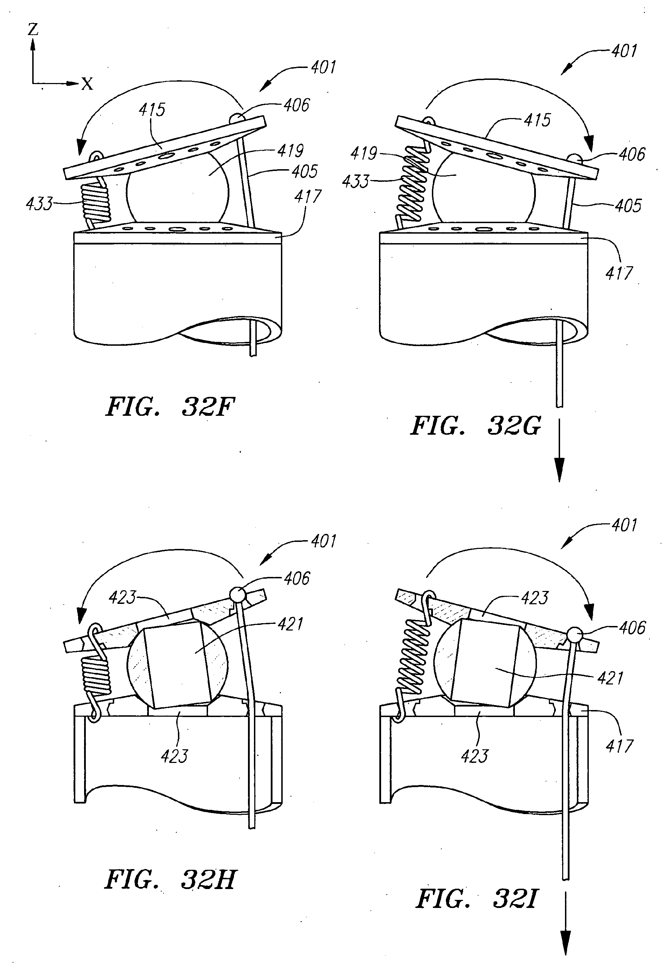

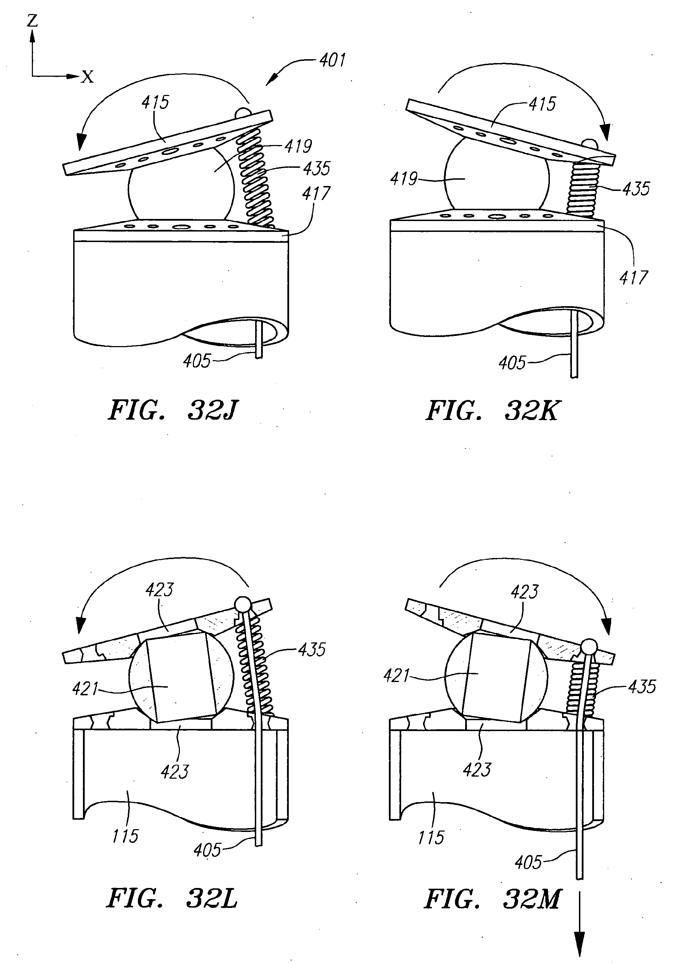

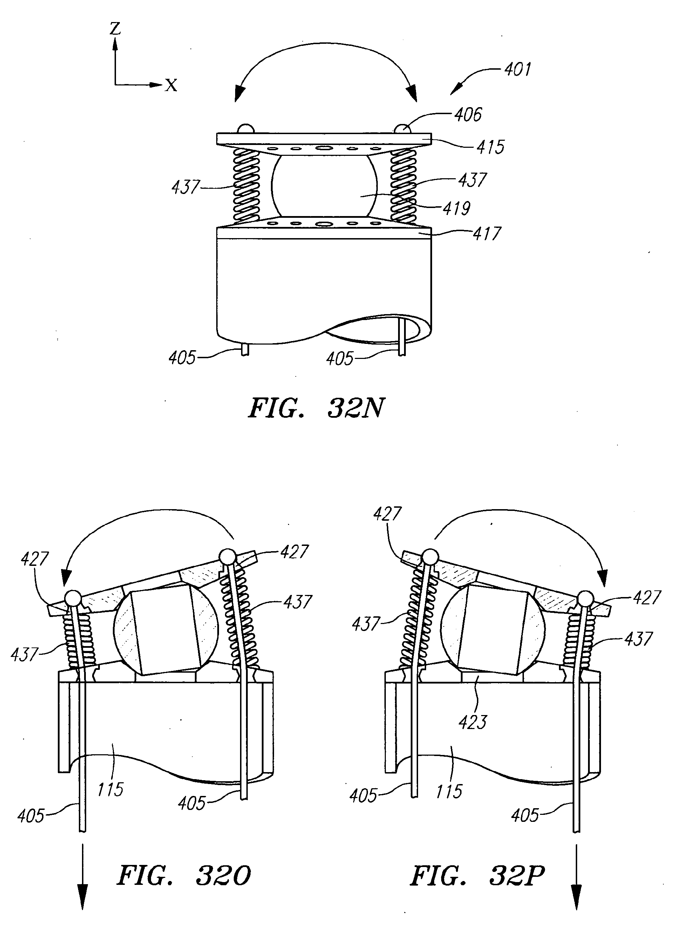

Figs. 32A-P illustrate configurations of an orientation platform or interface for a working instrument coupled to a distal end of a catheter having a ball and socket assembly, wherein Fig. 32A is a perspective view of a flexible catheter assembly constructed according to one embodiment, Fig. 32B further illustrates a distal portion of the assembly shown in Fig. 32A, Fig. 32C is an exploded view of assembly components shown in Figs. 32A-B, Fig. 32D is a perspective view of a platform constructed according to one configuration Fig. 32E is an exploded view of the platform shown in Fig. 32D, Figs. 32F-I illustrate how the platform shown in Figs. 32D-D can be controlled, and Figs. 32J-M illustrate how a platform constructed according to another configuration in which a control element extends through a spring may be controlled, and Figs. 32N-P illustrate how a platform constructed according to another embodiment in which a control elements extends through respective springs may be controlled;

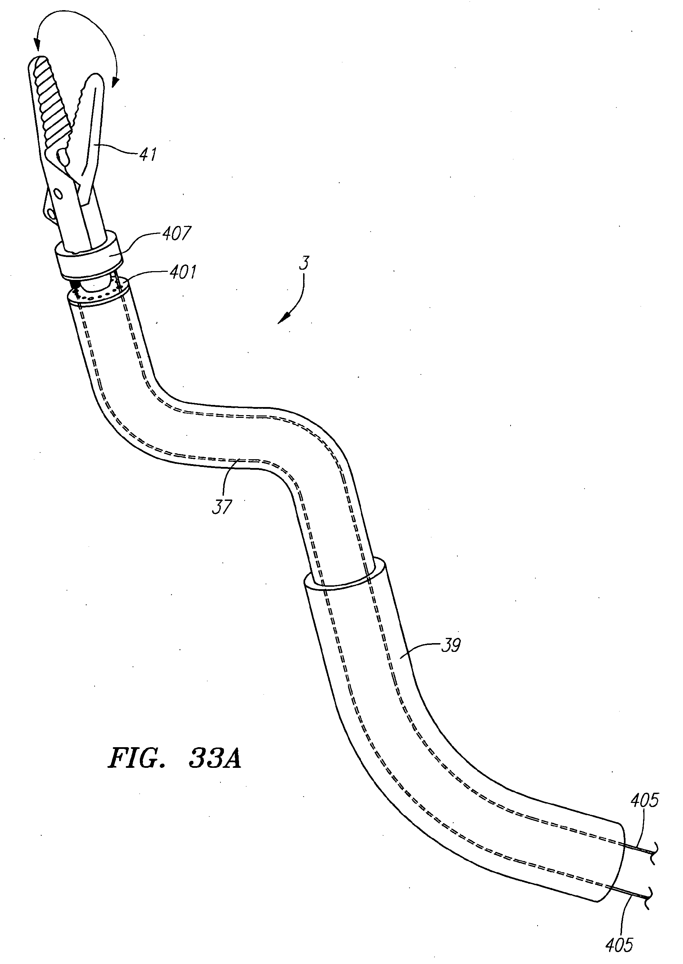

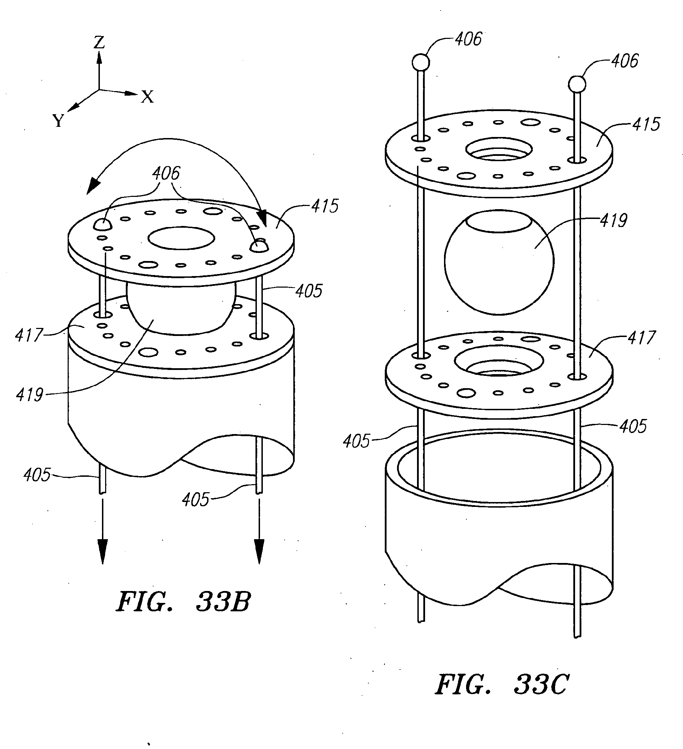

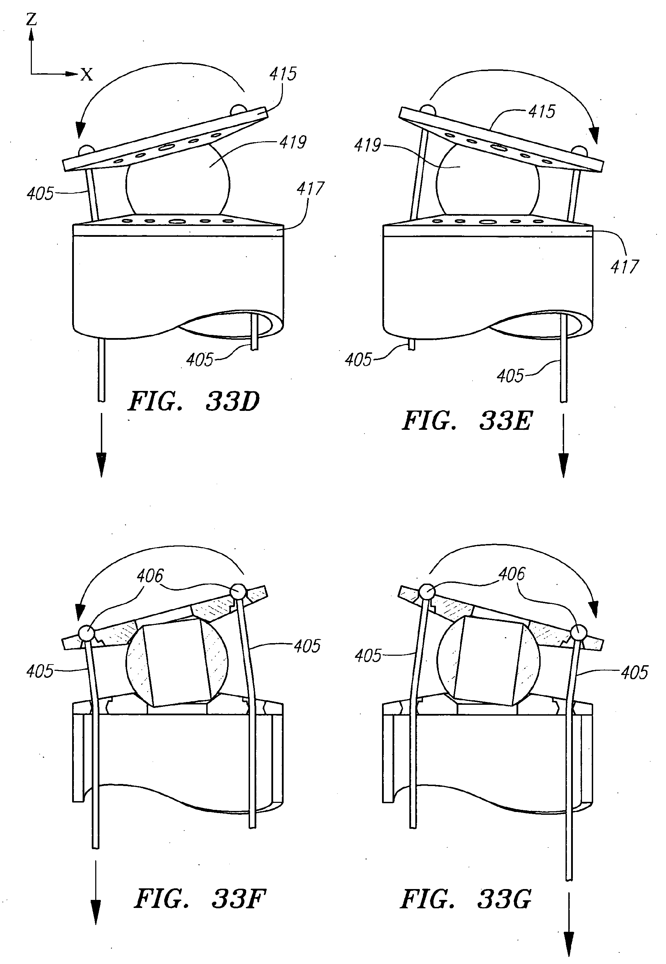

Figs. 33A-G illustrate another configuration of an orientation platform or interface constructed with a ball and socket assembly, wherein Fig. 33A is a perspective view of a flexible catheter assembly constructed according to one embodiment, Fig. 33B further illustrates a distal portion of the assembly shown in Fig. 33A, Fig. 33C is an exploded view of assembly components shown in Figs. 33A-B, Figs. 33D-G illustrate how the platform shown in Figs. 33B-C can be controlled;

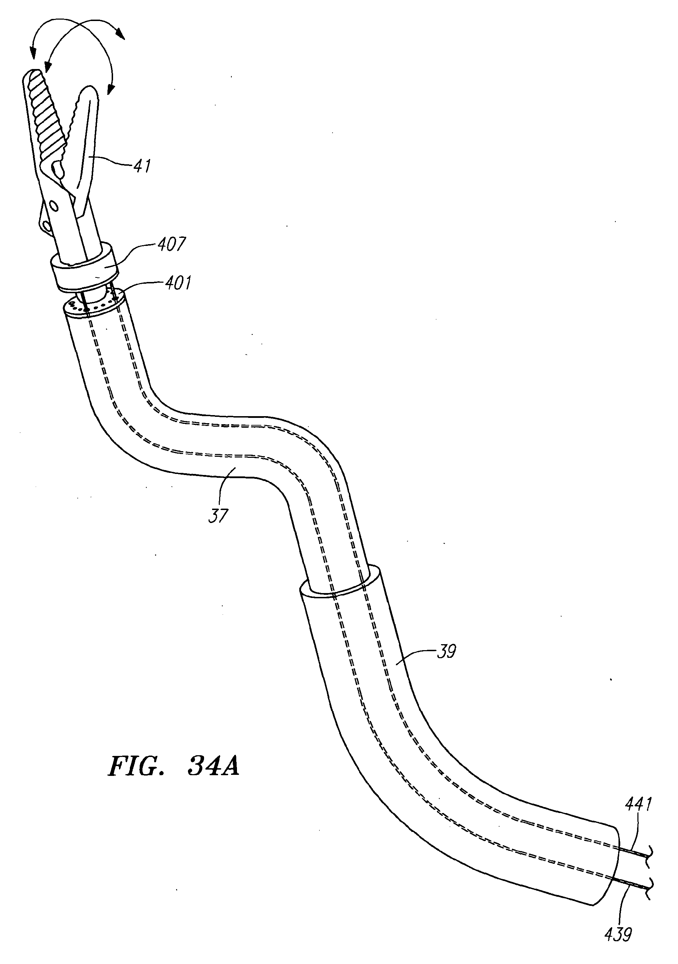

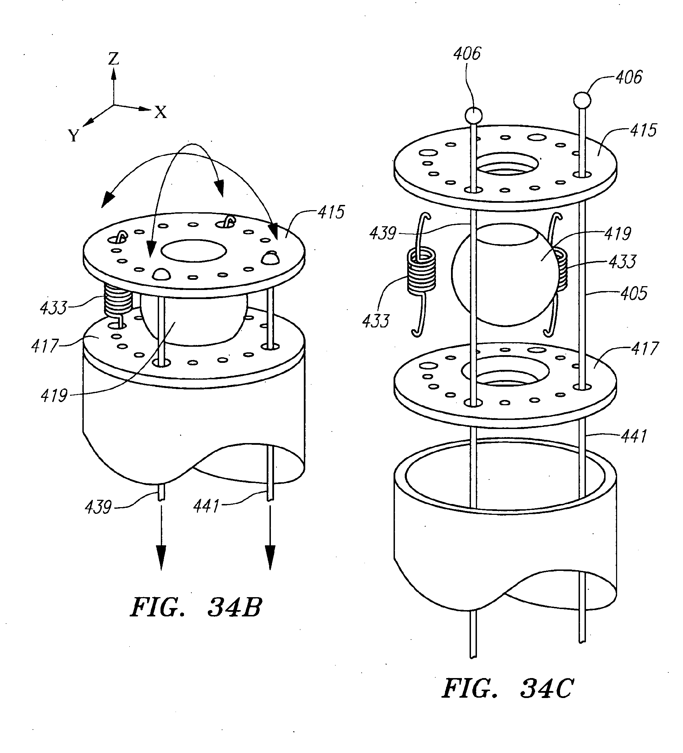

Figs. 34A-C illustrate yet another embodiment of an orientation platform or interface constructed a ball and socket assembly, wherein Fig. 34A is a perspective view of a flexible catheter assembly constructed according to one configuration, Fig. 34B further illustrates a distal portion of the assembly shown in Fig. 34A and including two springs, and Fig. 34C is an exploded view of assembly components shown in Figs. 34A-B;

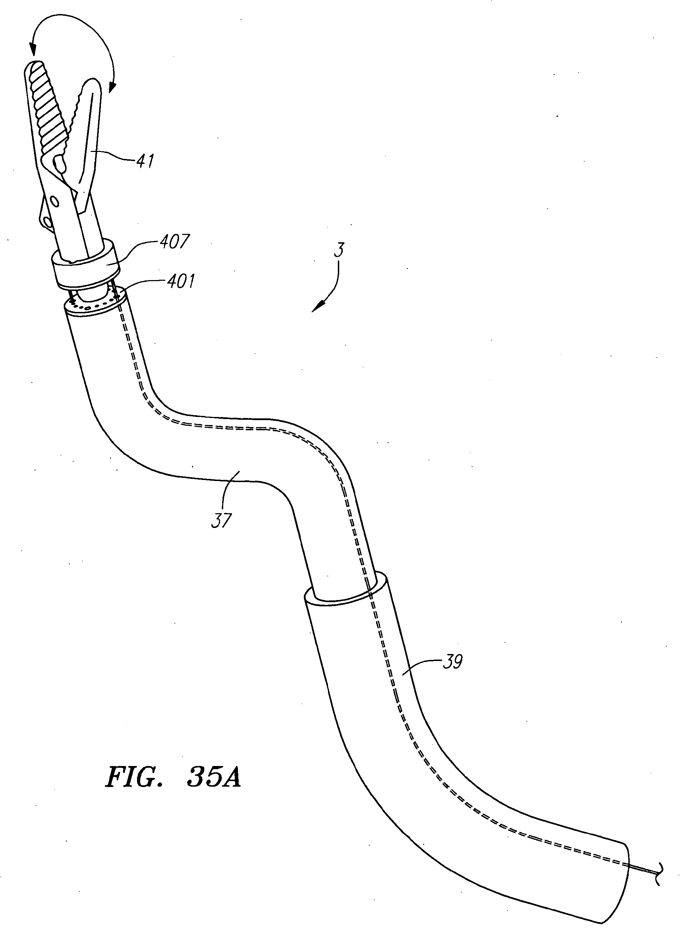

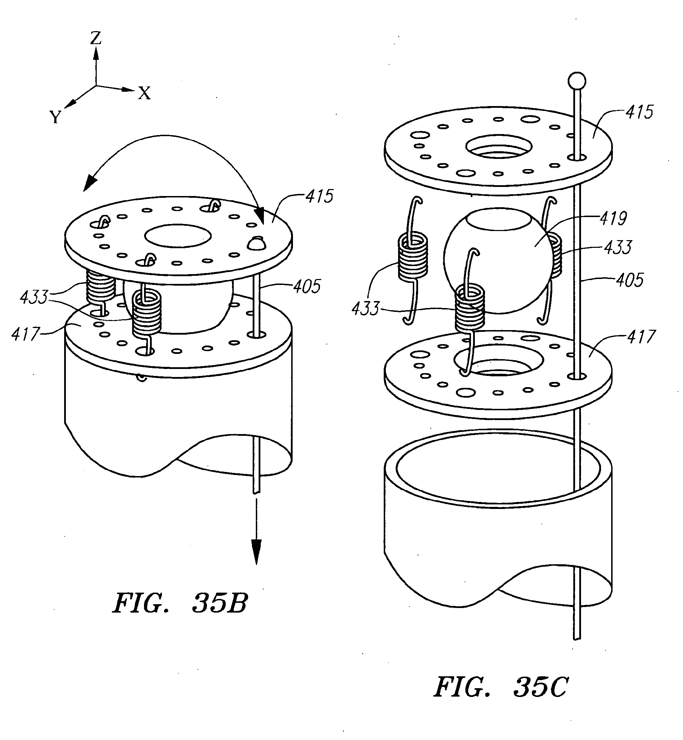

Figs. 35A-C illustrate still another embodiment of an orientation platform or interface constructed with a ball and socket assembly, wherein Fig. 35A is a perspective view of a flexible catheter assembly constructed according to one configuration, Fig. 35B further illustrates a distal portion of the assembly shown in Fig. 35A and including three springs and a control element, and Fig. 35C is an exploded view of assembly components shown in Figs. 35A-B;

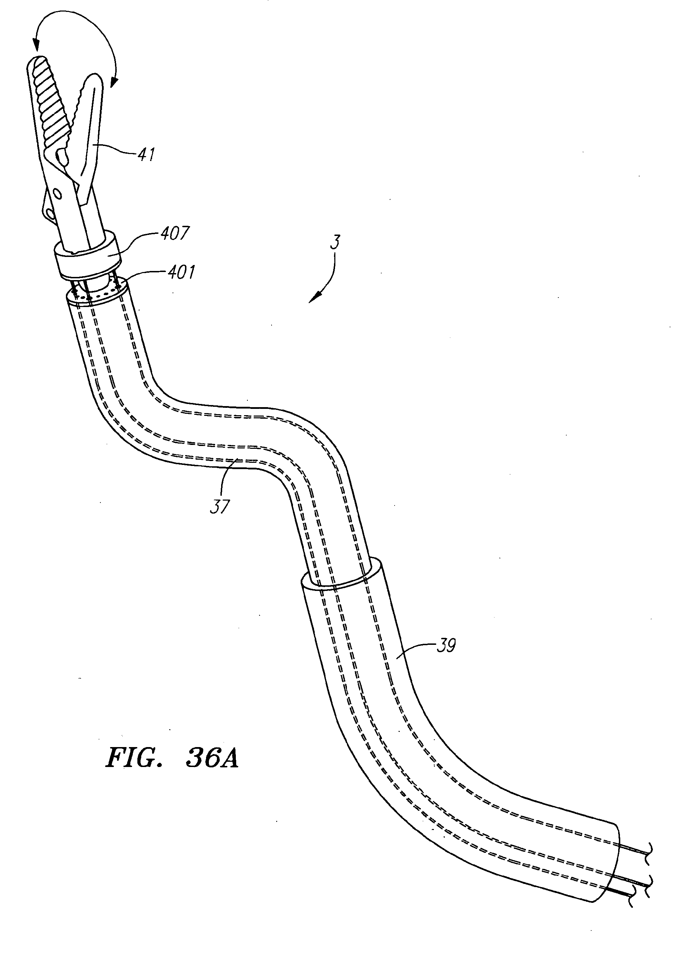

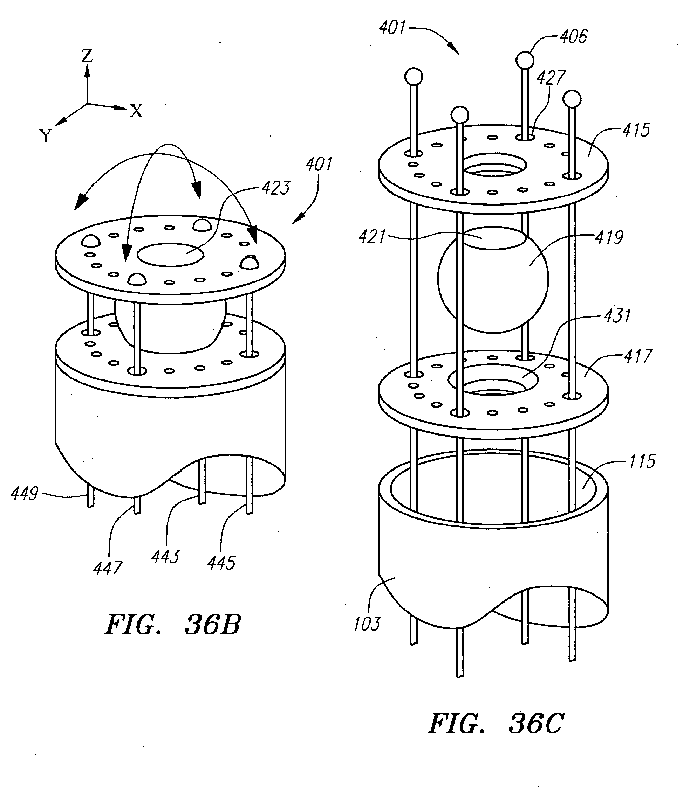

Figs. 36A-C illustrate a further configuration of an orientation platform or interface m constructed with a ball and socket assembly, wherein Fig. 36A is a perspective view of a flexible catheter assembly constructed according to one configuration, Fig. 36B further illustrates a distal portion of the assembly shown in Fig. 36A and including four equidistantly spaced control elements, and Fig. 36C is an exploded view of assembly components shown in Figs. 36A-B;

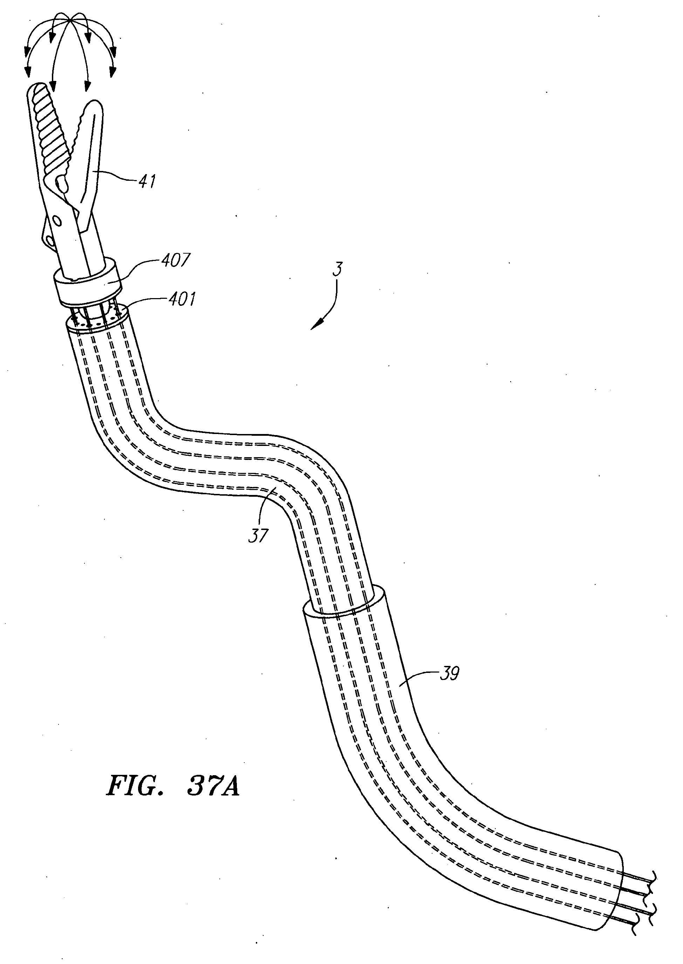

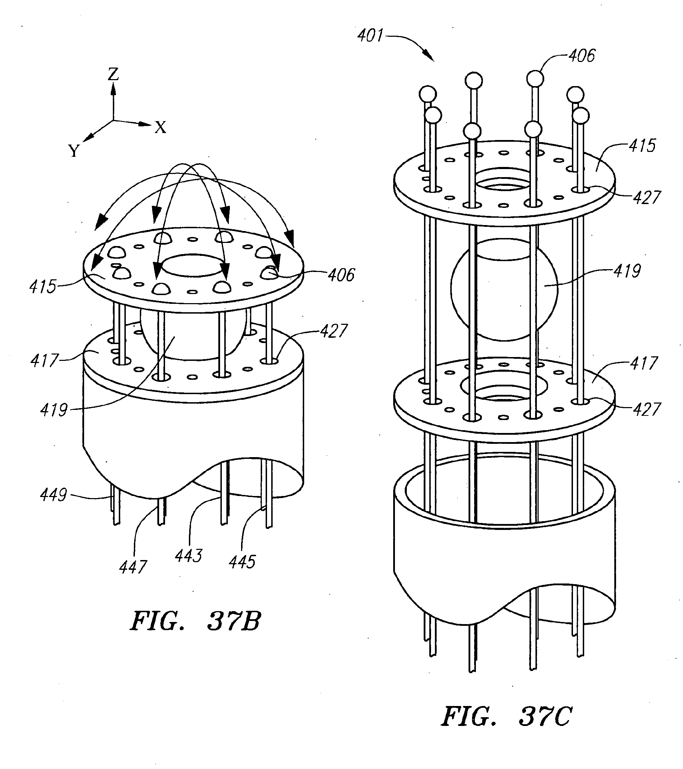

Figs. 37A-C illustrate yet another configuration of an orientation platform or interface constructed with a ball and socket assembly, wherein Fig. 37A is a perspective view of a flexible catheter assembly constructed according to one configuration Fig. 37B further illustrates a distal portion of the assembly shown in Fig. 36A and including eight equidistantly spaced control elements, and Fig. 37C is an exploded view of assembly components shown in Figs. 37A-B;

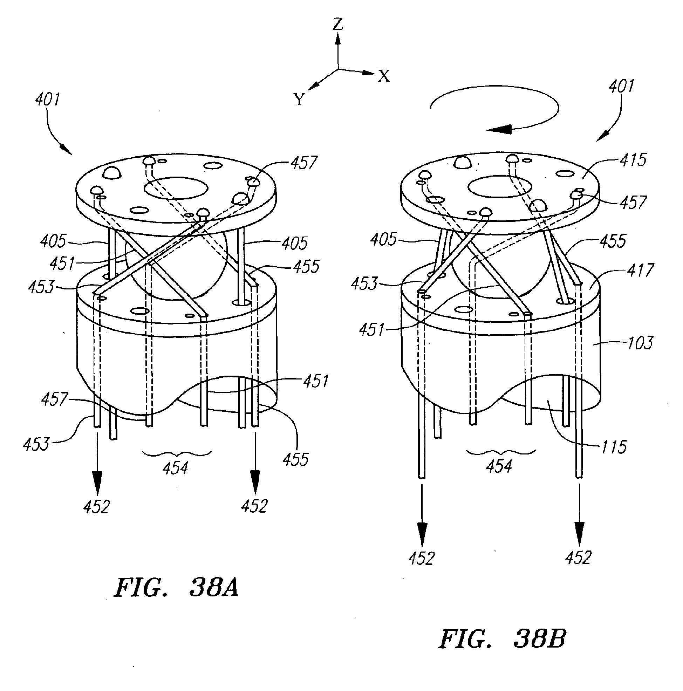

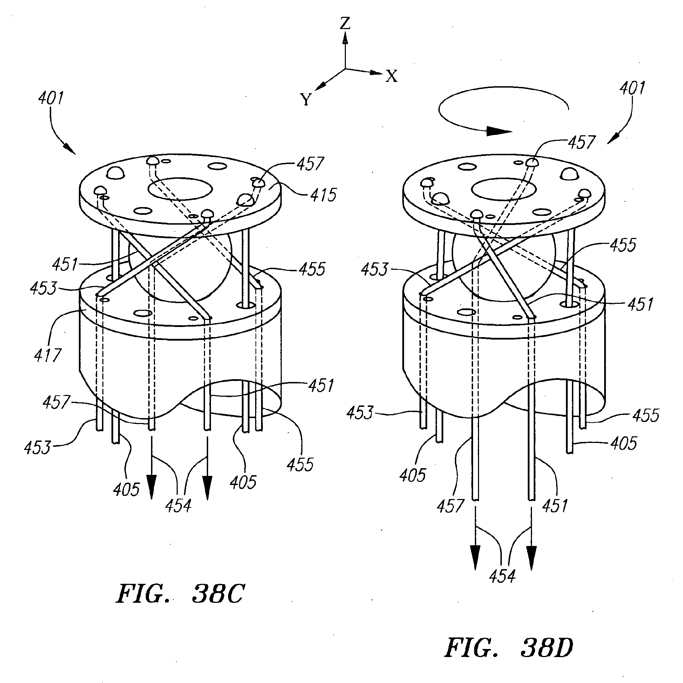

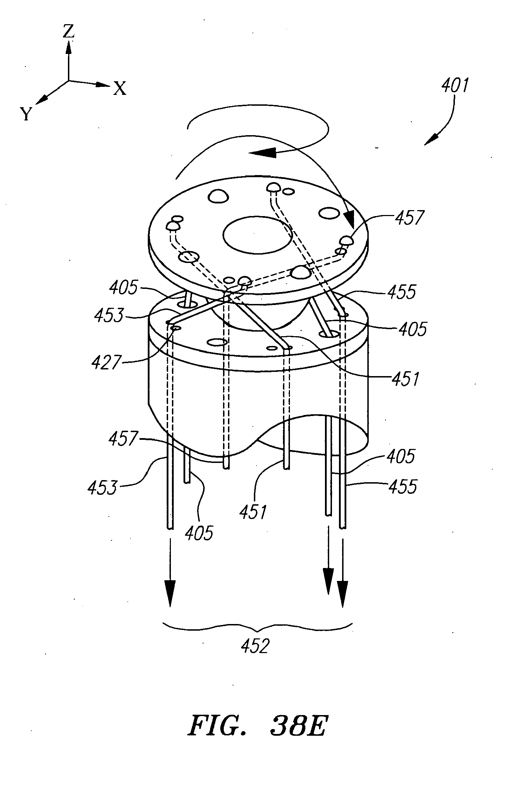

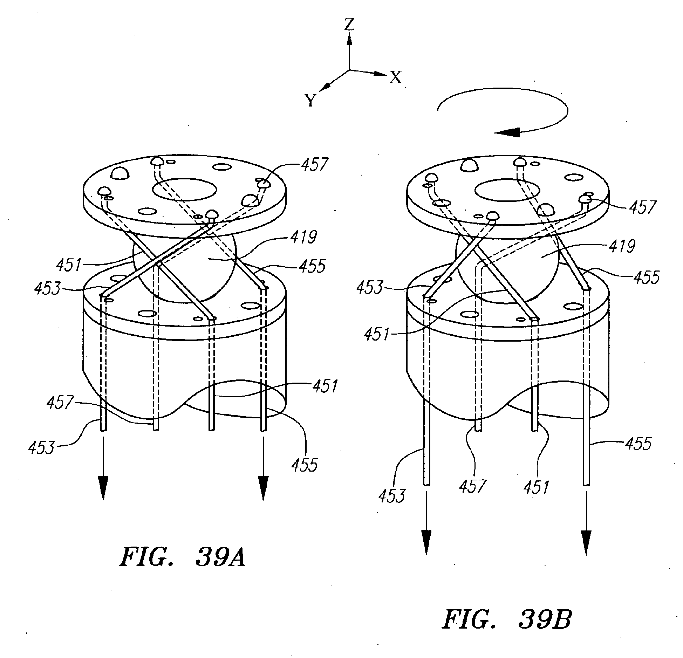

Figs. 38A-E illustrate a configuration of an orientation platform or interface constructed with a ball and socket assembly that includes non-crossing control elements and control elements in the form of crossing cables, wherein Figs. 38A-B illustrate a platform including crossing cables and clockwise platform rotation, Figs. 38C-D illustrate counter-clockwise platform rotation with the platform shown in Figs. 39A-B, and Fig. 38E illustrates the platform shown in Figs. 39A-B rotating clockwise with positive pitch;

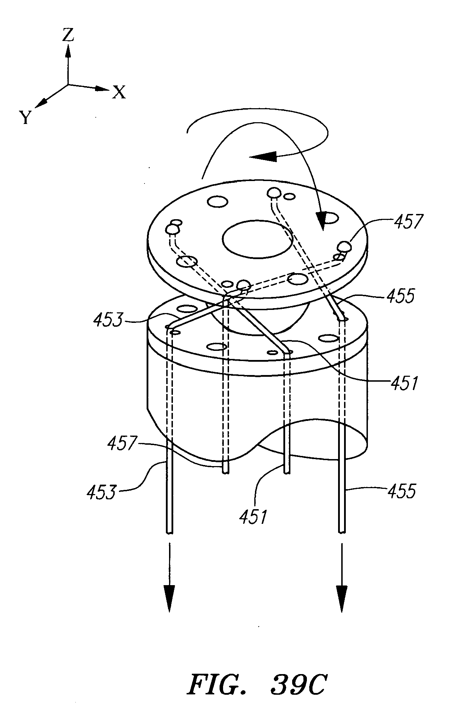

Figs. 39A-C illustrate an configuration of an orientation platform or interface constructed with a ball and socket assembly that includes control elements in the form of crossing cables, wherein Figs. 39A-B illustrate counter-clockwise platform rotation, and Fig. 39C illustrates clock-wise platform rotation with positive pitch;

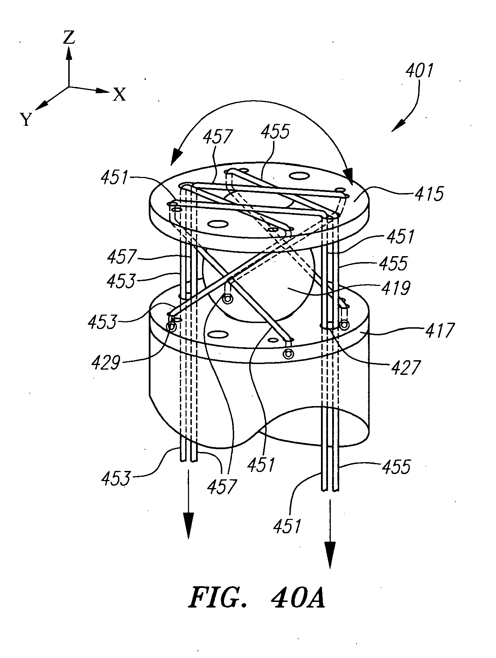

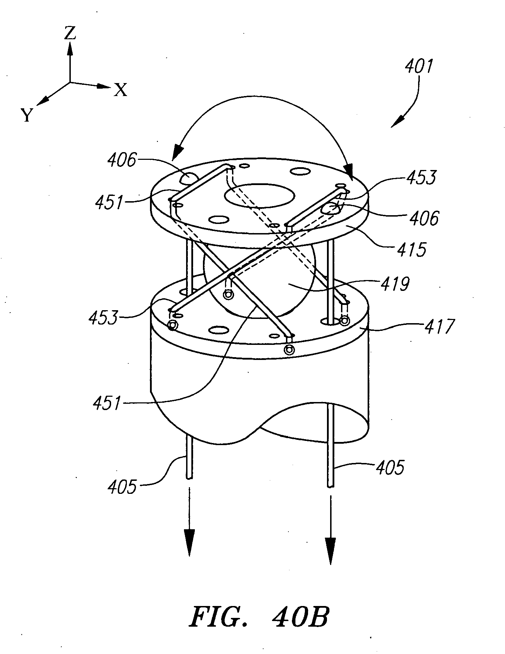

Figs. 40A-B illustrate yet another configuration of an orientation platform or interface constructed with a ball and socket assembly that includes crossing control elements and control elements extending across a distal platform surface, wherein Fig. 40A is a perspective view of a platform including only control cables, and Fig. 40B is a perspective view of a platform including both non-overlapping control elements and overlapping cables;

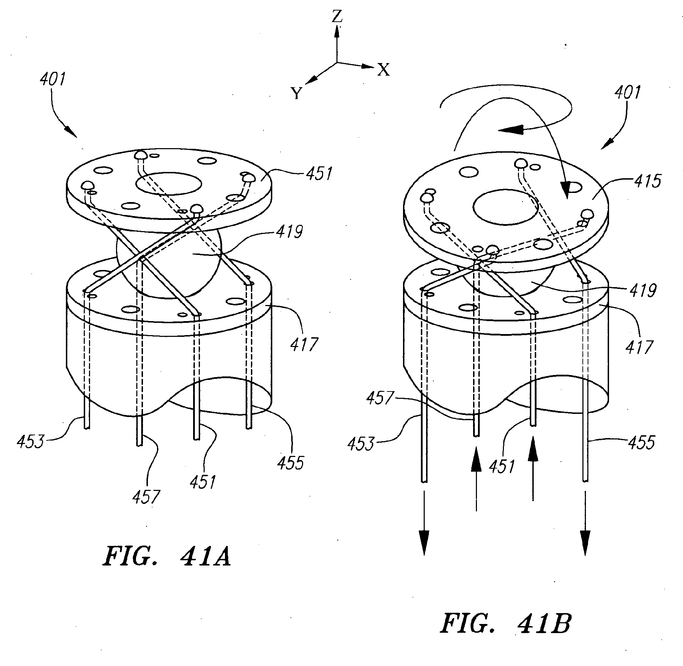

Figs. 41A-B illustrate a further configuration of an orientation platform or interface having a ball and socket configuration and crossing control elements and counter-clockwise rotation of the platform with positive pitch and positive yaw;

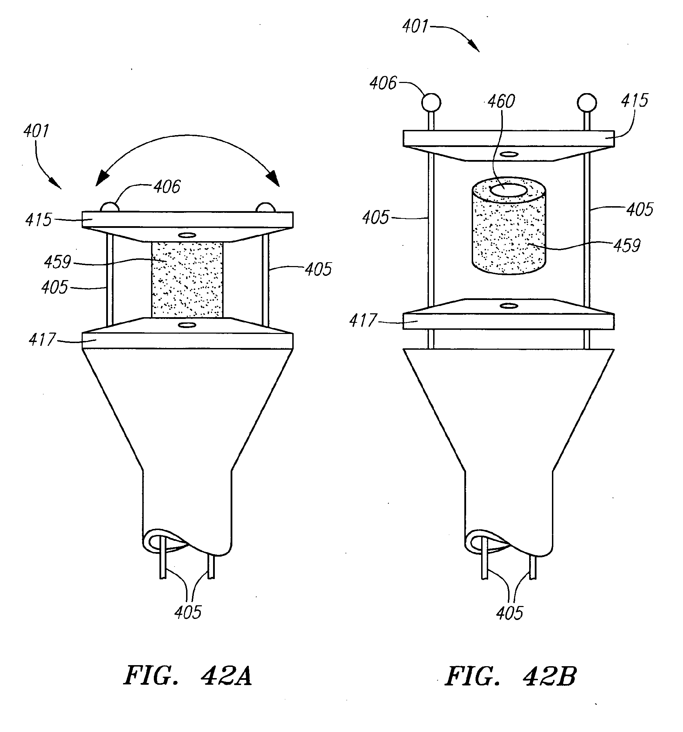

Figs. 42A-B illustrate another alternative configuration of an orientation platform or interface that includes a spacer element in the form of an elastomeric cylinder, wherein Fig. 42A is a side view of a platform according to another configuration, and Fig. 42B is an exploded view of the platform shown in Fig. 42A;

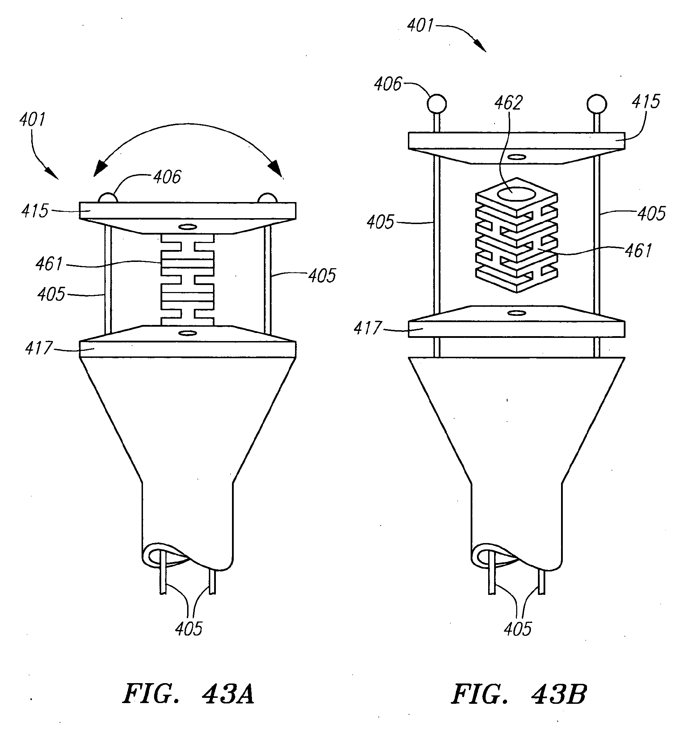

Figs. 43A-B illustrate a further alternative configuration of an orientation platform or assembly that includes a flexure spacer element, wherein Fig. 43A is a side view of a platform according to another configuration, and Fig. 43B is an exploded view of the platform shown in Fig. 43A;

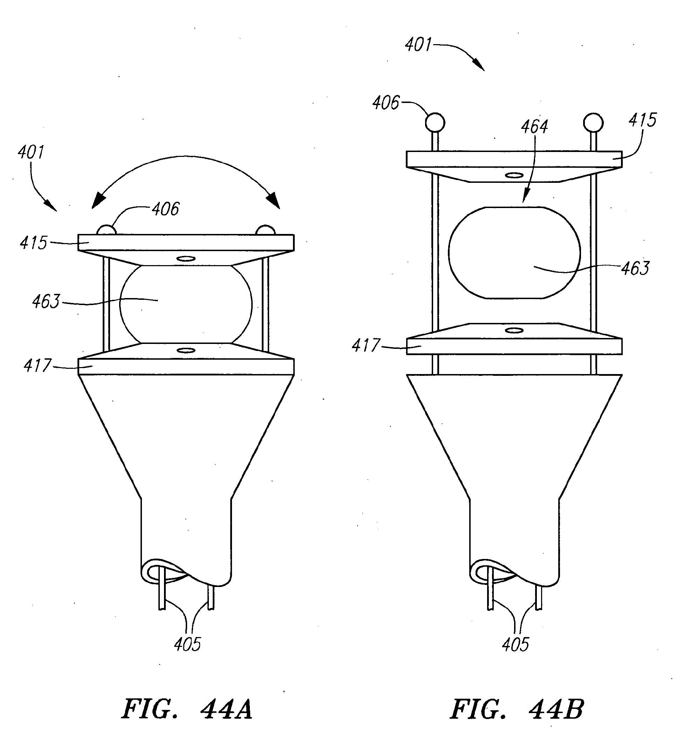

Figs. 44A-B illustrate a configuration of an orientation platform or interface that includes a non-spherical spacer element, wherein Fig. 44A is a side view of a platform according to another configuration, and Fig. 44B is an exploded view of the platform shown in Fig. 44A;

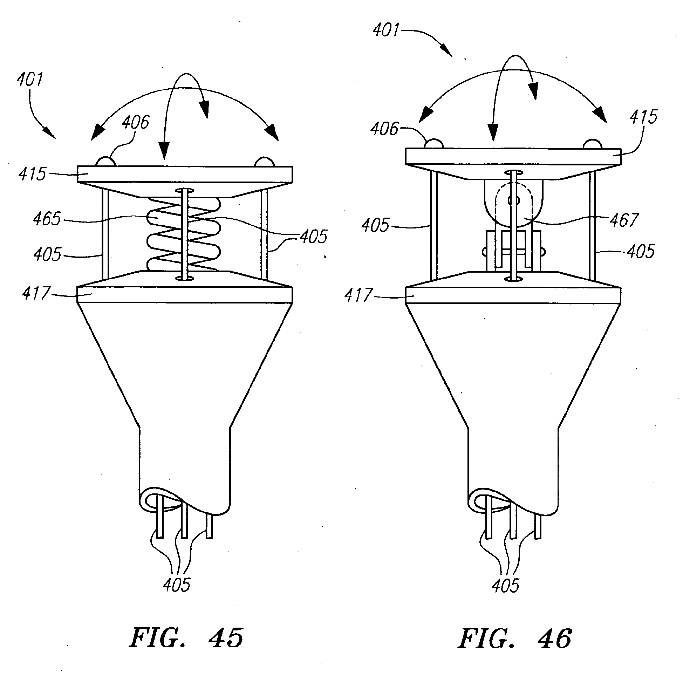

Fig. 45 is a side view of another alternative configuration of an orientation platform or interface that includes a flexible coil spacer element;

Fig. 46 is a side view of a further configuration of an orientation platform or interface employing a universal joint spacer element;

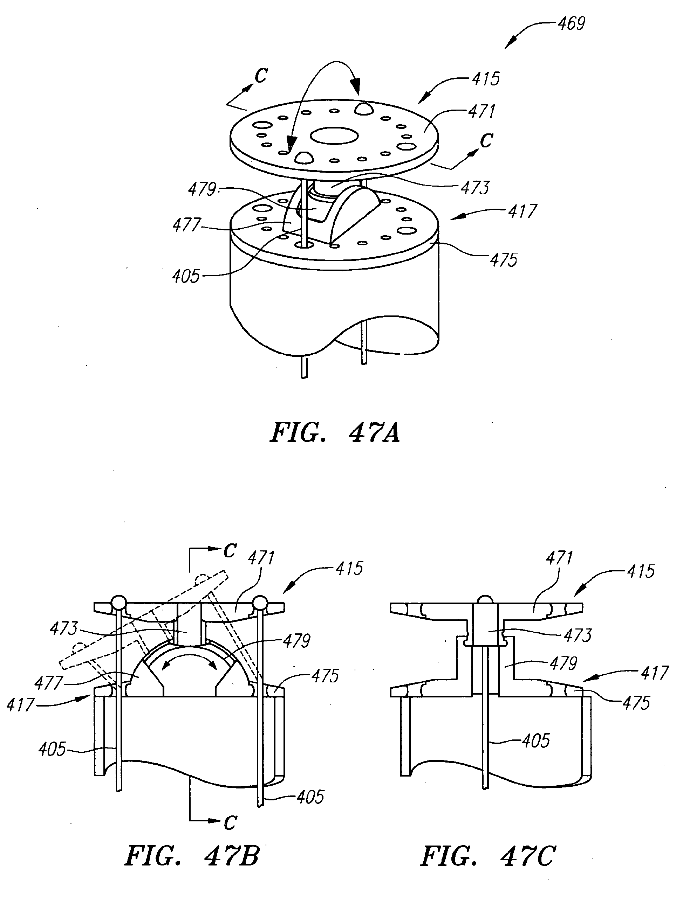

Figs. 47A-C illustrate a further alternative configuration of an orientation platform or interface including a spacer element in the form of a pin and groove arrangement, wherein Fig. 47A is s perspective view of a platform including a pin and groove arrangement, Fig. 47B is a cross-sectional side view of the platform shown in Fig. 47A along line C-C, and Fig. 47C a cross-sectional front view of the platform shown in Fig. 47B parallel to line C-C;

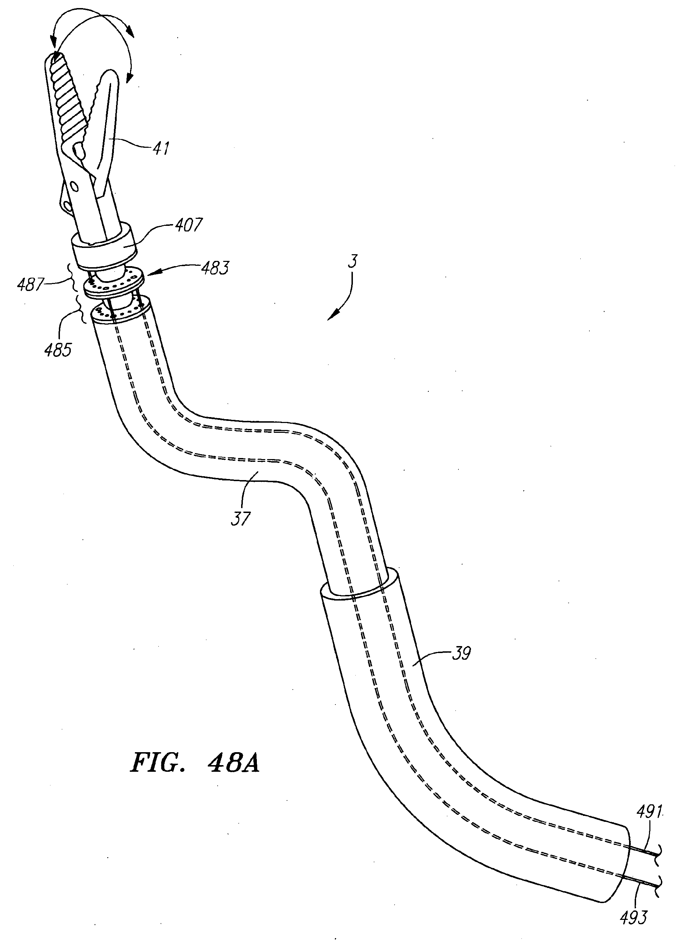

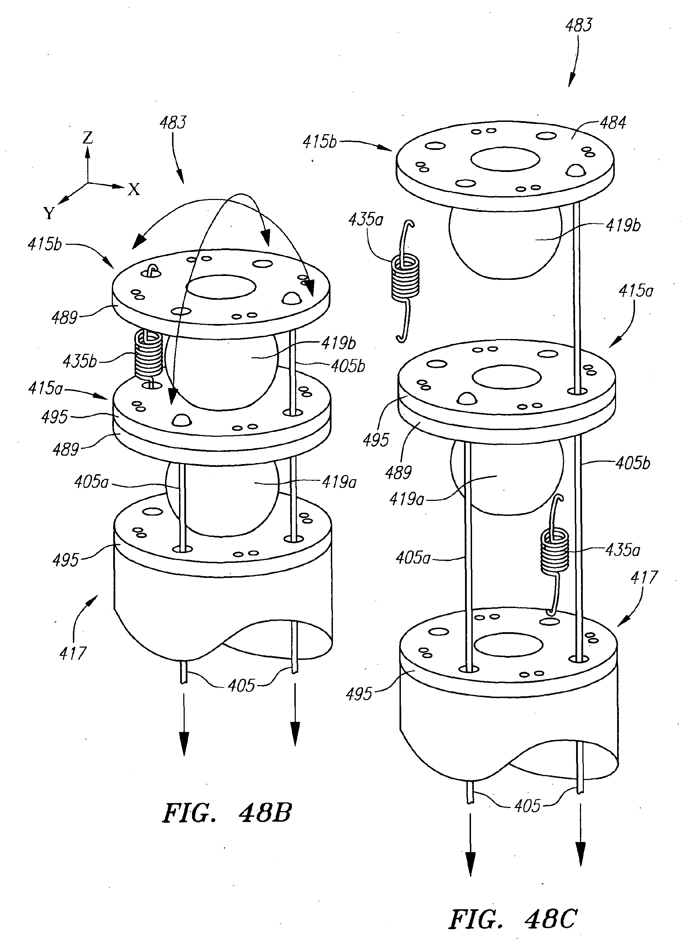

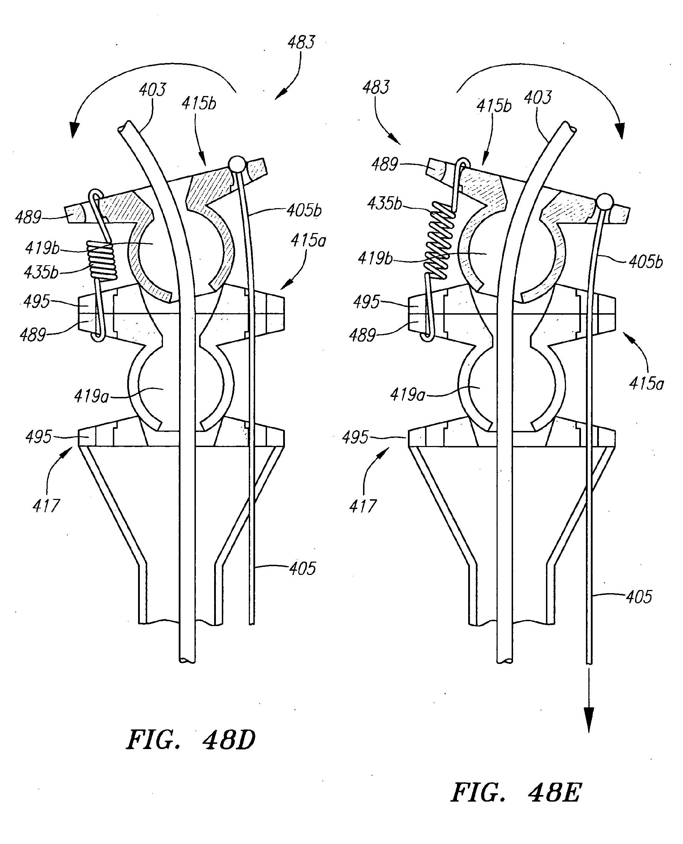

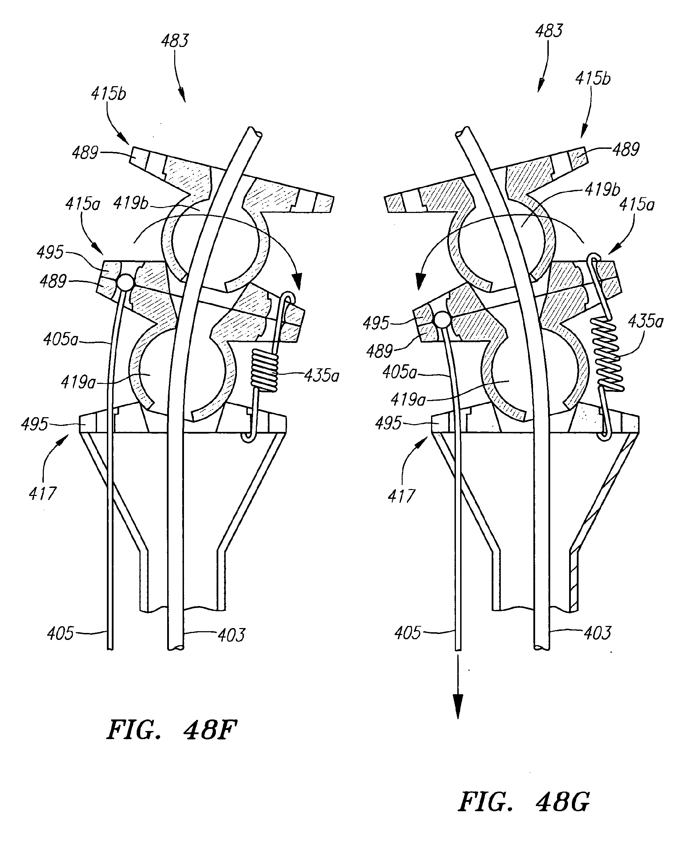

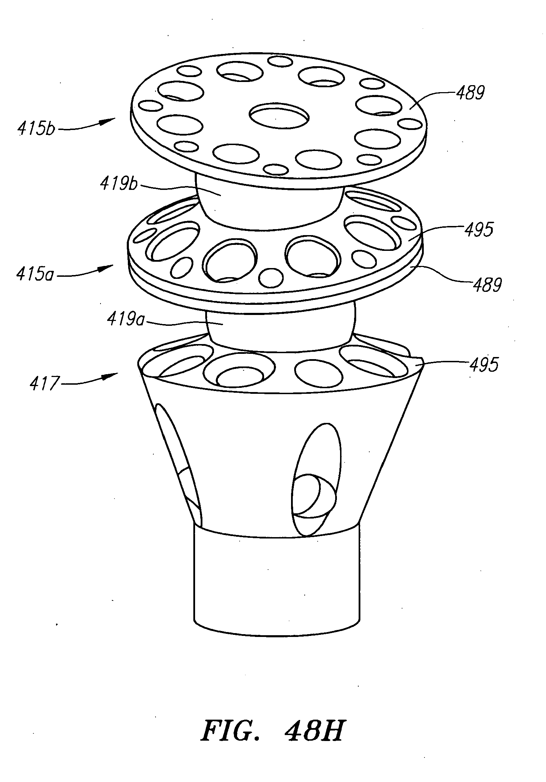

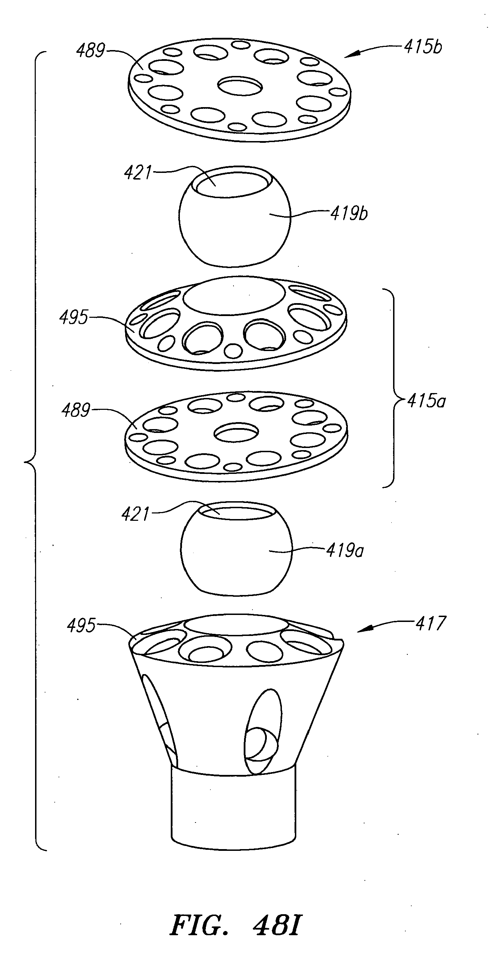

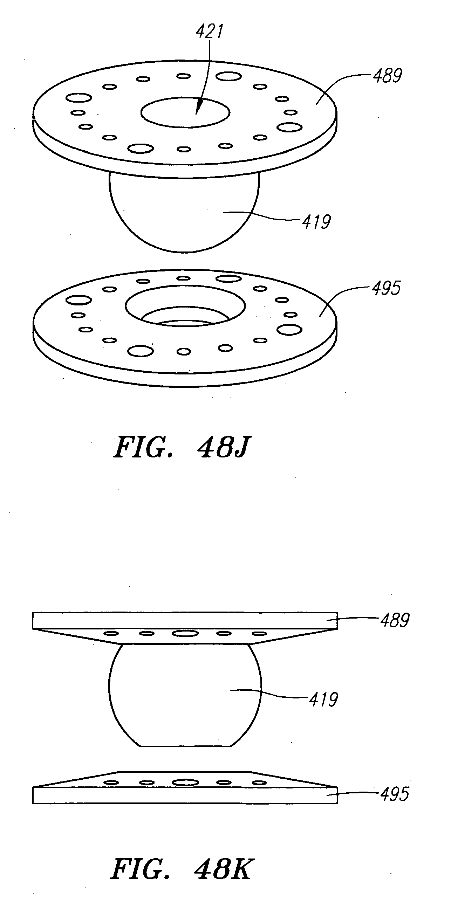

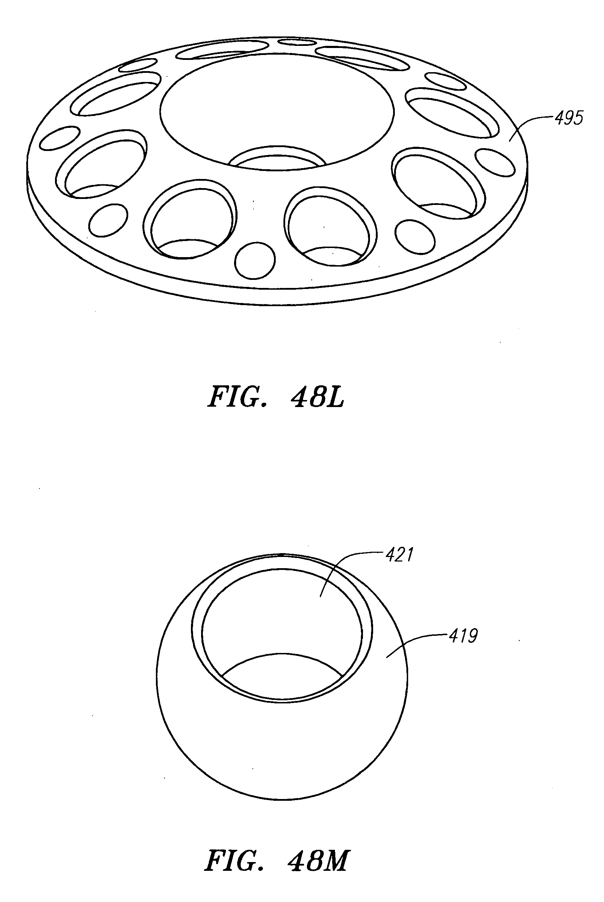

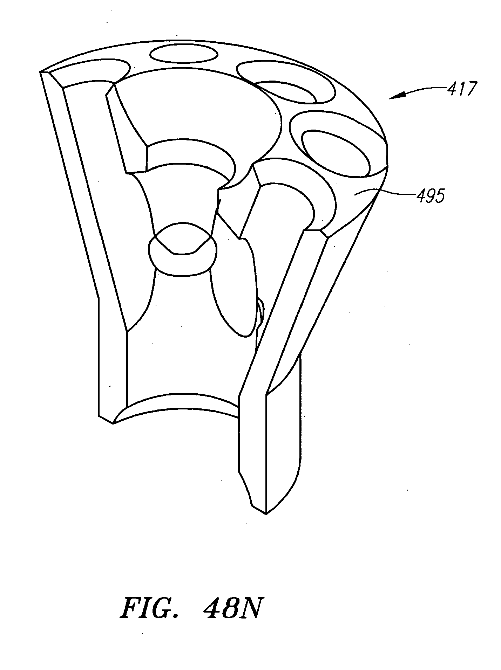

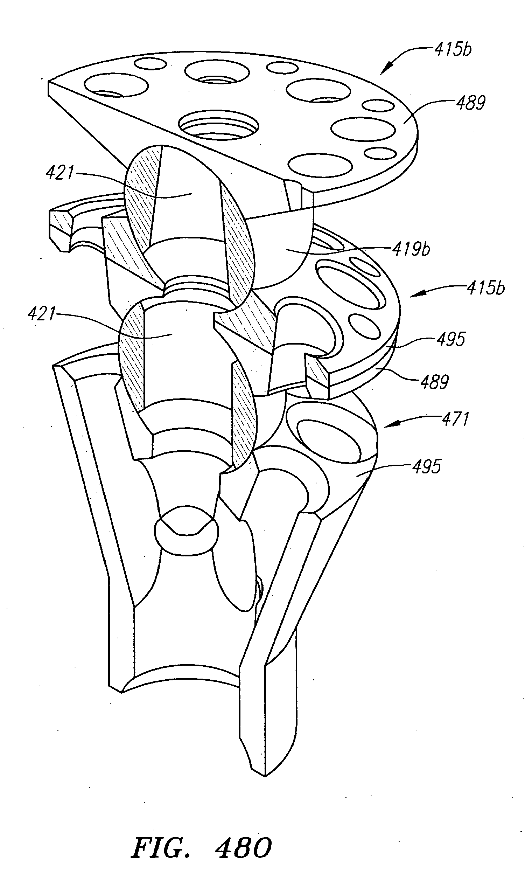

Figs. 48A-O illustrate a configuration of a multi-level platform or interface including multiple ball and socket assemblies and components thereof, wherein Fig. 48A is a perspective view of a flexible catheter assembly including a multi-stage or multi-level platform constructed according to another configuration, Fig. 48B further illustrates a distal portion of the multi-level platform shown in Fig. 48A, Fig. 48C is an exploded view of the multi-level platform shown in Figs. 48A-B Figs. 48D-E are cross-sectional views of the multi-level platform shown in Figs. 48A-C and pitch motion of the platform, Figs. F-G are cross-sectional views showing yaw motion of the platform, Fig. 48H illustrates platform components and different types of possible motion of first and second platform members; Fig. 48I is an exploded view of a platform constructed according to one embodiment; Figs. 48J-K further illustrate spacer element of a platform movably retained between plates; Fig. 48L illustrates a base member constructed according to one configuration, Fig. 48M illustrates a spacer element constructed according to one configuration, Fig. 48N is a cross-sectional view of a base member, Fig. 48O is a cross-sectional view of assembled platform components including a base member, platform members, and spacer elements;

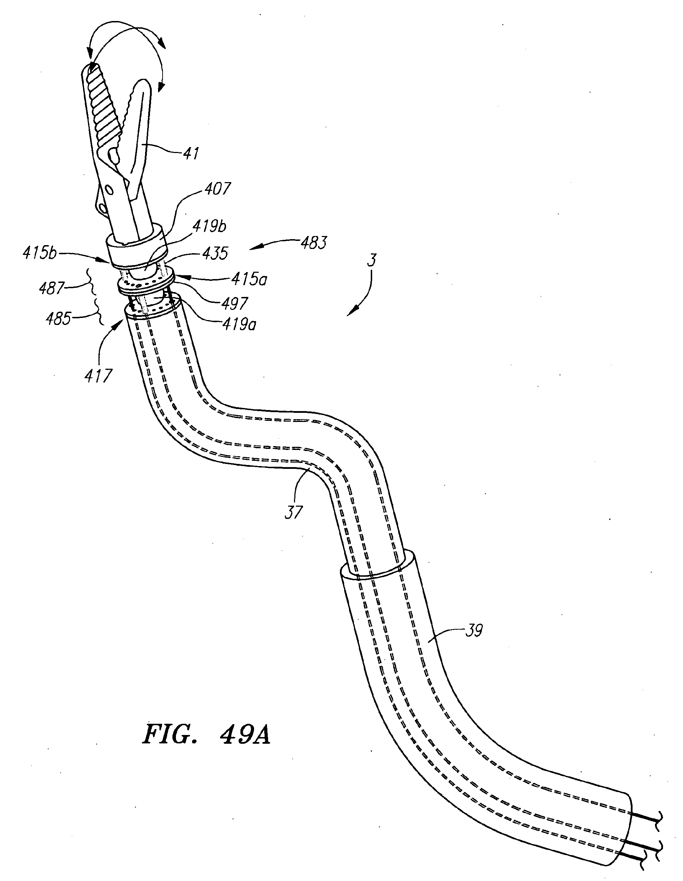

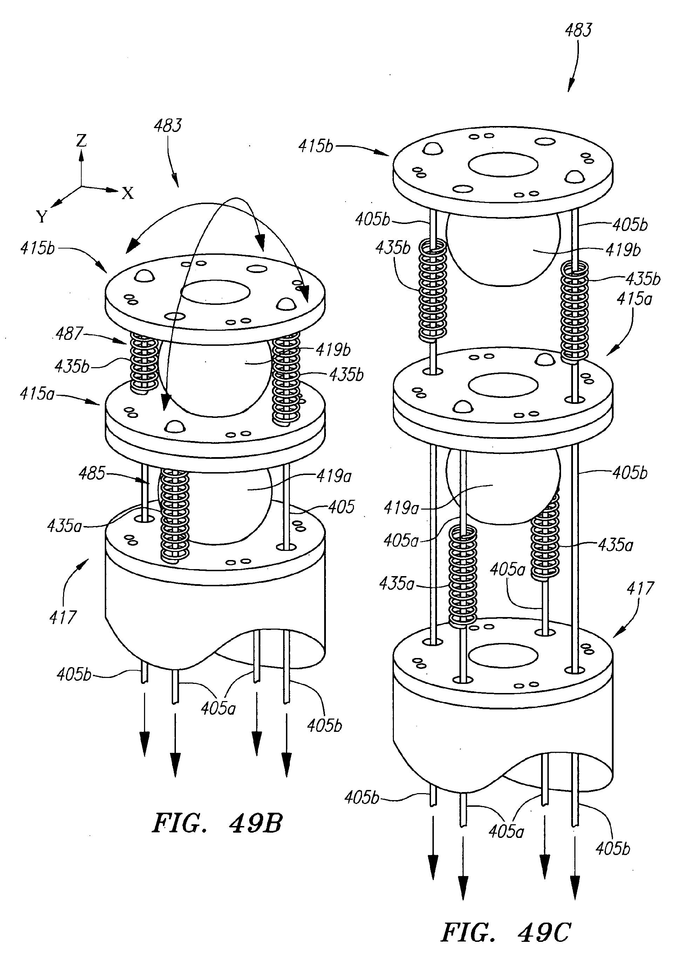

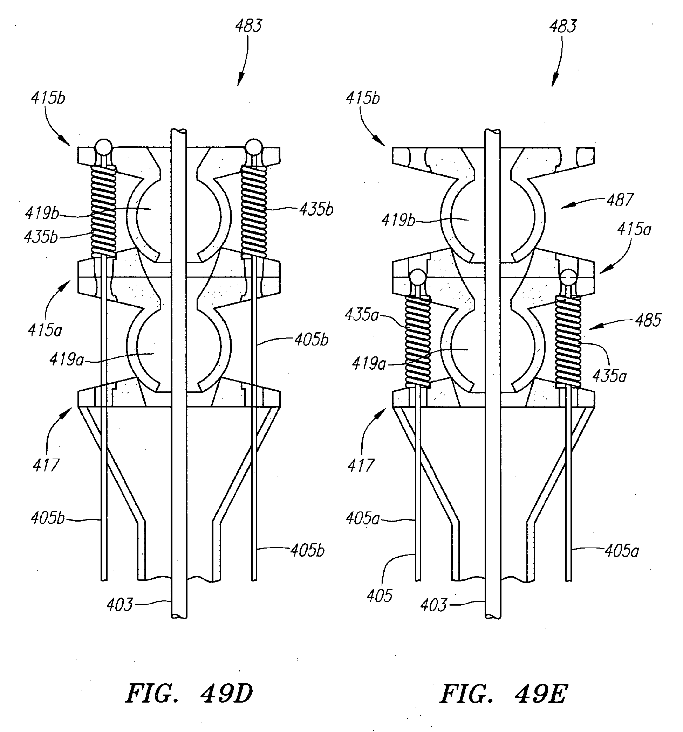

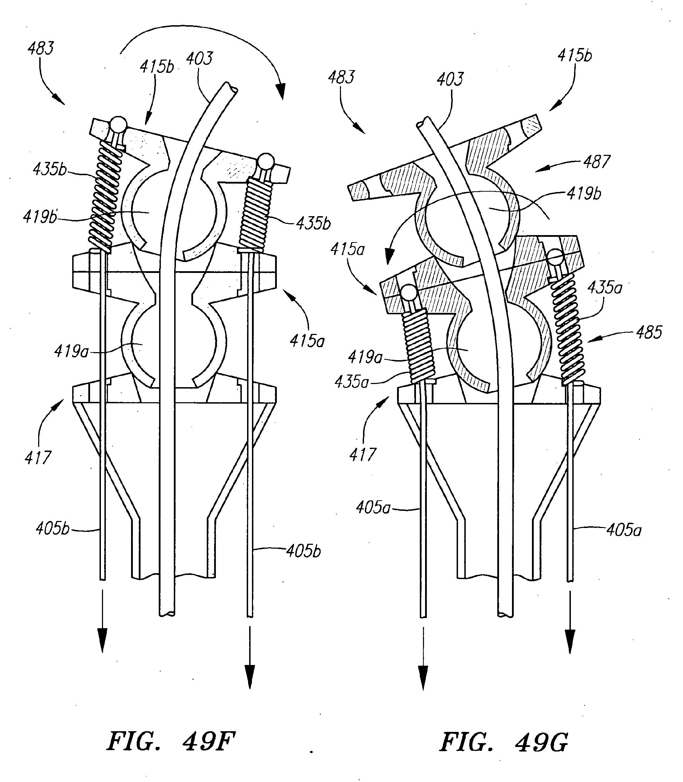

Figs. 49A-G illustrate another configuration of a multi-level platform or interface including multiple ball and socket assemblies, wherein 49A is s perspective view of a flexible catheter assembly including a multi-stage or multi-level platform constructed according to another configuration, Fig. 49B is a perspective view showing the platform in further detail, Fig. 49C is an exploded view of the platform shown in Fig. 49B, Fig. 49D is a front cross-sectional view of the platform shown in Fig. 49B, Fig. 49E is a side cross-sectional view of the platform shown in Fig. 49B, Fig. 49F is a cross-sectional view of the platform shown in Fig. 49D with pitch motion, and Fig. 49G is a cross-sectional view of the platform shown in Fig. 49E with yaw motion;

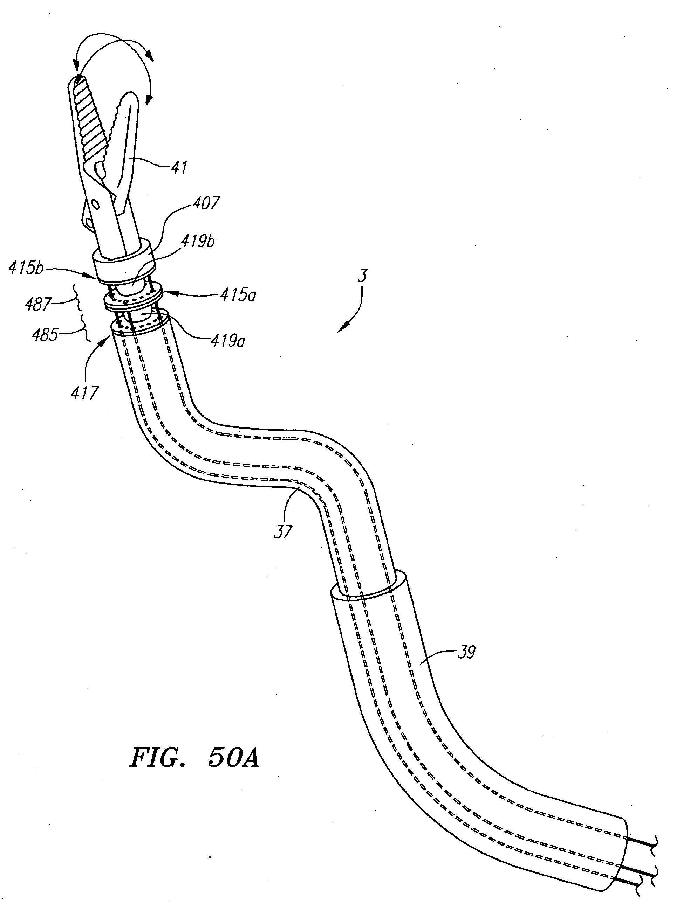

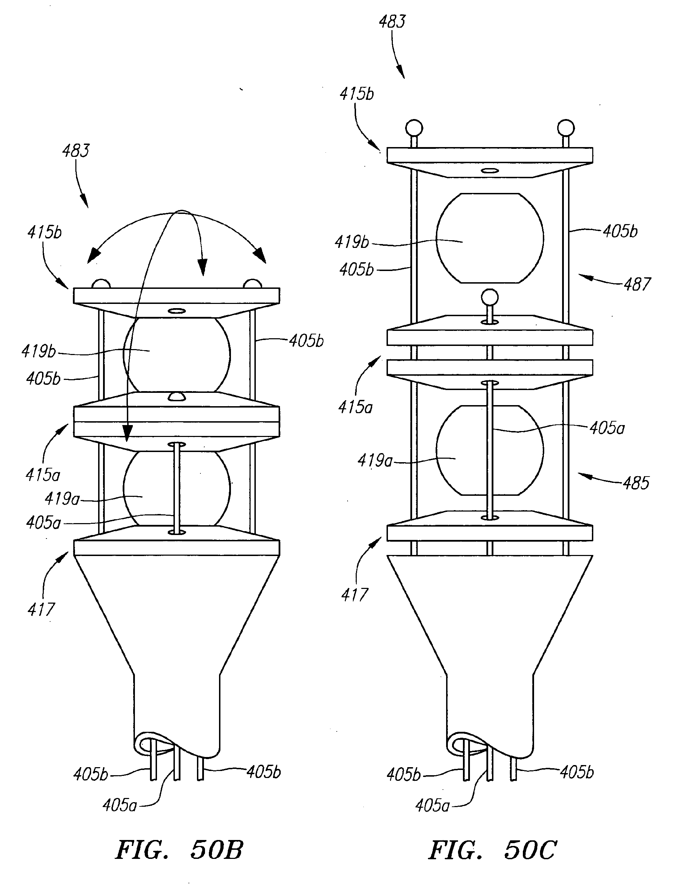

Figs. 50A-C illustrate a further alternative configuration of a multi-level platform or interface including spacer elements in the form of semi-spherical balls, wherein Fig. 50A is a perspective view of a flexible catheter assembly including a multi-stage or multi-level platform constructed according to another embodiment, Fig. 50B is a side view of the platform, Fig. 50C is an exploded view showing the platform components in further detail;

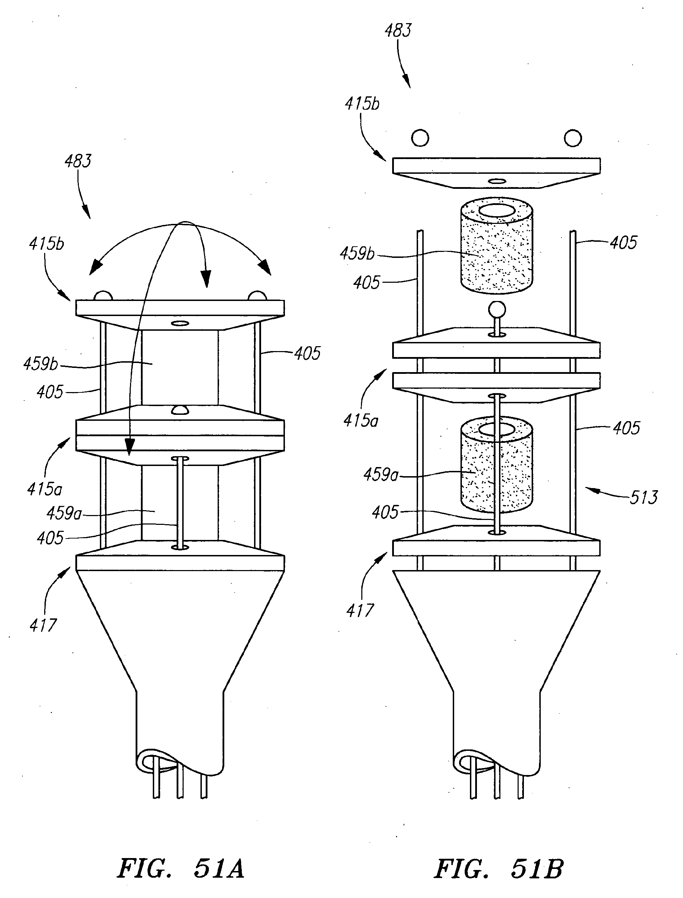

Figs. 51A-B illustrate another alternative configuration of a multi-level platform or interface including spacer elements in the form of elastomeric cylinders, wherein Fig. 51A is a side view of the platform, and Fig. 51B is an exploded view of the platform;

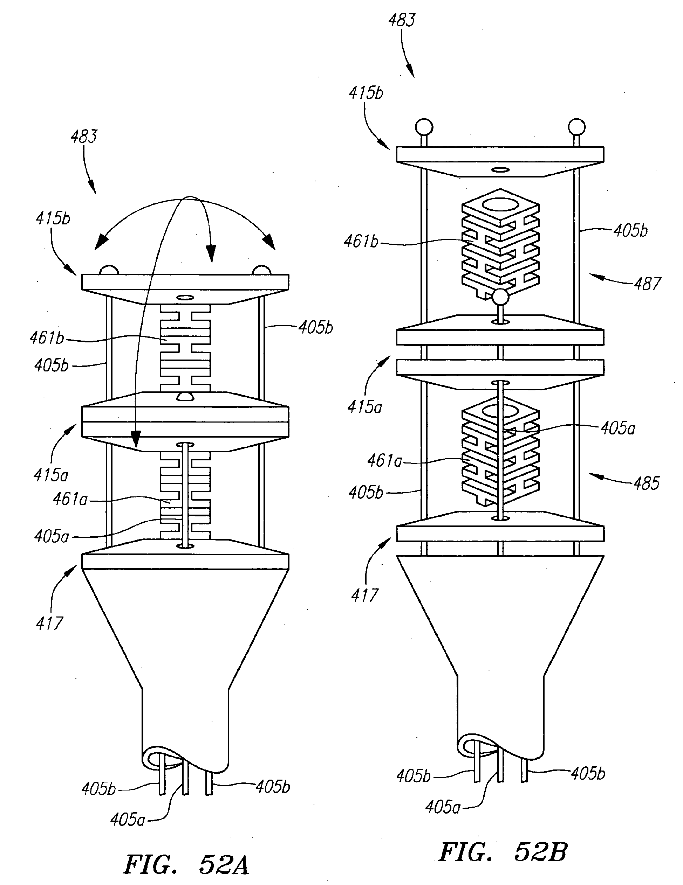

Figs. 52A-B illustrate one configuration of a multi-level platform or interface of a flexible catheter having multiple orientation platforms with spacer elements in the form of flexures, wherein Fig. 52A is a side view of the platform, and Fig. 52B is an exploded view of the platform;

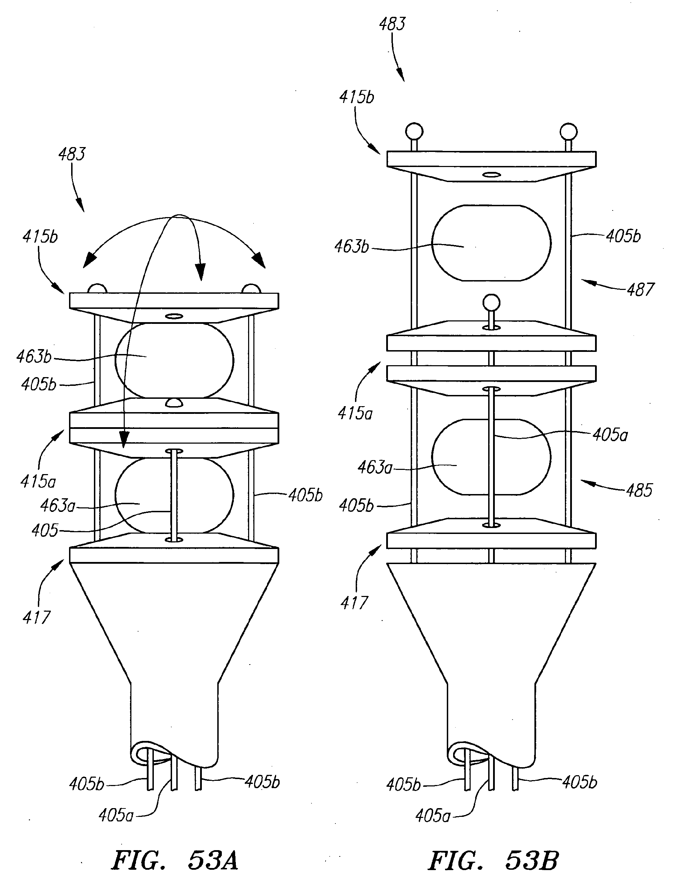

Figs. 53A-B illustrate another configuration of a multi-level platform or interface of a flexible catheter having spacer elements in the form of non-spherical balls, wherein Fig. 53A is a side view of the platform, and Fig. 53B is an exploded view of the platform;

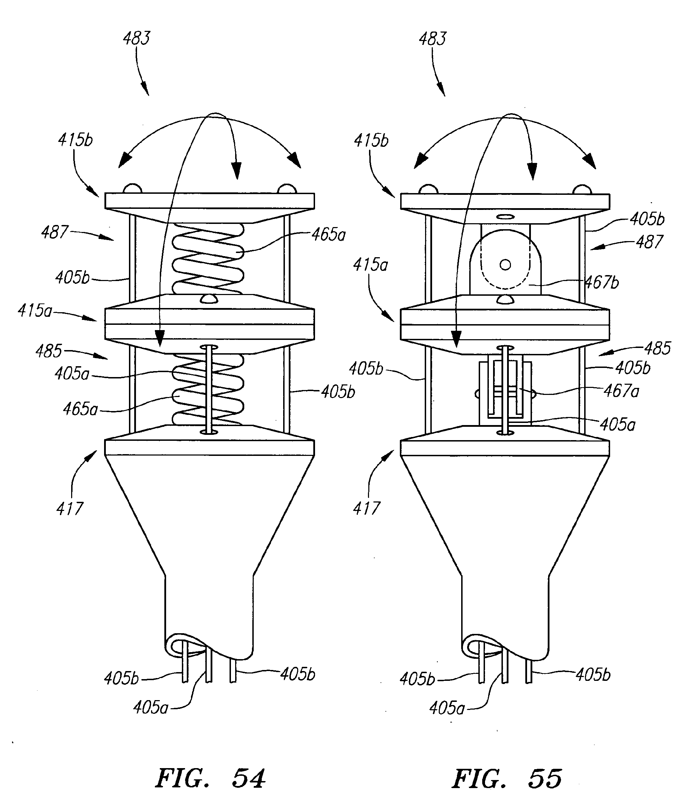

Fig. 54 is a side view of another configuration of a multi-level platform or interface of a flexible catheter having spacer elements in the form of flexible coils;

Fig. 55 is a side view of another configuration of a multi-level platform or interface of a flexible catheter having spacer elements in the form of universal joints;

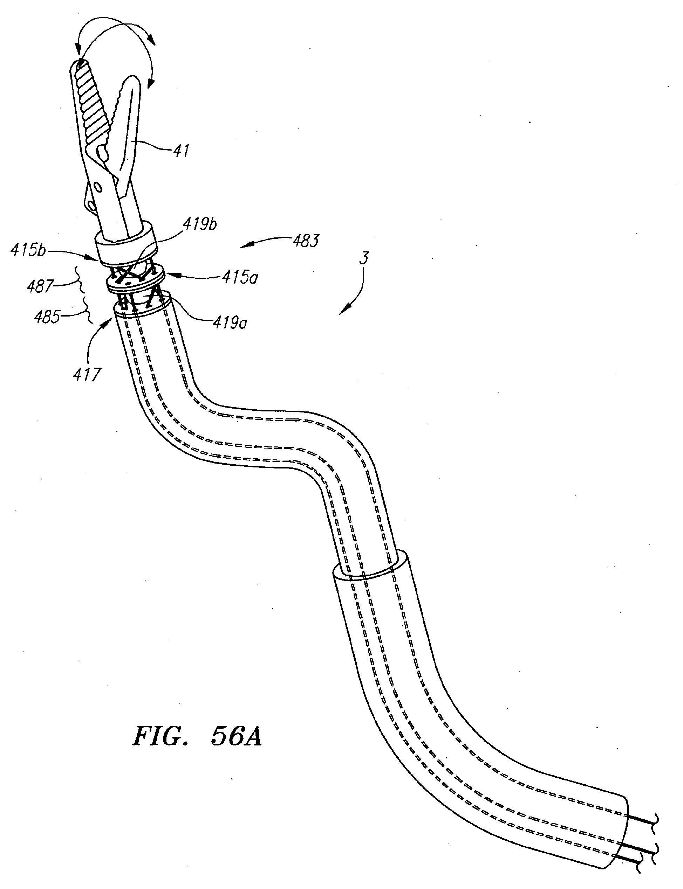

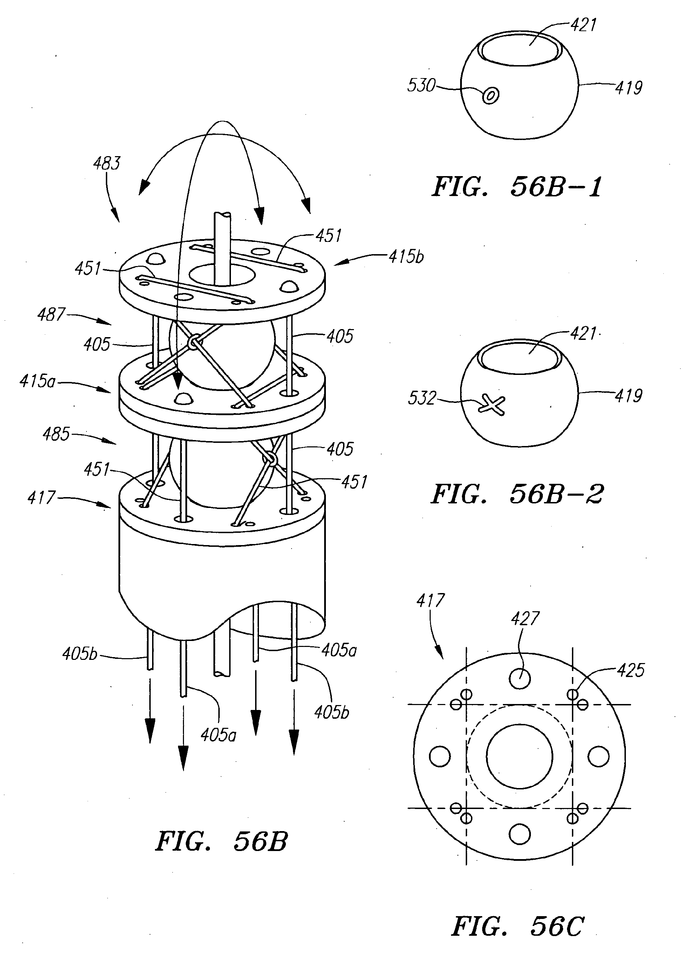

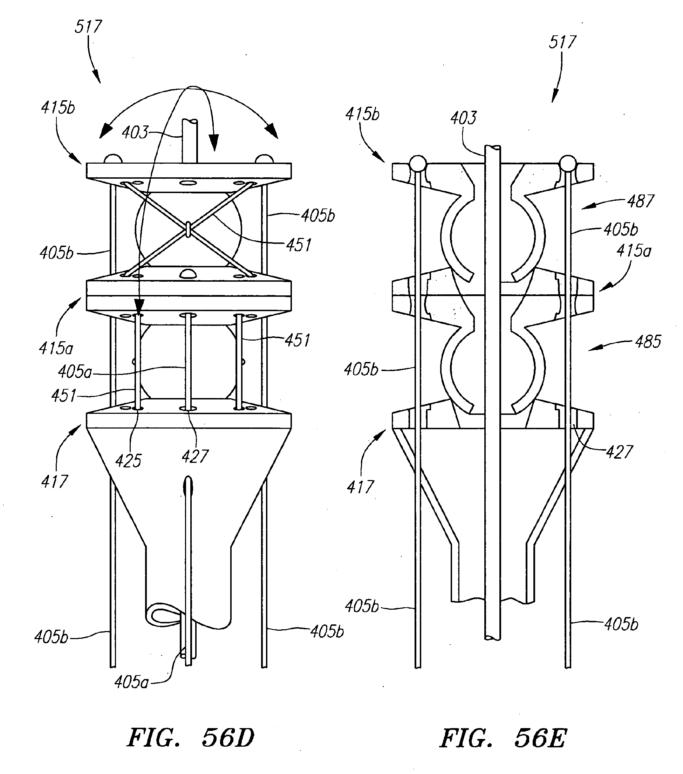

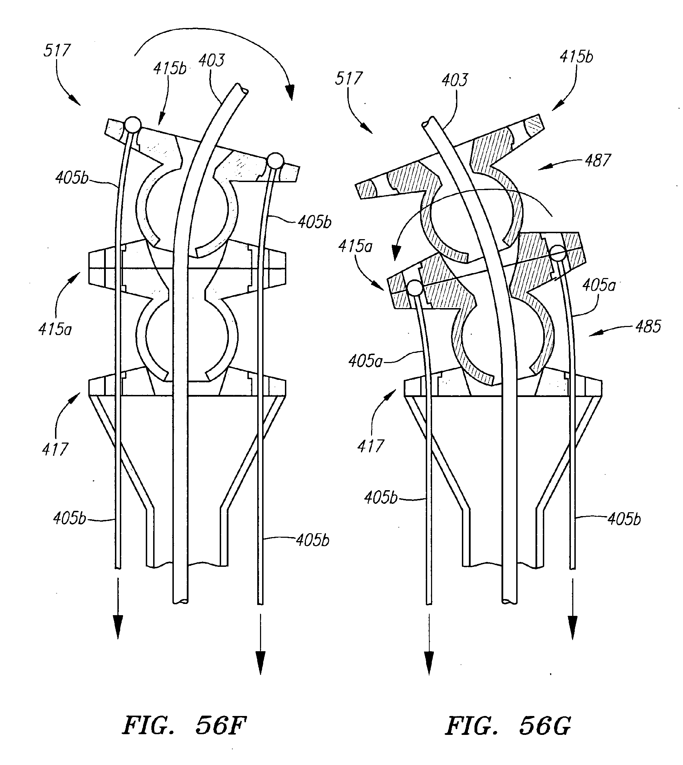

Figs. 56A-G illustrate a multi-level platform or interface constructed according to another configuration including crossing control elements and multiple ball and socket assemblies, wherein Fig. 56A is a perspective view of a flexible catheter assembly including a multi-stage or multi-level platform constructed according to another embodiment, Fig. 56B is a perspective view of the platform showing crossing cable elements, Fig. 56B-1 illustrates a spacer element having an eyelet for use in facilitating crossing or overlapping of control cables, Fig. 56B-2 illustrates a spacer element having a tie down element for use in facilitating crossing or overlapping of control cables, Fig. 56C is a top view of a platform base member, Fig. 56D is front view of the platform shown in Fig. 56B, Fig. 56E is a cross-sectional view of the platform shown in Fig. 56D, Fig. 56F is a cross-sectional view of the platform shown in Fig. 56E with pitch motion, Fig. 56G is a cross-sectional view of the platform shown in Fig. 56D with yaw motion;

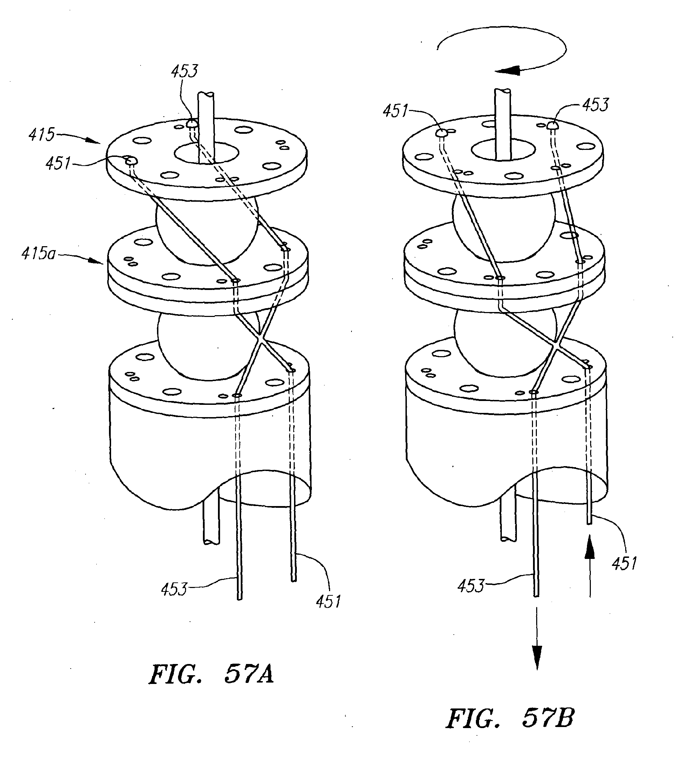

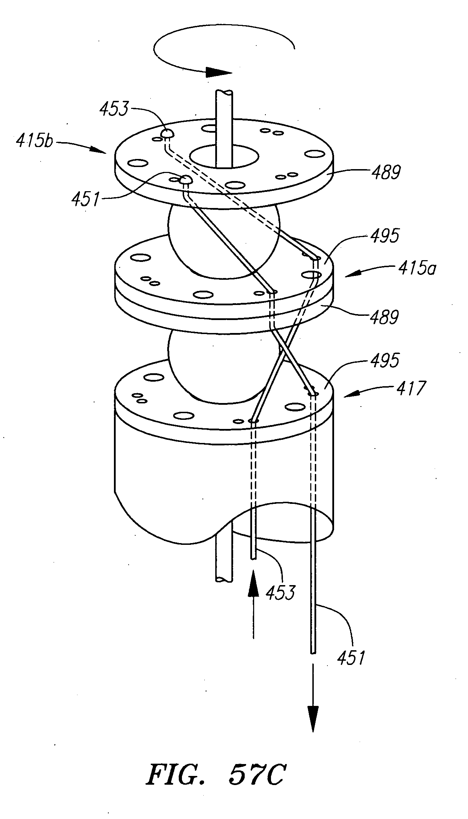

Figs. 57A-C illustrate another configuration of a multi-level platform or interface having crossing control elements and components thereof, wherein Fig. 57A is a perspective view of a multi-level platform constructed according to another embodiment, Fig. 57B illustrates how the platform shown in Fig. 57A can be rotated clockwise, and Fig. 57C illustrates how the platform shown in Fig. 57A can be rotated counter-clockwise;

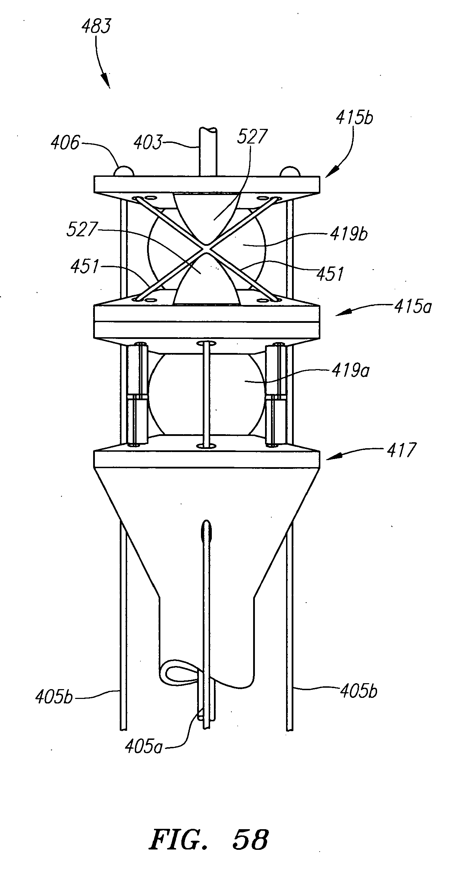

Fig. 58 is a side view of multi-level platform or interface having crossing control elements and cams to facilitate crossing arrangements according to another configuration, and

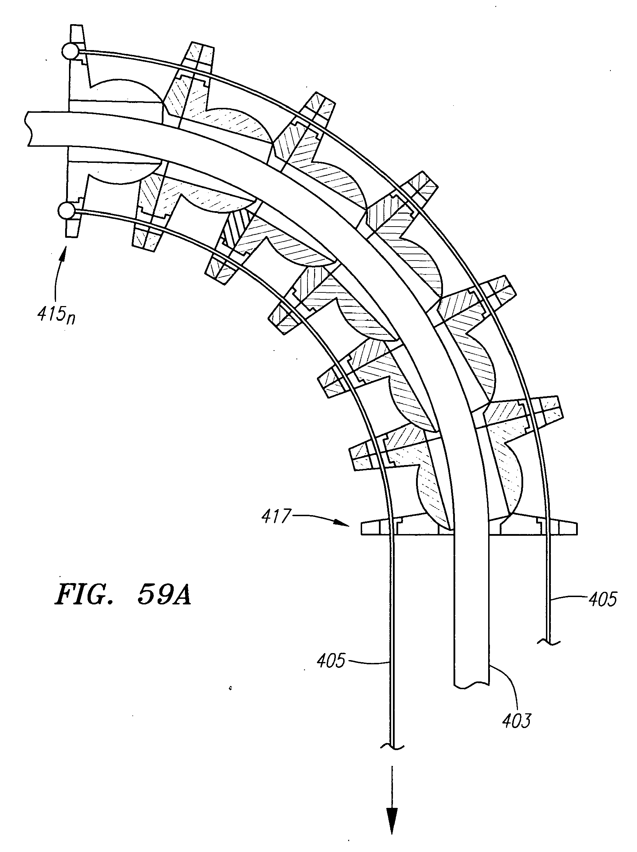

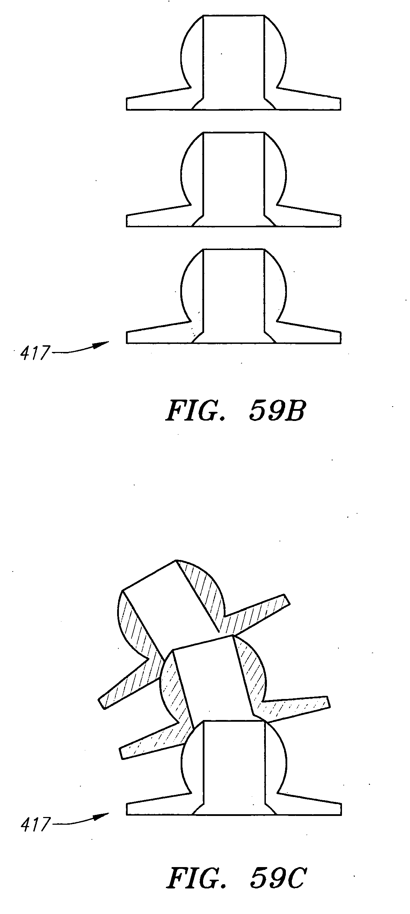

Figs. 59A-C illustrate how configurations of multi-level platform or interfaces can have different numbers of levels and bending of such structures, wherein Fig. 59A is a cross-sectional view of a multi-level platform constructed according to another configuration and including more than two levels or stages, and Figs. 59B-C illustrate a tri-level orientation platform or interface constructed according to another configuration.

## DETAILED DESCRIPTION OF ILLUSTRATED EMBODIMENTS

In the following "embodiment" does not relate to the invention unless specifically specified. Embodiments are directed to controlling a flexible distal portion or tip of a catheter or catheter assembly and mechanisms for controlling and orienting distal tip shapes and movement. Although embodiments are described in the context of robotically controlled catheters, embodiments may also be utilized with or applied to other types of surgical devices including, but not limited to, endoscopes and laparoscopes. Embodiments may thus be described with reference to various instruments including an "instrument member", a "flexible member", a "flexible instrument", a "catheter", a "catheter member", a "flexible catheter instrument", a "catheter assembly" and the like, which may, in some cases, be identified by different reference numbers in different embodiments / figures, but which are generally the same or substantially similar devices that include an elongated tubular member having a controllable distal working end that may carry one or more working instruments or tools, e.g., end effectors, to be surgically introduced into a cavity of a patient and be actuated by a surgeon at its proximal end external to the cavity. Thus, it should be understood that certain components and structures described with reference to certain embodiments and figures may also be applicable to other embodiments described with reference to other figures.

Fig. 1 illustrates one embodiment of a robotic catheter system (1) that utilizes or includes a flexible catheter assembly (3). The system (1) includes an operator workstation (5) located remotely from an operating table (7), an electronics rack (9), a bedside electronics box (11), a setup joint mounting brace (13), and an instrument driver (15). A surgeon (17) seated at the operator workstation (5) monitors a surgical procedure, patient vitals, and controls one or more flexible catheter assemblies (3). Although the various components of the system (1) are illustrated in close proximity to each other, in other embodiments, components may be separated from each other, e.g., in separate rooms. For example, the instrument driver (15), the operating table (7), and the bedside electronics box (11) may be located in the surgical area, whereas the operator workstation (5) and the electronics rack (9) may be located outside of the surgical area behind a shielded partition.

In one embodiment, system (1) components may communicate with other system (1) components via a network, thus allowing for remote surgery wherein the surgeon (17) may not even be in the same building or hospital site. For this purpose, a communication link may be provided to transfer signals between the operator control station (5) and the instrument driver (15). As shown in Fig. 1, the components are coupled together via a plurality of cables (19) for data communication. However, in another embodiment, one or more of the components may be equipped with a wireless transceiver (not shown in Fig. 1), thus allowing for wireless communication and reducing or eliminating cable connections.

Referring to Fig. 2A, the operator workstation (5) according to one embodiment includes three display screens (21), a touchscreen user interface (23), a control button console or pendant (25), and a master input device (MID) (27). Also depicted in Fig. 2A is a disable switch (29) configured to disable activity of the instrument temporarily. By manipulating the pendant (25) and MID (27), an operator (17) can cause an instrument driver (15) to remotely control flexible catheters (3) mounted to the instrument driver (15). The console (31) of this implementation is configurable for individual user preferences. For example, in the illustrated embodiment, the pendant (25) and the touchscreen (23) are shown as being positioned on the left side of the console (31), but these components may also be positioned on the right side of the console (31). An optional keyboard may be connected to the console (31) for inputting user data. In one embodiment, the workstation (5) is mounted on a set of casters or wheels (33) to allow easy movement of the workstation (5) from one location to another, e.g., within an operating room or catheter lab, such that the location of the operator control station may be located away from radiation sources, thereby advantageously decreasing the operator's exposure to radiation.

Fig. 2B illustrates one embodiment of an operator workstation (5) with inputs to control a flexible catheter assembly (3) can entered using a MID (27) and a pair of data gloves (35). The MID (27) and wireless data gloves (35) serve as user interfaces through which the operator (17) may control the operation of the instrument driver (15) and any instruments attached thereto. In this embodiment, a flexible catheter assembly (3) may be controlled by the operator (17) by manipulating either the master input device (27) or the pair of haptic gloves (35). Alternatively, each of the master input device (27) and gloves (35) may be configured to control a particular aspect of the flexible catheter assembly (3). For example, one of the input devices (27, 35) may be configured for control of the proximal portion or sheath (39) of the catheter assembly (3) while the other input device (27, 35) is configured for control of the distal portion or tip of a flexible catheter (37). Similarly, one input device (27, 35) may be configured for moving the overall flexible catheter assembly (3) whereas the other input device (27, 35) is configured for control of a tool mounted on an orientation platform at the distal tip of the catheter (37). In another implementation, one person may be seated at the workstation (5) to use the MID (27) while a second person may be using gloves (35) at another location.

As shown in Fig. 2B, the MID (27) may be located about the center of the operator workstation (5) and under the center screen (21). Data gloves (35) may be coupled to the workstation (5) via a cable. Depending on the particular implementation, the data gloves (35) may be wired to the workstation (5), or a wireless system may be utilized. In one embodiment, data gloves (35) are wireless devices and communicate wirelessly with the operator control station (5), thus allowing the operator (17) to work untethered from the station (5). It should be understood that while an operator (17) may robotically control one or more flexible catheter devices via an inputs device, in one or more embodiments, a computer of the robotic catheter system (1) may be activated to automatically position a catheter instrument and/or its distal extremity inside a patient or to automatically navigate the patient anatomy to a designated surgical site or region of interest.

The MID (27) in the illustrated embodiment is a multi-degree-of-freedom device having multiple joints and associated encoders. An operator interface (45) is configured for comfortable interfacing with human fingers. The depicted embodiment of the operator interface (45) is substantially spherical. Further, the MID (27) may have integrated haptics capability for providing tactile feedback to the operator (17). In various embodiments, the instrument driver (15) and associated flexible catheter assembly 3 and working instruments or end effectors 41 (e.g., as shown in Figs. 23A-Z) may be controlled by an operator (17) via the manipulation of the MID (27), a pair of data gloves (35), or a combination of both. For example, the insertion and removal of a flexible working instrument (41) mounted on the instrument driver (15) can be controlled via data gloves (35) and/or the MID (27). Flexible working instruments (41) that may be used with embodiments may include, for example, steerable catheters, endoscopes and other end-effectors (i.e., a working distal part located at the distal tip or working end of a catheter member for effecting an action). An end-effector may be an electrode or a blade. An end effector may include a single element or multiple elements, e.g., a grasper or scissors. In some embodiments, a catheter instrument or assembly (3) may include one or more lumens through which working instruments, such as tools, other catheters, optical fibers, illumination fibers, etc. may be deployed to a working or surgical site.

For ease of explanation, reference is primarily made to catheters (37) and catheter assemblies (3). Further, for ease of explanation, reference is made generally to a working instrument (41), which may be various instruments, tools and end-effectors. In some embodiments, data gloves (35) are configured to also control and manipulate working instruments (17). In other embodiments, the data gloves (35) control instrument (17) steering. Data gloves (35) can also maneuver an imaging fiber located at the tip of an instrument (17) such that the field of view can be changed based on movements of a data glove (35). A data glove (35) may also provide tactile feedback to the user in response to contact at the distal tip of a catheter (37).

Fig. 2E is a block diagram (47) illustrating the system architecture of one embodiment of a robotic catheter system (1). A master computer (49) oversees the operation of the system (1) and is coupled to receive user input from hardware input devices such as a data glove input device (35) and a haptic MID (27). The master computer (49) executes master input device software, data glove software, visualization software, instrument localization software, and software to interface with operator control station buttons and/or switches is depicted. Data glove software (53) processes data from the data glove input device (35), and master input device hardware and software (51) processes data from the haptic MID (27). In response to the processed inputs, the master computer (49) processes instructions to instrument driver computer (55) to activate the appropriate mechanical response from the associated motors and mechanical components to achieve the desired response from the flexible catheter assembly (3).

In one embodiment, the MID (27) software is a proprietary module packaged with an off-the-shelf master input device system, such as the Phantom® from SensAble Technologies, Inc., which is configured to communicate with the Phantom® Haptic Device hardware at a relatively high frequency as prescribed by the manufacturer. Other suitable MIDs (27) are available from suppliers such as Force Dimension of Lausanne, Switzerland. The MID (27) may also have haptics capability to facilitate feedback to the operator, and software modules pertinent to such functionality may be operated on the master computer (49). In one embodiment, data glove (35) software is a device driver or software model, such as a driver for the 5DT Data Glove. In other embodiments, software support for the data glove master input device is provided through application drivers such as Kaydara MOCAP, Discreet 3D Studio Max, Alias Maya, and SoftImage|XSI.

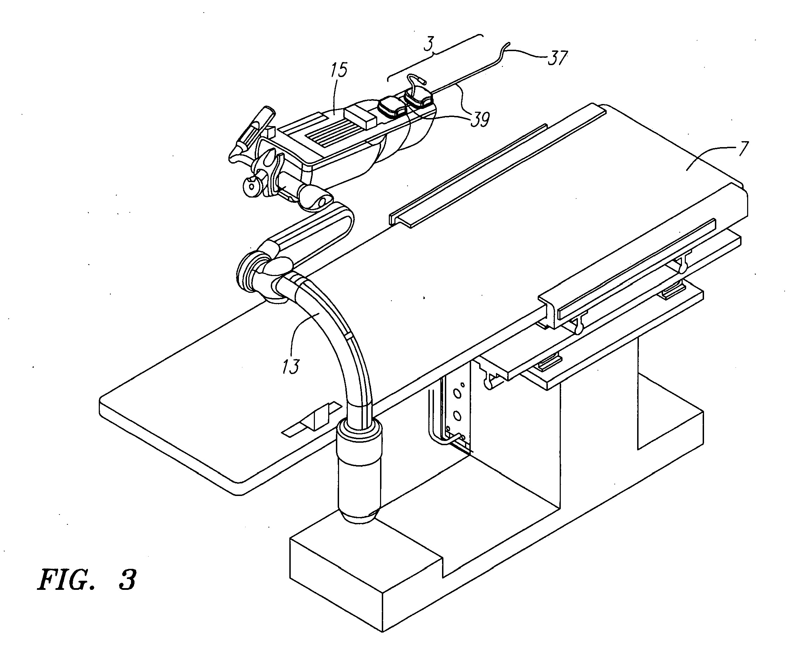

Fig. 3 illustrates one embodiment of a setup joint, instrument mounting brace or support assembly (13) (generally referred to as setup joint 13) that supports an instrument driver (15) above an operating table (7). In the illustrated embodiment, the setup joint (13) is an arcuate-shaped structure configured to position the instrument driver (15) above a patient lying on the table (7) for convenient access to desired locations relative to the patient. The support assembly (13) may also be configured to lock the instrument driver (15) into position. In this example, the setup joint (13) is mounted to the edge of a patient bed (7) such that a catheter assembly (3) mounted on an instrument driver (15) can be positioned for insertion into a patient and to allow for any necessary movement of the instrument driver (15) in order to maneuver the catheter assembly (3) during a surgical procedure. Although a single flexible guide catheter (37) and sheath assembly (39) are shown in Fig. 3 as being mounted on a single instrument driver (15), other embodiments may involve other configurations, e.g., a plurality of instrument drivers (15) on which a plurality of flexible catheter assemblies (3) may be controlled.

Figs. 4A-G illustrate one embodiment of a setup joint (13) in further detail. In the illustrated embodiment, the setup joint (13) is configured for mounting of a single instrument driver (15) to a mounting plate on a support member at a distal portion of the setup joint (13). A distal portion of the setup joint (13) includes a control lever (57) that may be manipulated to maneuver the setup joint (13).

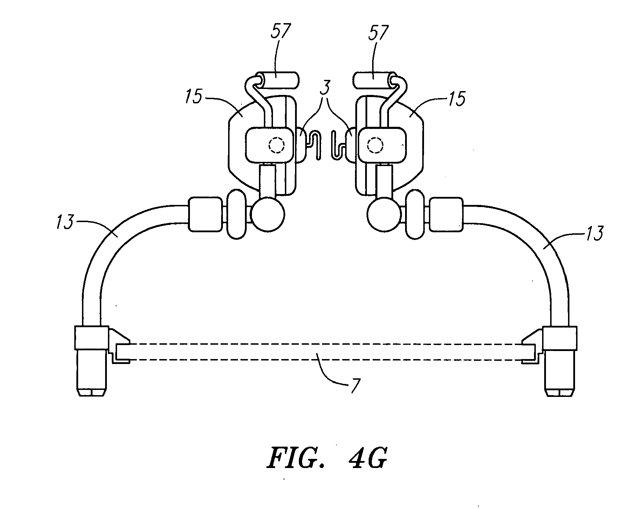

In the embodiment shown in Figs. 4G-H, a setup joint (13) is configured to support a pair of instrument drivers (15). In this embodiment, the setup joint (13) is in the form of an arch extending over the patient, and two instrument drivers (15) are mounted to the setup joint (13) towards a top portion of the arch, with their top surfaces facing each other and with their front surfaces or ends pointed in the same direction. With this arrangement, a pair of flexible catheter assemblies (3) may be mounted on respective instrument drivers (15) and inserted into a patient for use together during a surgical procedure, e.g., utilizing a sheath (39) or a common or "uber" sheath (63). Other embodiments may involve the use of more than two instrument drivers (15), e.g., three instrument drivers (15), to simultaneously deploy three flexible catheter assemblies (3) for use during a surgical procedure. In the illustrated example, the uber-sheath (63) is stand-alone component, but the uber-sheath (63) may also be coupled to its own instrument driver (15) and robotically controlled from a workstation (5) or manually maneuvered by a surgeon. Furthermore, the uber-sheath (63) may also be comprised of a flexible instrument member capable of traversing bends and curves.

During a procedure, an uber-sheath (63) may be inserted into a patient and directed to the anatomical region of interest, at which point flexible catheter instruments (37) may be deployed from a distal end of the uber-sheath (63). In one embodiment, one or more flexible catheter instruments (37) may be removed from the uber-sheath (63) and replaced with a different instrument or catheter (37) during a procedure without having to remove the uber-sheath (63) from the patient. Upon completion of the procedure, flexible catheter instruments (37) may be retracted into or back through the uber-sheath (63) and withdrawn from the patient.

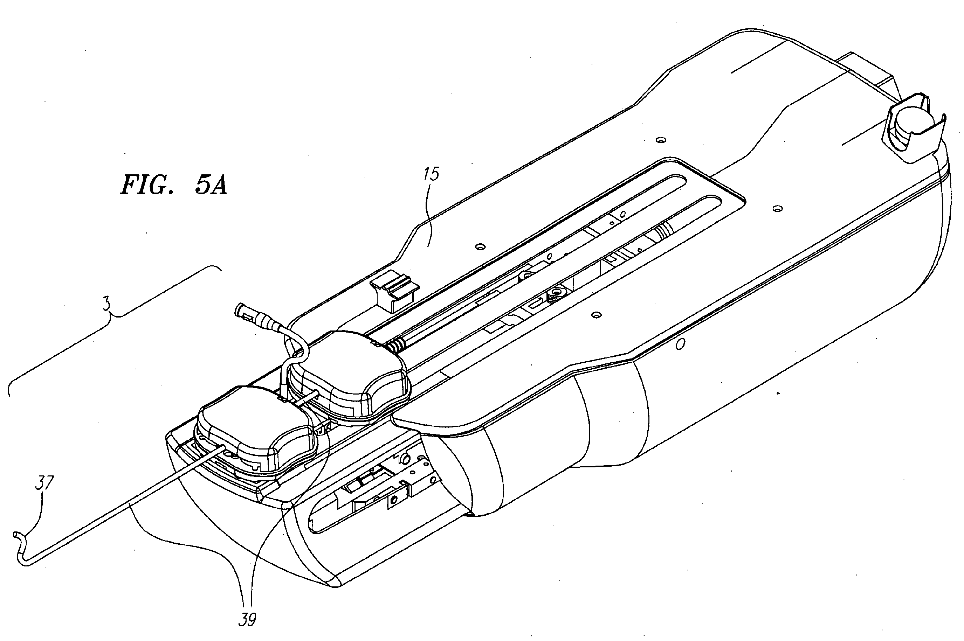

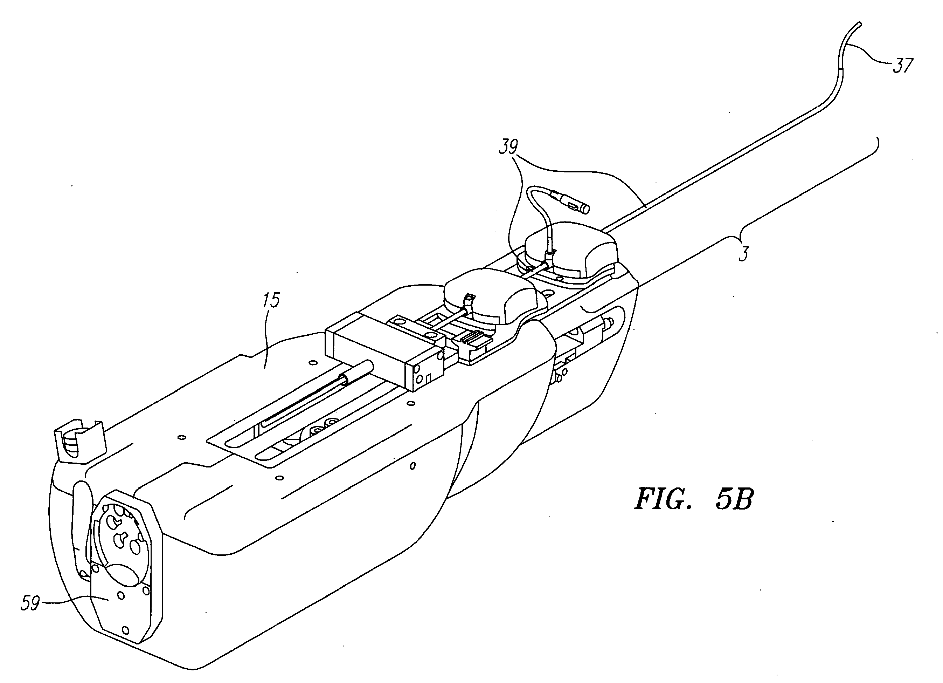

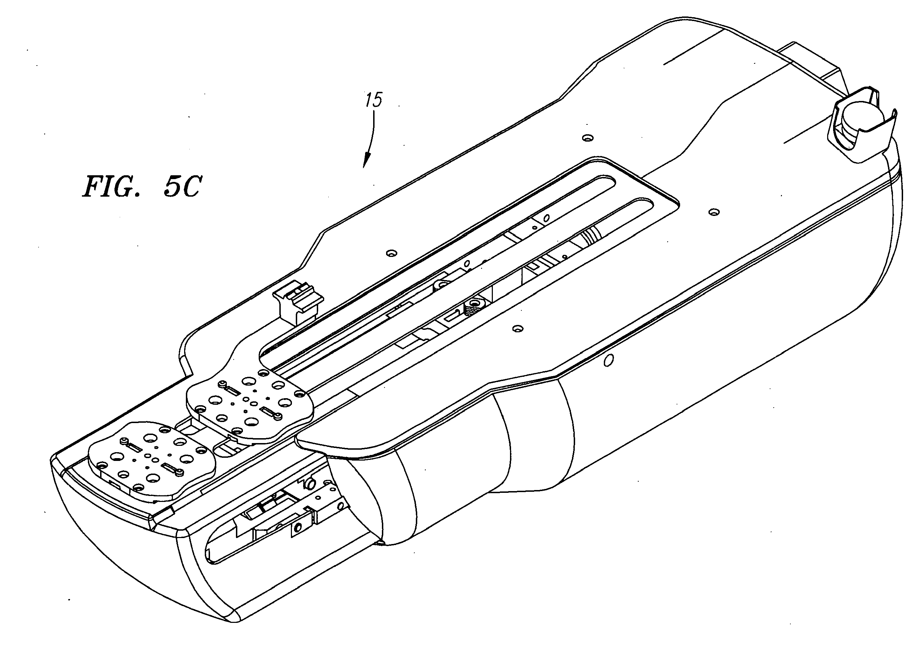

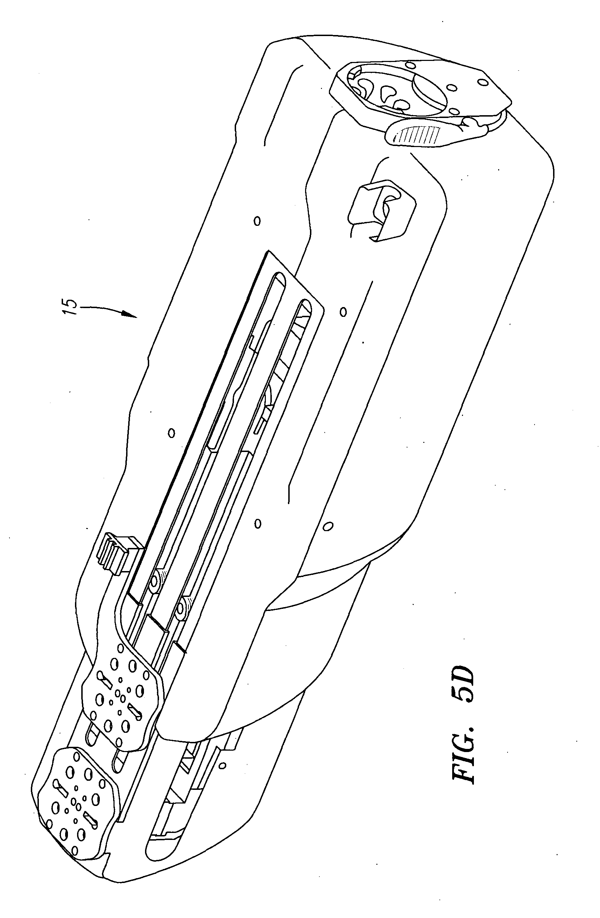

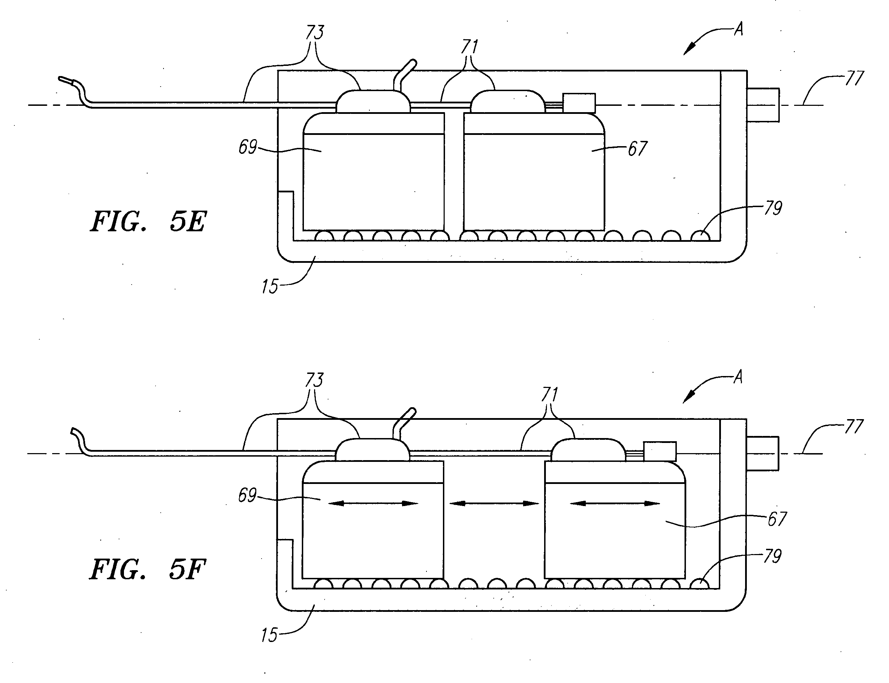

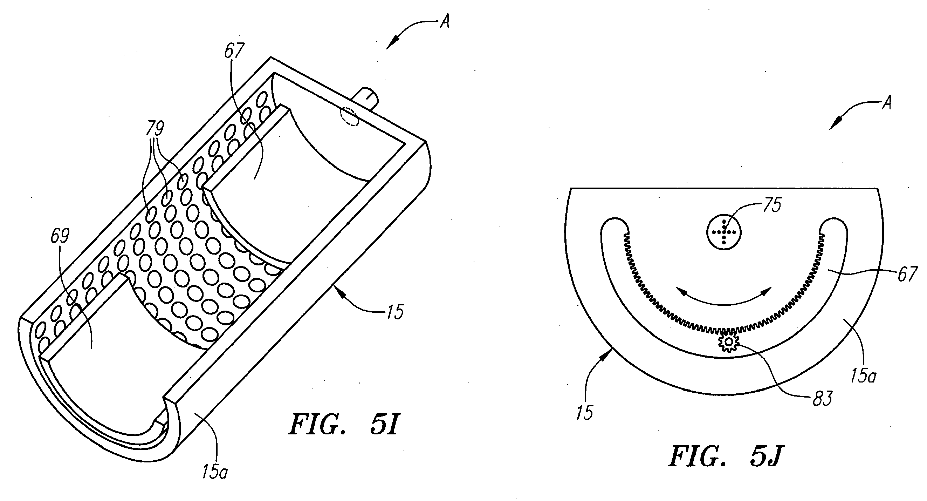

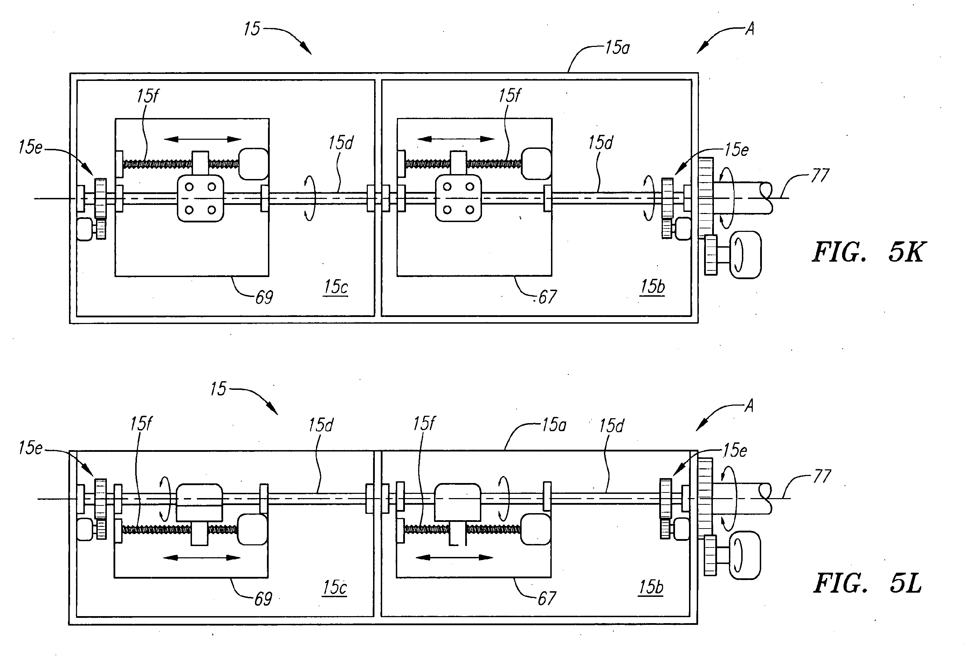

Figs. 5A-D illustrate an embodiment of an arrangement for controlling a flexible catheter assembly (3) using an instrument driver (15). In the illustrated embodiment, an instrument assembly (3) includes a flexible catheter instrument (37) and an associated flexible sheath instrument (39) attached to associated mounting plates on a top portion of an instrument driver (15). In one embodiment, the flexible catheter instrument (37) and sheath (39) are coaxially aligned, and the flexible catheter instrument (37) is inserted within a central lumen inside the sheath instrument (39). Movement of each instrument or catheter (37, 39) may be individually controlled and manipulated. As motors within the instrument driver (15) are activated, carriages coupled to mounting plates are driven forwards and backwards on bearings. As a result, the catheter (37) and sheath (39) can be controllably inserted and removed or retracted from the patient. Additional motors within the instrument driver (15) can be activated to control bending of the catheter (37) and sheath instruments (39), the orientation of the distal tips of these instruments (37, 39) and a working instrument (41) mounted to a distal tip of the flexible catheter (37).

Referring to Figs. 5E-L, an embodiment according to the invention is directed to an instrument driver assembly (A) that includes an instrument driver (15) for controlling a flexible catheter assembly (3). The instrument driver (15) includes a body, housing or frame (generally referred to as frame (15a) and carts or carriages (67, 69) that are positioned within the instrument driver frame (15a). In the illustrated embodiment, a first flexible instrument (71), such as a catheter assembly (3), is mounted to a first carriage (67), and a second flexible instrument (73), such as a sheath instrument (39), is mounted on a second carriage (69). The first and second carriages (67, 69) are advantageously controllably slidable or controllably movable along a longitudinal axis (77) of the instrument driver (15). Carriages (67, 69) are also controllably rotatable about the longitudinal axis (77), and independently rotatable relative to each other relative to the instrument driver (15).

Thus, both of the first and second carriages (67, 69) can be controlled such that only one carriage rotates, both carriages rotate in different directions, both carriages rotate in the same directions, the carriages are rotated at different speeds, or both carriages are stationary. Thus, for example, the first cart (67) and its flexible instrument (71) may be robotically controlled from the operator workstation (5) to slide along the longitudinal axis (77) and rotate in one direction, while the second cart (69) and its flexible instrument (73) may, for example, be controlled to be stationary, to slide along the longitudinal axis (77), and/or to rotate about the longitudinal axis (77) independently of the second carriage (69). Meanwhile, the instrument driver (15) itself may stationary or be caused to rotate in either direction, independently of the carriages (67, 69).

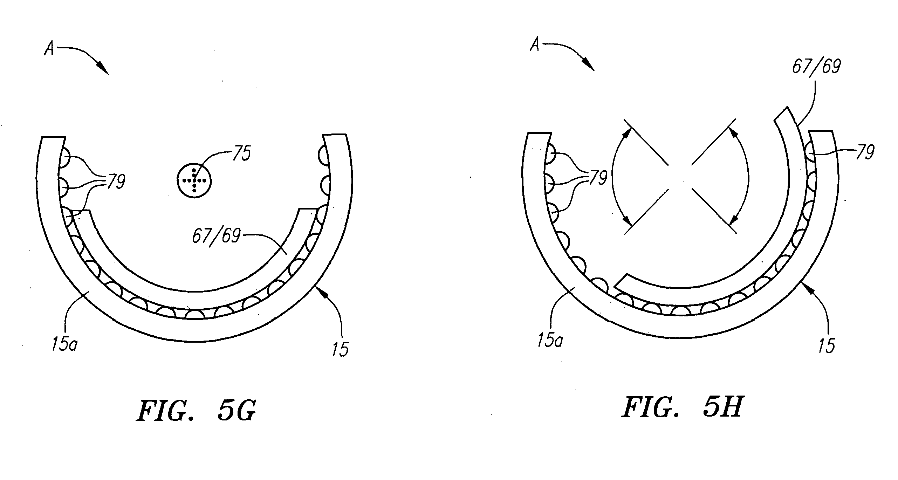

Referring to Fig. 5J, the dotted line cross (75) represents a central axis along the longitudinal axis (77) of the instrument driver (15), and referring to Fig. 5G, for example, a plurality of bearings (79) line an inner surface of the frame (15a) or are located about the inner surface of the instrument driver frame (15a). Carriages (67, 69) are parked on the bearings (79). By activating the appropriate control motors, a carriage may be driven forward and backwards along the longitudinal axis (77) of the instrument driver (15). Furthermore, through motor actuation, a carriage may also be controlled to rotate about the central axis (75) of the instrument driver (15), e.g., utilizing a drive screw or gear (83) shown in Fig. 5J, which includes teeth that may rotatably engage a groove or teeth structure of a carriage. Fig. 5H illustrates the degree of rotational freedom of a carriage that rotates about the instrument driver (15). In this example, because the instrument driver frame (15a) has a semi-circular shape, as a carriage travels on the bearings (79) upwards along the side of the frame (15a), the carriage rotates about the central axis (75) relative to the instrument driver (15).

A further embodiment of an instrument driver apparatus of a robotic medical system is illustrated in Figs. 5K-L. In the illustrated embodiment, the instrument driver (15) is designed to roll about its longitudinal axis (77) in response to motor driven gears located at the rear of its housing (to the right of the instrument driver (15) shown in Figs. 5K-L). The instrument driver (15) in the illustrated embodiment is bifurcated into a first chamber (15b) and a second chamber (15c). Located within each of the first and second chambers (15b, 15c) is a carriage (e.g., 67, 69) mounted to a rod (15d) that is positioned coaxially with the longitudinal axis (77) of the instrument driver (15). Each carriage (67, 69) has a motor and gear assembly (15e) in its chamber, which is configured to rotate about the longitudinal axis (77) as the rod (15d) is driven by the gear assembly (15e). In this embodiment, each carriage (67, 69) is configured to controllably rotate independently from the other carriage, and independently of the instrument driver (15), whether stationary or rotating. Thus an operator at the workstation (5) may cause rotation of whichever component desired.

Within each carriage (67, 69) is a lead screw (15f) extending parallel to the rod (15d) and fixedly coupled to the carriage. A mounting plate to receive a catheter splayer together with motors to actuate the catheter control elements are housed together on a movable platform that is coupled to the leadscrew (15f) with a rolling element nut. A motor assembly within each carriage (67, 69) may be configured to turn respective leadscrews (15f) which, in turn, causes its rolling element nut to travel forwards and backwards along the leadscrew (15f) depending on which direction the leadscrew (15f) is rotated. In alternative embodiments, the leadscrews (15f) may be mounted to the instrument driver frame (15a). Similarly, platforms may be configured to rotate on the rods (15d) without the use of a carriage.

Thus, with embodiments shown in Figs. 5E-L, an instrument driver (15) can be configured to receive at least two catheter instrument splayers or assemblies (3) and to be able to robotically maneuver catheter instruments (37) by controllably sliding each catheter splayer forwards and backwards along the longitudinal axis (77) of the instrument driver (15), and independently and controllably rotating each catheter splayer about the longitudinal axis (77) of the instrument driver (15). Further, with embodiments, carriages (67, 69) are independently controllable and rotatable relative to each other and relative to the instrument driver (15) such that the carriages are independently movable in at least three different directions, e.g., four different directions including towards a distal end of the instrument driver (15), towards a proximal end of the distal driver (15), rotated clockwise, and rotated counter-clockwise, with two different types of motion (linear or axial displacement, and rotational motion).

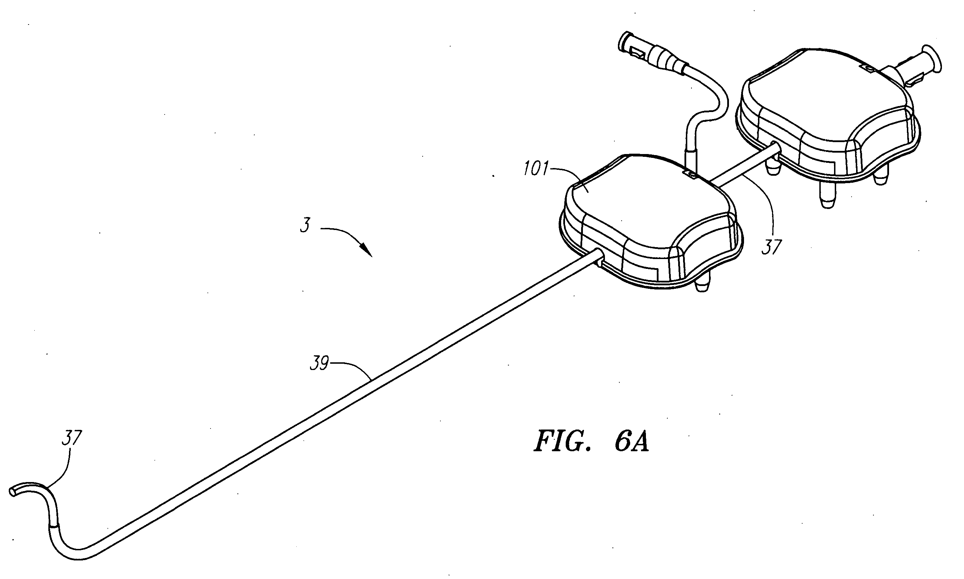

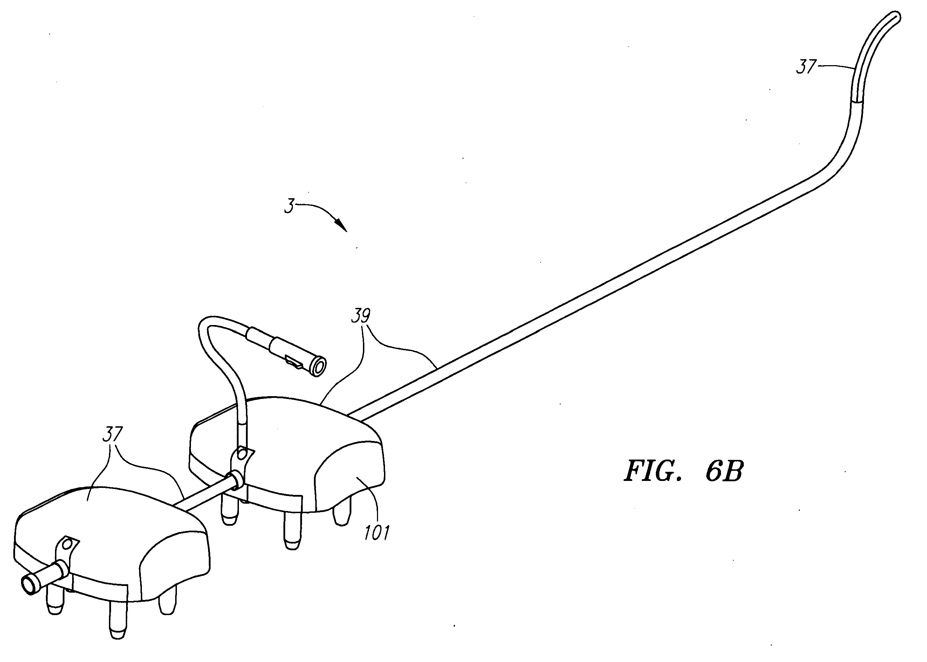

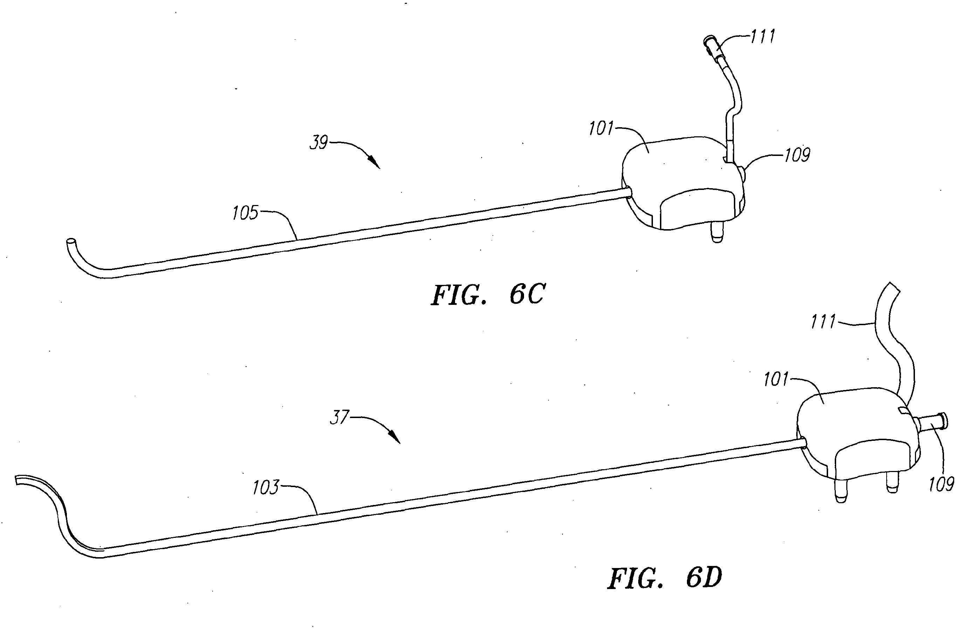

Figs. 6A-E illustrate various embodiments of a flexible catheter assembly (3) including a flexible catheter instrument (37) and a flexible sheath instrument (39). A sheath instrument (39) may include a splayer portion (101) having one or more control elements and a flexible sheath member (105) having a central lumen. Similarly, the catheter instrument (37) may also include a splayer portion (101), which would be located proximally of the splayer (101) for the sheath (39), and has one or more control elements and a flexible catheter instrument member (103). At a proximal end of the splayers (101), tubing (109) may be provided for insertion of another catheter device or valves (111) for the injection or removal of fluids. For example, the catheter instrument member (103) has a central lumen configured for passage of a working element or instrument, such as a tool, a scope, or another catheter, or a control cable for the same, which can be transported from the proximal end to the distal end of the catheter (37). The flexible catheter instrument member (103) may have a preconfigured surgical tool or end-effector mounted on an orientation platform at its distal tip.

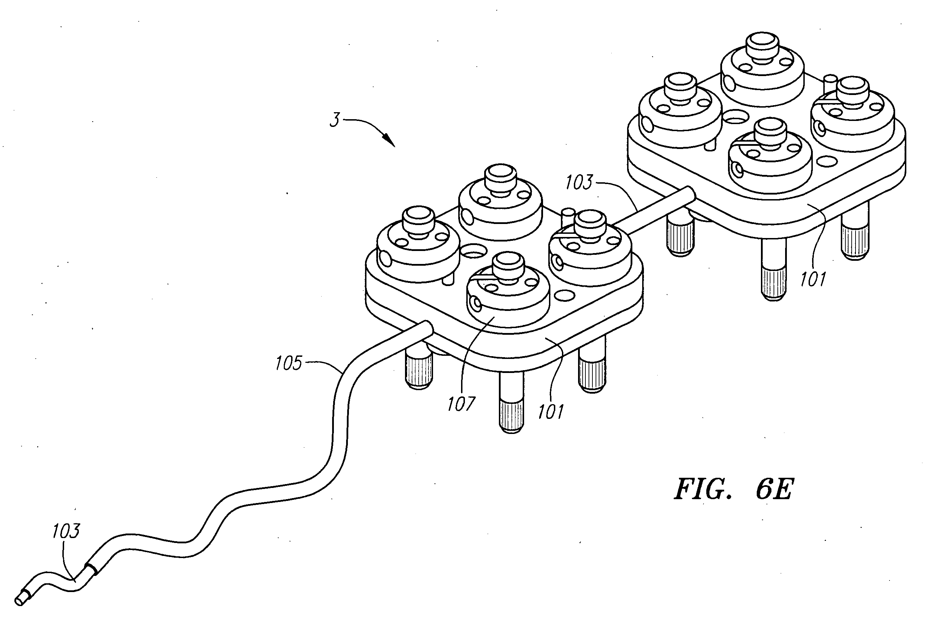

Prior to use of the catheter assembly (3) in a procedure, the catheter (37) is positioned proximally relative to the sheath (39) and the flexible catheter instrument member (103) is inserted into the sheath splayer (101), through the lumen of the sheath instrument member (105), such that the two instrument members are coaxially positioned. Both splayers (101) are mounted to respective mounting plates on the instrument driver (15). The splayers (101) can be controlled or adjusted using control knobs (107) (illustrated in Fig. 6E). Although each splayer (101) as illustrated includes four control knobs (107), other numbers of control knobs (107) may be utilized, and in some applications, they may be exposed for manual manipulation, and in others, they may covered by a housing. Further, the catheter instrument (37) and sheath instrument (39) may also differ in the number of control knobs (107) needed, e.g., depending on the number of control elements or pull wires needed to control the particular instrument.

For example, one embodiment of a flexible catheter instrument having a distal orientation platform and an end-effector can require a larger number of control elements whereas a simple 1 degree of freedom (DOF) sheath may require a smaller number of control elements. Similarly, a catheter instrument with numerous controllable portions or greater degrees of freedom may need to be wired with more control elements, each of which has to be robotically controlled by the instrument driver. For one embodiment, when the splayer for a flexible instrument is mounted onto the mounting plate of an instrument driver (15), an identification chip on the splayer is accessed by the instrument driver. By deciphering that information, the instrument driver (15) may be able to configure and pretension the control elements to a known state.

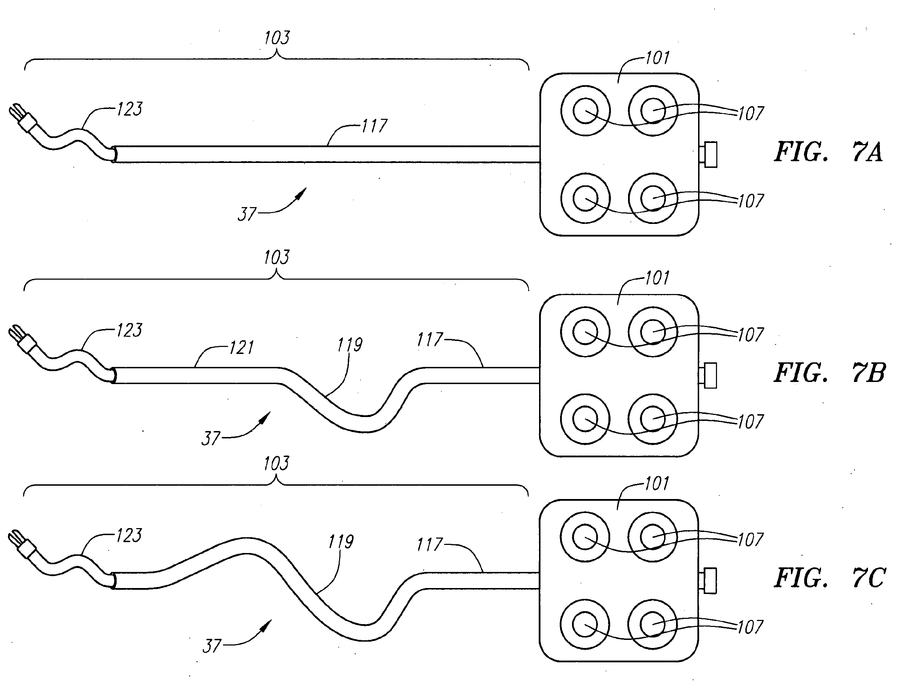

Figs. 7A-D illustrate various examples of flexible catheter embodiments having instrument members (103) with varying degrees of flexibility. Although the splayers (101) in Figs. 7A-D are each illustrated with four control knobs (107), the knobs shown here are illustrative in nature and are in no way restrictive of the number or placement of control knobs (107).

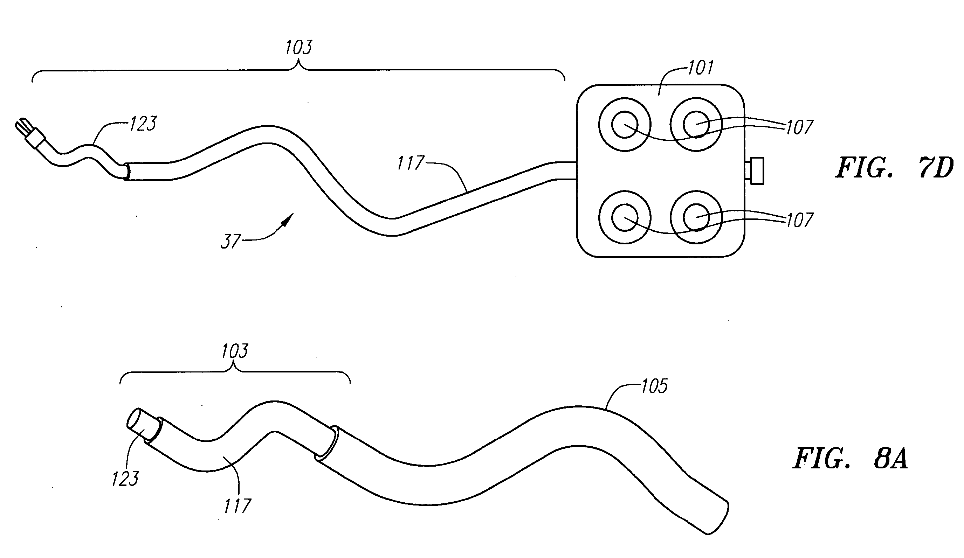

Referring to Fig. 7A, a catheter instrument (37) has a splayer (101) coupled to an instrument member (37) having two sections of different flexibility. The instrument member (103) shown in Fig. 7A has a first flexible distal section (121) coupled to rigid medial and proximal sections (117). In the implementation shown in Fig. 7B, the catheter instrument (37) includes a splayer (101) coupled to an instrument member (103) having a first rigid proximal section (117) coupled to a medial section that in turn has a first flexible medial subsection (119) and a second rigid medial subsection (121). Beyond the medial section is a flexible distal section (123). In this embodiment, although there are two flexible sections (119, 123) in the instrument member (103), the two sections (119, 123) may not necessarily be constructed to have the same degree of flexibility. For instance, the flexible distal section (123) may possess greater flexibility than the first flexible medial subsection (119). Referring to Fig. 7C, a catheter instrument (37) is shown having a splayer (101) coupled to an instrument member (103) that includes a first rigid proximal section (117), beyond which are a flexible medial section (119) and a flexible distal section (123). The medial and distal sections (119, 122) may have the same or different degrees of flexibility depending on the particular implementation. Referring to Fig. 7D, a catheter instrument (37) with a splayer (101) coupled to an instrument member (103) having a first flexible proximal section (117) and a second flexible distal section (123). Although the examples illustrated in Figs. 7A-D are described in the context of catheters instruments, the same techniques and concepts can be applied to other types of instruments such as sheaths.

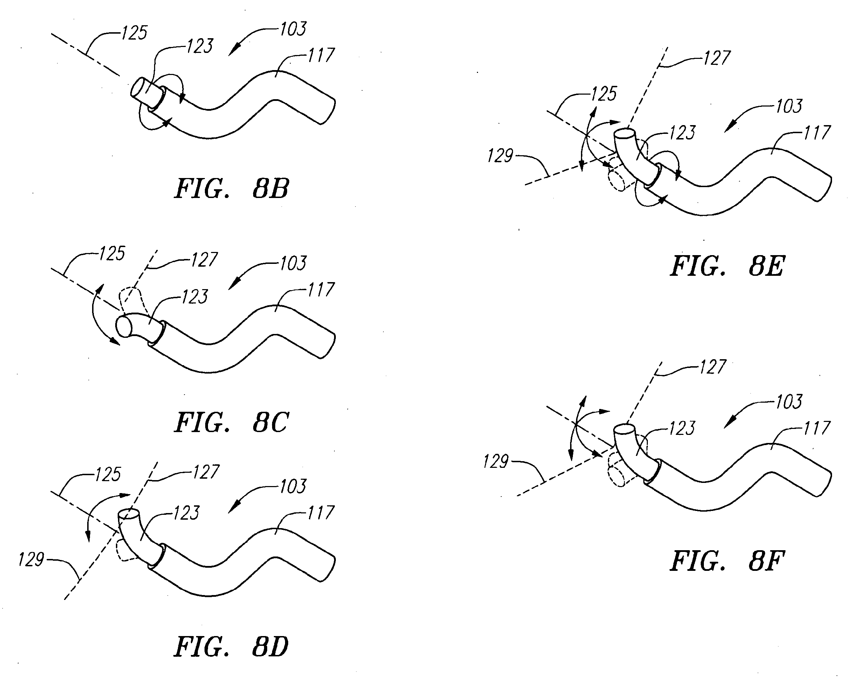

Figs. 8A-F illustrate various degrees of freedom of an embodiment of a flexible catheter instrument assembly (103), and instrument members (103, 105) are shown without splayers for clarity, and Figs. 8B-F illustrates a distal tip with its associated flexible catheter instrument. Referring to Fig. 8A, the distal section of the assembly starting from the left side of the illustration is a distal tip (123) of the flexible catheter instrument member. In one embodiment, the distal tip (123) may include one or more orientation platforms to which one or more tools or end-effectors may be mounted or attached. As shown in Fig. 8A, coupled to the right from the distal tip (123) is a flexible medial section (119) of the catheter member. The catheter member (103) is coaxially positioned within a flexible sheath member (105). As a result, certain sections of the catheter member (103) may mimic a similar curvature or path as that of the sheath member (105), especially the portions of the catheter member (103) that are located within the sheath member (105).

Referring to Fig. 8B, the distal tip (123) is shown as having a single degree of freedom relative to the catheter member (117). In this embodiment, the distal tip (123) can be controllably rotated about a central longitudinal axis (125) extending through the catheter member section (117). For example, the distal tip (123) and any attached working instrument or tool may freely rotate 360o about the longitudinal axis (125). In another implementation, the distal tip (123) may be configured to rotate 180o. The degree of axial rotation may depend on the particular design and application. Thus, examples discussed here are provided to illustrate how embodiments can be implemented in a non-limiting manner. Further, in some embodiments, the distal tip (123) may be implemented to rotate in a clockwise or counterclockwise manner, but may also be implemented to rotate in both a clockwise and counterclockwise manner.

Referring to Fig. 8C, a catheter member (103) may include a distal tip (123) capable of controlled pitching. In this example, the distal tip (123) can rotate about a lateral or transverse axis (127) that is perpendicular to the central longitudinal axis (125). For some embodiments, the distal tip (123) may have a positive (+) pitch or a negative (-) pitch, or even capable of both positive and negative (+/-) pitch. As shown in Fig. 8D, a catheter member (103) having a distal tip (123) capable of controlled yawing. In this example, the distal tip (123) can rotate about a transverse axis (129) that is perpendicular to both the central longitudinal axis (125) and the transverse axis (127) of pitch. For some embodiments, the distal tip (123) may have a positive (+) pitch or a negative (-) yaw, or even capable of both positive and negative (+/-) yaw.

Referring now to Fig. 8E, a catheter member (103) with a distal tip (123) having three degrees of freedom is illustrated. In this example, the distal tip (123) can rotate about a longitudinal axis (125), pitch about a first transverse axis (127), and yaw about a second transverse axis (129), wherein each of the three axes are perpendicular to the other two.

However, as discussed above with the embodiments of Figs. 8B-D, degrees of movement can vary depending on the particular implementation. It is contemplated that various combinations of varying degrees of movement may be implemented. For example, one distal tip may be designed to rotate 90o, have a +pitch, and a +yaw. The distal tip may also be configured to rotate 180o, have a +/- pitch and a +yaw. As show in Fig. 8F, the catheter member (123) of this example has a distal tip that pitches and yaws, but does not rotate.

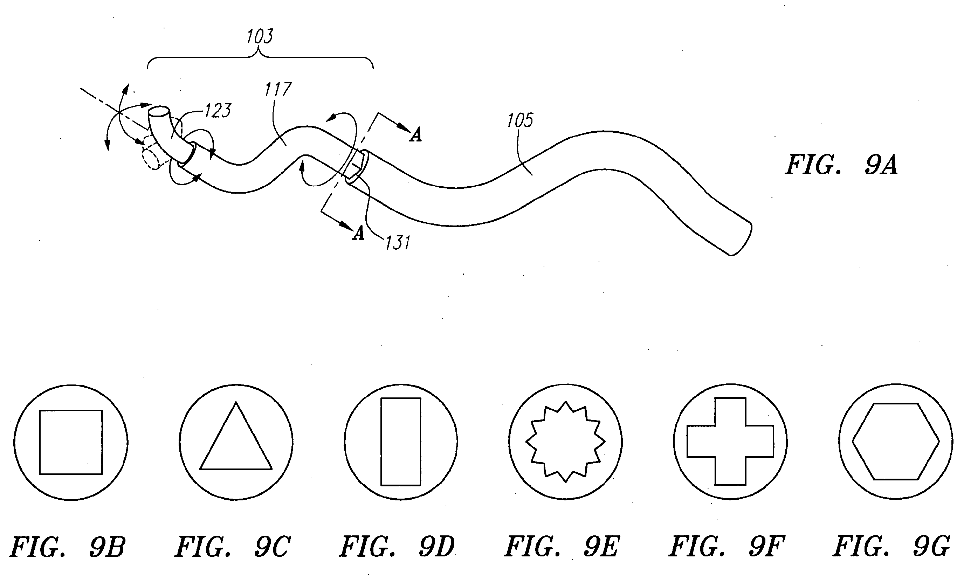

Additional degrees of freedom may be achieved in each of the examples described above in Figs. 8B-F by including an extra layer of mechanical control such as a sheath, as illustrated in Figs. 9A-G, which show the flexible catheter member (103) illustrated in Figs. 8B-F including an outer sheath (105) about the medial and proximal sections. For example, the catheter instrument member (103) may be extruded or retracted from the distal tip (131) of the sheath instrument member (105). Furthermore, the distal tip (131) of the sheath instrument (105) may be designed to allow for rotation about the longitudinal axis extending the length of the sheath instrument, thus providing an another degree of freedom. Depending on the particular configuration, the longitudinal axis of the catheter instrument may or may not coincide with the longitudinal axis of the sheath instrument. By rotating the sheath instrument distal tip (131), the catheter instrument member (103) located within and extending outwardly from the sheath instrument (105) will rotate in response to the sheath distal tip rotation.

Figs. 9A-F illustrate various shaped openings at the sheath instrument (105) distal tip as along A-A. By including a non-circular opening at the sheath instrument distal tip (131), the catheter instrument member (117) may be keyed to the opening. Thus rotation of the catheter instrumental (117) in response to the sheath instrument distal tip (131) rotation may be ensured.

In Fig. 9A, the opening is keyed in the shape of a square. Figs. 9B-G illustrate openings keyed in other shapes including a triangle, a rectangle, a star, a cross and a hexagon. Such shapes avoid slippage and allow the catheter instrument member to be physically or frictionally engaged with the sheath distal tip opening such that the desired coupled rotational movement occurs. Other shapes may also be utilized for this purpose, and a circular opening may be employed at the sheath distal tip if rotation is not desired.

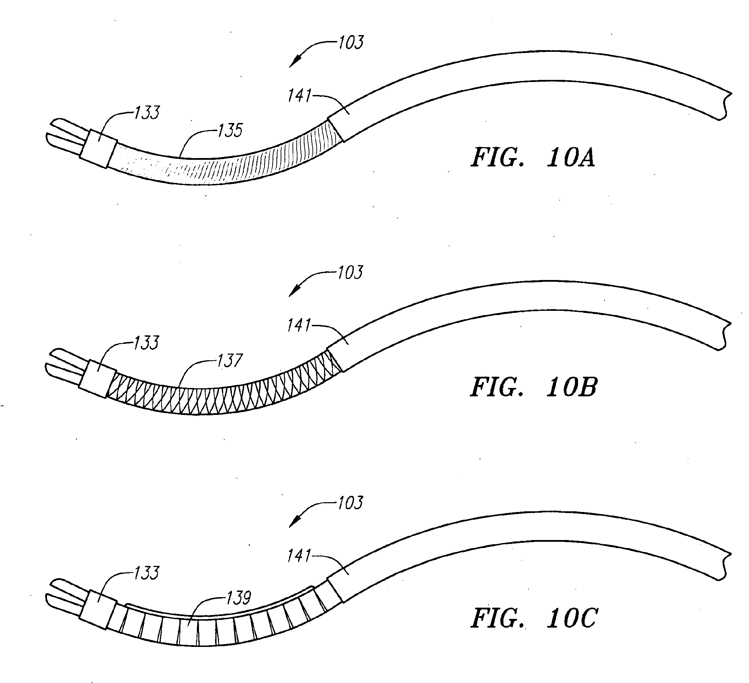

Figs. 10A-C illustrate the construction of various exemplary flexible catheter members (103). The flexible catheter members (103) are shown with a portion of the outer surface removed for purposes of illustration. Referring to Fig. 10A, the flexible catheter member (103) of one embodiment has a distal orientation platform (133) that includes an attached scissors-like tool. A distal portion of the instrument member having the exterior (141) omitted for clarity shows the coil construction in this embodiment. The coil spine (135) allows for flexible movement and bending of the catheter instrument member. Although the coil (135) of this illustration is shown wound in clockwise manner, the coils of other embodiments may be counterclockwise. Further, the number of coil layers may also vary. For example, it is possible to have a spine with multiple layers of coil wound in the same direction or alternate directions. Although not visible in Fig. 10A, the instrument member (103) of this embodiment includes a central lumen through with cables, tools, or other catheters may be deployed. Depending on the particular implementation, control elements for controlling the catheter flexing, distal tip, and end-effector may be routed within the coil spine, between the coil spine and the instrument member outer surface, or a combination of both.

Referring to Fig. 10B, a flexible catheter instrument member (103) is shown with a braid spine (137). The braid spine (137) of this embodiment forms a flexible tubular sheath with a central lumen and is constructed by interweaving a plurality of composite fibers or metal wires. However, it is contemplated that various methods for braiding and types of braids are possible.

Fig. 10C illustrates a catheter instrument member (103) having a spine constructed with a plurality of skeletal hinges (139). The skeletal hinges (139) of this embodiment are controlled with one or more control cables. These control cables may be activated to open or close certain hinges, thus resulting in the flexing of the catheter instrument member at certain junctures.

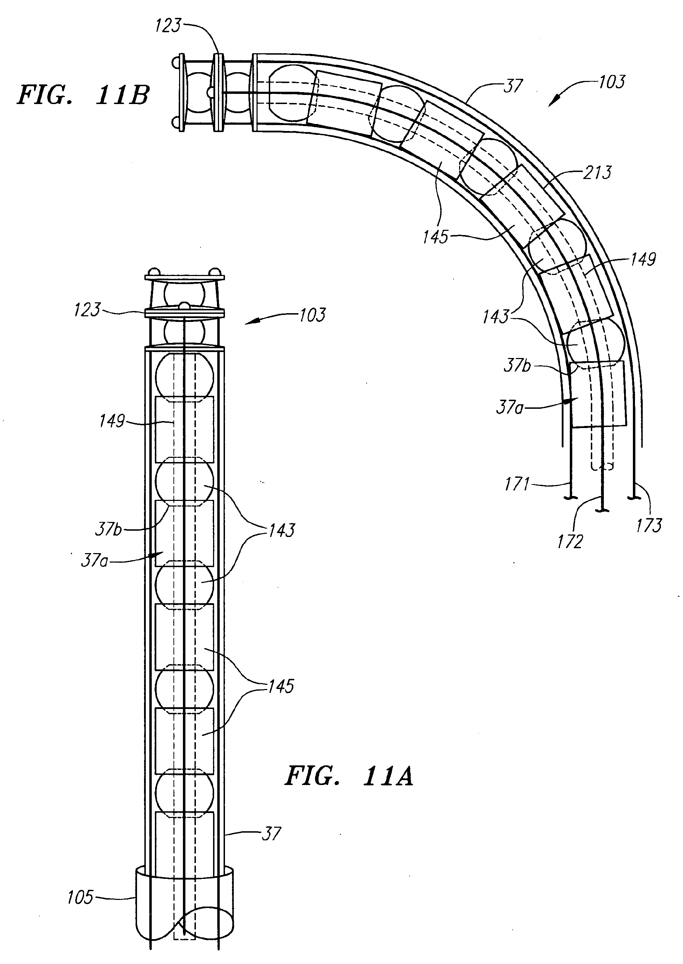

Referring to Figs. 11A-B, one embodiment a catheter instrument member (103) of catheter instrument (37) of a robotic medical system includes an elongate body (which may be disposed within a sheath instrument member (105) of a sheath (39) as shown in Fig. 11A), and an elongate, flexible support structure (37a), e.g., in the form of a spine-like structure, which extends through a lumen of the catheter (37). According to one embodiment, the support structure (37a) includes a plurality of substantially spherical elements (143) (generally referred to as spherical elements 143) and a plurality of non-spherical elements (145). In the illustrated embodiment, the support structure (37a) includes a series of alternating spherical or substantially spherical elements (143), e.g., metal or plastic balls, and non-spherical elements (145), e.g., cylindrical elements or cylinders (145).

As shown in Figs. 11A-B, a spherical element (143) may be seated and movable within indentations or concave cavities (37b) (shown as phantom limes) of adjacent non-spherical members (145). Embodiments of catheter support structures (37a) are configured such that the spherical elements (143) and cylindrical elements (145) may be retained or movably secured within the catheter (37), e.g., by tension applied by one or more control elements or wires (171-173) that extend through the catheter (37). In an alternative embodiment, a pair of tension rings or caps (not illustrated in Figs. 11A-B) at each end of the catheter instrument (37) may provide sufficient force to hold the support structure (37a) together.

In this manner, control elements (171-173) or other tension elements may be used to apply force that maintains the support structure (37a) components under tension for a given catheter flexure. Because the catheter instrument (37) is flexible, the various spherical elements (143) and cylinders (145) are arranged with sufficient freedom of movement such that they may slide, twist and/or bend as the catheter instrument member (103) bends and moves. Further, the catheter instrument member (103) may be substantially linear (as shown in Fig. 11A), or be placed under tension to bend (as shown in Fig. 11B), e.g., at an angle of about 45 degrees. With embodiments, the support structure (37a) collectively moves in a similar manner to follow bending of the catheter instrument member or catheter body (103).

Fig. 11A also illustrates a first flexible catheter instrument member (103) located coaxially within a sheath instrument member (105). Although spherical elements (143) and cylinders (145) are not shown beyond the distal tip of the sheath member (105), these components may extend along the length of the catheter member (103), and down into the sheath member (105), terminating at a catheter splayer (101). In other embodiments, the spherical elements (143) and cylinders (145) may begin and end at various locations within a catheter (37).

According to one embodiment, each spherical element (143) and each cylindrical element (145) may include a central lumen. In this manner, the plurality of substantially spherical elements (143) and the plurality of non-spherical elements (145) may be arranged or such that the individual lumens are aligned to collectively define a central, inner lumen within the catheter member (103) lumen. Such a lumen may be in communication with a catheter lumen such that a control cable for controlling a working instrument (41) may be inserted through the central, inner lumen. Further, in one embodiment, a spherical element (143) and/or the cylinder element (145) may be hollow in construction. In another embodiment, one or more of the pieces may be solid in nature except for the central lumen.

For example, as shown in phantom, a narrower, second flexible catheter instrument member (149) may be coaxially located within the collectively defined central lumen. As shown in Fig. 11B, the second catheter instrument (149) is bendable and may follow the bending of the outer catheter instrument member (103). Fig. 11B also shows the resulting repositioning of various spherical elements (143) and cylinders (145) at their interfaces in order to accommodate and move with bending of the catheter member (103). In the illustrated embodiment, each spherical element (143) is slightly tilted, and each cylinder (145) is moved relative to its original position as shown in Fig. 11A.

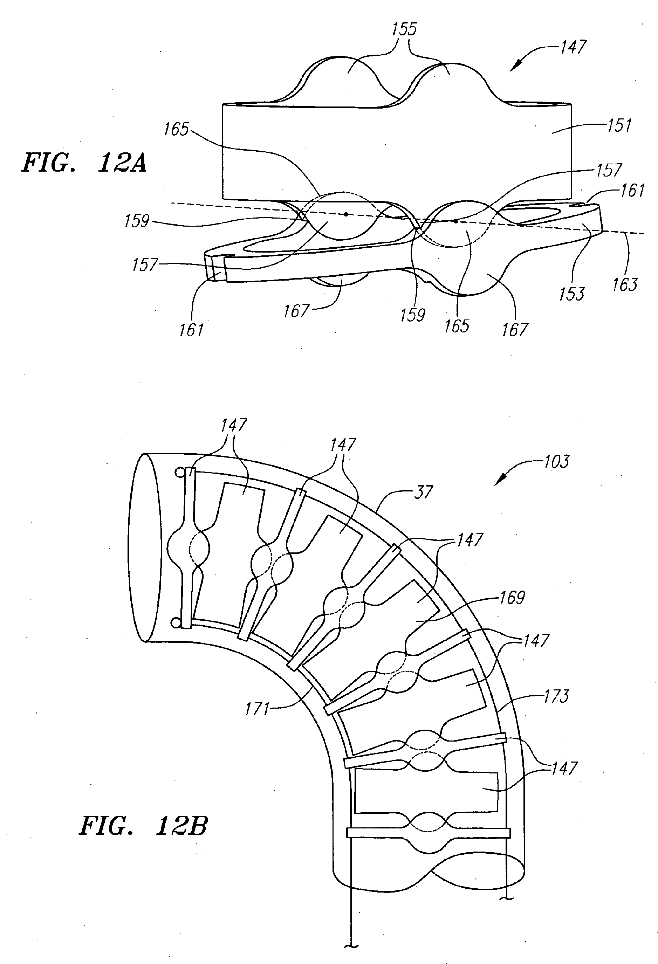

Figs. 12A-B illustrate an embodiment of a flexible catheter assembly (103) that includes a catheter instrument member (103) having a spine or support structure (37a) constructed according to another embodiment and including pivoting skeletal rings (147). As shown in Fig. 12A, a pair of skeletal rings are designed to mate together and result in pivoting movement. A first ring (151) is constructed as a circular band with two pairs of semi-circular teeth, a first pair (155) of teeth located on an upper edge of the first ring (151) and a second pair (157) located on the lower edge. In this embodiment, the upper and lower pairs of teeth (155, 157) are vertically aligned, but directed in opposite directions. In other words, top and bottom portions of the ring mirror each other.

With respect to each pair of teeth, each tooth in the pair is disposed on an opposite side of that particular ring edge and headed in the same direction. The second ring (153) is also constructed as a circular band with two pairs of semi-circular teeth, with one disposed on an upper edge of the second ring and one on the lower edge. However, each tooth on the second ring (153) of this embodiment abuts a indentation (159) or groove on the inside edge of the ring. This indentation is designed to receive a corresponding tooth from a first ring (151).

In one embodiment, the indentation (159) on the second ring is properly cut out to substantially match with the shape and thickness of a tooth on the first ring. Thus, when a first ring (151) and a second ring (153) are mated together as illustrated in Fig. 12A, a pair of teeth (157) from the first ring (151), the bottom pair in this example, are substantially lined up with the indentations for a pair of teeth (165) from the second ring (153), the top pair in this example, and the first ring teeth (157) can be slid into the second ring indentations (159). In this joined arrangement, the top ring (151) is allowed to pivot or rock relative to the second ring (153) about an axis (163) extending through the two pairs of opposing teeth. The second ring pair of teeth (165) in this example also helps to keep the first pair of teeth (157) in place at the indentations (159). When the rings are oriented in a coplanar manner, the physical contact between the rings is limited to portions of the surfaces of the mated teeth and space exists between the opposing ring surfaces. Thus, when the first ring is pivoted relative to the second ring, the space between the rings on one side should decrease and increase on the opposite side. In Fig. 12A, the space is located to the left and right sides of the rings (151, 153), and the upper ring (151) is slightly tilted towards the right such that the gap on the right side is less than that on the left side.

In the illustrated embodiment, the dimensions of the first ring (151) are different than the dimensions of the second ring (153), although in other embodiments the vertical heights, thicknesses, and/or diameters of the rings may be substantially similar. The second ring (153) of Fig. 12A is also shown with a notch (161) on its outer edge. This notch (161) serves as a channel along which a control element can be routed. In one embodiment, a control element, such as a pull wire, can freely move about this notch when the control element is activated, but is substantially held into place so that it does not migrate out of position. Depending on the particular implementation, some portions of the rings or the rings in their entirety may be constructed out of some type of metal or plastic. As with the spherical and cylindrical elements (143, 145) shown in Fig. 11A-B, these rings have a central opening or lumen such that a central lumen may be collectively defined when the plurality of rings are coupled together, thereby allowing a working instrument, cable or other device to be inserted through the central lumen.

With reference to Fig. 12B, a flexible catheter instrument member assembly (103) of a catheter (37) and a support structure or spine constructed with a plurality of rings (147) may be flexed to bend, e.g., at about 45 degrees. The spine (169) of this embodiment is comprised of an alternating stack of rings from Fig. 12A, and as shown in Fig. 12B, the teeth of the stack of rings are substantially aligned with the central longitudinal axis of the instrument member. In the illustrated embodiment, two control elements (171, 173) are located at opposite sides of the catheter (37). Each control element (171, 173) may be routed along the notches (161) discussed above on the second ring type (153) from a distal position back to the catheter splayer and associated control knobs. As the control element (171) on the left side of the catheter (37) is pulled downwardly, and the control element (173) on the right side is released or let out, the catheter (37) bends as the stack of rings (147) begins to pivot to the left.

Fig. 12B also illustrates how the space between opposing surfaces for adjacent rings vary depending on the degree of bending at that particular location. For instance, the left side of the rings is compressed together in response to the bending action, whereas the right side of the rings is spread apart and the space expanded. Based on how much the control elements (171, 173) are pulled and released, the amount of flexure can be controlled as desired. Although the spacing between the rings appear to be somewhat uniform in this illustration, the pivoting occurring at each of the ring interfaces may likely vary and the spacing may not necessarily be identical. In order to return the catheter (37) to a vertical configuration, the right control element (173) is pulled downwardly and the left control element (171) is released or let back out. Bending the catheter (37) to the right side can also be accomplished by manipulating the control elements (171, 173) appropriately. In one embodiment, control elements (171, 173) may be pre-tensioned before a surgical procedure to a known state and may be reset to a known state as necessary during a procedure.

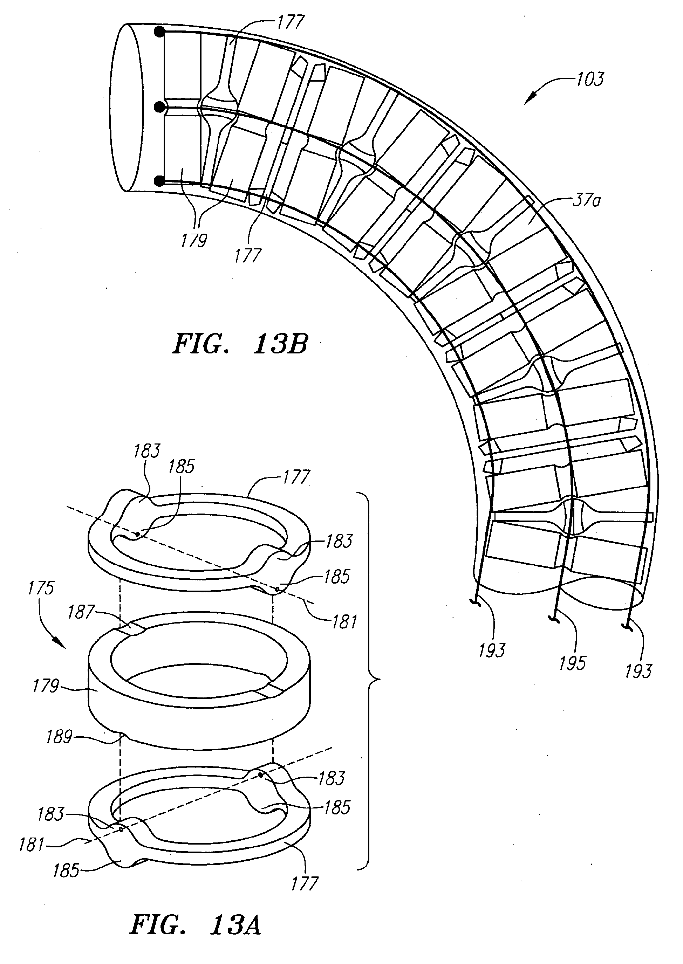

Figs. 13A-B illustrate another embodiment of a catheter instrument member (103) of a catheter (37) that includes a spine or support structure (37a) including pivoting skeletal rings. In the illustrated embodiment, rings (175) operate in a manner similar as described in Figs. 12A-B. However, the embodiment shown in Figs. 13A-B employs three types of rings. According to one embodiment, a first type of ring (177) is a circular band that includes a pair of circular wings (183, 185) or semi-circular teeth on opposite sides of the ring (177). A line (181) may be mentally drawn to connect the pair of circular wings (183, 185) as a first axis about which the ring (177) may rotate. A second type of ring (179) is a circular band with a first pair of rounded indentations on the upper surface and a second pair of rounded indentations in the bottom surface. The first pair of indentations (187) is on opposite sides of the ring along a first axis while the second pair of indentations (189) are on opposite sides of the ring along a second axis, wherein the first axis and second axis are orthogonal to the other. A third type of ring (177) may be the same as the first type of ring (177), but rotated 90o on its face. Thus, a line (181) drawn to connect the pair of circular wings (183, 185) on opposite side of the third type of ring may be a second axis about which this ring (177) may rotate. The first and third types of rings of this embodiment are designed to pitch or yaw, depending on their orientation, and the second type of ring is an interface piece between rings of the first and third types. During construction of a spine, the first type of ring is mated with the upper surface of the second ring type and a third ring type is mounted with the bottom surface of the second ring.

Fig. 13B illustrates a length of a catheter member (37) of a flexible catheter instrument member (103) including a spine (37a) constructed with rings shown in Fig. 13A. In the illustrated embodiment, the spine (37a) alternates between rings of the second type with rings of first or third type. For example, if "1" represents the first ring type, "2" represents the second ring type, and "3" represents the third ring type, then the repeating pattern in the spine (37a) shown in Fig. 13B, starting from the distal tip, is "2 - 1 - 2 - 3 - 2 - 1 - 2 - 3". The arrangement of the spine (37a) of this embodiment allows for pivoting and yawing of the catheter (37) about a central longitudinal axis extending along the length of the catheter (37). The illustrated embodiment includes four control elements (193, 195): a pair of control elements for controlling pitch motion, and a pair of control elements for controlling yaw motion. By tightening and slackening control element pairs, the catheter member (103) can be controlled to have various degrees of +/- pitch, +/- yaw, and combinations thereof. The amount of bending achievable may be physically limited by the clearance available between the rings, as shown in Fig. 13B. In alternative embodiments, the rings may be machined so that additional clearance may be available at certain edges of the rings.

Other embodiments may involve other patterns may be used in the spine (37a) construction. For example, rings shown in Fig. 13A may be used in a pattern such as "2 - 1 - 2 - 1 - 2 - 1 - 2" to construct a spine (37a) that allows for pitching. In an alternative embodiment, a pattern such as "2 - 3 - 2 - 3 - 2 - 3 - 2" may form a spine (37a) that allows for yawing. Furthermore, other embodiments may involve non-repeating patterns. For example, a spine (37a) may include different segments having different patterns in order to obtain other types of bends. For example, in one embodiment, a combination such as "2 - 1 - 2 - 1 - 2 - 1 - 2 - 3 - 2 - 3 - 2" may form a spine (37a) that allows for pitching in one segment and yawing in another segment. A virtual central lumen is also created within the catheter member (103) when a plurality of rings are stacked together.

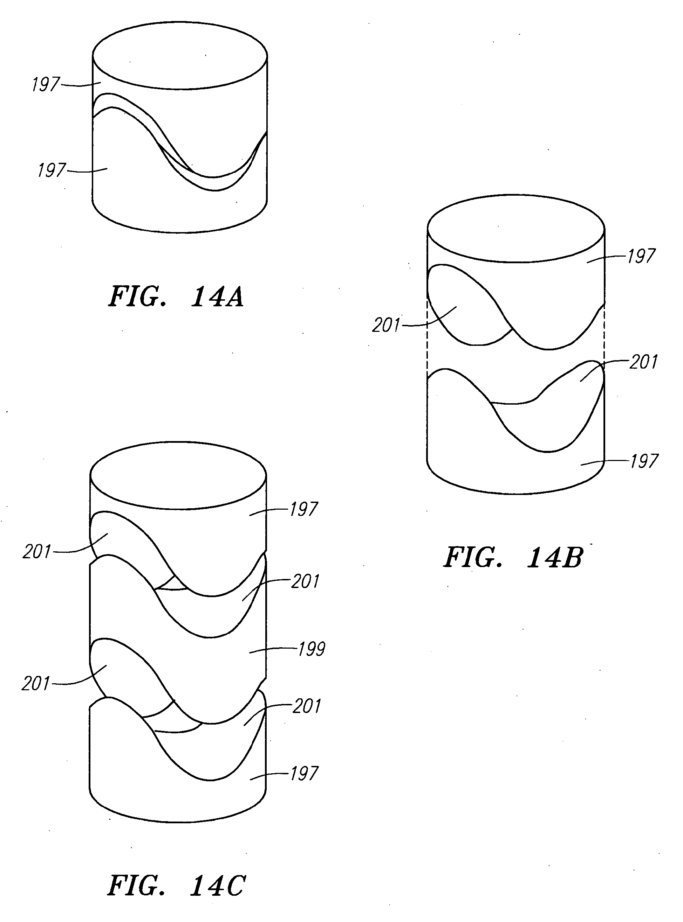

Figs. 14A-C illustrate another embodiment of a flexible catheter member (103) that includes a spine (37a) constructed with saddle joint type of segments. A first saddle joint segment (197) is comprised of a cylindrical outer surface coupled with an inner concavo-convex surface. The second saddle joint segment is also comprised of a cylindrical outer surface coupled with an inner concavo-convex surface. In one embodiment, the first and second segments (197) are identical in construction. In other embodiments, segments (197) may be constructed with different dimensions, materials, etc. The first and second saddle joint segments (197) may be joined together at their concavo-convex surfaces (201) when the surfaces are at a 90o offset from each other. This interface allows for movements such as flexion, extension, adduction, abduction, and circumduction between the two segments. One or more pairs of these saddle joint segments may be coupled together to serve as a spine for a flexible catheter instrument member. By attaching control elements to the saddle joint segments, a catheter instrument member may be flexed at various degrees of +/-pitch and +/yaw.

Figs. 14A-B illustrate a pair of segments (197) with flat end surfaces, and Fig. 14C includes an additional intermediate segment (199) comprising a cylindrical outer surface and two concavo-convex surfaces (201). In the illustrated embodiment, the two concavo-convex surfaces (201) are similar in shape and dimension, except that they are at 90o offset from each other. The first saddle joint segment (197) is coupled to the intermediate segment (199) at a first interface involving the first segment concavo-convex surface (201) and a first intermediate segment concavo-convex surface (201). The second saddle joint segment (197) is coupled to the intermediate segment (199) at a second interface involving the second segment concavo-convex surface (201) and a second intermediate segment concavo-convex surface (201). Utilizing a larger number of saddle joint segments increases the degree of bending obtainable from the spine (37a) and catheter member (103). Although not shown in Figs. 14A-C, each of the saddle joint segments (197, 199) is constructed with a central lumen such that segments may be aligned and stacked together into a spine to form a central lumen through the stack of segments.

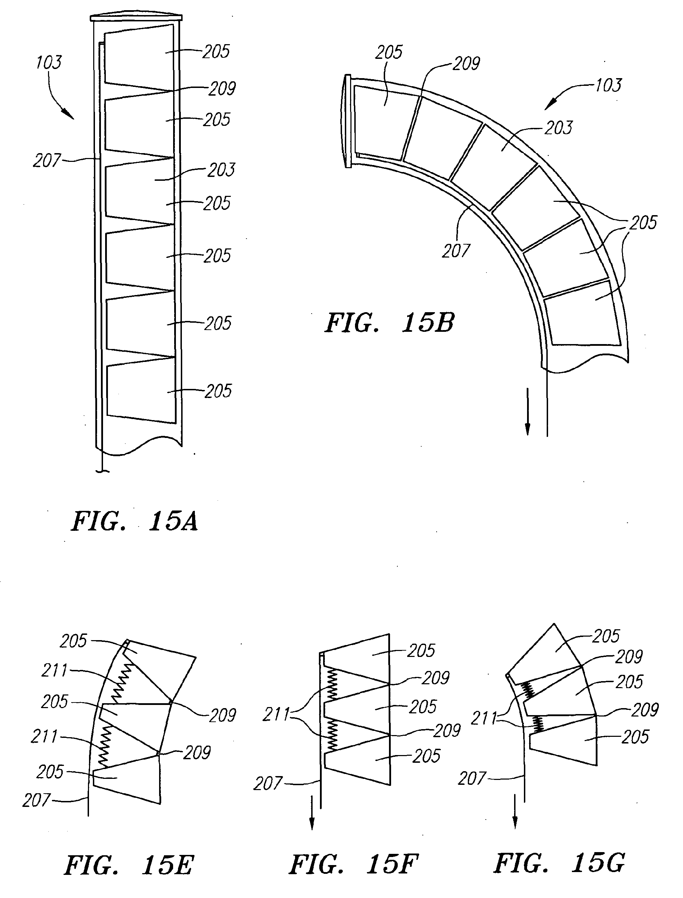

Figs. 15A-G illustrate various embodiments of flexible catheters with support structures or spines constructed of wedges. Fig. 15A illustrates a wedge spine (203) located within a flexible catheter instrument member (103) of a catheter instrument (103). In this embodiment, each of the wedges (205) has a cylindrical outer shape when viewed down the axis of the catheter member (103) and a trapezoidal shape when viewed from a first side and a rectangular shape when viewed from a second side. The trapezoidal shape is most relevant to this discussion and Fig. 15A reflects that elevation. A plurality of wedges (205) are stacked together as a spine (203) and a single control element (207) is coupled to the wedge closest to the distal tip of the catheter member (103). For this embodiment, a pivot point (209) exists where each wedge is in contact with an adjacent wedge along a single edge. When the control element (207) is pulled downward in this example, the wedges (205) revolve about their pivot points (209) and the space between the trapezoidal wedges is gradually reduced as the catheter assembly (103) bends to the left. As illustrated in Fig. 15B, when the space between the wedges (205) is completely eliminated, the catheter member (103) has reached its maximum bend radius and the control element is fully tensioned. To straighten the catheter member (103), the control element (207) may be released and pushed back up.

Figs. 15E-G illustrate the use of compression springs (211) to assist with the control and flexing of the catheter assembly (103). In this example, a spring (211) is coupled between each of the wedges (205) on the edge opposite from the pivot point (209). In Fig. 15E, a stack of three wedges (205) and the placements of the springs (211) are illustrated. The control element (207) is not being engaged in this instance, so the springs (211) not under load. Thus the springs (211) are shown pushing the wedges (205) open as the wedges (205) revolve about their pivot points (209).

In Fig. 15F, the catheter member (103) assumes the shape of a substantially straight line as the control element (207) is pulled downward to a specified tension. In one embodiment, the control element may be automatically pre-tensioned to such a designated tension so that the instrument member is in a known shape or configuration. Fig. 15G, illustrates the stack of wedges (205) bending to the left as the control element (207) is pulled downward more, which in turn causes the springs (211) to further compress than in Fig. 15F and the space between the wedges (205) to reduce.

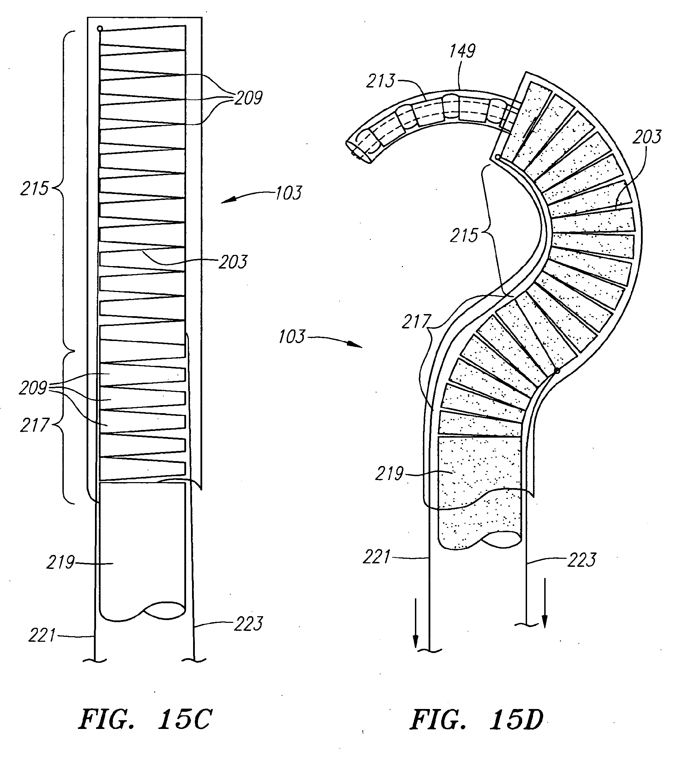

Figs. 15C-D illustrate the implementation of a catheter member (103) having a wedge spine (203) together with a second flexible catheter (199) having a ball and cylinder spine (213). As shown in Fig. 15C, the wedge spine (203) is constructed with a first series of wedges (215) all oriented in a first direction and a second series of wedges (217) all oriented in a second direction. By orienting the first series of wedges (215) all in a first direction, all their pivot points (209) are also aligned along one line. Similarly, for the second series of wedges, all their pivot points (209) are aligned along a second line. Beyond the second wedge series, the proximal portion of the spine (219) of this example may be constructed with tubing. A first control element (221) is coupled to the most distal wedge of the first wedge series and a second control element (223) is coupled to the first wedge of the second wedge series. By pulling the first control element (221) downward, the first wedge series (215) is bent into a first curvature. By also pulling on the second control element (223) downward, the second wedge series (217) is bent into a second curvature.

Fig. 15D illustrates the flexible catheter instrument member of Fig. 15C with the resultant overall curve of this example. In addition to the wedge spine (203), this example also includes a second flexible catheter (149) having a ball and cylinder spine (213) similar to that disclosed above with Figs. 11A-B. Here, the second flexible catheter member (149) snakes through a central lumen of the flexible catheter member (103). During a surgical procedure, the first flexible catheter member (103) may be introduced into the patient to a desired location whereupon the second flexible catheter member (149) may be deployed from the first flexible catheter instrument. The first flexible catheter instrument essentially serves as a sheath in this case. Thus a more rigorous first flexible catheter member (103) having limited bending capabilities may be used to deliver a second flexible catheter having greater degrees of bending. As discussed above, the second flexible catheter (149) may also include a lumen through which tools or another catheter may be passed. Although springs (211) are not illustrated in Figs. 15C-D, they may be employed between various wedge segments in accordance to the discussion above with Fig. 15E-G.

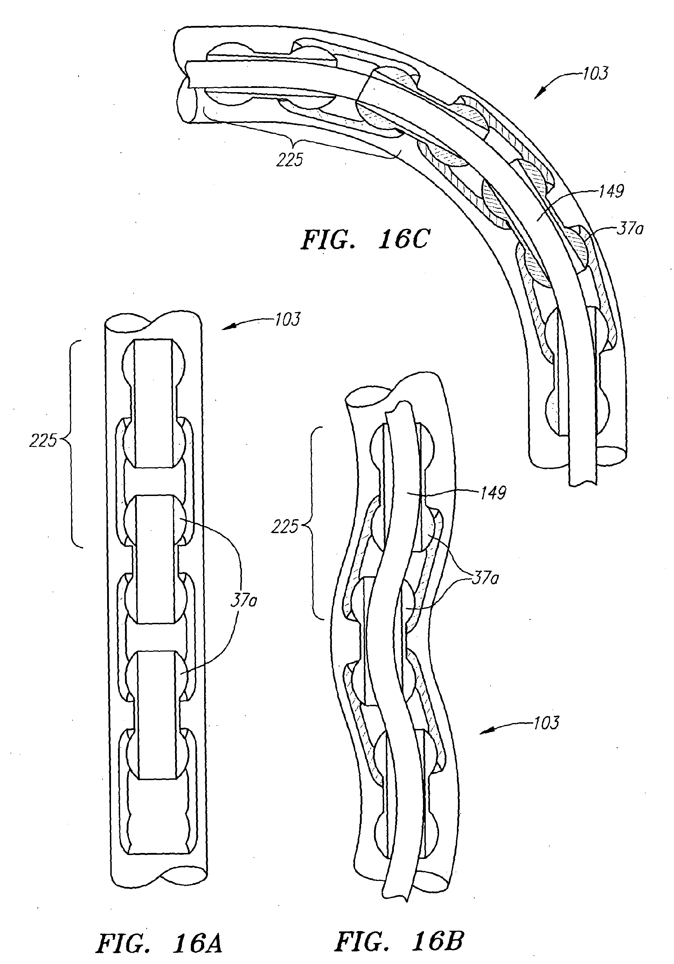

Figs. 16A-H illustrate various embodiments of catheter members or components (103) of a catheter instrument (37) having support structures (37a) constructed of spherical elements or ball links. Referring to Fig. 16A, a flexible catheter member (103) may include an inner spine (37a) comprised of a plurality links (225) in the form of spherical elements or balls. In the illustrated embodiment, two types of ball links are used: a female link (227) and a male link (229). Each female link (227) may have a substantially cylindrical shape and may be hollow in construction. Each end of the female link (227) may be a circular groove (231) to receive a ball member (233) of a male link (229).

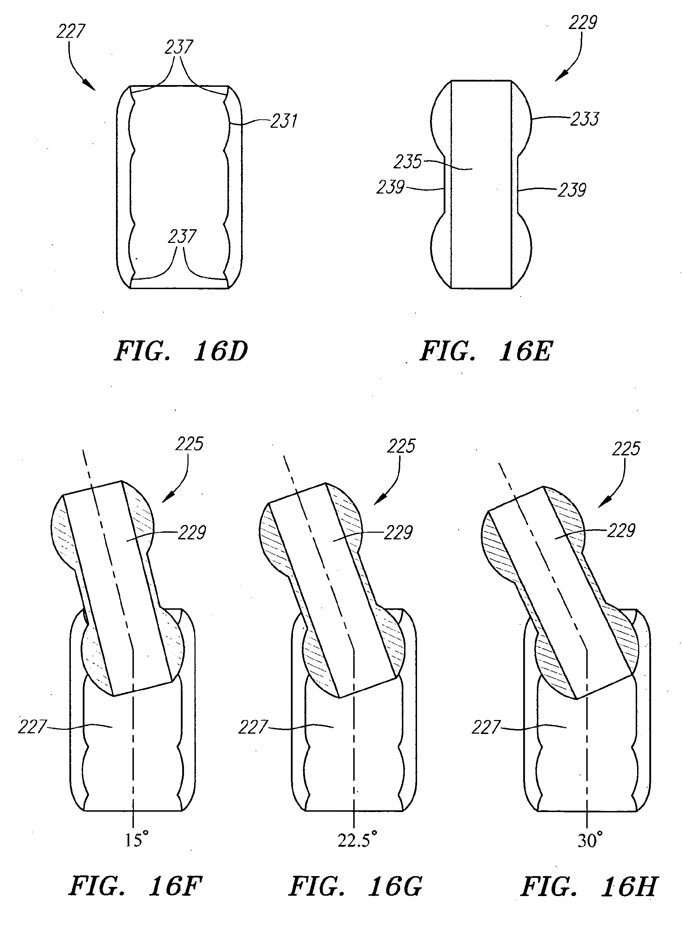

As shown in Fig. 16D illustrates a female link, and Fig. 16E illustrates a male link. According to one embodiment, each male link (229) has a dumbbell shape with a central lumen (235) along the longitudinal axis. Each ball member (233) of the male link (229) of this implementation may be inserted into the circular groove (231) of a female link (227). By mating a male link ball member (233) with a female link circular groove (231), the two links (227, 229) are joined together as a two link chain (225). To extend the chain, male (229) and female links (227) are alternately mated together as illustrated in Fig. 16A. Male link lumens and hollow centers of female links together form a central longitudinal lumen for the catheter instrument (37).

Figs. 16F-H illustrate bend angles that may be achieved at the male and female link interface of some implementations. In certain embodiments, the inside edge at each end of the female link (227) is notched with a slanted edge (237). This slanted edge is machined to match a desired maximum bend angle between a male (229) and a female (227) link at that particular link interface. For example, the implementation of Fig. 16F is designed to have a 15o bend angle as measured between the longitudinal axis of the male link (229) and the longitudinal axis of the female link (227). As visible in Fig. 16F, the notched slanted edge (237) of the female link (227) makes contact the male link bar member (239), thus preventing the links (227, 229) from bending further relative to each other beyond a 15o angle. For the implementation in Fig. 16G, the notched slanted edge (237) is machined for a 22.5o angle of bend between the links (227, 229). Similarly, the notched slanted edge (237) of the female link of Fig. 16H is designed for a 30o angle of bend between the links (227, 229).

Thus, as catheter member (103) is inserted into a patient, the spine (37a) may be allowed to slack and flex in order more easily navigate through tortuous paths. Fig. 16B illustrates how the instrument member of Fig. 16A may become flexed. Depending on the particular implementation of the notched slated edges on the female links (227), the ball link spine (37a), and its flexible instrument member (103) in turn, may be able to bend and curve as needed. Fig. 16C illustrates another embodiment of a spine (37a) wherein the ball links (225) are tilting together towards the left hand side such that the flexible instrument member (103) results in a gradual 90o curve to the left. For one embodiment, the bending of the instrument member may be controlled with one or more control elements coupled to various links in the chain. Also, shown in both Figs. 16B-C, another flexible catheter (149) may travel through the central lumen (235) of the ball link spine (37a) and illustrating how such a catheter (149) may be guided to follow the bending of the catheter instrument member (103).

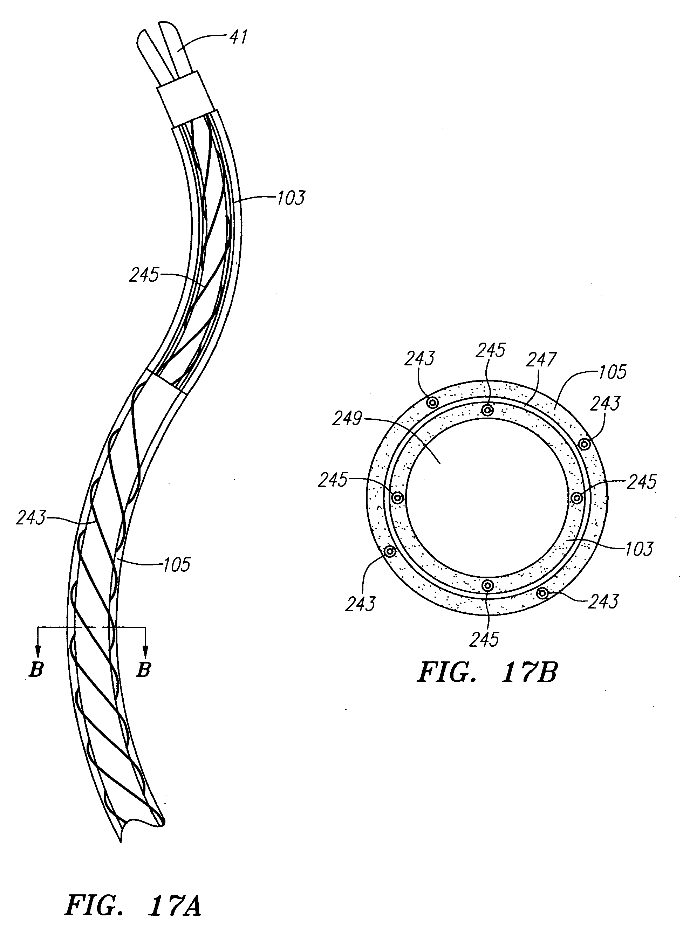

Figs. 17A-B illustrate routing of control elements for one embodiment of a flexible catheter arrangement. In the illustrated embodiment, two flexible catheters are coaxially positioned, with an outer catheter member (105) behaving like a sheath for an inner catheter member (103). A working instrument (41) such as an end-effector may be coupled to a distal end of the inner catheter member (103). Cutaway views of both catheter instrument members are illustrated in Fig. 17A.

As shown in Fig. 17A, inner catheter control elements (245) may be wound in a counterclockwise direction around the outside of the inner catheter member (103) when viewing the catheter in the direction of the catheter distal tip downwards towards the proximal end of the catheter. Further, the outer catheter control elements (243) may be wound in a clockwise direction around the outside of the outer catheter member (105) when viewing that catheter in the same direction as above. In the illustrated embodiment, the control elements of the different catheters are wound in different directions, but in other embodiments, they may be wound in a same direction. Further, each catheter may have varying numbers of control elements based on the particular implementation and the arrangement of such control elements may vary.

Referring to Fig. 17B, control elements for both flexible catheters are routed through lumens within their respective instrument members. For example, the outer catheter member (105) may have four control elements (243) spaced equi-distantly from each other, and the inner catheter member (103) may have four control elements (245) arranged equi-distantly from each other. Control elements may also be routed in channels or grooves on the exterior or interior of the walls in alternative embodiments. Dedicated shafts may also be used to house control elements in some embodiments. The inner catheter member (103) is disposed in the central lumen of the outer catheter instrument member (247). In the illustrated embodiment, the central lumen (249) of the inner catheter member (103) is available for items such as a catheter or tool to pass from the catheter proximal end to the distal tip.

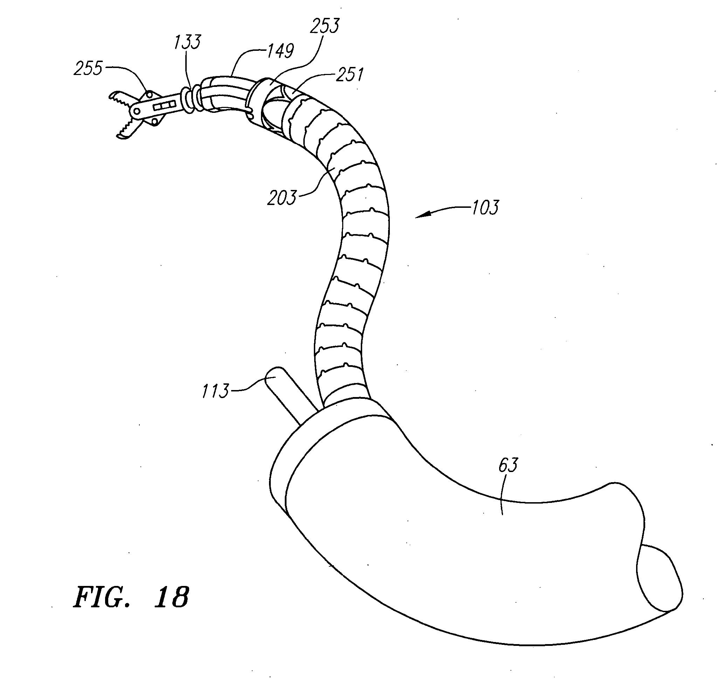

Referring to Fig. 18, according to another embodiment, an uber-sheath (63) includes a flexible instrument member (103) such as a flexible catheter member (103) and a scope (113). In the illustrated embodiment, the flexible catheter member (103) is constructed with an embodiment of a wedge spine (203). Towards the distal portion of the catheter member (103), an interface piece (251) couples the spine (203) and a rotational collar (253). A second inner flexible catheter instrument (149) is deployed coaxially within the central lumen of the first flexible catheter member (103). In the illustrated embodiment, the inner catheter instrument (149) is bolstered with a plurality of ball and socket segments. The distal ball and socket segment may also be referred to as an orientation platform (133) that may be controllably maneuvered as discussed in further detail with reference to Figs. 32A-59C. A grasper tool (255) is shown coupled to the orientation platform (133) of this example, although a multitude of other types of tools may be deployed at the distal tip of a flexible catheter. Depending on the particular implementation and type of surgical procedure, the scope (113) may be an optical device such as an endoscope, borescope, fiberscope, videoscope, or optical fiber through which objects or surroundings at its distal tip may be remotely viewed by a user. In certain embodiments, a proximal end of the scope may be coupled to an image capture device such as a camera in addition to an eyepiece. Some scopes may also include a light delivery system such as an illuminating optical fiber, a special optical lens such as a wide-angle lens, an image sensor such as a charge-coupled device (CCD), or a tube for delivery/removal of air, medication, fluids, etc. In other embodiments, the light delivery system may be deployed separately from the scope.

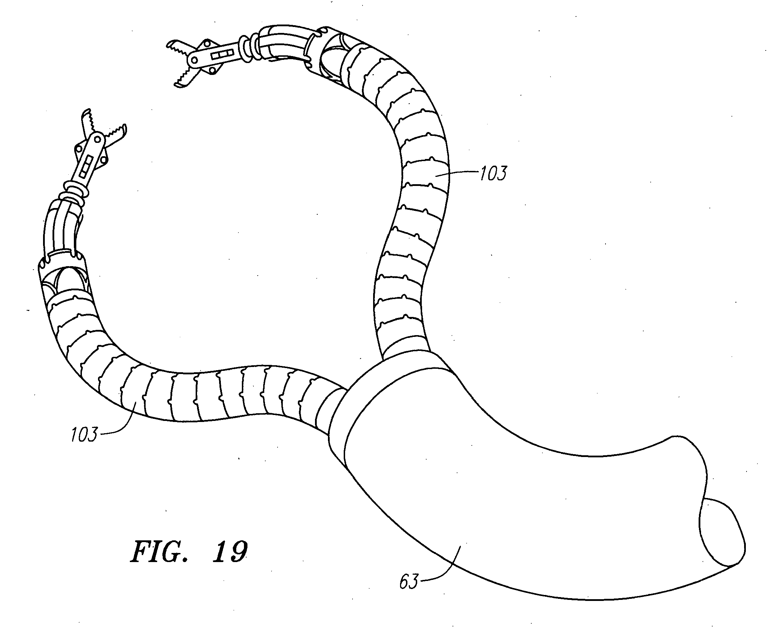

Fig. 19 illustrates another embodiment of an uber-sheath (63) including a pair of flexible catheter members (103) constructed with wedge spines. In contrast to Fig. 18, the embodiment shown in Fig. 19 involves replacing the scope (shown in Fig. 18) with an additional flexible catheter. For illustrative purposes, the catheter members (103) of this embodiment are similar in construction and both are fitted with working instruments in the form of graspers. In alternative embodiments, flexible catheters of different construction may be utilized. Each catheter may also have a different working instrument or tool that is deployed.

As the uber-sheath (63) of this implementation may be controlled by an operator from the system workstation described above, each flexible catheter member (103) and its associated devices may also be controlled by the operator independently from the uber-sheath and from each other. Thus, a surgeon may maneuver or navigate the uber-sheath to within a vicinity of the desired anatomical region, and then deploy one or more of the flexible catheters to perform a surgical procedure. During the procedure, one or both of the flexible catheters may have its working instrument or end-effector replaced with a different one or with a catheter device, such as a mapping catheter or ablation catheter. Furthermore, one or both of the flexible catheters themselves may be removed and replaced with another instrument such as scope, laser scalpel fiber, or insufflation device, etc. At the end of a procedure, the flexible catheter members (103) may be withdrawn back into the uber-sheath (63) and removed from its proximal end, and the uber-sheath (63) reverse navigating its way back out of the patient.

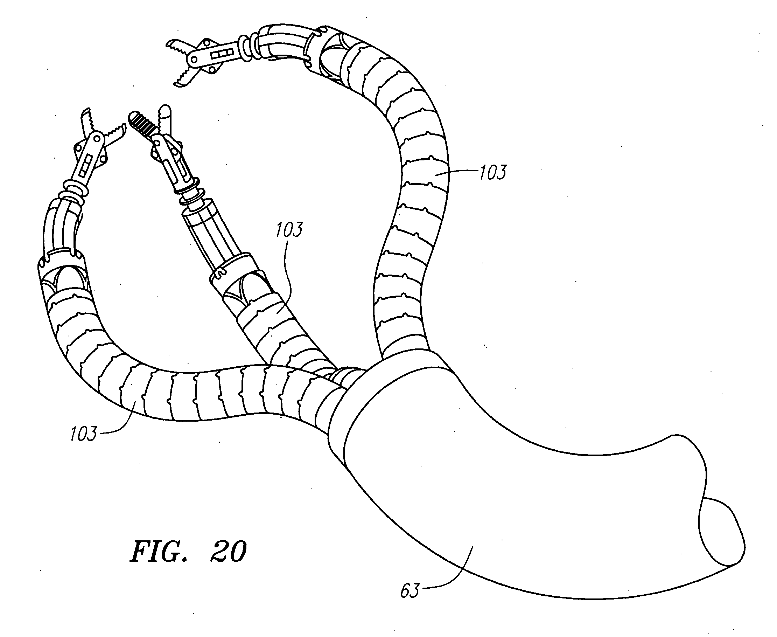

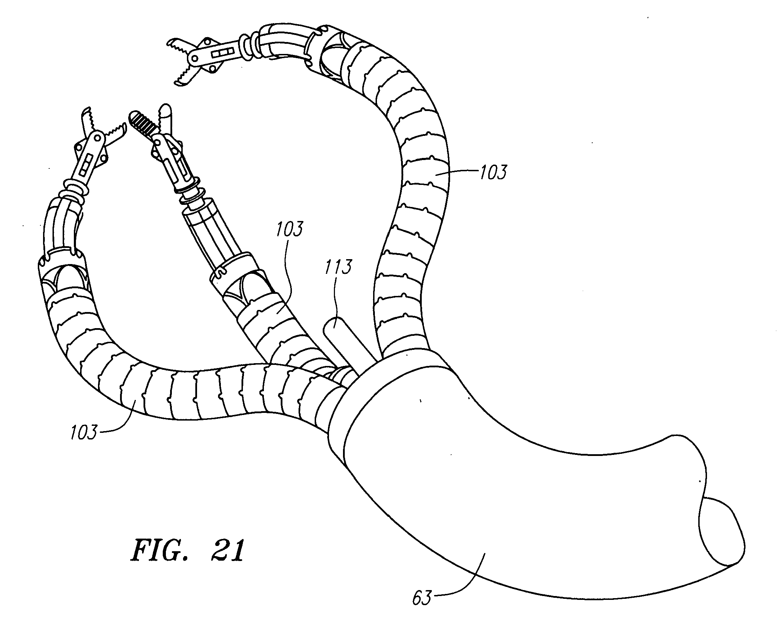

Fig. 20 illustrates a further embodiment of an uber-sheath (63) including three flexible catheters (103). Fig. 21 illustrates yet another embodiment of an uber-sheath (63) including three flexible catheter members (103) and a scope (113). Although the uber-sheath of this embodiment and embodiments described above have a specific number of through lumens along their longitudinal axes, alternative embodiments may involve different configurations. Further, each lumen of an uber-sheath may be configured with the same or different physical attributes such as lumen opening shape, dimensions, and placement. It should be understood that a multitude of uber-sheath (63) configurations may be utilized and may include various types and numbers of flexible catheters and instruments.

In one embodiment, when only some of the lumens on the uber-sheath are utilized or populated with an instrument, a vacant lumen may be covered with a flap or cap to prevent undesired entry of bodily fluids into the lumen. In an alternate embodiment, one or more of the lumen openings at the distal end of the uber-sheath may be covered with a unidirectional flow valve that behaves functionally like a heart valve or reed vale. Thus fluids and instruments may be allowed to enter the patient through the sheath, while the reverse flow of bodily fluids such as blood back into lumen is prevented.

Figs. 22A-Q illustrate the construction of a flexible catheter assembly (103) in accordance to one embodiment. The flexible catheter assembly of Fig. 22A is similar to those described with uber-sheaths in the examples of Figs. 18-21. In Fig. 22A, however, the outer flexible catheter instrument member (257) has a spine constructed with a plurality of wedges (205). In this illustration, the spine is shown exposed, without an outer layer such as a jacket. In certain embodiments, the spines may or may not be encapsulated with an outer layer of material. At the distal portion of the outer instrument member (257) is an interface piece (251) coupling the distal end of the spine with a rotational collar (253). An inner flexible catheter instrument member (149) is coaxially located within the central lumen of the outer flexible catheter (103). Although only the most distal ball and joint stage or orientation platform (133) of the inner catheter spine is visible in this figure, an entire spine comprised of a plurality of ball and joint stages extends from the distal tip of the inner catheter member to the proximal end at a splayer. Mounted on the orientation platform is a pair of graspers (255), but a variety of other tools or end-effectors may be mounted instead.

Fig. 22B illustrates a flexible catheter member (103) of a catheter instrument (37) of Fig. 22A and three pairs of control elements. A first pair (259) of control elements extends from a splayer at proximal end of the flexible catheter assembly (103) to termination points on the rotational collar (253). Second and third pairs (261, 263) extend from the splayer to termination points on the distal orientation platform (133). In some embodiments, the different pairs of control elements may be mounted to and controlled by different splayers, while a single splayer may control all the control elements of a flexible catheter in other embodiments. During a surgical procedure, an operator inputs commands to the system via the user interfaces on the workstation. The system processes the commands and communicates the control signals to activate the necessary motors and servos to cause the desired mechanical response on the catheter assembly. As the mechanical parts of the instrument driver respond to the commands, various control elements are actuated at the splayers, causing the relevant portion of the flexible catheter to move or flex.

In the embodiment involving three pairs of control elements, a first pair (254) may be manipulated to cause the rotational collar (253) and items located within its lumen, the inner flexible catheter instrument member (149) in this case, to controllably rotate either clockwise or counterclockwise. A second (261) pair may be manipulated to cause the distal orientation platform (133) to controllably pitch forward (+) or backward (-). A third pair (263) may be manipulated to cause the distal orientation platform (133) to yaw forward (+) or backward (-). In this exemplary embodiment, one or more control elements (265) for controlling the tool (255) extend from the tool down through a lumen of the inner flexible catheter to a splayer or servo at the proximal end of the catheter assembly (103). As these control elements (259, 261, 263, 265) are manipulated, the tool may be actuated to perform the desired movements. Depending on the complexity of the particular flexible instrument embodiment and the degrees of freedom achievable, varying numbers of control elements may be implemented to control these movements.

Further, in illustrated embodiments, the spine (203) of the outer catheter (257) is constructed with a plurality of interlocking wedges (205), as described with reference to Figs. 15A-G. These wedges (205) are circular in shape when viewed from a top or bottom angle. From a side perspective, the wedges may appear as trapezoidal in shape.

A series of three teeth elements (267) are equally spaced about the top face (271) of the wedge (205). Similarly, a series of six triangular notches (296) are equally spaced about the bottom face of the wedge (273). In the illustrated embodiment, teeth elements (267) are designed to fit into the notches (269). Thus, when teeth elements (267) of a first wedge are engaged with the notches (269) of a second wedge, adjacent wedges are locked together and prevented from rotating relative to each other. Although the number of teeth and notches differ in this embodiment, alternative embodiments may have an equal number of teeth and notches. By rotating the various wedges (205) of the spine (203), a user may be able to configure the spine to have a predefined amount of bending or to assume a desired curvature when the wedges (205) of the spine (203) are pulled together.

In this implementation, twelve lumens (277) that extend through the wall of a wedge from its top face (271) to its bottom face (273) are equally spaced about the circumferential face. When viewed from the top, it appears as if a series of four lumens (277) are located between adjacent teeth elements (267). When viewed from the bottom, these same lumens (277) appear distributed as pairs between adjacent notches (279). In the illustrated embodiment, lumens of adjacent wedges may align when the teeth and notches of these wedges are engaged together, and lumens of adjacent wedges may be combined together to form a longer lumen that may extend along a length of the wedge spine. Whereas the central lumen (275) of this wedge (205) forms part of the catheter instrument member lumen (247), lumens in the wedge wall (277) may be used to carry control elements or wiring from the catheter proximal end to some point along the catheter instrument member.

Figs. 22L-M illustrate an interface piece (251) of one embodiment. Fig. 22M illustrates a cross-sectional view of the interface piece (251) of Fig. 22L. Like the wedges described above with Figs. 22G-K, this interface piece (251) also includes three notches distributed about its bottom, face to engage with teeth (267) from a wedge (205). In this embodiment, the interface piece (251) caps the stack of wedges (205). Four sets of channels are located on the outer wall of this interface piece (251) for the routing of control elements from the top wedge piece (205) to the rotational collar piece (253). Each channel set starts as a groove (283) at the bottom edge of the piece (251) and then bifurcates into two curved grooves (281) sweeping out in opposite directions towards the top edge of the piece (251). In the illustrated embodiment, the eight curved grooves terminate at the top edge of the piece (251) at eight different points; but in alternative embodiments, some grooves may merge together, thus resulting in fewer points of termination. A recess (285) has been hollowed into the interior surface of the interface piece (251) of this example to receive a bottom section (287) of the rotational collar (253).

Fig. 22N is a perspective view of the rotational collar (253). This rotational collar (253) has a first section (287) for mating with the interface piece (251). Also visible in Fig. 22M is an deep groove (289) located circumferentially on the interior surface of the interface piece (251) approximate to the top edge of the piece. This deep groove (289) mates with a circumferential ridge (295) on the outside surface of the rotational collar (253). When the rotational collar (253) is fitted with the interface piece (251), the ridge (295) is allowed to rotatably glide within the groove (289) about a central axis (297). Both the interface piece (251) and the rotational collar (253) of this embodiment also have similarly sized central lumens (291, 293) extending along their longitudinal axis and which may be joined with the central lumen (275) of the associated wedge spine (203). Although the lumen opening (293) shown in Fig. 22N is circular, other embodiments may have openings of other shapes. For instance, Fig. 22O illustrates an opening having a square shape with rounded corners. Fig. 22P illustrates an opening having a triangular shape with rounded corners. Fig. 22Q illustrates an opening having a hexagon shape. It is contemplated that a plurality of other shapes may also be used for the openings of other embodiments.

The top section (288) of the rotational collar (253) includes four control element termination slots (301) to receive control elements routed from grooves (281) on the interface piece (251). In this embodiment, four slots (301) are arranged into a square shape. Each slot (301) is generally rectangular in shape and comprised of three substantially flat surfaces with one opened side as its top face. A control element (259) may be inserted into each of the slots and allowed to extend the length of the slot (301). At one end of each slot (301) is an enlarged circular notch (303) to receive the termination piece of its control element. In the embodiment shown in Fig. 22N, slots (301) are arranged such that each corner of the square shape are formed by similar slot ends - either both plain slot ends or both having a notch end. In one embodiment, each control element (259) is terminated with a metal solder ball or with a knot. Thus, when a control element (259) is laid into a slot, its termination piece may be seated into the circular notch for that slot and locked into place. The control element (259) is essentially locked together with the rotational collar (253). Thus, when the control element (259) is pulled at the distal end of the catheter, the tension is transferred along the length of that control element (259) through the spine (203) and interface piece (253) to the locked termination piece. That tension will cause the rotational piece (253) to move in the direction of the pulled controlled element. The curved path of the control element (259) causes the rotational collar (253) to rotatably slide about the interface piece (251) due to the control elements (259) traveling along the curved grooves (281) on the interface piece (251). The curved grooves of this embodiment serve to translate forces on the control elements (259) along the longitudinal axis of the catheter into partially transverse forces.

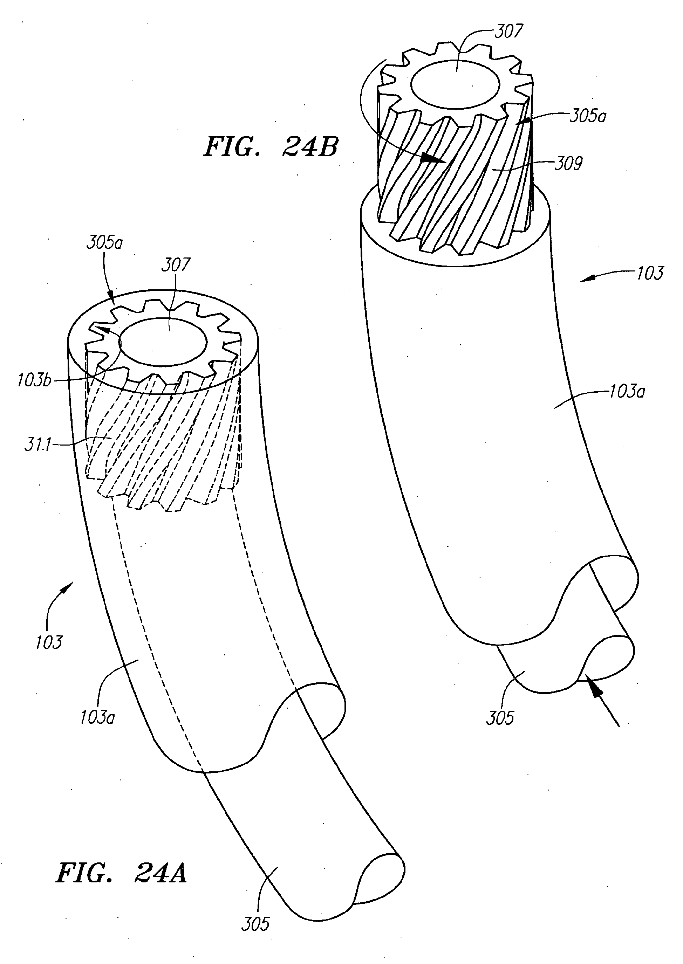

Figs. 24A-31K illustrate other alternative embodiments directed to actuation or drive elements of a distal end of a catheter (103) that result in rotation of the catheter, or simultaneous rotation and translation of the catheter or portion thereof. In this manner, the catheter or portion thereof and a working instrument may be controllably rotated.

Referring to Figs. 24A-B, in one embodiment, a catheter instrument member or assembly (103) of catheter instrument (e.g. as shown in Fig. 6D) of a robotic medical system includes an elongate catheter body (103a) and a catheter drive shaft (305) positioned within the lumen of the catheter body (103a). An inner surface (103b) of the distal end of the catheter body (103a) and an outer surface (305a) of a distal end of the catheter drive shaft (305) are operatively coupled or shaped such that axial displacement of the catheter drive shaft (305) relative to the catheter body (103a) causes a corresponding rotation of one of the drive shaft (305) and catheter body (103a) relative to the other. An orientation platform (e.g., as described with reference to Figs. 32A-59C) or a working instrument tool (41) (e.g., as shown in Figs. 23A-Z) may be mounted to the distal tip of catheter assembly 103 to controllably rotate and translate the platform or tool.

According to one embodiment, the outer surface of the distal end of the catheter drive shaft (305) and the inner surface of the distal end of the catheter body (103a) include complimentary threaded surfaces. In the embodiment illustrated in Figs. 24A-B, the threaded surfaces are helically threaded surfaces 311 including helical threads and helical teeth. In Fig. 24A, the distal portion of the flexible catheter body (103) is shown with the lower portion cutaway to expose an interior drive shaft (305), and only the top surface of the drive shaft (305) is visible, and helical teeth (309) on the distal end of the drive shaft (305) are hidden inside the instrument member (103) and represented as phantom lines. The outer surface of the helical distal portion 311 matingly engage a corresponding helically threaded inner surface such that the distal tip of the drive shaft (305) may be controllably extended beyond the distal tip of the shaft (305) (as shown in Fig. 24B) and be controllably retracted (as shown in Fig. 24A).

More particularly, when the drive shaft (305) is positioned inside of the catheter body (103a), the helical teeth and threads may be fitted together such that pushing the drive shaft (305) from its proximal end results in upward forces that move the shaft (305) upwardly. This axial motion also results in rotational motion due to the helically threaded surface (311) and corresponding helical teeth (309) of the drive shaft (305), resulting in translation of an upward force into a rotational force along the inclined surface. In other words, because the helical threads (311) are distributed about the inner shaft of the catheter body (103a), traversing the helical threads (311) results in rotation of the drive shaft (305) about the longitudinal axis (125), while the drive shaft (305) also translates upwardly.

In this manner, the drive shaft (305) may be translated upwardly such that its distal tip extends from the catheter body (103a), while being rotated in a counterclockwise direction (when viewed from the perspective of looking into the distal tip). Rotation in the opposite direction may also be utilized. The drive shaft distal tip (313) may also be retracted into the lumen of the catheter body (103a) (as shown in Fig. 24A), by pulling the drive shaft (305) downwardly, which causes the drive shaft (305) to rotate clockwise and translate downwardly along the helical surfaces (311). With embodiments, a user can robotically actuate simultaneous rotational and translational motion of the distal tip of a flexible catheter body (103a). Further, in certain embodiments, rotational interaction of the helical gear elements may also cause some rotational movement or twisting to occur on the drive shaft member below the helical gear arrangement.

The drive shaft (305) may also include a lumen (307) that extends from its distal end to its proximal end at the catheter splayer. The lumen may be used to house or deliver a cable connected to a working instrument or a control element.

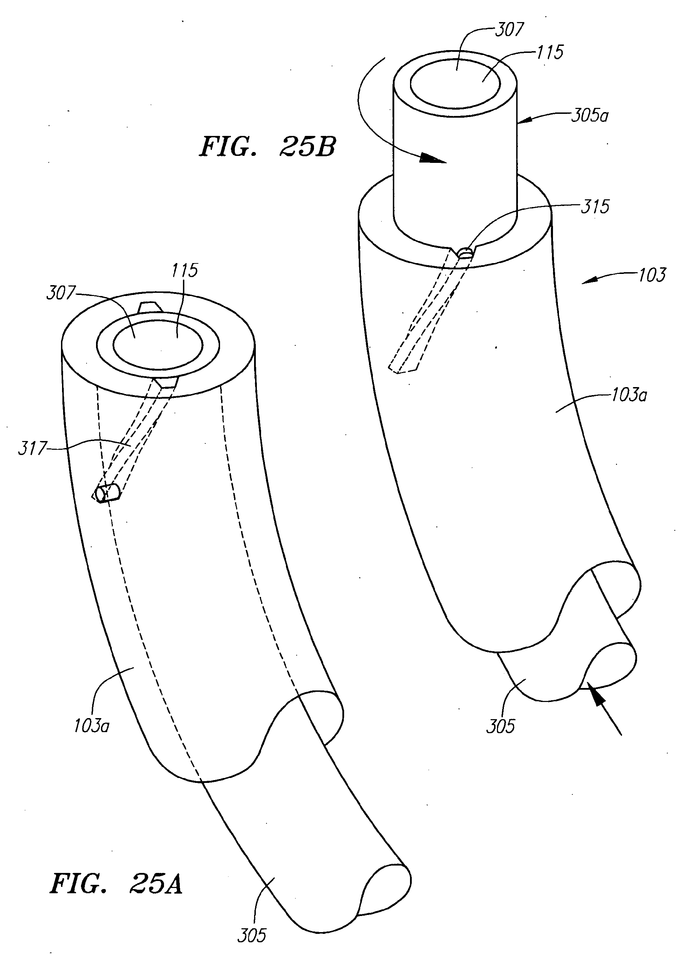

Figs. 25A-B illustrate a catheter assembly that operates in a similar manner as described with reference to Fig. 24A except that the embodiment shown in Fig. 24B includes a different type of translational / rotational drive element. In the embodiment illustrated in Figs. 25A-B, the outer surface of the distal end of the catheter drive shaft (305) and the inner surface of the distal end of the catheter body (103a) form a connector that is in the form of a Bayonet Neill-Concelman (BNC) connector or drive element.

As shown in Fig. 25A, the distal portion of a flexible catheter body (103a) is shown with the lower portion cutaway to expose and interior drive shaft (305). The drive shaft (305) of this embodiment is coaxially located in the central lumen (115) of the catheter (103a) along the longitudinal axis of the catheter (103). In one embodiment, the outer surface of the distal end of the catheter drive shaft (305) includes an outwardly extending pin (315), and the inner surface of the distal end of the catheter body (103a) defines an arcuate groove (317) or female mating surface configured to receive the pin (315). The female mating surface may include a spring that maintains a clamping force. More particularly, to couple the two surfaces, a pin 315 on the male surface is aligned with and inserted within a slot 317 on the female surface. Once the pin 315 reaches the bottom or end of the slots 317, the two surfaces may be turned in opposite directions to guide the pin 315 into a perpendicular slot that prevents or restricts removal of the pin 315 from the slot 317, e.g. utilizing one or more springs then hold the pin 315 in position within the slot 317 to prevent backing out of the pin 315. To disconnect the two surfaces, they are pushed together to overcome the springs, and the locking turn is reversed.

Thus, with such a BNC drive shaft or element 305, a user may be able to robotically actuate rotational and translational movements at the distal tip of a flexible catheter body (103a). In alternative embodiments, the female receptor slots317 on the inside surface of the catheter body (103a) may be configured to cause a clockwise rotation. Furthermore, in some embodiments, the rotational interaction of the male pin elements may also cause some rotational movement or twisting to occur on the drive shaft member below the bayonet connector arrangement. The drive shaft distal tip (313) may be controllably extended from and controllably retracted into the catheter body (103a) by pushing / pulling the drive shaft (305), thereby causing rotational and translational motion of the drive shaft (305).

An orientation platform, e.g., as described in Figs. 32A-59C, or a working instrument (41) may be mounted to the distal tip of the drive shaft (305). Further, the drive shaft (305) may include a lumen (307) extending from its distal end to its proximal end at the catheter splayer, e.g., for a cable to control a working instrument (41).

Referring to Figs. 26A-H, another embodiment is directed to a catheter assembly (103) of a robotic medical system includes an elongate catheter body or tubular body (321), an actuation element (319) coaxial with the tubular body (321) and positioned within the tubular body (321) lumen, and a control element (327), such as a pull wire, that extends through the tubular body (321). The actuation element (319) is coupled to an internal portion of the tubular body (321). Manipulation of the control element (327) causes the actuation element (319) and the catheter or tubular body (321) to rotate together.

According to one embodiment, as illustrated in Figs. 26A-H, the actuation element (319) is in the form of a ratchet drive or reciprocating pin / cam drive that rotates a distal segment of a tubular body (321). In Fig. 26A, the outer portion of the distal tip of the tube (321) is removed to illustrate the actuation element (319) (as represented by phantom lines). According to one embodiment, the actuation element (319) includes a gear, such as a helical gear (319), having a plurality of teeth and defining a plurality of corresponding grooves, a guide or track (325) (generally referred to as guide 325) that is disposed on an inner surface of the distal end of the tube (321) adjacent to the gear, and a pin (335) that is movable along the guide (325), e.g., within a slot defined by the guide (325). A control element (327) is attached to the pin (335) such that manipulation of the control element (327) results in movement of the pin (335) along the guide (325) and within a groove defined by teeth of the gear (319), resulting in rotation of the actuation element (319) and the tubular body (321). In the illustrated embodiment, rotation is counterclockwise (represented by arrow), but components can be configured for clockwise rotation.

Referring to Figs. 26A-D, in one embodiment, the helical gear (319) is affixed, attached or couple to a length of the catheter body or tube (321) having a ridge (331) that interfaces with a groove (333) inside of the catheter body (103a) at its first end. As the helical gear (319) and tube (321) rotate, the ridge (331) is allowed to move within the groove (333), thus allowing the tube (321) to also rotate coaxially within the catheter (103a). In the illustrated embodiment, the centers of the helical gear (314) and the tube (321) include a hollow portion or lumen that allows access to a lumen (115) defined by the catheter assembly

(103). An orientation platform or tool (not illustrated in Fig. 26A) may be mounted to the tube of this instrument member and controlled by running one or more control elements through the lumen (307) to the proximal end.

Figs. 26C-D are croas-sectional views illustrating the placement of a helical gear (319) and its associated pin (335). A pin (335) for actuating rotation of this helical gear (319) resides inside a slotted track (325) on the inside surface of the tube (321). The slotted track (325) in this embodiment has triangle shaped structure as illustrated in Fig. 26A. In this implementation, rotation of the helical gear (319) may be actuated by sequentially pulling and releasing a control element (327) coupled to the pin (323).

Fig. 26C shows the pin (335) at a first position on the slotted guide (325). As the pin (335) travels downwardly on the slotted guide (325) in response to the downward force on the control element (327), the helical gear (319) is caused to rotate counterclockwise (as viewed from the perspective of looking into the distal tip) as shown in Fig. 26D. However, the slotted guide (325), according to one embodiment, has a non-uniform thickness or depth.

More particularly, Fig. 26E is a cross-sectional, stretched out view of one embodiment a non-uniform surface (325a) of a guide (325). The bottom edge (325b) of the cross-sectional view of Fig. 26E represents the outer surface of the tube (321) or catheter body (103) of the catheter instrument assembly (103), and the top edge (325a) represents the uneven, non-uniform or undulating inner surface (325a). With this configuration, as the pin (335) traverses the surface (325a), e.g., within a slot formed in the guide (325) through which the pin (335) may extend, the pin (335) is caused to rise and drop in accordance to the undulating surface (325a).

Fig. 26B illustrates how the pin (335) extend outwardly to engage with threads of the helical gear (319) when the pin (335) is at a raised or thicker portion of the guide (325), and how the pin (335) withdraws into the sidewall of the catheter or tubular body (321) when the pin (335) is at a lowered or thinner portion of the guide (325).

Referring to Figs. 26F-H, movement of the pin (335) along the guide (325), and how the helical gear (319) is moved to the right (i.e., rotated counterclockwise in the example illustrated in Figs. 26A-D) as the pin (335) traverses along the slotted guide (325) is further illustrated. For reference, the lowercase letters identifying different portions of the surface (325a) of the guide (325) in Fig. 26E are provided in Figs. 26F-H to show how the pin (335) is extended and retracted relative to the helical gear (319).

In the illustrated embodiment, the pin (335) is configured to traverse or slide along the guide (325) in a single direction (as indicated by directional arrow in Fig. 26E). The taller or thicker the guide (325) surface, the more the pin (335) will extend outwardly from the sidewall of the catheter or tubular body (321) since the guide 325 is attached to, disposed on, or formed in an inner surface or side wall of the body (321) as shown in Fig. 26F.

Referring to Fig. 26F, assume, for example that the initial position of the pin (335) is position 'd' at which the pin (335) is forced outwardly and does not engage any teeth of the gear (319). In this example, force may be applied to a control element (327) to cause the pin (335) to move from position 'd' to position 'e', rounding the corner or vertex of the a guide that may have a triangular shape. As the pin (335) rounds the first vertex on the right side of the triangle approximately at position 'e', and with further reference to Fig. 26E, the pin (335) retracts into the sidewall and slides down the sloped track segment to position 'f'. More particularly, as shown in Fig. 26E, the height of the surface (325) at position 'd' is higher than position 'e' and, therefore, the pin follows the surface down to a lower level, thereby resulting in retraction of the pin (335). In one embodiment, movement of the pin (335) between positions 'e' and 'f' may be assisted by gravity. In another embodiment, the pin (335) may be biased with a spring force.

Downward force may be applied to the control element (327) to force the pin (335) outwardly from the sidewall when moving from position 'f' (which, in the illustrated embodiment, is at the same level as position 'e') to position 'a' at the second corner or vertex. More particularly, as the pin (335) traverse the surface (325a), the pin is extended outwardly as it approaches position 'a' at which point the pin (335) contacts a left side of a tooth, i.e. the third or middle tooth (identified by crosshatching) of the gear (319). By pulling the control element (327), the attached pin (335) is pulled along the guide (325) from position 'a' to position 'b'. In the embodiment illustrated in Fig. 26E, the level of the surface (325a) between positions 'a' and 'b' remains the same, and the pin (335) continues to engage the left surface of the third gear tooth element. As such, downward force along the left inclined face or surface of this tooth is translated into a rightward or rotational force that causes the gear (319) to move towards the right, as illustrated in Figs. 26G-H.

Referring to Fig. 26G, the gear (319) moves or rotates as the pin (335) traverses the guide (325) between positions 'a' and 'b'. But because the gear (319) is a wheel about the longitudinal axis of the catheter instrument, the gear (319) is caused to rotate towards the right (or counterclockwise) in this example. Referring to Fig. 26H, it can be observed that the second vertex of the slotted guide (325) is now positioned between the first and second gear teeth, whereas the second vertex was previously positioned between the second and third gear teeth before the gear rotation as shown in Fig. 26F. As the pin (335) moves past position 'b' and approaches the third vertex at position 'c', the pin (335) retracts into the sidewall and becomes disengaged from the gear (319) as a result of the change in the surface (325a) of the guide (325), as shown in Fig. 26E.

By releasing or slacking the control element (327), the pin (335) is allowed to travel from position 'c' to position 'd' while the pin (335) remains in a retracted position and out of contact from the gear (319). Upward movement of the pin (335) from position 'c' to position 'd' may be facilitated with a spring urging the pin (335) upwardly and thus also pulling the control element (327) upwardly. In one implementation, the control element (327) is biased with an upward force so that the pin (335) may be actuated by applying downward force as the control element (327) is pulled.

Although one embodiment has been described with reference to specific physical attributes of a gear (319) and non-uniform, undulating guide surface (325a), other embodiments may be implemented with other actuation element or gear designs, and other surface (325a) attributes. Further, in the illustrated embodiment, the guide (325) is triangular, but other shapes may also be utilized. Similarly, the particular surface (325a) profile and height levels of different portions of the surface (325a) may vary. For ease of explanation, however, one embodiment has been described with reference to an actuation element that includes a single pin (335), a guide (325) having a triangular shape, and a control element (327) in the form of a pull wire. Further, although embodiments are described with reference to the helical gear (319) rotating in a counterclockwise direction, the actuation element may also be configured such that the gear (319) rotates in a clockwise direction. Moreover, in other alternative embodiments, a distal portion of a catheter member or assembly (103) may include multiple actuation elements. For example, two actuation elements may be utilized, as shown in further detail in Figs. 27A-E.

Referring to Figs. 27A-E, according to another embodiment, a catheter assembly (103) of a robotic medical system constructed according to another embodiment includes an elongate catheter or tubular body (321), multiple actuation elements and multiple control elements (327). Portions of the actuation elements are coupled to internal portions of the body (321) such that rotation of the actuation element results in rotation of the catheter body (103a).

In the illustrated embodiment, a catheter assembly (103) includes two actuation elements that are positioned within the catheter or tubular body (321) and positioned within the body (321) lumen, one actuation element being positioned at the distal end of the catheter member (103) body. During use, one or both of the actuation elements are rotatable together with the catheter member (103) such in response to manipulation of at least one of the first and second control elements (327, 340).

In the illustrated embodiment, a the catheter assembly includes the same components as described with reference to Figs. 26A-H, except one actuation element includes a gear (319) configured to rotate in a first direction, and the other actuation element includes a gear (323) configured to rotate in a second direction, e.g., as a reversing or dual reciprocating pin / cam drive. During use, both gears (319, 323) may rotate independently of each other, one gear may be rotated at a time, or both gears may be rotated at the same time. In practice, meaningful movement at the distal tip may be obtained when one gear is rotated.

More particularly, referring to Fig. 27A, an outer portion of the distal tip is illustrated in phantom such that inner components of the apparatus are visible. In the illustrated embodiment, a first gear (323) is shown positioned coaxially inside of a central lumen of a flexible catheter or tubular body (123) just below the distal tip portion of the body (123). A second gear (319) is shown positioned coaxially inside of the tubular body (123), proximally of and coaxial with the first gear (323).

In the illustrated embodiment, the gears (319, 323) are helically threaded gears. Further, in the illustrated embodiment, the helical gears (319, 323) are attached or affixed to a length of tube (321) having a ridge (331) that interfaces with a groove (333) inside the instrument member at its first end and extends out the distal tip of the instrument member at its second end. As the helical gears (319, 323) and tube (321) rotate, the ridge (331) is allowed to move within the groove (333), thus allowing the tube (321) to also rotate coaxially within the instrument member. In this embodiment, the centers of the helical gears (319, 323) and tube (321) include a hollow portion that allows access to instrument lumen (307) from the distal tip. Although not illustrated here, an orientation platform or tool may be mounted to the tube of this instrument member and controlled by running one or more control elements through the lumen (307) to the proximal end.

Figs. 27B-C further illustrate the how first and second helical gears (329, 323) and their associated pins (335, 337) are configured. Actuation of a first pin (337) causes rotation of the first helical gear (323) in a counterclockwise direction (as viewed from the perspective of looking into the distal tip) as shown in Fig. 27B and indicated by a counter-clockwise directional arrow. Actuation of the second pin (335) causes rotation of the second helical gear (319) in a clockwise direction as shown in Fig. 27C and indicated by a clockwise directional arrow. Because both gears (319, 323) are affixed or attached to the tube (321), rotation of one gear causes the tube (321) and the other gear to also rotate in the same manner.

In the illustrated embodiment, the first pin (337) resides inside a first slotted track or guide (339) dispose on or formed within the inside surface of the catheter or tubular member (123), and the second pin (335) resides inside a second slotted guide or track (325). In the illustrated embodiment, the guides (325, 339) have the same shape and are triangle-shaped guides that face opposite directions. Each guide may function in the manner described with reference to Figs. 26A-H. n alternative embodiments, the slotted guides (325, 339) may have other shapes and orientation, and the guides may be the same or different shapes and sizes. For ease of explanation, the structure of the guides (325, 339) of the illustrated embodiment are similar to the guide (325) described in Fig. 26E.

During use, as pins (335, 337) traverse respective guides (325, 339), each pin rises and falls as it follows the non-uniform surface (e.g., surface 325a shown in Fig. 26E), of its guide. Rotation of a helical gear may be actuated by sequentially pulling and releasing a control element coupled to its pin. In the illustrated embodiment, control element or wire (340) is coupled to pin (337) carried by guide (339) and that engages gear (323), and control element or wire (327) is coupled to pin (335) carried by guide (325) and that engages gear (319).

Fig. 27B shows the first pin (337) driving the gear (323) in a counter-clockwise direction as the control element (340) is pulled downwardly, and the second pin (335) is disengaged from the second gear (319). Fig. 27C shows the second pin (335) driving the gear (319) in a clockwise direction as the control element (327) is pulled downwardly, and the first pin (337) is disengaged from the first gear (323). Figs. 27D-E further illustrate how the gears (319, 323) may be moved depending on whether respective pins (335, 337) engage the gear based on the guide surface (325a).

More specifically, Fig. 27D illustrates how the first helical gear (323) is moved to the right (or rotated counter-clockwise in the context of Figs. 27A-C) as a first pin (337) traverses the guide or track (339), and a second pin (335) is disengaged from the second gear (319). In the illustrated embodiment, the first pin (337) is configured to travel in a single direction along the first track (339) as is noted in Fig. 27D by a directional arrow. As discussed above with reference to the track of Fig. 26E, the taller or thicker the surface (325a) of the guide (325), the more the pin will extend outwardly from the sidewall of the catheter or tubular member (321) to engage the gear (323).

With further reference to Fig. 26E, in the illustrated example, assuming the first pin (337) is initially positioned at 'd' (at which the first pin (337) is forced outwardly to engage the gear (323). Moving the pin (337) from position 'd' to 'e' results in the pin (337) rounding the first corner or vertex on the right side of the triangle-shaped guide (339). As a result, the first pin (337) slides down the sloped guide surface (325a) to a lower level, resulting in retraction of the pin (337) from the gear (323) and remains at this level between positions 'e' and 'f'. Application of downward force to the first control element (340) forces the first pin (337) to move along the guide (339) from position 'f' to position 'a' thereby resulting in the pin (337) being extended outwardly from the sidewall of the catheter or tubular body (123). At position 'a', the pin (337) is extended to engage the gear (323). In the illustrated example, the pin (337) contacts the left hand surface of the fifth gear tooth element (shown with crosshatching) on the first gear (323). By pulling the first control element (340), the attached first pin (337) is pulled along the guide (339) from position 'a' to position 'b'. As the first pin (337) traverses the guide (339) between positions 'a' and 'b', the pin (337) engages with the left surface of the fifth gear tooth element and the downward force along the left surface is translated by the inclined, angled or helical tooth surface into a rightward that causes the first gear (323) to move towards the right and rotate.

Thus, because the first gear (323) is a wheel-like structure that is movable about the longitudinal axis of the catheter or tubular body (123), the first gear (323) rotates counterclockwise in this illustrated example. Upon the first pin (337) reaching position 'b' on its guide (339), the second vertex of the first guide (339) is now positioned between the third and fourth gear teeth, whereas the second vertex was previously positioned between the fourth and fifth gear teeth before gear (323) rotation. As the first pin (337) traverses the guide (339) and moves past position 'b' and approaches the third vertex at position 'c', the first pin (337) retracts into the sidewall of the catheter or tubular body (123) and disengages the first gear (323). By releasing or slacking the first control element (340), the first pin (337) is allowed to travel from position 'c' to position 'd' while the first pin (337) is out of contact from the first gear (323).

The second gear (319) is moved by a second slotted guide or track (325) in a similar manner, except that in this example, the teeth of the gear (319) and the guide (325) are oriented in a different manner such that the gear (319) rotates clockwise as the second pin (335) traverses the second guide (325), and the first pin (337) disengages from the first gear (323). Thus, the rotational direction of the catheter or tubular member (321) may be reversed relative to rotational motion resulting from the first gear (319) by the second gear (319). In this embodiment, the second pin (335) is also configured to travel in a single direction along the second guide (325) as shown by a directional arrow in Fig. 27E. For ease of explanation, and given the similar structural configurations shown in Figs. 26A-H and Figs. 27A-E, further details regarding the manner in which the second pin (335) traverses the guide (325) are not repeated.

In this manner, a distal tip of a catheter member or assembly (103) may be controllably rotatable. Further, depending on which gear is rotated, a tool or orientation platform mounted to the distal tip of the catheter member (103) may also be controllably rotatable.

Figs. 28A-C illustrate another embodiment of a catheter assembly of a robotic medical system that includes a harmonic drive element (341) that may be used to rotate a segment, such as the distal end, of a catheter member (103) or catheter body or tube (123). In the illustrated embodiment, a harmonic drive element (341) includes a harmonic wave generator (343), a flexible spline or gear (345) and an outer circular spline or gear (347). The harmonic wave generator has an elliptical shape and is rotatable within a bore of the flexible spine 345 to impart an elliptical shape to the flexible spline 345, which is positioned within a bore of the outer or circular spline (347). Components of the harmonic drive element (341) may be made of stainless steel, plastic, polycarbonate, aluminum, copper, metal and other suitable materials. The manner in which the harmonic drive element functions may be based on principles involving high mechanical leverage being achieved by generating a traveling deflection wave in a flexing spline element.

In the illustrated embodiment, the wave generator (343) is an elliptical cam that is enclosed within an anti-friction ball bearing assembly and functions as a rotating input element. For this purpose, the wave generator (343) may be coupled to a primary power source or servomotor (not shown in Figs. 28A-C). As the servomotor operates, the wave generator (343) serves as a high efficiency torque converter. More particularly, when the wave generator (343) is inserted into the bore (349) of the flexspline (345), the wave generator (343) imparts its elliptical shape to the flexspline (345), thereby causing the external teeth (351) of the flexspline (345) to engage with the internal teeth (353) of the circular spline (347) at locations. In the illustrated embodiment, these locations are at opposite ends of the wave generator (343), i.e. separated by 180o, thus forming a positive gear mesh at these engagement points. In another embodiment, the wave generator (343) may be an assembly comprising a bearing and a steel disk known as a wave generator plug. The ball bearing is pressed around the carefully machined elliptical shape of the wave generator plug, causing the bearing to conform to the same elliptical shape of the wave generator plug. For ease of explanation, reference is made to the structural configuration shown in Figs. 28A-C.

The flexspline (345) according to one embodiment is a flexible, thin-walled cylindrical cup with gear teeth that are machined into an outer surface of the flexspline (345) near the open end of the cup near the brim. This structural configuration allows the walls of the cup to be radially compliant, yet remain torsionally stiff as the cup has a larger diameter. In the illustrated embodiment, the flexspline (345) is slightly smaller in circumference and has two less teeth than the circular spline (347). The cup in Fig. 28A has a rigid boss at one end to provide a rugged mounting surface. For this example, a platform, such as an orientation platform on which a tool may be mounted, is coupled to the flexspline (345).

The circular spline (347) may be a thick-walled, rigid ring with internal spline teeth. The circular spline (347) is usually attached to the housing and often functions as the fixed or non-rotating member, but may be utilized as a rotating output element as well in certain applications. Although the flexspline (345) is often the rotating output element as in this implementation, it can also be utilized as a fixed, non-rotating member when output is through the circular spline (347).

During assembly of the harmonic drive element (341), the wave generator (343) is inserted inside the flexspline (345) such that the bearing is at the same axial location as the flexspline teeth (351). The flexspline (345) wall near the brim of the cup conforms to the same elliptical shape of the bearing, thus causing the teeth (351) on the outer surface of the flexspline (345) to conform to this elliptical shape. Effectively, the flexspline (345) now has an elliptical gear pitch diameter on its outer surface. The circular spline (347) is located such that its teeth (353) mesh with those of the flexspline (345). The now elliptical tooth pattern of the flexspline (345) engages the circular tooth profile of the circular spline (345) along the major axis of the ellipse, in a manner that is similar to an ellipse inscribed concentrically within a circle. Figs. 28B-C illustrate cross-sectional views of the harmonic drive element (341) relative to cross section B-B. An inscribed ellipse will contact a circle at two points; however, as a practical matter, the gear teeth of this embodiment have a finite height so there may be two regions of teeth engagement instead of simply two points. Moreover, in other embodiments, approximately 30% of the teeth may be engaged at all times.

The pressure angle of the gear teeth transforms the tangential force of the output torque into a radial force that acts upon the wave generator (343) bearing. The teeth of the flexspline (345) and circular spline (347) are engaged near the major axis of the ellipse and disengaged at the minor axis of the ellipse. Referring to Fig. 28B, as the wave generator (343) begins to rotate in a clockwise direction in response to its servomotor, a continuously moving elliptical form or wave-like motion is imparted to the flexspline (345). An initial position (335) on the flexspline (345) is marked with a small arrow in Fig. 28B. This motion causes the meshing of the external teeth (351) of the flexspline (345) with the internal teeth (353) of the circular spline (347) at their two equidistant points of engagement and allows for a full tooth disengagement at the two points along the minor axis of the wave generator (343). Thus the zones of tooth engagement travel with the major elliptical axis of the wave generator (343).

When the wave generator (343) has rotated 180o clockwise, the flexspline (347) has regressed by one tooth relative to the circular spline (347). In this embodiment, each complete revolution of the wave generator (343) displaces the flexspline (345) two teeth counter-clockwise relative to the circular spline (347). Fig. 28C illustrates the displacement of the marked position (355) on the flexspline (345) relative to Fig. 28B in a counter-clockwise direction in response to clockwise revolutions of the wave generator (343). This displacement is in the opposite direction of the rotation of the wave generator (343) such that if the wave generator (343) of this example rotates in a counter-clockwise direction, then the two tooth per revolution displacement of the flexspline (345) will be in a clockwise direction.

A harmonic drive element (341) may also allow for finer rotational control of a distal platform coupled thereto since this type of drive element also functions as a speed reducer. In contrast to high speed input from a power source to the wave generator (343), the considerably slower flexspline (345) causes a two-tooth per revolution displacement. The resulting reduction ratio may be calculated by dividing the number of teeth on the flexspline (345) by the difference between the number of teeth on the circular spline (347) and the flexspline (345) as follows:

$$\text{Reduction Ratio} = \frac{\# \text{teeth}_{\text{Flexspline}}}{\# \text{teeth}_{\text{Flexspline}} - \# \text{teeth}_{\text{Circular Spline}}}$$

In this example, the reduction ratio is calculated as:

$$\text{Reduction Ratio} = \frac{\#\,\text{teeth}_{\text{Flexspline}}}{\#\,\text{teeth}_{\text{Flexspline}} - \#\,\text{teeth}_{\text{Circular Spline}}} = \frac{98}{98-100} = -49:1$$

The negative sign in the above expression indicates that the input and output are turning in opposite directions. It is contemplated that the reduction ratio in other embodiments will be different as the difference between the number of teeth of the flexspline (345) and the number of teeth of the circular spline (347) may vary.

Figs. 29A-E illustrate another embodiment of a catheter assembly of a robotic medical system that includes an elongate catheter or tubular body and a wobble plate drive element (357) that is coaxial with the catheter body and located at the distal end of the catheter body. The wobble plate drive element (357) is operable to rotate a segment, such as the distal end, of the catheter body. As with other embodiment discussed above, including the ratchet-type drive element, embodiments of a wobble plate drive element (357) may be positioned at a distal tip of a flexible catheter instrument member and utilized to controllably rotate a segment of the catheter.

According to one embodiment, a wobble plate drive element (357) includes a rotatable shaft (367), a first, stationary gear element (361), a second gear element (359) that is coaxial with the shaft (367) and rotatable about the first gear element (361) and around the shaft (367), a compression element, such as a spring (363), disposed between the first and second gear elements (361, 359) that urges the second gear element (359) away from the first gear element (361), and a cam drive member or element (365) configured to manipulate or rotate the second gear element (359) to urge a portion of the second gear element (359), against the force of the spring (363), to engage a portion of the first gear element (361), while an opposite portion of the second gear element (359) does not engage the first gear element (361). In the illustrated embodiment, the first and second gear elements (361, 359) may be in the form of gear plates, which may be made of stainless steel, plastic, polycarbonate, aluminum, metal, and other suitable materials.

The drive shaft (367) may extend down into a central lumen of a catheter or other instrument member to a power source, such as a servomotor, at the proximal end of the catheter. In some embodiments, a micro-motor may be employed proximate to the wobble plate drive element (357) itself.

The cam drive element (365) shown in Fig. 29B, according to one embodiment, includes an angled arm or finger element (369) that is secured to the end of the drive shaft (367) such that when the drive shaft (367) rotates, the arm or finger element (369) also rotates together with the shaft (367) and in the same direction. The arm or finger element (369) is in contact with a portion of a top surface of an upper or distal gear element (359), which is coaxially located about the drive shaft (367) and includes a plurality of teeth or gear elements extending proximally towards the first, stationary gear element (361). According to one embodiment, the gear element (359) includes 100 teeth, and includes more teeth than the other gear element (361). Although the drive shaft (367) passes through the center of the first gear element (359), the drive shaft (367) is configured to freely rotate without directly causing rotational movement of the first gear (359).

Also coaxially located about the drive shaft (367) and below the first gear element (359) is the second, bottom gear element (361) that is stationary and has a plurality of teeth. For example, the second gear (361) may be attached or affixed to a catheter or other instrument. According to one embodiment, the gear element (361) includes 98 teeth on a top surface thereof, i.e., less than the other gear element (359). The spring (363) coaxially located about the drive shaft (367) between the first gear (369) and the second gear (361) serves to urge the two gears apart.

Figs. 29C-E illustrate how the wobble plate drive element (357) functions during use. To engage the drive element (357), a combination of tensional and rotational forces may be imparted onto the drive shaft (367). By pulling the drive shaft (367) in downward direction, the resulting tensional force causes the arm or finger element (369) to press down on a portion of a top surface of the first gear (359), which serves to compress the spring (363). As the requisite amount of downward force is supplied, a portion of the teeth on the first gear (359) positioned below the arm or finger element (369) engage and mesh with certain teeth on the second gear (361). In Fig. 29C, for example, the teeth on the left sides of the gear elements (361, 359) are engaged, whereas teeth on the other side are not engaged. During use, the shaft (367) is rotated in either a clockwise or counterclockwise direction which, in turn, causes the arm or finger element (369) to turn about the central axis of the drive element (357), as generally represented by a curved arrow in Fig. 29C. In the illustrated example, the drive shaft (367) rotates counter-clockwise (as viewed from the top of the device). The associated counter-clockwise rotation of the arm or finger element (369) causes a tip (371) to circle about and press down the top surface of the first gear (359). Because the first gear (359) is tilted relative to the second gear (361) (due to the spring (363) exerting upward force on other portions of the gear element (359)), this motion causes the first gear (359) to "wobble" over the second gear (361). As the tip (371) continues to circle about the gear element (361), the wobbling action forces the different portions of teeth from the first gear (359) and the second gear (361) to temporarily engage or mesh as the incline on the first gear (359) changes as shown in Figs. 29C-E.

Further, because the first and second gears (359, 361) have a different number of teeth and full tooth disengagement is achieved, each complete revolution of the tip (371) results in a predetermined displacement between the two gears (359, 361) in the opposite direction of the rotation. In one embodiment, the second gear (361) has two less teeth than the first gear (359) such that a two tooth displacement in a clockwise direction is obtained with each complete counter-clockwise revolution, resulting in rotational motion as the top gear element (359) wobbles over the bottom gear element (361). Although embodiments are described with reference to gear elements (361, 359) having 100 and 98 teeth, respectively, other embodiments may involve gear elements having different numbers of teeth. Further, the teeth number difference may also vary such that the wobble effects and reduction ratios can be adjusted.

The first gear element (359) may be coupled to a distal tip platform or orientation platform on which a tool may be deployed. In this manner, the rotational motion generated by the wobble plate element can be imparted to the platform or tool. Further, in another embodiment, a lumen may extend through the drive assembly to allow a cable to link to a working instrument or provide a passage of another catheter device or fiber.

Referring to Figs. 30A-D, a wobble plate drive element (357) constructed according to another embodiment is similar to the embodiment shown in Figs. 29A-E except that rather than using a cam drive 365 as shown in Figs. 29A-E, this embodiment actuated through the sequencing of control elements or tension cables (373). Referring to Fig. 30A, and similar to the components discussed above, the wobble plate drive (357) includes a first gear plate (359), a compression spring (363), a second gear plate (361), and a central shaft (375). The first gear (359) has a set of teeth on its bottom surface and the second gear (361) has a set of teeth on its top surface. The number of teeth on the first gear (359) differs from the number of teeth on the second gear (361). The first gear (359) and the second gear (361) are each coaxially coupled with the central shaft (375), with the spring located on the coaxially on the shaft between the two gears (359, 361). The spring (363) serves to urge the two gears apart.

A set tension cables (373), e.g., six tension cables (373) labeled 'A' through 'F', are distributed about the circumferential edge of the first gear element (359). Each tension cable (373) is connected to the first gear element (359) at one end while the other end extends downwardly to a proximal end of a catheter through a catheter lumen. In one embodiment, each tension cable (373) is routed through its own individual lumen defined in a sidewall of a catheter or other instrument. In another embodiment, one or more tension cables may be grouped together and routed through a central lumen. For ease of explanation, reference is made to tension cables (373) that are attached to equidistantly spaced locations on the top gear element (359).

With this configuration, and as with the wobble drive element (357) shown in Figs. 29A-E, a platform or working instrument coupled to the wobble drive element (357) shown in Figs. 30A-D is rotated by wobbling the first gear (359) on top of the second gear (361). With this example, a user sequentially tensions each cable (373) by pulling each cable downward with enough force to overcome the spring (363) and to cause a portion of the gear teeth on the first gear (359) proximate to that particular cable to mesh with a portion of the teeth underneath on the second gear (361). During operation of the drive (357), the cables (373) are sequentially tensioned in either a clockwise or counterclockwise direction. Fig. 30A illustrates how the tension cables are sequenced in counterclockwise manner (when viewing the drive from above) with a pattern of "A-B-C-D-E-F-A". In response to this counterclockwise sequencing of the cables (373), the first gear (359) gradually becomes displaced in a clockwise direction relative to the second gear (361). For a clockwise sequencing, the displacement would be in a counterclockwise direction.

Figs. 30B-D illustrate the displacement of the first gear (359) in response to the sequential tension of the cables (373). As indicated by the arrows pointing down in Fig. 30B-D, cables 'A', 'B', and 'C' are each pulled downward to tilt the first gear (359) as it wobbles over the second gear (361). Because the first and second gears (359, 361) have a different number of teeth and full tooth disengagement is achieved, each complete revolution of the first gear (359) results in a predetermined displacement between the two gears (359, 361) in the opposite direction of the wobbling and cable sequencing, thereby resulting in rotational motion.

Referring to Fig. 31, a catheter assembly of a robotic medical system constructed according to another embodiment includes an elongate catheter body having a proximal end and a controllable and flexible distal end, the catheter body having a longitudinal axis and defining a lumen, and a planetary gear drive element (377) that is coaxial with the catheter body and located at the distal end of the catheter body. The planetary gear drive element (377) is operable to rotate a segment, such as the distal end, of the catheter body and any platform or working instrument attached thereto.

A planetary gear element (377) constructed according to one embodiment includes at least three components: a central sun gear (379), one or more planet gears (381) of the same size, and a ring gear (383). The various drive components may be made of stainless steel, plastic, polycarbonate, aluminum, metal, etc. or combinations thereof, but are not such restricted.

The sun (379) and planet gears (381) are located inside the ring gear (383), which may also be referred to as the annulus. Because the entire planetary gear element (377) is only as large as the largest gear, the system may be very compact. The teeth of the ring gear (383) are located on an inside surface such that they can mesh with the planet gears (381) within the ring gear (383). In this embodiment, gear teeth of all of the gears are clearly visible. In some embodiments, the gear teeth may be of smaller dimensions or knurls may be implemented in lieu of teeth.

The sun gear (379) is coaxially located in the center of the ring gear (383). Located between the sun gear (379) and the ring gear (383) are the one or more planet gears (381), whose gear teeth mesh with the teeth both the sun (379) and the ring (383). When a plurality of planet gears (381) are used in such a drive, there are several points of contact where the teeth on the planet gears (381) mesh simultaneously with those of the two coaxial gears (379, 383). The more teeth that are meshed, the strong the arrangement is and the greater the ability to handle very high torques. In the illustrated embodiment, planet gears (381) are held into place by a disc or planet carrier, and are free to turn on pinions (382) that attach the planet gears (381) to the planet carrier. Although not shown in Fig. 31, the planet carrier is located coaxially with the sun gear (379) and the ring gear (383). In some instances, a planetary gearing system may also be referred to as an epicyclic gearing system.

A planetary gear drive element (377) may be implemented using a number of configurations. For example, each of the three components can be the input, the output, or held maintained as stationary. Thus, there are six possible combinations, although three of these provide velocity ratios that are reciprocals of the other three. Choosing which piece plays which role determines the gear ratio for the gearset. Locking any two of the three components together will lock up the whole device at a 1:1 gear reduction. The ratio of input rotation to output rotation is dependent upon the number of teeth in the ring gear (383) and the sun gear (379), and upon which component is held stationary. However, the ratios are independent of the number of planets (381) or the number of teeth on each planet (381).

During operation of the drive in one implementation, input power drives one member of the assembly, a second member is driven to provide the output, and the third member is fixed. If the third member is not fixed, no power is delivered. For one configuration, the sun gear (379) is used as the input, the planet carrier is locked in position so it cannot rotate but its planet gears (381) can rotate on their pinions (382), and the ring gear (383) is the output. In this case, the ring gear (383) will rotate in the opposite direction from the sun gear (379), and the gear ratio will be the ring gear over the sun gear (379):

$$\text{Gear Ratio} = -\frac{\#teeth_{Ring}}{\#teeth_{Sun}}$$

For another configuration, the sun gear (379) is used as the input, the ring gear (383) is held stationary, and the planet carrier is used as the output, with the planet carrier rotating in the same direction as the sun gear (379). The resulting ratio is:

$$\text{Gear Ratio} = 1 + \frac{\#teeth_{Ring}}{\#teeth_{Sun}}$$

because the planet carrier has to circle the sun one additional time in the same direction it is spinning. Furthermore, in other embodiments, planetary gear drive elements (377) may include different number of teeth, and the pitch of the various gear teeth may also vary in different embodiments.

Referring again to Fig. 31, the ring gear (383) or annulus is mounted coaxially in the central lumen of the catheter instrument member (103). In one embodiment, the ring gear (383) may be fixedly coupled to the sidewall of the catheter instrument member (103) such that ring gear (383) and catheter instrument member (103) rotate or move together. In another embodiment, the ring gear (383) may be held into place in the catheter instrument member (103) with a set of retaining rings or grooves. In yet another embodiment, the ring gear (383) may be built into the sidewall such that the teeth of the ring gear (383) jut out of the sidewall. In this example, the sun gear (379) is illustrated with a counterclockwise rotation on its shaft whereas the three planets (381) rotate clockwise on their pinions (382). Because of these rotational movements, the ring gear (383) is caused to rotate in a clockwise direction. By reversing the direction of rotation at the input, the directions of all these components become reversed also.

Because of the varying gear ratios that can be achieved from the different combinations, it may be possible to achieve an output speed that is slower than the input speed, an output speed that is faster than the input speed, or an output direction that is reverse from the input direction. Although the planetary gear drive elements (377) disclosed are in the context of a single drive unit, in other embodiments, a planetary gear drive element (377) may include multiple stages. For example, multiple planet and sun gear units may be placed in series within the same ring gear housing such that the output shaft of the first stage becomes the input shaft of the next stage, thus providing a larger (or smaller) gear ratio. In the present implementation, any of the ring gear (383), planet carrier, or the sun gear (379) may be coupled to a distal tip platform or orientation platform on which working instrument or tool may be deployed. In another embodiment, a lumen may extend through the drive assembly to link with a catheter or instrument member central lumen to allow passage of another catheter device or fiber.

Whereas each of the components in Fig. 30 includes a set of teeth to mesh with other gears, the sun member (385) and the ring member (387) of the implementation illustrated in Figs. 31A-K are tubular lengths of shafts without teeth. The four planet gears (381) illustrated in Fig. 31A are fabricated with knurled patterns. In the illustrated embodiment, the planet gears (381) have straight patterns as shown in Fig. 31C. In other embodiments, the knurled surface may have a pattern similar resembling diamond-shapes (crisscross), bumps, straight ridges, helices, or combinations thereof.

Furthermore, a planet gear (381) may also be manufactured with an irregular gripping surface. With this configuration, knurled surfaces (384) of the planet gears (381) grip or bite into the surfaces of the sun member (385) and the ring member (387) as the planet gears (381) rotate, thus causing the sun member (385) and the ring member (387) to also rotate. The components of this planetary gear drive element (377) are assembled together in a manner such that the planet gears (381) are sufficiently tight against both the sun member (385) and the ring member (387), but still allowing for rotational motion by the planet gears (381).

In this embodiment, the motor input is provided through the planet gears (381), the central shafts of which are flexible and extend downwardly through the catheter or instrument member to a motor block at the proximal end of the catheter instrument. Thus, by rotating these axles at a proximal location, the planet gears (381) may be driven to rotate at a distal location. These central shafts of one embodiment are flexible, sleeved cables such as speedometer cables. In another embodiment, the motor input may be provided through a planet carrier via the planet gears (381).

As shown in Fig. 31A, a first dot on the ring member (387) marks its starting position and a second dot on the sun member (385) marks its starting position. Figs. 31C-D illustrate cross-sectional views of the drive assembly within a flexible instrument member. As the planet gears (381) begin to turn in a counterclockwise rotation as shown in Figs. 31A and 31C, the sun member (385) beings to rotate in a counterclockwise direction and the ring member (387) turns in a clockwise direction. Referring now to Fig. 31B, the sun member (385) and ring member (387) can both be seen slightly rotated in response to the revolving planet gears (381) as the marks have shifted counterclockwise and clockwise, respectively.

As shown in Fig. 31D, a platform is attached to the sun member (385) in this example, but in alterative embodiments, any of the ring member (387), planet carrier, or the sun member (385) may be coupled to a distal tip platform or orientation platform on which a working instrument or tool may be deployed. In another embodiment, a lumen may extend through the drive assembly, as with the sun member (385) of Fig. 31D, to link with an instrument member central lumen to allow passage of another catheter device or fiber.

The planetary gear drive element (377) shown in Fig. 31D is built into its own flexible catheter instrument member (103) and has been inserted into through the lumen (115) of the catheter member (103) and locked in position when the sun member (385) is installed. Thus, in this embodiment, the planetary gear drive element (377) may be removed from the distal tip of the catheter instrument member (103), if desired, by extracting the sun member (385) from the assembly.

Various planetary drive element components of different embodiments may be constructed out of stainless steel, plastic, polycarbonate, aluminum, metal, etc. or combinations thereof, but are not restricted as such. Component materials may be selected so that the knurled surfaces (384) of the planet gears (381) are able to firmly grip or bite into the surfaces of the ring member (387) and the sun member (385). Further, although the planetary gear drive element (377) components in one embodiment may be designed with the same height dimensions at their contact surfaces, in other embodiments, the components may be fashioned with different heights so long as the desired rotational actions and drive functionality are achieved. For example, the various components of the drive assembly shown in Figs. 31C-D may not necessarily have the height dimensions. The sun member (385), planet gears (381), and ring member (387) each have a different height in Fig. 31C. In Fig. 31D, the planet gears (381) and the ring member (387) are of one height while the sun member has a different height.

Figs. 31E-K illustrate a planetary gear drive element (377) constructed according to another embodiment. Figs. 31E-F are perspective views of this embodiment without a catheter instrument, but as with the various drive assemblies disclosed in this document, embodiments may be installed into or at the distal tip of a flexible catheter instrument member in order to rotate a platform, tool, or segment of a catheter instrument. The planetary gear drive element (377) of this embodiment is also constructed with a sun band piece (389), four planet gears (381), and a ring band piece (391). More specifically, the sun piece (389) is coaxially located inside the ring piece (391) and the planet gears (381) are located between the sun piece (389) and the ring piece (391). Each of the planet gears (381) are in simultaneous contact with sun piece (389) and the ring piece (391). The planet gears (381) of this implementation are held into place with the drive assembly with a pair retention discs (393) and collars on the planet gear drive shafts (382).

As shown in Fig. 31K, a sun band piece (389) may include a through lumen and an offset lip about its circumferential edge. In other embodiments, the sun band piece (389) may or may not include one or more physical characteristics such as a lumen, ridges, grooves, etc. Two retention discs (393), which also serve as part of the planet carrier in this embodiment, are shown in Fig. 31G. Fig. 31J illustrates a closer view of a retention disc (393) with a plurality of circumferential holes (395) through which planet gears (381) may be positioned and a central through hole (397) that overlaps with the sun band through lumen. Depending on the particular design, one or more of the holes (395) may be left vacant if the number of planet gears needed is fewer than the number of holes. In one embodiment, a retention disc (393) may be fabricated to include only the needed number of holes. A first retention disc (393) fits over the top portion of the drive assembly (377) and the second disc (393) fits over the bottom portion of the drive assembly, thus sandwiching the sun piece (389), ring piece (391), and the planet gears (381). The present example includes four planet gears (381), but it is contemplated that more or less planet gears (381) may be used in other embodiments. Fig. 31L illustrates one embodiment of a planet gear component (381) constructed in this manner.

In this embodiment, each planet gear component (381) is comprised of shaft member (382) having a gear portion (384) knurled with a straight pattern about a first end and a hole to receive a dowel pin about a second end. The hole or aperture in Fig. 31L is transverse to the longitudinal axis of the shaft member and allows for the dowel pin to pass completely through the shaft. In one embodiment, a flexible cable such as a speedometer cable is coupled to the shaft member via the dowel pin. In another embodiment, the cable may be fastened to the shaft by a clamp collar. Alternatively, a cable may be threaded through the hole and held into place with a solder ball or a knot. Sandwiching the knurled gear portion (384) of the shaft member are ridged sleeves, both of which assist with keeping the retention discs together (393). The ridge sleeve in some embodiments may be a cap, clamp, collar clamp, lock washer, ring, or any fastener which may lock into position on the shaft member.

Fig. 31I illustrates one example of such a planetary gear drive element (377). In assembling the drive of one embodiment, the sun piece (389) has a lipped portion seated with a central hole or aperture of a retention disc (393). Planet gears (381) are inserted through the designated circumferential holes of that retention disc (393) and held into place with clamp pieces (399). A ring band is fitted onto the retention disc (393) around the planet gears (381) and sun piece (389). A second retention disc (393) is placed over this subassembly, with the planet gears (381) aligning with and fitted through circumferential holes of this second retention disc (393). Additional clamp pieces are fastened onto the planet gear pieces (382) to hold this retention disc (393) to the other pieces. The planet gear shaft members (382) may be coupled to a motor block for providing input via flexible drive cables. The drive may now be coupled with a flexible instrument member to provide rotational action.

Figs. 32A-P illustrate embodiments of an interface or orientation platform (401) for controlling a working instrument (41) (one example of which is illustrated) coupled to a distal end of a catheter instrument (37) (e.g. as shown in Fig. 6D) or other instrument assembly (3) of a robotic medical system, e.g., a sheath (39) covered catheter (37). According to one embodiment, an interface or platform (401) includes a base member or socket plate (417) configured for coupling to a distal end of catheter instrument member (103), a spacer element (419) and another socket plate or platform member (415). The spacer element (419) is retained or interposed between, and separates, the base member (417) and the platform member (415). The platform member (415) is movable relative to the base member (417) about the spacer element (419). The interface or platform (401) also includes a control element (405), such as a pull wire, that extends through the catheter member (103), through an aperture defined by the base member (417), and terminating at the platform member (415).

Embodiments may be utilized to control an orientation of the platform member (415) and an orientation of the working instrument (41) are controllably adjustable by manipulation of the control member (405). For example, in the embodiment shown in Figs. 32A-C, a catheter assembly (3) includes a first flexible catheter instrument (37) coaxially disposed in a flexible sheath instrument (39). A tool actuation cable (403) and a platform control element (405) are routed through one or more lumens inside the instruments (37) to a proximal portion of the assembly (3). An interface or platform (401) servers as a controllable interface between the distal end of the catheter (37) and the working instrument (41).

More particularly, in the illustrated embodiment, an interface or orientation platform (401) is shown coupled to the distal tip of the catheter instrument member (103). A mating ring (407) is provided for attaching a working instrument or tool (41) to the orientation platform (401), and the tool (41) may be coupled to the mating ring (407). In the illustrated embodiment, the mating ring (407) includes a pair receptors with female slots (409) to engage with a pair corresponding male pins (411) located on the tool (41), and in one embodiment, the fastening mechanism for removably connecting the tool (41) to the instrument member (103) in this example is a type of bayonet mount.

To install a tool (41), pins (411) on the male side are aligned with the slots (409) on the female receptor and the two surfaces are pushed together. Once the pins (411) reach the end of the slots (409), the two surfaces are turned in opposite directions to guide each pin (411) into a perpendicular portion of the slot (409) that prevents it from slipping. A spring in the mating ring (407) maintains a clamping force at the mating surfaces. To disconnect the tool (41), the two surfaces are pushed together to overcome the spring force and the locking turn is reversed. A tool actuation cable (403) with a eyehook at one end connects to the tool (41) in this implementation and is used to control the opening and closing action of the grasping tool. As shown in Fig. 32C, this actuation cable (403) passes through the mating ring (407), a lumen (413) in the orientation platform (401), and the catheter instrument member (103) to a control knob or motor at the proximal end of the catheter assembly (3).

According to one embodiment, as shown in, for example, Figs. 32D-E, the interface or platform (401) includes a ball and socket assembly. According to one embodiment, a ball and socket assembly is formed by a spacer element (419) that is in the form of a spherical element or ball, which is secured within indentations of adjacent socket plates (417, 415). In this embodiment, controlled pitching action is accomplished by the application of force on one or more control elements (405) together with one or more connectors or springs (433).

An interface or orientation platform (401) that includes base and platform members (417, 415) in the form of socket plates, the spacer element (419) may be in the form of a ball-like, semi-spherical structure, or a spherical structure. The spacer element (417) may define a lumen (421) through which, for example, a control cable (403) for a working instrument (41) may be inserted. In one embodiment, the first and second socket plates (415, 417) are identical and may be inverted versions of each other, and each socket plate (415, 417) includes a concave cup cavity (431) configured to receive and interface with a spherical spacer unit (419). The socket plate (415, 417) also includes a larger center aperture (423) and a plurality of smaller apertures (425) distributed about its circumferential portion of the disc. In this illustration, four apertures (427) that are positioned at approximately 90o apart are slightly larger in size than each of the three apertures (429) located between adjacent 90o holes (427). However, other embodiments may include apertures of similar dimensions or of a variety of different dimensions.

With the embodiment illustrated in Figs. 32D-E, the interface or orientation platform (401) is assembled by inserting the spacer element or ball unit (419) into the concave cavities (431) of the base 417 and platform 415 members or socket plates. The ball unit (419) may be adjusted to ensure alignment of its lumen (421) with the center apertures or apertures (423) of the first and second socket plates (415, 417). Similarly, the plates (415, 417) may be adjusted to ensure that the 90o apertures (427) on the first plate (415) are aligned with the corresponding apertures (427) on the second plate (417). One end of a tension spring (433) is hooked into one of the large apertures (427) on the first socket plate (415) and a second end is hooked into the large aperture (427) on the second socket plate (417) directly below the first aperture. A control element (405) with a ball termination (406) that terminates at the platform member (415) is threaded through a 90o apertures (427) of the socket plates (417, 415), and through a lumen (115) in the instrument member (103) to a splayer at the proximal end of the catheter assembly. Although the control element (405) shown in Fig. 32E is located within a lumen of instrument, other embodiments of an instrument member may have one or more dedicated lumens for containing control elements and tool actuation cables.

Referring to Figs. 32F-H, thee orientation platform (401) is designed for a pitch degree of freedom. The XYZ orientation compass associated with Fig. 32D indicates that this orientation platform may perform a pitching motion by rotating about the Y axis in a XZ plane. In one embodiment, the spring (433) may be calibrated to provide a preset amount of tension force in its neutral state and the control element (405) also has to be pre-tensioned to counterbalance that force such that the orientation platform (401) may naturally assume a known state or position. For example, sufficient downward force may be applied to the control element (405) to cause the top or platform member (415) to have 0o of tilt relative to the longitudinal axis of the instrument or to be parallel to the second plate (417) (as shown in Fig. 32D).

Referring to Figs. 32F and 32H, because this spring (433) is biased to compress, the first plate or platform member (415) of the orientation platform (401) is caused to tilt or pitch to the left in a pitch-direction when the control element (405) is slack or applies insufficient force. Fig. 32H shows that not only is the top plate or platform member (415) moving, but the spacer element (419) also rotates counter-clockwise as the orientation platform (401) tilts down on the left side. It can also be observed that the lumen (421) of the spacer element (419) may become slightly misaligned with the center holes (423) of the base and platform members (417, 415), but there is sufficient overlap such that the a cable, an instrument, a tool, etc. may still pass from a catheter and through the orientation platform (401). Preferably, the center apertures (423) and lumen (421) are dimensioned such that when the orientation platform (401) is utilized, the central lumen or passage does not become unduly constricted or a situation wherein an instrument or cable in the passage may become undesirably crimped is not created. The center holes (423) and lumen (421) of different embodiments may have various shapes an sizes to allow for sufficient clearance as components traverse through this passage when the orientation platform (401) is pitching. The control element (405) may also flex or bend as the orientation platform (401) moves.

Referring to Figs. 32G and 32I, pulling down on the platform control element (405) results in a downward force conveyed by the cable tension. The control element (405) flexes as the space between the plates (415, 417) narrow on the right side whereas the coils of the spring (433) are stretched apart due to the load caused the downward force on the control element (405). If the force is sufficient to counteract the spring (433) force, the right edge of the platform member (415) proximate to where the termination (406) of the control element (405) is engaged to tilt downward and pitch to the right in a pitch+ direction. Similar to the pitch- discussion above, the illustration in Fig. 32I shows that in addition to the platform member (415) moving, the spacer element (419) also rotates clockwise as the orientation platform (401) tilts downwardly on the right side. Here, the lumen (421) of the spacer element (419) may also become slightly misaligned with the center holes (423) of the base and platform members (417, 415), but there is sufficient overlap in these openings such that material may still pass from the catheter or instrument member lumen and through the orientation platform (401).

Figs. 32J-M illustrate another embodiment of an interface or platform (401) that includes the same components discussed above except that the interface (401) does not include a tension spring (433). Certain aspects of this embodiment are not repeated since the configuration and operation of the embodiment shown in Figs. 32D-1 applies.

As shown in Fig. 32J, in the illustrated embodiment, a compression spring (435) replaces the tension spring (433) to provide known amount of compressive force in its neutral state. The control element (405) is also pre-tensioned to counter-balance that force such that the orientation platform (401) may naturally assume a known state or position. For example, sufficient downward force may be applied to the control element (405) to cause the platform member (415) of the orientation platform (401) to have a 0o of tilt to be parallel to the second plate (417). The compression spring (435) and the control element (405) are coaxially located on the same side of the orientation platform (401). One end of the spring (435) is coupled to the platform member (415) and the other end is coupled to the base member (417). A control element (405) with a termination (406) at one end is threaded through a 90o hole (427) of the platform member (415), through the spring (435), through a corresponding 90o hole (427) underneath on the second plate (417), and through a lumen (115) defined by the catheter or instrument to a splayer at the proximal end of the catheter assembly. The compression spring (435) of this embodiment is designed to provide a known amount force to push apart the first and second socket plates (415, 417) in its neutral state as illustrated in Fig. 32J.

Thus, when a sufficient amount of force is applied to control element (405) to pull the top plate (415) downward to compress the spring (435), the spring force may be counteracted and the orientation platform placed in a neutral position wherein the orientation platform may have a 0o of tilt relative to the longitudinal axis of the instrument. But because the spring (435) is biased to expand, the platform member (415) of the interface or platform (401) tilts or pitches to the left in a pitch- direction when tension on the control element (405) is slackened or if insufficient compression force is applied to the cable (405) to counteract the spring force. Fig. 2L shows that not only is the partition member (415) moves, but the spacer element (419) also rotates counter-clockwise as the platform (401) tilts down on the left side. The control element (405) may also flex or bend as the orientation platform (401) moves.

Referring to Figs. 32K-M, when an amount of force sufficient to overcome the spring force is applied to the control element (405), the platform member (415) may be pulled downward beyond a 0o of tilt position to compress the compression spring (435) as illustrated in Figs. 32K and 32M. Thus by pulling down on the control element (405), the overwhelming downward force conveyed by the cable tension causes the right edge of the platform member (415) proximate to the ball termination (406) to tilt downwardly and pitch to the right in a pitch+ direction when sufficient force has been exerted to counteract the spring force.

Figs. 32N-P illustrate another embodiment of an interface or platform (401) that includes many of the same component as discussed above and that operate in the same or substantially similar manner, but the embodiment shown in Figs. 32N-P includes two similar springs (437), and a control element (405) that extends through each spring (437). This embodiment is also designed for a pitch degree of freedom. In its neutral state, the two springs (437) are configured such that one spring (437) counteracts the spring force of the opposing spring (437). For example, if both springs are tension springs, then the force of the left spring (437) in Fig. 32N pushing upward to pivot the top plate (415) about the spherical element (419) towards the right side while the right spring (437) exerts an upward force to pivot the top plate (415) about the spherical element (419) towards the left side. However, because the forces are equal, the top plate or platform member (415) remains in an equilibrium state with a 0o of tilt. If either of the control elements (405) are manipulated, the platform member (415) can be caused to pitch in a predetermined direction, as shown in Figs. 32O-P.

Figs. 33A-G illustrate another embodiment of an orientation platform or interface (401) constructed with a ball and socket assembly as described above. Many of the components shown in Figs. 33A-G are the same as components discussed above and function in the same manner and, therefore, are not repeated. In this embodiment, however, the platform or interface (401) does not include any springs (tension or compression) and instead includes multiple control elements (405). Thus, the illustrated embodiment is designed for a pitch degree of freedom, and the XYZ orientation compass associated with Fig. 33D indicates that this orientation platform may perform a pitching motion by rotating about the Y axis in a XZ plane. In one implementation, the control elements (405) are pre-tensioned to a predetermined setting during setup such that the orientation platform is in a known state (i.e., 0o of pitch). In one embodiment, the orientation platform (401) is maintained in a 0o pitch position while the forces on the control elements (405) are balanced. During a procedure, the control elements (405) may be tensioned or slackened to cause the orientation platform to controllably pitch as needed in a positive or negative direction. Figs. 33D and 33F show a platform member (415) being controllably tilted or pitched about the Y axis toward the left in a pitch- direction when the left control element (405) is tensioned with a downward force that overcomes the downward force applied on the right control element (405), or if the right control element (405) is slackened. Because each control element (405) is coupled to the platform member (415) with a ball termination (406), a force pulling on the control element (405) may be transferred to the platform member (415) via the ball terminations (406). By tensioning the right control element (405), the pitching action may be stopped or reversed.

Further, if the right control element (405) is tensioned with a downward force sufficient to overcome the force on the left control element (405) or if the left control element (405) is slackened, the platform member (415) may be brought back to a 0o of pitch position. Figs. 33E and 33G illustrate the right control element (405) tensioned by a downward force, causing the orientation platform (401) to pitch in a pitch+ direction.

Figs. 34A-C illustrate yet another embodiment of an orientation platform (401). In this embodiment, controlled pitching action is accomplished by the application of force on two control elements (439, 441) and two tension springs (433). Figs. 35A-C illustrate yet another embodiment of an orientation platform (401). In this embodiment, controlled pitching action is accomplished by the application of force on one control element (405) and three tension springs (433). Other numbers and combinations of tension springs (433) and control elements (405) may also be utilized. Further, embodiments that do not include any springs may include different numbers and arrangements of control elements.

For example, Figs. 36A-C illustrate an embodiment of an interface or platform (401) including four control elements. A first control element (443) with a ball termination (406) at one end is threaded through an aperture (427), on the platform member (425), through a corresponding aperture (427) underneath on the base member (417), and through a first lumen (115) in a catheter instrument member (103) to a splayer (101) at a proximal end of the catheter (37). Second, third and fourth control elements (445, 447, 449) are arranged in a similar manner. Thus, in viewing the orientation platform from above in Fig. 36B, the first control element (443) may be view as being at the 0o position, the second control element (445) at the 90o position, the third control element (447) at the 180o position, and the fourth control element (449) at the 270o position. However, it is contemplated that the control elements may be also located in other positions relative to each other. In one embodiment, the orientation platform (401) is maintained in a 0o tilt position while the forces on the four control elements are balanced. However, during a procedure, the control elements may be tensioned or slackened to cause the orientation platform to controllably tilt as needed.

For example, if the intention is to pitch the orientation platform (401), the platform (401) may be controllably pitched in the pitch- direction by tensioning the pitch- control element (449) with a downward force and slackening the tension on the pitch+ control element (445). Conversely, if the intention is to pitch in the pitch+ direction, the pitch+ control element (445) is tensioned and the pitch- control element (449) slackened. Similarly, if the intention is to yaw the orientation platform (401), the platform (401) may be controllably yawed in the yaw- direction by tensioning the yaw- control element (443) and slackening the yaw+ control element (447). For a tilt in the yaw+ direction, the yaw+ control element (447) is tensioned and the yaw- control element (443) slackened. Furthermore, by manipulating a combination of the pitch and yaw control elements (443, 445, 447, 449), it is possible to cause the orientation platform to both pitch and yaw to varying degrees. Further, although manipulation of the control elements have been described in the context of tensioning one element as another is slackened, it is contemplated that one or more slackening actions may be avoided if that amount of force being applied to the control element being tensioned is sufficient to overcome any tensioning force on the control elements formerly described as being slackened.

Figs. 37A-C illustrate another embodiment of an orientation platform (401) that is similar to the embodiment shown in Figs. 36A-C except that the embodiment shown in Figs. 37A-C includes eight control elements. Other embodiments can include other numbers and arrangements of control elements. During a procedure, the eight control elements may be tensioned or slackened to cause the orientation platform (401) to controllably tilt as needed. For example, if the intention is to pitch the orientation platform (401), the platform (401) may be controllably pitched in the pitch- direction by tensioning the pitch- control element (449) with a downward force and slackening the tension on the pitch+ control element (445). Conversely, if the intention is to pitch in the pitch+ direction, the pitch+ control element (445) is tensioned and the pitch- control element (449) slackened. By manipulating a combination of the pitch and yaw control elements (443, 445, 447, 449), it is possible to cause the orientation platform to both pitch and yaw to varying degrees.

Figs. 38A-E illustrate another embodiment of an interface or platform (401) for controlling an orientation of a working instrument coupled to a distal end of a flexible catheter of a robotic medical system. The interface or platform (401) includes a base member or first plate (417) configured for coupling to the distal end of the flexible catheter, a spacer element, e.g., a spherical element or ball (419), a platform member or second plate (415) arranged such that the spacer element (419) is retained between and separates the base member (417) and the platform member (415). Control elements (451, 453, 455, 457 (generally 451) extend through the catheter and through apertures (427) defined by the base member (417). The control elements (451) are arranged such that at least one control element extends between the base and platform members (417, 415) at an angle, i.e., not parallel to the longitudinal axis of the base member (417). In other words, an angle, e.g., at least 30 degrees, and other angles as appropriate, may be defined between the longitudinal axis of the base member (417) and a longitudinal axis of the control element.

Overlapping or crossing control elements are referred to as control cables (451). Thus, the term "control elements" as used in this specification is defined to include a control element that is not arranged in a criss-cross pattern (e.g., as shown in Figs. 33B-C), and also control elements in the form of control cables (451) that cross or overlap with at least one other control cable (451) in an angular arrangement. Such control cables (451) are identified with heavier or dark lines compared to non-crossing or non-overlapping control elements, which may be illustrated as non-filled or lighter lines. Such control cables and their associated overlapping or crossing patterns provide different control characteristics compared to non-overlapping control elements when the control cables (451) are placed in tension or slackened.

More particularly, an embodiment of a platform (401) constructed according to one embodiment includes, for example, a spherical or semi-spherical spacer element (419), may be assembled by inserting the spacer element (419) into the concave cavities (431) of the base and platform members (417, 415). A first control element (405) with a ball termination (406) at one end is threaded through the platform member (415), through a corresponding hole (427) underneath on the base member (417), and through a first lumen (115) in the instrument or catheter member (103) to a splayer at the proximal end of the catheter assembly. A second control element (405) is similarly threaded through the first plate (415), the second plate (417), and through a second lumen (115) in the instrument member (103). In this example, the first and second control elements (405) are positioned oppositely from each other on the first plate (415), or offset by 180o.

Control elements in the form of four control cables (451, 453, 455, 457) (generally 451) are also threaded through apertures (427) defined by the platform member (415), apertures (427) defined by base member (417), and down through the catheter instrument member (103). Unlike the other control elements (405), however, the control elements in the form of control cables (451, 453, 455, 457) are, in one embodiment, arranged in an overlapping or crossing or criss-cross manner, as illustrated in Fig. 38A. In one embodiment, overlapping or crossing control cables (451) extend across a substantial width of the base member (417). Overlapping or crossing control cables (451) may or may not contact each other depending on, for example, the configuration of the base and platform members (417, 415) and the location of the misaligned apertures (427). For purposes of illustration, control cables (451) are illustrated with heavier lines compared to non-overlapping or non-crossing control elements.

These crossing patterns result from control cables (451) extending through misaligned apertures (427) of the base member (417) and the platform member (415). In other words, at least one control cable (451) extends through a base member (417) aperture and through a platform member (415) aperture that is not directly above, or in-line with, the base member (417) aperture. In this manner, all of the cables (451) may extend through misaligned apertures (427) of the base and platform members (417, 415), or some of the cables (451) may extend through misaligned apertures (427), whereas one or more other control elements (405) do not. Instead, control elements (405) and extend through aligned apertures (427) of the base and platform members (417, 415). Embodiments utilizing these arrangements may result in some type of overlapping or criss-cross cable configuration involving a control cable (451).

One manner in which embodiments may be implemented is illustrated in Figs. 38A-B. A first control cable (451) extends through misaligned apertures (427) of the base and platform members (417, 415) and crosses the second control cable (453), and a second control cable (453) crosses the first control cable (451). In essence, the control cables (451, 453) have swapped second plate holes (427) compared to the routing scheme of the control elements (405), which extend through aligned apertures and are parallel to the longitudinal axis of the catheter instrument (103), i.e., perpendicular to surfaces of the base and platform members (417, 415).

As shown in Figs. 38A-B, pulling or tensioning a first opposing pair (452) of control cables (453, 455) and slackening a second opposing pair (454) of control cables (455, 457) results in the platform member (415) rotating in a clockwise manner as illustrated in Fig. 38B (represented by directional arrow). On the other hand, pulling or tensioning the pair (454) of control cables (451, 457) and slackening the pair (452) of control cables (453, 455), the platform member (415) rotates in a counter-clockwise manner, as illustrated in Fig. 38D.

Further, as shown in Fig. 38E, by performing a combination of pulling or tension a first opposing pair (452) of control cables (453, 455), slackening the second opposing pair (454) of control cables (451, 457), and tensioning the pitch+ control element (405), the platform member (415) may be caused to pitch and rotate in a clockwise manner. Thus, Figs. 38A-E illustrate how control elements may be manipulated in various ways, by pulling and slackening various combinations of elements (405) and cables (451), for desired pitch and rotation.

Figs. 39A-C illustrate another embodiment of an interface or platform (401) in which the platform (401) is controlled with control elements in the form of a set of four control elements in the form of cables (451, 453, 455, 457) (generally cable 451) that are also arranged in an overlapping or crossing manner, without non-crossing control / pitch elements (405). The control cables (451) can be manipulated in various ways to rotate and tilt the platform (401). For example, clockwise rotation can be achieved by pulling control cables 453, 455 (as shown in Fig. 39B), and clockwise rotation and positive pitch can be achieved by pulling one or more control cables (e.g., 453, 455) while stabilizing a counter rotation line so rotation is stopped.

Fig. 40A illustrate another embodiment of an interface or platform (401) in which the platform (401) is controlled with a set of control elements in the firm of four control cables (451, 453, 455, 457) (generally 451) that may cross or overlap, but no non-crossing control elements. Further, the control cables (451) are woven in a more complex criss-cross fashion and routed through larger apertures (427) and smaller apertures (429). Also, in the illustrated embodiment, multiple control cables may be threaded through a single aperture (427). Moreover, control cables may be threaded through an aperture (427) defined through a top or distal surface of the platform member (415), traverse or pass over the distal or top surface of the platform member (415), then be threaded back through the platform member (415) and the base member (417).

Referring to Fig. 40B, in another embodiment, the orientation platform (401) is controlled with four control elements - two non-crossing control elements (405) that terminate at (406) on the platform member (415), and two control cables (451, 451). The control elements (405) are controlled from the proximal end of the catheter instrument (as discussed above), and the two control cables (451, 453) are woven in a crossing or criss-cross manner in which both ends of each control cable (451, 453) extend through the base and platform members (417, 415), traverse a top surface of the platform member (415), then extend from the platform member (415) to the base member (417) such that each control cable extends along opposite sides of the intermediate spacer element (419). Each control cable (451, 453) terminate at the base member (417), e.g., on a bottom surface or underside of the base member (417).

In another embodiment, referring to Figs. 41A-B, an interface or platform (401) may include a different crossing cable (451) arrangement in which the platform (401) may be controlled with a set of four control cables (451, 453, 455, 457) without the need for any control elements (405). In this embodiment, the control cables (451) may be woven in a crossing or overlapping manner, and one end of each control cable (451) may terminate on a top surface of the platform member (415). Figs. 41A-B illustrate an example of omnidirectional motion by pulling cable (453) and slackening cables (451, 455, 457), thereby resulting in rotation, pitch and yaw motion, positive yaw being slightly larger than positive pitch in this example.

Various embodiments described with reference to Figs. 32A-41B include a spacer element in the form of a spherical element or ball (419), e.g., as part of a ball and socket assembly. Other embodiments, however, may utilize different types of spacer elements.

For example, referring to Figs. 42A-B illustrate one embodiment of an orientation platform (401) employing a spacer element in the form of an elastomeric cylinder (459). An elastomeric cylinder (459) suitable for embodiments may be semi-flexible and may allow for bending as the orientation platform (401) if caused to move in response to manipulation of the control elements (405). Similar to the spherical spacer element (419), the elastomeric cylinder may also define a lumen (460) for passage of, e.g., a cable for a working instrument (41) or other component or a working substance. The manner in which control elements (405) may be manipulated to achieve desired rotation and orientation of the interface or platform (401) is described in detail with respect to a spherical spacer element (419), and the same principles generally apply to the embodiment shown in Figs. 42A-B that utilizes an elastomeric cylinder (459) as a spacer element.

In a further alternative embodiment, the spacer element may be in the form of a flexure element (461), as shown in Figs. 43A-B. A flexure (461) for use in embodiments may be semi-flexible and allow for bending as the orientation platform (401) if caused to move in response to the control elements (405). Similar to the spherical spacer element (419), the flexure (461) may also define a lumen (462) for passage of, e.g., a cable for a working instrument (41) or other component or a working substance. The manner in which control elements (405) may be manipulated to achieve desired rotation and orientation of the platform (401) is described in detail with respect to a spherical spacer element (419), and the same principles generally apply to the embodiment shown in Figs. 43A-B having a flexure (461) as a spacer element.

Referring to Figs. 44A-B, in yet another alternative embodiment, the spacer element may be in the form of a non-spherical element or ball (463) rather than a spherical ball or element (419). In the illustrated embodiment, surfaces of the non-spherical element have planar faces that interface with surfaces of the base and platform members (417, 415). Similar to the spherical spacer element (419), a non-spherical spacer element (463) may also define a lumen (464) for passage of, e.g., a cable for a working instrument (41) or other component or a working substance. The manner in which control elements (405) may be manipulated to achieve desired rotation and orientation of the interface or platform (401) is described in detail with respect to a spherical spacer element (419), and the same principles generally apply to the embodiment shown in Figs. 43A-B that a non-spherical spacer element.

Fig. 45 illustrates another alternative embodiment of an orientation platform (401) employing a flexible coil (465) as a spacer element. The flexible coil (465) for use in embodiments may be semi-flexible and may allow for bending as the orientation platform (401) is caused to tilt in a variety of ways in response to the control elements (405). The discussion above regarding how control elements (405) may be manipulated to achieve desired rotation and orientation of the platform (401) is described in detail above, and the same principles generally apply to the embodiment shown in Fig. 45 that includes a flexible coil (465) spacer element.

While various spacer units are described and may be utilized within an interface or platform (401), the various spherical elements (419, 463), elastomeric cylinder (459), flexure (461), and flexible coil (465) may be fabricated from a variety of materials, preferably a material that is inert and suitable for medical procedures. Suitable materials for certain embodiments may include, for example, Buna-N (nitrile), propylene (EPDM), silicone, cast polyurethane, chloroprene (Neoprene), fluorocarbon (Viton, Fluorel), fluorosilicone, liquid silicone rubber, etc., but are not so limited.

Referring to Fig. 46, according to another embodiment, an orientation platform (401) includes a universal joint (467) as a spacer element. The universal joint (467) of this embodiment is controlled with a plurality of control elements (405) in a similar manner as discussed above and may be manipulated to tilt as the orientation platform (401) in response to manipulation of the control elements (405).

Figs. 47A-C illustrate one embodiment of an orientation platform (401) employing a pin and groove arrangement (469) as a spacer element. The pin and groove (469) of the illustrated embodiment includes a platform member (415) in the form of a first plate (471) having a cylindrical pin element (473) on its bottom face. The base member (417) is in the form of a second plate (475) that includes a semi-circular structure (477) disposed on its top face. This semi-circular structure (477) may be fabricated as a half disc with a groove or channel (479) extending partway along its edge. The orientation platform (401) is constructed by mating the pin element of the first plate (471) into the half disc channel (477) of the second plate (475). Control elements (405) are threaded through the first and second plates (471, 475) on opposite sides of the orientation platform (401). In this embodiment, the pin element (473) may freely slide within the groove (479) on the disc surface, thus tilting the top plate (471). Control elements (405) can be manipulated to control tilting action of the proximal end of the instrument.

Embodiments described with reference to Figs. 32A-47C include a "single-level" interface or platform (401). Alternative embodiments of an orientation interface or platform (401) may include multiple levels.

For example, referring to Figs. 48A-O, a multi-level platform or interface (483) for coupling to a distal end of flexible catheter having a lower level or stage 487 and an upper level or stage 485. In the illustrated embodiment, each level (485, 487) is structured in a manner that is similar to the platform (401) shown in Figs. 32D-I.

In the embodiment illustrated in Figs. 48A-M, the multi-level platform (483) includes two "ball and socket" spacer elements (419a, 419b) (generally 419). A first spherical spacer element is disposed between a base member (417) and a first platform member (415a), and a second spherical spacer element (419b) is disposed between the first platform member (415a) and a second, distal platform member (415b). In the illustrated embodiment, the first platform member (415a) is constructed to include with multiple components to interface between the first and second levels (485, 487). In the illustrated embodiment, the first platform member (415a) includes a first plate 489 that interfaces with a lower spacer element (419a), and a second, top plate (495) that interface with the upper spacer element (419b)

The lower stage (485) is controllably yawed in a positive or negative direction by tensioning or slackening a control element (405a) that terminates at the first platform member (415a) to counterbalance a tension spring (433a) (shown in Fig. 48C). Similarly, the upper stage (487) of the orientation platform (483) is controllably pitched in a positive or negative direction by tensioning/slackening a control element (405b) that terminates at the second platform member (415b) to counterbalance a tension spring 433(b). Because the lower stage (485) is rotated relative to the upper stage (487) by 90o, the pitch degree of freedom in the upper stage (487) has become a yaw degree of freedom for the lower stage (485). By manipulating the first and second control elements (405a, 405b) in combination, the distal tip of this flexible catheter may be caused to controllably pitch and yaw in a variety of directions.

Figs. 49A-G illustrate another embodiment of a flexible catheter having a multi-level interface or platform (483) that includes first and second stages (485, 487) in which the stages (485, 487) are constructed in a manner that is similar to the orientation platform (401) including compression springs (435) and control elements (405) that extend through respective compression springs (435) as described with reference to Figs. 32N-P. The lower stage (485) of the platform (483) is controllably yawed in a positive or negative direction by tensioning or slackening of control elements (405a) to counterbalance compression springs (435a). The upper stage (487) is controllably pitched in a positive or negative direction by tensioning or slackening control elements (405b) to counterbalance compression springs (435b). Because the lower stage (485) is rotated relative to the upper stage (487) by 90o, the pitch degree of freedom of the upper stage (487) has become a yaw degree of freedom for the lower stage (485). By manipulating the first and second control elements (405a, 405b), the distal tip of this flexible catheter may be caused to pitch and yaw in a variety of directions.

Figs. 50A-C illustrate another embodiment of a flexible catheter having a multi-level interface or platform (483) that includes spacer elements in the form of spherical elements or balls (419). Each level (485, 487) is constructed in a manner that is similar to the platform (401) structure described with reference to Figs. 33A-G, in which control elements (405), but not any springs, are used to manipulate the platform. In the illustrated embodiment, the lower stage (485) of the orientation platform (483) is controllably yawed in a positive or negative direction by tensioning or slackening of control elements opposing control elements (405a) that terminate at the first platform member (415a). The upper stage (487) is controllably pitched in positive or negative directions by tensioning or slackening control elements (405b) that terminate at the second or distal platform member (415b). Because the lower stage (485) is rotated relative to the upper stage (487) by 90o, the pitch degree of freedom of the upper stage (487) has become a yaw degree of freedom for the lower stage (513). By manipulating the control elements (405a,b), the distal tip of this flexible catheter may be caused to pitch and yaw in various directions.

Referring to Figs. 51A-B, a further alternative embodiment of a multi-level orientation interface or platform (483) including multiple elastomeric cylinders (459a,b). The stages (485, 487) of this embodiment are structured in a manner that is similar to the orientation platform (401) described with reference to Figs. 42A-B. The lower stage (485) of the orientation platform (483) is controllably yawed in a positive or negative direction by tensioning or slackening control elements (405a). The upper stage (487) of the orientation platform (483) is controllably pitched in a positive or negative direction by tensioning or slackening control elements (405b). Because the lower stage (485) is rotated relative to the upper stage (487) by 90o, the pitch degree of freedom of the upper stage (487) has become a yaw degree of freedom for the lower stage (513). The distal tip of this flexible catheter may be caused to pitch and yaw in a variety of directions by manipulating control elements (405a,b).

Referring to Figs. 52A-B, another alternative embodiment of a multi-level orientation interface or platform (483) including multiple stages (485, 487) includes flexures (461a,b). The stages (485, 487) of this embodiment are structured in a manner that is similar to the orientation platform (401) described with reference to Figs. 43A-B. The lower stage (485) of the orientation platform (483) is controllably yawed in a positive or negative direction by tensioning or slackening of control elements (405a), and the upper stage (487) is controllably pitched in a positive or negative direction by tensioning or slackening of control elements (405b). Because the lower stage (485) is rotated relative to the upper stage (487) by 90o, the pitch degree of freedom of the upper stage (487) has become a yaw degree of freedom for the lower stage (485). The control elements (405a,b) can be manipulated to cause pitch and yaw motions of the distal tip of this flexible catheter in various directions.

Figs. 53A-B illustrate a further alternative embodiment of a multi-level orientation interface or platform (483) for a flexible catheter and that includes non-spherical elements or balls (463a,b). The lower and upper stages (485, 487) of this embodiment are structured in a manner that is similar to the orientation platform (401) described with reference to Figs. 44A-B. The lower stage of the platform (483) is controllably yawed in a positive or negative direction by tensioning or slackening control elements (405a), and the upper stage (487) is controllably pitched in a positive or negative direction by tensioning or slackening control elements (405b). Because the lower stage (485) is rotated relative to the upper stage (487) by 90o, the pitch degree of freedom of the upper stage (487) has become a yaw degree of freedom for the lower stage (485). The control elements (405a,b) can be manipulated to cause the distal tip of a flexible catheter to pitch and yaw in various ways.

Fig. 54 illustrates another alternative embodiment of a multi-level orientation interface or platform (483) for a flexible catheter and that includes flexible coils (465a,b). The lower and upper stages (485, 487) of this embodiment are structured in a manner that is similar to the orientation platform (401) descried with reference to Fig. 45. The lower stage (485) of the orientation platform (483) is controllably yawed in a positive or negative direction by tensioning or slackening of control elements (405a), and the upper stage (487) is controllably pitched in a positive or negative direction by tensioning or slackening control elements (405b). Because the lower stage (485) is rotated relative to the upper stage (487) by 90o, the pitch degree of freedom of the upper stage (487) has become a yaw degree of freedom for the lower stage (485). By manipulating the control elements (405a,b), the distal tip of this flexible catheter may be caused to pitch and yaw in a variety of directions.

Fig. 55 illustrates another embodiment of a multi-level orientation interface or platform (483) for a flexible catheter and that includes multiple universal joints (467a,b). The lower and upper stages or levels (485, 487) of this embodiment are structured in a manner that is similar to the orientation platform (401) described with reference to Fig. 46. The lower stage (485) of the orientation platform (483) is controllably yawed in a positive or negative direction by tensioning or slackening control elements (405a), and the upper stage (487) is controllably pitched in a positive or negative direction by tensioning or slackening control elements (405b). Because the lower stage (485) is rotated relative to the upper stage (487) by 90o, the pitch degree of freedom in the upper stage (487) has become a yaw degree of freedom for the lower stage (485). By manipulating the control elements (405a,b) the distal tip of this flexible catheter may be caused to pitch and yaw in a variety of directions.

Figs. 56A-G illustrate a further embodiment of a multi-level orientation platform or interface (483) and components thereof. The first and second stages (485, 487) may be constructed such that they include only crossing control cables (generally 451), or a combination of crossing control cables (451) and non-crossing control elements (405) similar to various embodiments previously described, e.g. as in Fig. 40B. Spacer elements, e.g., in the form of a spherical element (419) or other element described in other embodiments, may include an eyelet or loop (530) or other tying structure (532) for facilitating crossing or overlapping control cables (451) within a multi-level structure as necessary. Manipulation of motion and positioning of distal tip of a flexible catheter may be achieved by manipulation of control elements (405a,b) and control cables (451).

Other crossing patterns within a multi-level platform (483) that may be implemented with embodiments are illustrated in Figs. 57A-D. As shown in these figures, control cables (451) may cross within one level, e.g., the lower level (485), but not cross in another level, e.g., the upper level (487). Other control cable (451) patterns may be utilized. Alternatively, control cables (451) may cross within each level (485, 487). Further, as shown in Fig. 58, cams (527) maybe provided to assist with the routing of the various control cables (529).

Although embodiments are described as having a single level (Figs. 32A-47C) or two levels (Figs. 48A-58), embodiments may also be implemented with additional levels as necessary. For example, Figs. 59A-C illustrate alternative embodiments of a flexible catheter that may include multiple ball and socket orientation platforms and how such platforms may bend as needed by manipulating control elements (405) and/or control cables (451). Thus, the embodiments described above are provided as examples of how embodiments may be implemented.

Although particular embodiments have been shown and described, it should be understood that the above discussion is not intended to limit the scope of these embodiments. While embodiments and variations of the many aspects of the invention have been disclosed and described herein, such disclosure is provided for purposes of illustration only. Many combinations and permutations of the disclosed embodiments are useful in minimally invasive surgery, and the system is configured to be flexible. Thus, various changes and modifications may be made without departing from the scope of the claims.

For example, although embodiments are advantageously suited for minimally invasive procedures, they may also be utilized in other, more invasive procedures. Further, while embodiments of the invention are described with reference to a robotic instrument system, such as a robotic catheter system available from Hansen Medical of Mountain View, CA, certain embodiments may also be used with other types of computer or robotically controlled surgical systems such as, for example, the da Vinci® surgical system available from Intuitive Surgical Inc. of Sunnyvale, CA, the NIOBE Magnetic Navigation System and associated Magnetic GentleTouch Catheters, available from Stereotaxis, Inc. of St. Louis, MO; the Mako Haptic Guidance System available from Mako Surgical, Inc. of Ft. Lauderdale, FL; and the surgical platform available from NeoGuide Systems Inc. of Los Gatos, CA.

Because one or more embodiments of the flexible catheter instruments disclosed in above may be used in minimally invasive surgical procedures, the distal portions of these instruments may not be easily visible to the naked eye. As such, embodiments of the invention may be utilized with various imaging modalities such as magnetic resonance (MR), ultrasound, computer tomography (CT), X-ray, fluoroscopy, etc. may be used to visualize the surgical procedure and progress of these instruments. It may also be desirable to know the precise location of any given catheter instrument and/or tool device at any given moment to avoid undesirable contacts or movements. Thus, embodiments may be utilized with localization techniques that are presently available may be applied to any of the apparatuses and methods disclosed above. For example, one or more localization coils may be built into a flexible catheter instrument. In other implementations, a localization technique using radio-opaque markers may be used with embodiments of the invention. Similarly, a fiber optic Bragg sensing fiber may be built into the sidewall of a catheter instrument to sense position and temperature. Further, a plurality of sensors, including those for sensing patient vitals, temperature, pressure, fluid flow, force, etc., may be combined with the various embodiments of flexible catheters and distal orientation platforms disclosed herein.

Embodiments involving catheter components may made with materials and techniques similar to those described in detail in U.S. Patent Application publication number 2006-0100610. Further, various materials may be used to fabricate and manufacture different flexible catheters and orientation platforms. For example, it is contemplated that in addition to that disclosed above, materials including, but not limited to, stainless steel, copper, aluminum, nickel-titanium alloy (Nitinol), Flexinol® (available from Toki of Japan), titanium, platinum, iridium, tungsten, nickel-chromium, silver, gold, and combinations thereof, may be used to manufacture components such as control elements, control cables, spine elements, gears, plates, ball units, wires, springs, electrodes, thermocouples, etc. Similarly, non-metallic materials including, but not limited to, polypropylene, polyurethane (Pebax®), nylon, polyethylene, polycarbonate, Delrin®, polyester, Kevlar®, carbon, ceramic, silicone, Kapton® polyimide, Teflon® coating, polytetrafluoroethylene (PTFE), plastic (nonporous or porous), latex, polymer, etc. may be used to make the various parts of a catheter, orientation platform, tool, etc.

Additionally, certain embodiments are described as having lumens that are configured for carrying or passage of control elements, control cables, wires, and other catheter instruments. Such lumens may also be used to deliver fluids such as saline, water, carbon dioxide, nitrogen, helium, for example, in a gaseous or liquid state, to the distal tip. Further, some embodiments may be implemented with a open loop or closed loop cooling system wherein a fluid is passed through one or more lumens in the sidewall of the catheter instrument to cool the catheter or a tool at the distal tip.

Further, although embodiments are described with reference to examples of working instruments such as end effectors shown in Figs. 23A-Z, embodiments may be utilized with other types of tools and end-effectors including, for example, a Kittner dissector, a multi-fire coil tacker, a clip applier, a cautery probe, a shovel cautery instrument, serrated graspers, tethered graspers, helical retraction probe, scalpel, basket capture device, irrigation tool, needle holders, fixation device, transducer, and various other graspers may be utilized with embodiments.

A number of other catheter type instruments may also be utilized together with certain embodiments including, but not limited to, a mapping catheter, an ablation catheter, an ultrasound catheter, a laser fiber, an illumination fiber, a wire, transmission line, antenna, a dilator, an electrode, a microwave catheter, a cryo-ablation catheter, a balloon catheter, a stent delivery catheter, a fluid/drug delivery tube, a suction tube, an optical fiber, an image capture device, an endoscope, a Foley catheter, Swan-Ganz catheter, fiberscope, etc. Thus, it is contemplated that one or more catheter instruments may be inserted through one or more lumens of a flexible catheter instrument, flexible sheath instrument, or any catheter instrument to reach a surgical site at the distal tip. Similarly, it is contemplated that one or more catheter instruments may be passed through an orientation platform to a region of interest.

Accordingly, embodiments are intended to cover alternatives, modifications, and equivalents that may fall within the scope of the claims.

**Claims**

1. An instrument driver operable to control coaxially aligned elongate catheter instruments in response to control signals generated by an input device, the instrument driver comprising:

    a frame (15a);

a first carriage (67) movably coupled to the frame and configured to support a first catheter instrument (71); **characterized in that** it further comprises

a second carriage (69) movably coupled to the frame and configured to support a second catheter instrument (73), the first and second carriages being independently rotatable about respective longitudinal axes of the first and secured catheter instruments.

2. The instrument driver of claim 1, wherein the first carriage is positioned relative to the second carriage such that the first catheter instrument, when coupled to the first, carriage, may be positioned within a lumen of the second catheter instrument, when coupled to the second carriage.

3. The instrument driver of claim 2, wherein the first and second carriages are positioned within the frame such that the respective longitudinal axes or the first and second catheter instruments are aligned with a longitudinal axis of the frame.

4. The instrument driver of any of claims 1-3, wherein the first and second carriages are independently and simultaneously rotatable about a longitudinal axis of the frame.

5. The instrument driver of any of claims 1-3, wherein the first and second carriages are independently and simultaneously rotatable in different directions about a longitudinal axis of the frame.

6. The instrument driver of any of claims 1-3, wherein the frame is rotatable about a support structure, and wherein the first and second carriages are independently rotatable relative to each other and to the frame when the frame is rotated about the support structure.

7. The instrument driver of any of claims 1-6, the frame comprising an arcuate inner surface including a plurality of bearings (79), wherein the first and second carriages are rotatable relative to the frame by sliding along the plurality of bearings.

8. The instrument driver of claim 7, wherein the first and second carriages each have a semi-circular shape, and the arcuate inner surface has a corresponding semi-circular shape.

9. The instrument driver of any of claims 1-6, each of the first and second carriages comprising a respective drive element controllable to rotate the respective carriage relative to the frame.

10. The instrument of claim 1, the first and second carriages being independently movable in at least three different directions with at least two different types of motion.

11. The instrument driver of claim 10, wherein one of the at least two different types of motion is rotational motion about the respective longitudinal axis, and another of the at least two different types of motion is linear motion along the respective longitudinal axis.

**Patentansprüche**

1. Ein Instrumentenantrieb, welcher zur Steuerung koaxial ausgerichteter länglicher Katheterinstrumente in Antwort auf Steuerungssignale, welche durch ein Eingabegerät erzeugt werden, ausgebildet ist, wobei der Instrumentenantrieb Folgendes umfasst:

ein Gehäuse (15a);
einen ersten Schlitten (67), welcher bewegbar mit dem Gehäuse verbunden ist und zum Tragen eines ersten Katheterinstruments (71) ausgebildet ist;

**dadurch gekennzeichnet, dass** dieser weiterhin Folgendes umfasst:

einen zweiten Schlitten (69), welcher bewegbar mit dem Gehäuse verbunden ist und zum Tragen eines zweiten Katheterinstruments (73) ausgebildet ist, wobei der erste und der zweite Schlitten unabhängig um entsprechende longitudinale Achsen des ersten und des zweiten Katheterinstruments rotierbar sind.

**2.** Der Instrumentenantrieb gemäß Anspruch 1, wobei der erste Schlitten relativ zu dem zweiten Schlitten derart angeordnet ist, dass das erste Katheterinstrument, wenn es mit dem ersten Schlitten verbunden ist, innerhalb eines Hohlraums des zweiten Katheterinstruments angeordnet werden kann, wenn das zweite Katheterinstrument an den zweiten Schlitten gekoppelt ist.

**3.** Der Instrumentenantrieb gemäß Anspruch 2, wobei der erste und der zweite Schlitten innerhalb des Gehäuses angeordnet sind, so dass die entsprechenden longitudinalen Achsen des ersten und des zweiten Katheterinstruments entlang einer longitudinalen Achse des Gehäuses ausgerichtet sind.

**4.** Der Instrumentenantrieb gemäß einem der Ansprüche 1-3, wobei der erste und der zweite Schlitten unabhängig und simultan um eine longitudinale Achse des Gehäuses rotierbar sind.

**5.** Der Instrumentenantrieb gemäß einem der Ansprüche 1-3, wobei der erste und der zweite Schlitten unabhängig und simultan in verschiedene Richtungen um eine longitudinale Achse des Gehäuses rotierbar sind.

**6.** Der Instrumentenantrieb gemäß einem der Ansprüche 1-3, wobei das Gehäuse um eine Stützstruktur rotierbar ist und wobei der erste und der zweite Schlitten unabhängig relativ zueinander und relativ zu dem Gehäuse rotierbar sind, wenn das Gehäuse um die Stützstruktur rotiert wird.

**7.** Der Instrumentenantrieb gemäß einem der Ansprüche 1-6, wobei das Gehäuse eine gebogene innere Oberfläche umfasst, welche eine Vielzahl von Lagern (79) umfasst, wobei der erste und der zweite Schlitten relativ zu dem Gehäuse durch Gleiten entlang der Vielzahl der Lager rotierbar sind.

**8.** Der Instrumentenantrieb gemäß Anspruch 7, wobei der erste und der zweite Schlitten jeweils eine halbkreisförmige Form aufweisen und die gebogene innere Oberfläche eine entsprechende halbkreisförmige Form aufweist.

**9.** Der Instrumentenantrieb gemäß einem der Ansprüche 1-6, wobei jeder des ersten und des zweiten Schlittens ein entsprechendes Antriebselement umfasst, welches zum Rotieren des entsprechenden Schlittens relativ zum Gehäuse steuerbar ist.

**10.** Der Instrumentenantrieb gemäß Anspruch 1, wobei der erste und der zweite Schlitten unabhängig in mindestens drei verschiedenen Richtungen mit zumindest zwei verschiedenen Bewegungsarten bewegbar sind.

**11.** Der Instrumentenantrieb gemäß Anspruch 10, wobei eine der mindestens zwei verschiedenen Bewegungsarten eine rotatorische Bewegung um die entsprechende longitudinale Achse ist und eine andere der mindestens zwei verschiedenen Bewegungsarten eine lineare Bewegung entlang der entsprechenden longitudinalen Achse ist.

**Revendications**

**1.** Un mécanisme de commande d'instrument, apte à contrôler des instruments allongés alignés de manière coaxiale et du type cathéter en réponse à des signaux de contrôle générés par un périphérique d'entrée, le mécanisme de commande comprenant :

    un châssis (15a) ;
    un premier chariot (67) couplé de manière mobile au châssis et configuré pour supporter un premier instrument cathéter (71) ;

    **caractérisé en ce qu'**il comprend en plus :

    un second chariot (69) couplé de manière mobile au châssis et configuré pour supporter un second instrument cathéter (73), le premier et le second chariots étant mobiles en rotation de manière indépendante autour des axes longitudinaux respectifs des premier et second instruments cathéter.

**2.** Le mécanisme de commande d'instrument de la revendication 1, où le premier chariot est positionné de manière relative au second chariot de manière à ce que le premier instrument cathéter, lorsqu'il est couplé au premier chariot, puisse être positionné dans une lumière du second instrument cathéter, lorsqu'il est couplé au second chariot.

**3.** Le mécanisme de commande d'instrument de la revendication 2, où les premier et second chariots sont positionnés dans le châssis de manière à ce que les axes longitudinaux respectifs des premier et second instruments cathéter soient alignés avec un axe longitudinal du châssis.

**4.** Le mécanisme de commande d'instrument de l'une des revendications 1 à 3, où les premier et second chariots sont mobiles en rotation de manière indépendante et simultanément autour d'un axe longitudinal du châssis.

**5.** Le mécanisme de commande d'instrument de l'une des revendications 1 à 3, où les premier et second chariots sont mobiles en rotation de manière indépendante et simultanément dans des directions différentes autour d'un axe longitudinal du châssis.

**6.** Le mécanisme de commande d'instrument de l'une des revendications 1 à 3, où le châssis est mobile en rotation autour d'une structure de support, et où les premier et second chariots sont mobiles en rotation de manière indépendante l'un par rapport à l'autre et par rapport au châssis lorsque le châssis est déplacé en rotation autour de la structure de support.

**7.** Le mécanisme de commande d'instrument de l'une des revendications 1 à 6, le châssis comprenant une surface courbée vers l'intérieur incluant une pluralité de roulements (79), où les premier et second chariots sont mobiles en rotation par rapport au châssis en glissant le long de la pluralité de roulements.

**8.** Le mécanisme de commande d'instrument de la revendication 7, où les premier et second chariots ont, chacun, une forme semi-circulaire, et la surface courbée vers l'intérieur a une forme semi-circulaire correspondante.

**9.** Le mécanisme de commande de l'une des revendications 1 à 6, chacun des premier et second chariots comprenant un élément de commande respectif contrôlable pour déplacer en rotation le chariot respectif par rapport au châssis.

**10.** Le mécanisme de commande de la revendication 1, les premier et second chariots étant mobiles de manière indépendante dans au moins trois directions différentes avec au moins deux types de mouvement.

**11.** Le mécanisme de commande de la revendication 10, où un des au moins deux différents types de mouvement est un mouvement de rotation autour de l'axe longitudinal respectif, et un autre des au moins deux différents types de mouvement est un mouvement linéaire selon l'axe longitudinal respectif.

FIG. 1

**FIG. 2A**

**FIG. 2B**

FIG. 2C

47

53 DATA GLOVE SOFTWARE

55 CONTROL AND INSTRUMENT DRIVER COMPUTER

49 MASTER COMPUTER

51 MASTER INPUT DEVICE HARDWARE/SOFTWARE

35 DATA GLOVE INPUT DEVICE

27 HAPTIC MASTER INPUT DEVICE

*FIG. 2D*

FIG. 3

FIG. 4A

FIG. 4B

13

*FIG. 4C*

FIG. 4D

FIG. 4E

EP 2 124 800 B1

FIG. 4F

FIG. 4G

FIG. 4H

EP 2 124 800 B1

FIG. 4I

FIG. 4J

EP 2 124 800 B1

FIG. 5A

FIG. 5B

FIG. 5C

FIG. 5D

FIG. 5E

FIG. 5F

FIG. 5H

FIG. 5G

*A*

67

75

15a

83

15

*FIG. 5J*

*A*

67

79

69

15

15a

*FIG. 5I*

*FIG. 5K*

*FIG. 5L*

FIG. 6A

37

39

101

3

37

*FIG. 6B*

FIG. 6C

FIG. 6D

FIG. 6E

FIG. 7A

FIG. 7B

FIG. 7C

EP 2 124 800 B1

FIG. 7D

FIG. 8A

EP 2 124 800 B1

FIG. 8E

FIG. 8F

FIG. 8B

FIG. 8C

FIG. 8D

73

FIG. 9A

FIG. 9B    FIG. 9C    FIG. 9D    FIG. 9E    FIG. 9F    FIG. 9G

74

FIG. 10A

103
141
135
133

FIG. 10B

103
141
137
133

FIG. 10C

103
141
139
133

FIG. 11B

FIG. 11A

*FIG. 12A*

*FIG. 12B*

FIG. 13B

FIG. 13A

FIG. 14A

FIG. 14B

FIG. 14C

FIG. 15A

FIG. 15B

FIG. 15E

FIG. 15F

FIG. 15G

FIG. 15C

FIG. 15D

**FIG. 16C**

**FIG. 16A**   **FIG. 16B**

**FIG. 16D**

**FIG. 16E**

15°

22.5°

30°

**FIG. 16F**

**FIG. 16G**

**FIG. 16H**

FIG. 17A

FIG. 17B

FIG. 18

FIG. 19

FIG. 20

EP 2 124 800 B1

FIG. 21

FIG. 22A

EP 2 124 800 B1

FIG. 22B

*103*

**FIG. 22C**

**FIG. 22D**

EP 2 124 800 B1

FIG. 22E

FIG. 22F

FIG. 22G

FIG. 22H

FIG. 22J

FIG. 22I

FIG. 22K

FIG. 22G-1

FIG. 22M

FIG. 22L

EP 2 124 800 B1

FIG. 22N

FIG. 22O

FIG. 22P

FIG. 22Q

FIG. 23A

FIG. 23B

FIG. 23C

FIG. 23D

FIG. 23E

FIG. 23F

FIG. 23G

FIG. 23H

FIG. 23I

FIG. 23J

FIG. 23K

EP 2 124 800 B1

FIG. 23P

FIG. 23O

FIG. 23N

FIG. 23M

FIG. 23L

FIG. 23U

FIG. 23T

FIG. 23S

FIG. 23R

FIG. 23Q

FIG. 23Z

FIG. 23Y

FIG. 23X

FIG. 23W

FIG. 23V

*FIG. 24B*

307

305a

309

103

103a

305

305a

307

103b

31.1

103

103a

*FIG. 24A*

305

FIG. 25B

FIG. 25A

*FIG. 26B*

319

321

319 115

313

325

335

B

B

335

103

327

321

*FIG. 26A*

**FIG. 26C**

**FIG. 26D**

**FIG. 26E**

FIG. 26F

FIG. 26G

FIG. 26H

FIG. 27A

FIG. 27B          FIG. 27C

FIG. 27D

FIG. 27E

FIG. 28A

**FIG. 28B**

**FIG. 28C**

357

**FIG. 29A**

369
365
359
363
361
357
367

**FIG. 29B**

369
371
359
361
367

**FIG. 29C**

359
369
361
367

**FIG. 29D**

369
371
359
361
367

**FIG. 29E**

**FIG. 30A**

**FIG. 30B**          **FIG. 30C**          **FIG. 30D**

FIG. 31

377

387

381

385

**FIG. 31A**

377

387

381

385

**FIG. 31B**

FIG. 31C

FIG. 31D

FIG. 31F

FIG. 31E

*377*

393

389

391

393

381

381

381

384

382

**FIG. 31G**

**FIG. 31L**

**FIG. 31H**

FIG.  31I

**FIG. 31J**

**FIG. 31K**

FIG. 32A

FIG. 32B

FIG. 32C

FIG. 32D

FIG. 32E

FIG. 32F

FIG. 32G

FIG. 32H

FIG. 32I

**FIG. 32J**

**FIG. 32K**

**FIG. 32L**

**FIG. 32M**

FIG. 32N

FIG. 32O

FIG. 32P

FIG. 33A

**FIG. 33B**

**FIG. 33C**

FIG. 33D

FIG. 33E

FIG. 33F

FIG. 33G

FIG. 34A

FIG. 34B

FIG. 34C

FIG. 35A

FIG. 35B

FIG. 35C

FIG. 36A

**FIG. 36B**

**FIG. 36C**

FIG. 37A

FIG. 37B

FIG. 37C

FIG. 38A

FIG. 38B

FIG. 38C

FIG. 38D

FIG. 38E

FIG. 39A

FIG. 39B

FIG. 39C

FIG. 40A

FIG. 40B

FIG. 41A

FIG. 41B

FIG. 42A          FIG. 42B

FIG. 43A

FIG. 43B

FIG. 44A

FIG. 44B

FIG. 45

FIG. 46

FIG. 47A

FIG. 47B

FIG. 47C

FIG. 48A

FIG. 48B

FIG. 48C

**FIG. 48D**

**FIG. 48E**

FIG. 48F

FIG. 48G

FIG. 48H

*FIG. 48I*

FIG. 48J

FIG. 48K

*FIG. 48L*

*FIG. 48M*

*FIG. 48N*

FIG. 480

FIG. 49A

FIG. 49B

FIG. 49C

FIG. 49D

FIG. 49E

FIG. 49F

FIG. 49G

FIG. 50A

483

415b

419b
405b

415a

419a

405a

417

405b          405b
405a

**FIG. 50B**

483

415b

419b
405b

405b

487

415a

405a

419a

485

417

405b          405b
405a

**FIG. 50C**

**FIG. 51A**    **FIG. 51B**

FIG. 52A

FIG. 52B

FIG. 53A

FIG. 53B

483

415b

487

405b

415a

485

405a

465a

417

465a

405b

405b

405a

405b

**FIG. 54**

483

415b

415a

405b

417

405b

405a

405b

487

467b

485

405b

467a

405a

405b

**FIG. 55**

*FIG. 56A*

*FIG. 56B-1*

*FIG. 56B-2*

*FIG. 56B*

*FIG. 56C*

FIG. 56D

FIG. 56E

FIG. 56F

FIG. 56G

FIG. 57A

FIG. 57B

FIG. 57C

FIG. 58

$415n$

$417$

FIG. 59A

$405$

$405$

$403$

417

FIG. 59B

417

FIG. 59C

**REFERENCES CITED IN THE DESCRIPTION**

**Patent documents cited in the description**

- US 20060084945 A

- US 20060100610 A